# EUROPEAN PATENT SPECIFICATION

(11) **EP 3 645 743 B1**
(45) Date of publication and mention of the grant of the patent: **18.08.2021**
(21) Application number: 18745740.3
(22) Date of filing: 29.06.2018
(51) Int. Cl.: C12Q 1/6806, C12Q 1/6853

(54) **LIBRARY PREPARATION METHODS AND COMPOSITIONS AND USES THEREFOR**
VERFAHREN UND ZUSAMMENSETZUNGEN ZUR ZUSAMMENSTELLUNG EINER BIBLIOTHEK UND VERWENDUNGEN DAFÜR
PROCÉDÉS DE PRÉPARATION DE BIBLIOTHÈQUE AINSI QUE COMPOSITIONS ET UTILISATIONS CORRESPONDANTES

(30) Priority: 30.06.2017 US 201762527893 P; 06.01.2018 US 201862614362 P; 15.06.2018 US 201862685424 P
(43) Date of publication of application: 06.05.2020
(73) Proprietor: Life Technologies Corporation, Carlsbad, CA 92008 (US)
(72) Inventor: ANDERSEN, Mark, Carlsbad California 92008 (US); MAZUR, Daniel, Carlsbad California 92008 (US); CHEN, Sihong, Carlsbad California 92008 (US); LUO, Guobin, Carlsbad California 92008 (US); PENG, Xinzhan, Carlsbad California 92008 (US)
(74) Representative: Thermo Fisher Scientific- Life Sciences Solutions Group
(86) International application number: PCT/US2018/040432
(87) International publication number: WO 2019/006392

(56) References cited:
- WO-A1-2013/081864
- VARLEY KATHERINE ELENA ET AL: "Nested Patch PCR enables highly multiplexed mutation discovery in candidate genes", GENOME RESEARCH, COLD SPRING HARBOR LABORATORY PRESS, US , vol. 18, no. 11 1 November 2008 (2008-11-01), pages 1844-1850, XP002678933, ISSN: 1088-9051, DOI: 10.1101/GR.078204.108 Retrieved from the Internet: URL:http://genome.cshlp.org/content/18/11/ 1844 [retrieved on 2008-10-10]

## Description

### FIELD OF THE INVENTION

The present invention relates to methods of preparing a library of target nucleic acid sequences and compositions and uses therefor. WO 2013/081864 A1 discloses e.g. in Fig 1 target specific multiplex amplification using primers which are removed by digestion followed by a separate adapter ligation step and universal amplification. Varley et al. disclose in GENOME RESEARCH, COLD SPRING HARBOR LABORATORY PRESS, US vol. 18, no. 11, pages 1844-1850 a"Nested Patch PCR enables highly multiplexed mutation discovery in candidate genes". It involves target specific primers which are cleaved from the amplicons by the addition of heat-labile uracil DNA glycosylase, endonuclease VIII, and single-strand-specific exonuclease I. Then nested patch oligonucleotides are annealed to the target amplicons and serve as a patch between the correct amplicons and universal primers.

### BRIEF SUMMARY OF THE INVENTION

Provided are methods for preparing a library of target nucleic acid sequences. Disclosed as well are compositions and uses therefor. Methods comprise contacting a nucleic acid sample with a plurality of adaptors capable of amplification of one or more target nucleic acid sequences under conditions wherein the target nucleic acid(s) undergo a first amplification; digesting the resulting first amplification products; repairing the digested target amplicons; and amplifying the repaired products in a second amplification, thereby producing a library of target nucleic acid sequence. Each of the plurality of adaptor compositions comprise a handle and a targeted nucleic acid sequence and optionally one or more tag sequences. Provided methods may be carried out in a single, addition only workflow reaction, allowing for rapid production of highly multiplexed targeted libraries, optionally including unique tag sequences. Resulting library compositions are useful for a variety of applications, including sequencing applications.

One aspect of the invention comprises methods for preparing a library of target nucleic acid sequences. In certain embodiments the methods comprise contacting a nucleic acid sample with a plurality of adaptors wherein each of a pair of adaptors are capable of amplification of one or more target nucleic acid sequences in the sample under conditions wherein the target nucleic acid(s) undergo a first amplification. The methods further comprise digesting the resulting first amplification products to reduce or eliminate any primer dimers resulting in the reaction and preparing partially digested amplicons, thereby preparing resulting gapped, double stranded partially digested amplicons. The methods further comprise repairing the partially digested target amplicons; then amplifying the repaired products in a second amplification using universal primers to thereby produce a library of target nucleic acid sequences. Each of the plurality of adaptors used in the provided methods comprise a 5' universal handle sequence and a 3' target nucleic acid sequence and a cleavable moiety. Two or more target specific adaptor pairs are included for use in provided methods, wherein each of the 3' target specific sequences comprise cleavable moieties. Optionally, one or more tag sequences are included.

In another aspect of the invention methods for preparing a library of target nucleic acid sequences having unique tag sequences is provided. In certain embodiments the methods comprise contacting a nucleic acid sample with a plurality of adaptors wherein each of a pair of adaptors are capable of amplification of one or more target nucleic acid sequences in the sample under conditions wherein the target nucleic acid(s) undergo a first amplification. The methods further comprise digesting the resulting first amplification products to reduce or eliminate any primer dimers resulting in the reaction and preparing partially digested amplicons, thereby preparing resulting gapped, double stranded partially digested amplicons. The methods further comprise repairing the partially digested target amplicons; then amplifying the repaired products in a second amplification using universal primers to thereby produce a library of target nucleic acid sequences. Each of the plurality of adaptors used in the provided methods comprise a 5' universal handle sequence, one or more unique tag sequences and a 3' target nucleic acid sequence and a cleavable moiety. Two or more target specific adaptor pairs are included for use in provided methods, wherein each of the 3' target specific sequences comprise cleavable moieties, each tag sequence is flanked by cleavable moieties, and each universal handle is without cleavable moieties.

In a further aspect, compositions are provided. In some embodiments provided are compositions comprising nucleic acid libraries generated by the methods described herein. In other embodiments, compositions comprising a plurality of nucleic acid adaptors are provided, wherein each of the plurality of adaptors comprise a 5' universal handle sequence, one or more unique tag sequences, and a 3' target nucleic acid sequence wherein each adaptor comprises a cleavable moiety. In certain embodiments the target nucleic acid sequence of the adaptor includes at least one cleavable moiety, cleavable moieties are included flanking either end of the tag sequence and the universal handle sequence does not include the cleavable moiety. In certain embodiments, compositions include at least two and up to one hundred thousand target specific adaptor pairs.

Still further, uses of provided compositions and kits comprising provided compositions for analysis of sequences of the nucleic acid libraries are additional aspects of the invention. In some embodiments, analysis of the sequences of the resulting libraries enables detection of low frequency alleles in a sample of interest.

### BRIEF DESCRIPTION OF THE DRAWINGS

Efficient methods for production of targeted libraries from complex samples is desirable for a variety of nucleic acid analyses. The present invention provides, *inter alia,* methods of preparing libraries of target nucleic acid sequences, allowing for rapid production of highly multiplexed targeted libraries, optionally including unique tag sequences; and resulting library compositions are useful for a variety of applications, including sequencing applications. Novel features of the invention are set forth with particularity in the appended claims; and a complete understanding of the features and advantages of the present invention will be obtained by reference to the following detailed description that sets forth illustrative embodiments, in which the principles of the invention are utilized, and the accompanying drawings of which:
FIGURE 1 depicts a workflow method of the invention that enables efficient rapid, highly multiplexed library preparation.
FIGURE 2 depicts results from the experimental description in Example 2A.
FIGURE 3 depicts results from the experimental description in Example 2B.
FIGURE 4A-4C depicts results from the experimental description in Example 4.
FIGURE 5 depicts results from the experimental description in Example 5.
FIGURE 6A-6C depicts results from the experimental description in Example 6.
FIGURE 7 depicts an additional aspect of the workflow of the invention that enables addition of adaptor sequences to facilitate bidirectional sequencing
FIGURE 8 depicts an additional aspect of the workflow of the invention that enables sequencing on Illumina platforms

### DESCRIPTION OF THE INVENTION

Section headings used herein are for organizational purposes only and are not to be construed as limiting the described subject matter in any way. When definitions of terms in cited references like literature and similar materials cited in this application, including but not limited to, patents, patent applications, articles, books, treatises, and internet web pages appear to differ from the definitions provided in the present teachings, the definition provided in the present teachings shall control. It will be appreciated that there is an implied "about" prior to the temperatures, concentrations, times, etc discussed in the present teachings, such that slight and insubstantial deviations are within the scope of the present teachings herein. In this application, the use of the singular includes the plural unless specifically stated otherwise. It is noted that, as used in this specification, singular forms "a," "an," and "the," and any singular use of a word, include plural referents unless expressly and unequivocally limited to one referent. Also, the use of "comprise", "comprises", "comprising", "contain", "contains", "containing", "include", "includes", and "including" are not intended to be limiting. It is to be understood that both the general description is exemplary and explanatory only and not restrictive of the invention.

Unless otherwise defined, scientific and technical terms used in connection with the invention described herein shall have the meanings that are commonly understood by those of ordinary skill in the art. Further, unless otherwise required by context, singular terms shall include pluralities and plural terms shall include the singular. Generally, nomenclatures utilized in connection with, and techniques of, cell and tissue culture, molecular biology, and protein and oligoor polynucleotide chemistry and hybridization used herein are those well-known and commonly used in the art. Standard techniques are used, for example, for nucleic acid purification and preparation, chemical analysis, recombinant nucleic acid, and oligonucleotide synthesis. Enzymatic reactions and purification techniques are performed according to manufacturer's specifications or as commonly accomplished in the art or as described herein. Techniques and procedures described herein are generally performed according to conventional methods well known in the art and as described in various general and more specific references that are cited and discussed throughout the instant specification. *See, e.g.,* Sambrook et al., Molecular Cloning: A Laboratory Manual (Third ed., Cold Spring Harbor Laboratory Press, Cold Spring Harbor, N.Y. 2000). Unless specifically provided, any nomenclature utilized in connection with, and laboratory procedures and techniques described herein are those well-known and commonly used in the art. As utilized in accordance with embodiments provided herein, the following terms, unless otherwise indicated, shall be understood to have the following meanings:

As used herein, "amplify", "amplifying" or "amplification reaction" and their derivatives, refer generally to an action or process whereby at least a portion of a nucleic acid molecule (referred to as a template nucleic acid molecule) is replicated or copied into at least one additional nucleic acid molecule. The additional nucleic acid molecule optionally includes sequence that is substantially identical or substantially complementary to at least some portion of the template nucleic acid molecule. A template target nucleic acid molecule may be single-stranded or double-stranded. The additional resulting replicated nucleic acid molecule may independently be single-stranded or double-stranded. In some embodiments, amplification includes a template-dependent in vitro enzyme-catalyzed reaction for the production of at least one copy of at least some portion of a target nucleic acid molecule or the production of at least one copy of a target nucleic acid sequence that is complementary to at least some portion of a target nucleic acid molecule. Amplification optionally includes linear or exponential replication of a nucleic acid molecule. In some embodiments, such amplification is performed using isothermal conditions; in other embodiments, such amplification can include thermocycling. In some embodiments, the amplification is a multiplex amplification that includes simultaneous amplification of a plurality of target sequences in a single amplification reaction. At least some target sequences can be situated on the same nucleic acid molecule or on different target nucleic acid molecules included in a single amplification reaction. In some embodiments, "amplification" includes amplification of at least some portion of DNA- and/or RNA-based nucleic acids, whether alone, or in combination. An amplification reaction can include single or double-stranded nucleic acid substrates and can further include any amplification processes known to one of ordinary skill in the art. In some embodiments, an amplification reaction includes polymerase chain reaction (PCR). In some embodiments, an amplification reaction includes isothermal amplification.

As used herein, "amplification conditions" and derivatives (e.g., conditions for amplification, etc.) generally refers to conditions suitable for amplifying one or more nucleic acid sequences. Amplification can be linear or exponential. In some embodiments, amplification conditions include isothermal conditions or alternatively include thermocycling conditions, or a combination of isothermal and thermocycling conditions. In some embodiments, conditions suitable for amplifying one or more target nucleic acid sequences includes polymerase chain reaction (PCR) conditions. Typically, amplification conditions refer to a reaction mixture that is sufficient to amplify nucleic acids such as one or more target sequences, or to amplify an amplified target sequence ligated or attached to one or more adaptors, e.g., an adaptor-attached amplified target sequence. Generally, amplification conditions include a catalyst for amplification or for nucleic acid synthesis, for example a polymerase; a primer that possesses some degree of complementarity to the nucleic acid to be amplified; and nucleotides, such as deoxyribonucleoside triphosphates (dNTPs) to promote extension of a primer once hybridized to a nucleic acid. Amplification conditions can require hybridization or annealing of a primer to a nucleic acid, extension of the primer and a denaturing step in which the extended primer is separated from the nucleic acid sequence undergoing amplification. Typically, though not necessarily, amplification conditions can include thermocycling. In some embodiments, amplification conditions include a plurality of cycles wherein steps of annealing, extending and separating are repeated. Typically, amplification conditions include cations such as Mg⁺⁺ or Mn⁺⁺ (e.g., MgCh, etc) and can also optionally include various modifiers of ionic strength.

As used herein, "target sequence" "target nucleic acid sequence" or "target sequence of interest" and derivatives, refers generally to any single or double-stranded nucleic acid sequence that can be amplified or synthesized according to the disclosure, including any nucleic acid sequence suspected or expected to be present in a sample. In some embodiments, the target sequence is present in double-stranded form and includes at least a portion of the particular nucleotide sequence to be amplified or synthesized, or its complement, prior to the addition of target-specific primers or appended adaptors. Target sequences can include the nucleic acids to which primers useful in the amplification or synthesis reaction can hybridize prior to extension by a polymerase. In some embodiments, the term refers to a nucleic acid sequence whose sequence identity, ordering or location of nucleotides is determined by one or more of the methods of the disclosure.

The term "portion" and its variants, as used herein, when used in reference to a given nucleic acid molecule, for example a primer or a template nucleic acid molecule, comprises any number of contiguous nucleotides within the length of the nucleic acid molecule, including the partial or entire length of the nucleic acid molecule.

As used herein, "contacting" and its derivatives, when used in reference to two or more components, refers generally to any process whereby the approach, proximity, mixture or commingling of the referenced components is promoted or achieved without necessarily requiring physical contact of such components, and includes mixing of solutions containing any one or more of the referenced components with each other. The referenced components may be contacted in any particular order or combination and the particular order of recitation of components is not limiting. For example, "contacting A with B and C" encompasses embodiments where A is first contacted with B then C, as well as embodiments where C is contacted with A then B, as well as embodiments where a mixture of A and C is contacted with B, and the like. Furthermore, such contacting does not necessarily require that the end result of the contacting process be a mixture including all of the referenced components, as long as at some point during the contacting process all of the referenced components are simultaneously present or simultaneously included in the same mixture or solution. For example, "contacting A with B and C" can include embodiments wherein C is first contacted with A to form a first mixture, which first mixture is then contacted with B to form a second mixture, following which C is removed from the second mixture; optionally A can then also be removed, leaving only B. Where one or more of the referenced components to be contacted includes a plurality (e.g., "contacting a target sequence with a plurality of target-specific primers and a polymerase"), then each member of the plurality can be viewed as an individual component of the contacting process, such that the contacting can include contacting of any one or more members of the plurality with any other member of the plurality and/or with any other referenced component (e.g., some but not all of the plurality of target specific primers can be contacted with a target sequence, then a polymerase, and then with other members of the plurality of target-specific primers) in any order or combination.

As used herein, the term "primer" and its derivatives refer generally to any polynucleotide that can hybridize to a target sequence of interest. In some embodiments, the primer can also serve to prime nucleic acid synthesis. Typically, a primer functions as a substrate onto which nucleotides can be polymerized by a polymerase; in some embodiments, however, a primer can become incorporated into a synthesized nucleic acid strand and provide a site to which another primer can hybridize to prime synthesis of a new strand that is complementary to the synthesized nucleic acid molecule. A primer may be comprised of any combination of nucleotides or analogs thereof, which may be optionally linked to form a linear polymer of any suitable length. In some embodiments, a primer is a single-stranded oligonucleotide or polynucleotide. (For purposes of this disclosure, the terms 'polynucleotide" and "oligonucleotide" are used interchangeably herein and do not necessarily indicate any difference in length between the two). In some embodiments, a primer is double-stranded. If double stranded, a primer is first treated to separate its strands before being used to prepare extension products. Preferably, the primer is an oligodeoxyribonucleotide. A primer must be sufficiently long to prime the synthesis of extension products. Lengths of the primers will depend on many factors, including temperature, source of primer and the use of the method. In some embodiments, a primer acts as a point of initiation for amplification or synthesis when exposed to amplification or synthesis conditions; such amplification or synthesis can occur in a template-dependent fashion and optionally results in formation of a primer extension product that is complementary to at least a portion of the target sequence. Exemplary amplification or synthesis conditions can include contacting the primer with a polynucleotide template (e.g., a template including a target sequence), nucleotides and an inducing agent such as a polymerase at a suitable temperature and pH to induce polymerization of nucleotides onto an end of the target-specific primer. If double-stranded, the primer can optionally be treated to separate its strands before being used to prepare primer extension products. In some embodiments, the primer is an oligodeoxyribonucleotide or an oligoribonucleotide. In some embodiments, the primer can include one or more nucleotide analogs. The exact length and/or composition, including sequence, of the target-specific primer can influence many properties, including melting temperature (Tm), GC content, formation of secondary structures, repeat nucleotide motifs, length of predicted primer extension products, extent of coverage across a nucleic acid molecule of interest, number of primers present in a single amplification or synthesis reaction, presence of nucleotide analogs or modified nucleotides within the primers, and the like. In some embodiments, a primer can be paired with a compatible primer within an amplification or synthesis reaction to form a primer pair consisting or a forward primer and a reverse primer. In some embodiments, the forward primer of the primer pair includes a sequence that is substantially complementary to at least a portion of a strand of a nucleic acid molecule, and the reverse primer of the primer of the primer pair includes a sequence that is substantially identical to at least of portion of the strand. In some embodiments, the forward primer and the reverse primer are capable of hybridizing to opposite strands of a nucleic acid duplex. Optionally, the forward primer primes synthesis of a first nucleic acid strand, and the reverse primer primes synthesis of a second nucleic acid strand, wherein the first and second strands are substantially complementary to each other, or can hybridize to form a double-stranded nucleic acid molecule. In some embodiments, one end of an amplification or synthesis product is defined by the forward primer and the other end of the amplification or synthesis product is defined by the reverse primer. In some embodiments, where the amplification or synthesis of lengthy primer extension products is required, such as amplifying an exon, coding region, or gene, several primer pairs can be created than span the desired length to enable sufficient amplification of the region. In some embodiments, a primer can include one or more cleavable groups. In some embodiments, primer lengths are in the range of about 10 to about 60 nucleotides, about 12 to about 50 nucleotides and about 15 to about 40 nucleotides in length. Typically, a primer is capable of hybridizing to a corresponding target sequence and undergoing primer extension when exposed to amplification conditions in the presence of dNTPS and a polymerase. In some instances, the particular nucleotide sequence or a portion of the primer is known at the outset of the amplification reaction or can be determined by one or more of the methods disclosed herein. In some embodiments, the primer includes one or more cleavable groups at one or more locations within the primer.

As used herein, "target-specific primer" and its derivatives, refers generally to a single stranded or double-stranded polynucleotide, typically an oligonucleotide, that includes at least one sequence that is at least 50% complementary, typically at least 75% complementary or at least 85% complementary, more typically at least 90% complementary, more typically at least 95% complementary, more typically at least 98% or at least 99% complementary, or identical, to at least a portion of a nucleic acid molecule that includes a target sequence. In such instances, the target-specific primer and target sequence are described as "corresponding" to each other. In some embodiments, the target-specific primer is capable of hybridizing to at least a portion of its corresponding target sequence (or to a complement of the target sequence); such hybridization can optionally be performed under standard hybridization conditions or under stringent hybridization conditions. In some embodiments, the target-specific primer is not capable of hybridizing to the target sequence, or to its complement, but is capable of hybridizing to a portion of a nucleic acid strand including the target sequence, or to its complement. In some embodiments, the target-specific primer includes at least one sequence that is at least 75% complementary, typically at least 85% complementary, more typically at least 90% complementary, more typically at least 95% complementary, more typically at least 98% complementary, or more typically at least 99% complementary, to at least a portion of the target sequence itself; in other embodiments, the target-specific primer includes at least one sequence that is at least 75% complementary, typically at least 85% complementary, more typically at least 90% complementary, more typically at least 95% complementary, more typically at least 98% complementary, or more typically at least 99% complementary, to at least a portion of the nucleic acid molecule other than the target sequence. In some embodiments, the target-specific primer is substantially non-complementary to other target sequences present in the sample; optionally, the target-specific primer is substantially non-complementary to other nucleic acid molecules present in the sample. In some embodiments, nucleic acid molecules present in the sample that do not include or correspond to a target sequence (or to a complement of the target sequence) are referred to as "non-specific" sequences or "non-specific nucleic acids". In some embodiments, the target-specific primer is designed to include a nucleotide sequence that is substantially complementary to at least a portion of its corresponding target sequence. In some embodiments, a target-specific primer is at least 95% complementary, or at least 99% complementary, or identical, across its entire length to at least a portion of a nucleic acid molecule that includes its corresponding target sequence. In some embodiments, a target-specific primer can be at least 90%, at least 95% complementary, at least 98% complementary or at least 99% complementary, or identical, across its entire length to at least a portion of its corresponding target sequence. In some embodiments, a forward target-specific primer and a reverse target-specific primer define a target-specific primer pair that can be used to amplify the target sequence via template-dependent primer extension. Typically, each primer of a target-specific primer pair includes at least one sequence that is substantially complementary to at least a portion of a nucleic acid molecule including a corresponding target sequence but that is less than 50% complementary to at least one other target sequence in the sample. In some embodiments, amplification can be performed using multiple target-specific primer pairs in a single amplification reaction, wherein each primer pair includes a forward target-specific primer and a reverse target-specific primer, each including at least one sequence that substantially complementary or substantially identical to a corresponding target sequence in the sample, and each primer pair having a different corresponding target sequence. In some embodiments, the target-specific primer can be substantially non-complementary at its 3' end or its 5' end to any other target-specific primer present in an amplification reaction. In some embodiments, the target-specific primer can include minimal cross hybridization to other target-specific primers in the amplification reaction. In some embodiments, target-specific primers include minimal cross-hybridization to non-specific sequences in the amplification reaction mixture. In some embodiments, the target-specific primers include minimal self-complementarity. In some embodiments, the target-specific primers can include one or more cleavable groups located at the 3' end. In some embodiments, the target-specific primers can include one or more cleavable groups located near or about a central nucleotide of the target-specific primer. In some embodiments, one of more targets-specific primers includes only non-cleavable nucleotides at the 5' end of the target-specific primer. In some embodiments, a target specific primer includes minimal nucleotide sequence overlap at the 3'end or the 5' end of the primer as compared to one or more different target-specific primers, optionally in the same amplification reaction. In some embodiments 1, 2, 3, 4, 5, 6, 7, 8, 9, 10 or more, target-specific primers in a single reaction mixture include one or more of the above embodiments. In some embodiments, substantially all of the plurality of target-specific primers in a single reaction mixture includes one or more of the above embodiments.

As used herein, the term "adaptor" denotes a nucleic acid molecule that can be used for manipulation of a polynucleotide of interest. In some embodiments, adaptors are used for amplification of one or more target nucleic acids. In some embodiments, the adaptors are used in reactions for sequencing. In some embodiments, an adaptor has one or more ends that lack a 5' phosphate residue. In some embodiments, an adaptor comprises, consists of, or consist essentially of at least one priming site. Such priming site containing adaptors can be referred to as "primer" adaptors. In some embodiments, the adaptor priming site can be useful in PCR processes. In some embodiments an adaptor includes a nucleic acid sequence that is substantially complementary to the 3' end or the 5' end of at least one target sequences within the sample, referred to herein as a gene specific target sequence, a target specific sequence, or target specific primer. In some embodiments, the adaptor includes nucleic acid sequence that is substantially non-complementary to the 3' end or the 5' end of any target sequence present in the sample. In some embodiments, the adaptor includes single stranded or double-stranded linear oligonucleotide that is not substantially complementary to an target nucleic acid sequence. In some embodiments, the adaptor includes nucleic acid sequence that is substantially non-complementary to at least one, and preferably some or all of the nucleic acid molecules of the sample. In some embodiments, suitable adaptor lengths are in the range of about 10-75 nucleotides, about 12-50 nucleotides and about 15-40 nucleotides in length. Generally, an adaptor can include any combination of nucleotides and/or nucleic acids. In some aspects, adaptors include one or more cleavable groups at one or more locations. In some embodiments, the adaptor includes sequence that is substantially identical, or substantially complementary, to at least a portion of a primer, for example a universal primer. In some embodiments, adaptors include a tag sequence to assist with cataloguing, identification or sequencing. In some embodiments, an adaptor acts as a substrate for amplification of a target sequence, particularly in the presence of a polymerase and dNTPs under suitable temperature and pH.

As used herein, "polymerase" and its derivatives, generally refers to any enzyme that can catalyze the polymerization of nucleotides (including analogs thereof) into a nucleic acid strand. Typically but not necessarily, such nucleotide polymerization can occur in a template-dependent fashion. Such polymerases can include without limitation naturally occurring polymerases and any subunits and truncations thereof, mutant polymerases, variant polymerases, recombinant, fusion or otherwise engineered polymerases, chemically modified polymerases, synthetic molecules or assemblies, and any analogs, derivatives or fragments thereof that retain the ability to catalyze such polymerization. Optionally, the polymerase can be a mutant polymerase comprising one or more mutations involving the replacement of one or more amino acids with other amino acids, the insertion or deletion of one or more amino acids from the polymerase, or the linkage of parts of two or more polymerases. Typically, the polymerase comprises one or more active sites at which nucleotide binding and/or catalysis of nucleotide polymerization can occur. Some exemplary polymerases include without limitation DNA polymerases and RNA polymerases. The term "polymerase" and its variants, as used herein, also refers to fusion proteins comprising at least two portions linked to each other, where the first portion comprises a peptide that can catalyze the polymerization of nucleotides into a nucleic acid strand and is linked to a second portion that comprises a second polypeptide. In some embodiments, the second polypeptide can include a reporter enzyme or a processivity-enhancing domain. Optionally, the polymerase can possess 5' exonuclease activity or terminal transferase activity. In some embodiments, the polymerase can be optionally reactivated, for example through the use of heat, chemicals or re-addition of new amounts of polymerase into a reaction mixture. In some embodiments, the polymerase can include a hot-start polymerase and/or an aptamer based polymerase that optionally can be reactivated.

The terms "identity" and "identical" and their variants, as used herein, when used in reference to two or more nucleic acid sequences, refer to similarity in sequence of the two or more sequences (e.g., nucleotide or polypeptide sequences). In the context of two or more homologous sequences, the percent identity or homology of the sequences or subsequences thereof indicates the percentage of all monomeric units (e.g., nucleotides or amino acids) that are the same (i.e., about 70% identity, preferably 75%, 80%, 85%, 90%, 95%, 98% or 99% identity). The percent identity can be over a specified region, when compared and aligned for maximum correspondence over a comparison window, or designated region as measured using a BLAST or BLAST 2.0 sequence comparison algorithms with default parameters described below, or by manual alignment and visual inspection. Sequences are said to be "substantially identical" when there is at least 85% identity at the amino acid level or at the nucleotide level. Preferably, the identity exists over a region that is at least about 25, 50, or 100 residues in length, or across the entire length of at least one compared sequence. A typical algorithm for determining percent sequence identity and sequence similarity are the BLAST and BLAST 2.0 algorithms, which are described in Altschul et al, Nuc. Acids Res. 25:3389-3402 (1977). Other methods include the algorithms of Smith & Waterman, Adv. Appl. Math. 2:482 (1981), and Needleman & Wunsch, J. Mol. Biol. 48:443 (1970), etc. Another indication that two nucleic acid sequences are substantially identical is that the two molecules or their complements hybridize to each other under stringent hybridization conditions.

The terms "complementary" and "complement" and their variants, as used herein, refer to any two or more nucleic acid sequences (e.g., portions or entireties of template nucleic acid molecules, target sequences and/or primers) that can undergo cumulative base pairing at two or more individual corresponding positions in antiparallel orientation, as in a hybridized duplex. Such base pairing can proceed according to any set of established rules, for example according to Watson-Crick base pairing rules or according to some other base pairing paradigm. Optionally there can be "complete" or "total" complementarity between a first and second nucleic acid sequence where each nucleotide in the first nucleic acid sequence can undergo a stabilizing base pairing interaction with a nucleotide in the corresponding antiparallel position on the second nucleic acid sequence. "Partial" complementarity describes nucleic acid sequences in which at least 20%, but less than 100%, of the residues of one nucleic acid sequence are complementary to residues in the other nucleic acid sequence. In some embodiments, at least 50%, but less than 100%, of the residues of one nucleic acid sequence are complementary to residues in the other nucleic acid sequence. In some embodiments, at least 70%, 80%, 90%, 95% or 98%, but less than 100%, of the residues of one nucleic acid sequence are complementary to residues in the other nucleic acid sequence. Sequences are said to be "substantially complementary" when at least 85% of the residues of one nucleic acid sequence are complementary to residues in the other nucleic acid sequence. In some embodiments, two complementary or substantially complementary sequences are capable of hybridizing to each other under standard or stringent hybridization conditions. "Non-complementary" describes nucleic acid sequences in which less than 20% of the residues of one nucleic acid sequence are complementary to residues in the other nucleic acid sequence. Sequences are said to be "substantially non-complementary" when less than 15% of the residues of one nucleic acid sequence are complementary to residues in the other nucleic acid sequence. In some embodiments, two non-complementary or substantially non-complementary sequences cannot hybridize to each other under standard or stringent hybridization conditions. A "mismatch" is present at any position in the two opposed nucleotides are not complementary. Complementary nucleotides include nucleotides that are efficiently incorporated by DNA polymerases opposite each other during DNA replication under physiological conditions. In a typical embodiment, complementary nucleotides can form base pairs with each other, such as the A-T/U and G-C base pairs formed through specific Watson-Crick type hydrogen bonding, or base pairs formed through some other type of base pairing paradigm, between the nucleobases of nucleotides and/or polynucleotides in positions antiparallel to each other. The complementarity of other artificial base pairs can be based on other types of hydrogen bonding and/or hydrophobicity of bases and/or shape complementarity between bases.

As used herein, "amplified target sequences" and its derivatives, refers generally to a nucleic acid sequence produced by the amplification of/amplifying the target sequences using target-specific primers and the methods provided herein. The amplified target sequences may be either of the same sense (the positive strand produced in the second round and subsequent even-numbered rounds of amplification) or antisense (i.e., the negative strand produced during the first and subsequent oddnumbered rounds of amplification) with respect to the target sequences. For the purposes of this disclosure, amplified target sequences are typically less than 50% complementary to any portion of another amplified target sequence in the reaction.

As used herein, terms "ligating", "ligation" and derivatives refer generally to the act or process for covalently linking two or more molecules together, for example, covalently linking two or more nucleic acid molecules to each other. In some embodiments, ligation includes joining nicks between adjacent nucleotides of nucleic acids. In some embodiments, ligation includes forming a covalent bond between an end of a first and an end of a second nucleic acid molecule. In some embodiments, for example embodiments wherein the nucleic acid molecules to be ligated include conventional nucleotide residues, the ligation can include forming a covalent bond between a 5' phosphate group of one nucleic acid and a 3' hydroxyl group of a second nucleic acid thereby forming a ligated nucleic acid molecule. In some embodiments, any means for joining nicks or bonding a 5'phosphate to a 3' hydroxyl between adjacent nucleotides can be employed. In an exemplary embodiment, an enzyme such as a ligase can be used.

As used herein, "ligase" and its derivatives, refers generally to any agent capable of catalyzing the ligation of two substrate molecules. In some embodiments, the ligase includes an enzyme capable of catalyzing the joining of nicks between adjacent nucleotides of a nucleic acid. In some embodiments, a ligase includes an enzyme capable of catalyzing the formation of a covalent bond between a 5' phosphate of one nucleic acid molecule to a 3' hydroxyl of another nucleic acid molecule thereby forming a ligated nucleic acid molecule. Suitable ligases may include, but not limited to, T4 DNA ligase; T7 DNA ligase; Taq DNA ligase, and *E. coli* DNA ligase.

As defined herein, a "cleavable group" generally refers to any moiety that once incorporated into a nucleic acid can be cleaved under appropriate conditions. For example, a cleavable group can be incorporated into a target-specific primer, an amplified sequence, an adaptor or a nucleic acid molecule of the sample. In an exemplary embodiment, a target-specific primer can include a cleavable group that becomes incorporated into the amplified product and is subsequently cleaved after amplification, thereby removing a portion, or all, of the target-specific primer from the amplified product. The cleavable group can be cleaved or otherwise removed from a target-specific primer, an amplified sequence, an adaptor or a nucleic acid molecule of the sample by any acceptable means. For example, a cleavable group can be removed from a target-specific primer, an amplified sequence, an adaptor or a nucleic acid molecule of the sample by enzymatic, thermal, photo-oxidative or chemical treatment. In one aspect, a cleavable group can include a nucleobase that is not naturally occurring. For example, an oligodeoxyribonucleotide can include one or more RNA nucleobases, such as uracil that can be removed by a uracil glycosylase. In some embodiments, a cleavable group can include one or more modified nucleobases (such as 7-methylguanine, 8-oxo-guanine, xanthine, hypoxanthine, 5,6-dihydrouracil or 5-methylcytosine) or one or more modified nucleosides (i.e., 7-methylguanosine, 8-oxo-deoxyguanosine, xanthosine, inosine, dihydrouridine or 5-methylcytidine). The modified nucleobases or nucleotides can be removed from the nucleic acid by enzymatic, chemical or thermal means. In one embodiment, a cleavable group can include a moiety that can be removed from a primer after amplification (or synthesis) upon exposure to ultraviolet light (i.e., bromodeoxyuridine). In another embodiment, a cleavable group can include methylated cytosine. Typically, methylated cytosine can be cleaved from a primer for example, after induction of amplification (or synthesis), upon sodium bisulfite treatment. In some embodiments, a cleavable moiety can include a restriction site. For example, a primer or target sequence can include a nucleic acid sequence that is specific to one or more restriction enzymes, and following amplification (or synthesis), the primer or target sequence can be treated with the one or more restriction enzymes such that the cleavable group is removed. Typically, one or more cleavable groups can be included at one or more locations with a target-specific primer, an amplified sequence, an adaptor or a nucleic acid molecule of the sample.

As used herein, "digestion", "digestion step" and its derivatives, generally refers to any process by which a cleavable group is cleaved or otherwise removed from a target-specific primer, an amplified sequence, an adaptor or a nucleic acid molecule of the sample. In some embodiments, the digestion step involves a chemical, thermal, photo-oxidative or digestive process.

As used herein, the term "hybridization" is consistent with its use in the art, and generally refers to the process whereby two nucleic acid molecules undergo base pairing interactions. Two nucleic acid molecule molecules are said to be hybridized when any portion of one nucleic acid molecule is base paired with any portion of the other nucleic acid molecule; it is not necessarily required that the two nucleic acid molecules be hybridized across their entire respective lengths and in some embodiments, at least one of the nucleic acid molecules can include portions that are not hybridized to the other nucleic acid molecule. The phrase "hybridizing under stringent conditions" and its variants refers generally to conditions under which hybridization of a target-specific primer to a target sequence occurs in the presence of high hybridization temperature and low ionic strength. As used herein, the phrase "standard hybridization conditions" and its variants refers generally to conditions under which hybridization of a primer to an oligonucleotide (i.e., a target sequence), occurs in the presence of low hybridization temperature and high ionic strength. In one exemplary embodiment, standard hybridization conditions include an aqueous environment containing about 100 mm magnesium sulfate, about 500 mM Tris-sulfate at pH 8.9, and about 200 mM ammonium sulfate at about 50-55°C., or equivalents thereof.

As used herein, the term "end" and its variants, when used in reference to a nucleic acid molecule, for example a target sequence or amplified target sequence, can include the terminal 30 nucleotides, the terminal 20 and even more typically the terminal 15 nucleotides of the nucleic acid molecule. A linear nucleic acid molecule comprised of linked series of contiguous nucleotides typically includes at least two ends. In some embodiments, one end of the nucleic acid molecule can include a 3' hydroxyl group or its equivalent, and can be referred to as the "3' end" and its derivatives. Optionally, the 3' end includes a 3' hydroxyl group that is not linked to a 5' phosphate group of a mononucleotide pentose ring. Typically, the 3' end includes one or more 5' linked nucleotides located adjacent to the nucleotide including the unlinked 3' hydroxyl group, typically the 30 nucleotides located adjacent to the 3' hydroxyl, typically the terminal 20 and even more typically the terminal 15 nucleotides. Generally, the one or more linked nucleotides can be represented as a percentage of the nucleotides present in the oligonucleotide or can be provided as a number of linked nucleotides adjacent to the unlinked 3' hydroxyl. For example, the 3' end can include less than 50% of the nucleotide length of the oligonucleotide. In some embodiments, the 3' end does not include any unlinked 3' hydroxyl group but can include any moiety capable of serving as a site for attachment of nucleotides via primer extension and/or nucleotide polymerization. In some embodiments, the term "3' end" for example when referring to a target-specific primer, can include the terminal 10 nucleotides, the terminal 5 nucleotides, the terminal 4, 3, 2 or fewer nucleotides at the 3'end. In some embodiments, the term "3' end" when referring to a target-specific primer can include nucleotides located at nucleotide positions 10 or fewer from the 3' terminus. As used herein, "5' end", and its derivatives, generally refers to an end of a nucleic acid molecule, for example a target sequence or amplified target sequence, which includes a free 5' phosphate group or its equivalent. In some embodiments, the 5' end includes a 5' phosphate group that is not linked to a 3' hydroxyl of a neighboring mononucleotide pentose ring. Typically, the 5' end includes to one or more linked nucleotides located adjacent to the 5' phosphate, typically the 30 nucleotides located adjacent to the nucleotide including the 5' phosphate group, typically the terminal 20 and even more typically the terminal 15 nucleotides. Generally, the one or more linked nucleotides can be represented as a percentage of the nucleotides present in the oligonucleotide or can be provided as a number of linked nucleotides adjacent to the 5' phosphate. For example, the 5' end can be less than 50% of the nucleotide length of an oligonucleotide. In another exemplary embodiment, the 5' end can include about 15 nucleotides adjacent to the nucleotide including the terminal 5' phosphate. In some embodiments, the 5' end does not include any unlinked 5' phosphate group but can include any moiety capable of serving as a site of attachment to a 3' hydroxyl group, or to the 3'end of another nucleic acid molecule. In some embodiments, the term "5' end" for example when referring to a target-specific primer, can include the terminal 10 nucleotides, the terminal 5 nucleotides, the terminal 4, 3, 2 or fewer nucleotides at the 5'end. In some embodiments, the term "5' end" when referring to a target-specific primer can include nucleotides located at positions 10 or fewer from the 5' terminus. In some embodiments, the 5' end of a target-specific primer can include only non-cleavable nucleotides, for example nucleotides that do not contain one or more cleavable groups as disclosed herein, or a cleavable nucleotide as would be readily determined by one of ordinary skill in the art. A "first end" and a "second end" of a polynucleotide refer to the 5' end or the 3'end of the polynucleotide. Either the first end or second end of a polynucleotide can be the 5' end or the 3' end of the polynucleotide; the terms "first" and "second" are not meant to denote that the end is specifically the 5' end or the 3' end.

As used herein "tag," "barcode," "unique tag" or "tag sequence" and its derivatives, refers generally to a unique short (6-14 nucleotide) nucleic acid sequence within an adaptor or primer that can act as a 'key' to distinguish or separate a plurality of amplified target sequences in a sample. For the purposes of this disclosure, a barcode or unique tag sequence is incorporated into the nucleotide sequence of an adaptor or primer. As used herein, "barcode sequence" denotes a nucleic acid fixed sequence that is sufficient to allow for the identification of a sample or source of nucleic acid sequences of interest. A barcode sequence can be, but need not be, a small section of the original nucleic acid sequence on which the identification is to be based. In some embodiments a barcode is 5-20 nucleic acids long. In some embodiments, the barcode is comprised of analog nucleotides, such as L-DNA, LNA, PNA, etc. As used herein, "unique tag sequence" denotes a nucleic acid sequence having at least one random sequence and at least one fixed sequence. A unique tag sequence, alone or in conjunction with a second unique tag sequence, is sufficient to allow for the identification of a single target nucleic acid molecule in a sample. A unique tag sequence can, but need not, comprise a small section of the original target nucleic acid sequence. In some embodiments a unique tag sequence is 2-50 nucleotides or base-pairs, or 2-25 nucleotides or base-pairs, or 2-10 nucleotides or base-pairs in length. A unique tag sequence can comprise at least one random sequence interspersed with a fixed sequence.

As used herein, "comparable maximal minimum melting temperatures" and its derivatives, refers generally to the melting temperature (Tm) of each nucleic acid fragment for a single adaptor or target-specific primer after digestion of a cleavable groups. The hybridization temperature of each nucleic acid fragment generated by an adaptor or target-specific primer is compared to determine the maximal minimum temperature required preventing hybridization of a nucleic acid sequence from the target-specific primer or adaptor or fragment or portion thereof to a respective target sequence. Once the maximal hybridization temperature is known, it is possible to manipulate the adaptor or target-specific primer, for example by moving the location of one or more cleavable group(s) along the length of the primer, to achieve a comparable maximal minimum melting temperature with respect to each nucleic acid fragment to thereby optimize digestion and repair steps of library preparation.

As used herein, "addition only" and its derivatives, refers generally to a series of steps in which reagents and components are added to a first or single reaction mixture. Typically, the series of steps excludes the removal of the reaction mixture from a first vessel to a second vessel in order to complete the series of steps. Generally, an addition only process excludes the manipulation of the reaction mixture outside the vessel containing the reaction mixture. Typically, an addition-only process is amenable to automation and high-throughput.

As used herein, "polymerizing conditions" and its derivatives, refers generally to conditions suitable for nucleotide polymerization. In typical embodiments, such nucleotide polymerization is catalyzed by a polymerase. In some embodiments, polymerizing conditions include conditions for primer extension, optionally in a template-dependent manner, resulting in the generation of a synthesized nucleic acid sequence. In some embodiments, the polymerizing conditions include polymerase chain reaction (PCR). Typically, the polymerizing conditions include use of a reaction mixture that is sufficient to synthesize nucleic acids and includes a polymerase and nucleotides. The polymerizing conditions can include conditions for annealing of a target-specific primer to a target sequence and extension of the primer in a template dependent manner in the presence of a polymerase. In some embodiments, polymerizing conditions can be practiced using thermocycling. Additionally, polymerizing conditions can include a plurality of cycles where the steps of annealing, extending, and separating the two nucleic strands are repeated. Typically, the polymerizing conditions include a cation such as MgCl₂. Generally, polymerization of one or more nucleotides to form a nucleic acid strand includes that the nucleotides be linked to each other via phosphodiester bonds, however, alternative linkages may be possible in the context of particular nucleotide analogs.

As used herein, the term "nucleic acid" refers to natural nucleic acids, artificial nucleic acids, analogs thereof, or combinations thereof, including polynucleotides and oligonucleotides. As used herein, the terms "polynucleotide" and "oligonucleotide" are used interchangeably and mean single-stranded and double-stranded polymers of nucleotides including, but not limited to, 2'-deoxyribonucleotides (nucleic acid) and ribonucleotides (RNA) linked by intemucleotide phosphodiester bond linkages, *e.g.* 3'-5' and 2'-5', inverted linkages, *e.g.* 3'-3' and 5'-5', branched structures, or analog nucleic acids. Polynucleotides have associated counter ions, such as H⁺, NH₄⁺, trialkylammonium, Mg²⁺, Na⁺ and the like. An oligonucleotide can be composed entirely of deoxyribonucleotides, entirely of ribonucleotides, or chimeric mixtures thereof. Oligonucleotides can be comprised of nucleobase and sugar analogs. Polynucleotides typically range in size from a few monomeric units, *e.g.* 5-40, when they are more commonly frequently referred to in the art as oligonucleotides, to several thousands of monomeric nucleotide units, when they are more commonly referred to in the art as polynucleotides; for purposes of this disclosure, however, both oligonucleotides and polynucleotides may be of any suitable length. Unless denoted otherwise, whenever a oligonucleotide sequence is represented, it will be understood that the nucleotides are in 5' to 3' order from left to right and that "A" denotes deoxyadenosine, "C" denotes deoxycytidine, "G" denotes deoxyguanosine, "T" denotes thymidine, and "U' denotes deoxyuridine. As discussed herein and known in the art, oligonucleotides and polynucleotides are said to have "5' ends" and "3' ends" because mononucleotides are typically reacted to form oligonucleotides via attachment of the 5' phosphate or equivalent group of one nucleotide to the 3' hydroxyl or equivalent group of its neighboring nucleotide, optionally via a phosphodiester or other suitable linkage.

As used herein, the term "polymerase chain reaction" ("PCR") refers to the method of K. B. Mullis U.S. Pat. Nos. 4,683,195 and 4,683,202, which describe a method for increasing the concentration of a segment of a polynucleotide of interest in a mixture of genomic DNA without cloning or purification. This process for amplifying the polynucleotide of interest consists of introducing a large excess of two oligonucleotide primers to the DNA mixture containing the desired polynucleotide of interest, followed by a precise sequence of thermal cycling in the presence of a DNA polymerase. The two primers are complementary to their respective strands of the double stranded polynucleotide of interest. To effect amplification, the mixture is denatured and the primers then annealed to their complementary sequences within the polynucleotide of interest molecule. Following annealing, the primers are extended with a polymerase to form a new pair of complementary strands. The steps of denaturation, primer annealing and polymerase extension can be repeated many times (i.e., denaturation, annealing and extension constitute one "cycle"; there can be numerous "cycles") to obtain a high concentration of an amplified segment of the desired polynucleotide of interest. The length of the amplified segment of the desired polynucleotide of interest (amplicon) is determined by the relative positions of the primers with respect to each other, and therefore, this length is a controllable parameter. By virtue of repeating the process, the method is referred to as the "polymerase chain reaction" (hereinafter "PCR"). Because the desired amplified segments of the polynucleotide of interest become the predominant nucleic acid sequences (in terms of concentration) in the mixture, they are said to be "PCR amplified". As defined herein, target nucleic acid molecules within a sample including a plurality of target nucleic acid molecules are amplified via PCR. In a modification to the method discussed above, the target nucleic acid molecules can be PCR amplified using a plurality of different primer pairs, in some cases, one or more primer pairs per target nucleic acid molecule of interest, thereby forming a multiplex PCR reaction. Using multiplex PCR, it is possible to simultaneously amplify multiple nucleic acid molecules of interest from a sample to form amplified target sequences. It is also possible to detect the amplified target sequences by several different methodologies (e.g., quantitation with a bioanalyzer or qPCR, hybridization with a labeled probe; incorporation of biotinylated primers followed by avidin-enzyme conjugate detection; incorporation of ³²P-labeled deoxynucleotide triphosphates, such as dCTP or dATP, into the amplified target sequence). Any oligonucleotide sequence can be amplified with the appropriate set of primers, thereby allowing for the amplification of target nucleic acid molecules from genomic DNA, cDNA, formalin-fixed paraffin-embedded DNA, fine-needle biopsies and various other sources. In particular, the amplified target sequences created by the multiplex PCR process as disclosed herein, are themselves efficient substrates for subsequent PCR amplification or various downstream assays or manipulations.

As defined herein "multiplex amplification" refers to selective and non-random amplification of two or more target sequences within a sample using at least one target-specific primer. In some embodiments, multiplex amplification is performed such that some or all of the target sequences are amplified within a single reaction vessel. The "plexy" or "plex" of a given multiplex amplification refers generally to the number of different target-specific sequences that are amplified during that single multiplex amplification. In some embodiments, the plexy can be about 12-plex, 24-plex, 48-plex, 96-plex, 192-plex, 384-plex, 768-plex, 1536-plex, 3072-plex, 6144-plex or higher.

### Methods of Preparing Nucleic Acid Libraries

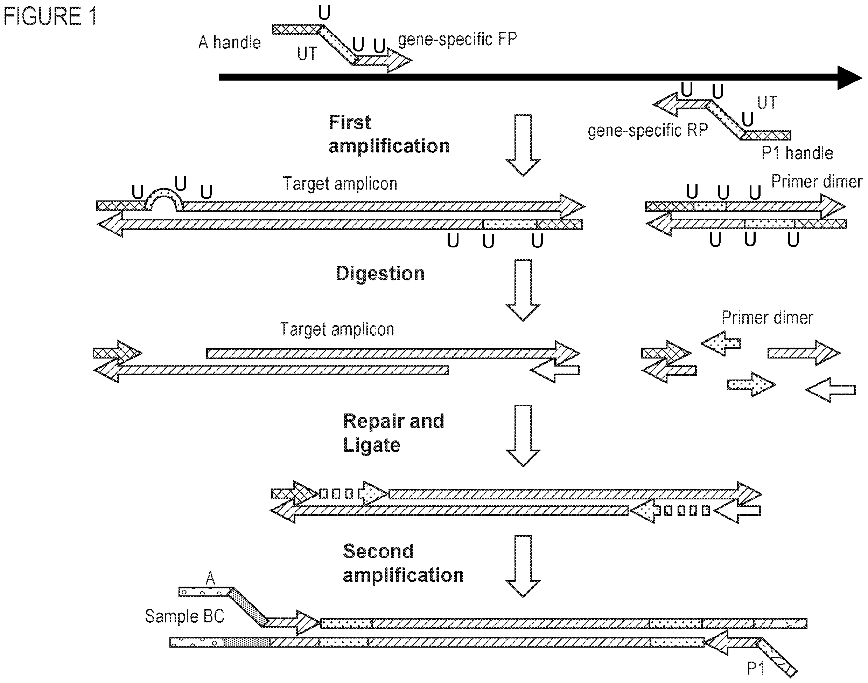

Provided methods of the invention comprise efficient procedures which enable rapid preparation of highly multiplexed libraries suitable for downstream analysis. See FIG. 1. The methods optionally allow for incorporation of one or more unique tag sequences, if so desired. Certain methods comprise streamlined, addition-only procedures conveying highly rapid library generation.

In one aspect of the invention, methods for preparing a library of target nucleic acid sequences are provided. In some embodiments, methods comprise contacting a nucleic acid sample with a plurality of adaptors capable of amplification of one or more target nucleic acid sequences in the sample under conditions wherein the target nucleic acid(s) undergo a first amplification; digesting resulting first amplification products to reduce or eliminate resulting primer dimers and prepare partially digested target amplicons, thereby producing gapped, double stranded amplicons. The methods further comprise repairing the partially digested target amplicons; then amplifying the repaired target amplicons in a second amplification using universal primers, thereby producing a library of target nucleic acid sequences. Each of the plurality of adaptors used in the methods herein comprise a universal handle sequence and a target nucleic acid sequence and a cleavable moiety and optionally one or more tag sequences. At least two and up to one hundred thousand target specific adaptor pairs are included in the provided methods, wherein the target nucleic acid sequence of each adaptor includes at least one cleavable moiety and the universal handle sequence does not include the cleavable moiety. In some embodiments where an optional tag sequence is included in at least one adaptor, the cleavable moieties are included in the adaptor sequence flanking either end of the tag sequence.

In one aspect of the invention, methods for preparing a tagged library of target nucleic acid sequences are provided. In some embodiments, methods comprise contacting a nucleic acid sample with a plurality of adaptors capable of amplification of one or more target nucleic acid sequences in the sample under conditions wherein the target nucleic acid(s) undergo a first amplification; digesting resulting first amplification products to reduce or eliminate resulting primer dimers and prepare partially digested target amplicons, thereby producing gapped, double stranded amplicons. The methods further comprise repairing the partially digested target amplicons; then amplifying the repaired target amplicons in a second amplification using universal primers, thereby producing a library of target nucleic acid sequences. Each of the plurality of adaptors used in the methods herein comprise a universal handle sequence and a target nucleic acid sequence and a cleavable moiety and one or more tag sequences. At least two and up to one hundred thousand target specific adaptor pairs are included in the provided methods, wherein the target nucleic acid sequence of each adaptor includes at least one cleavable moiety, the universal handle sequence does not include the cleavable moiety, and the cleavable moieties are included flanking either end of the tag sequence.

In certain embodiments, the comparable maximal minimum melting temperature of each universal sequence is higher than the comparable maximal minimum melting temperature of each target nucleic acid sequence and each tag sequence present in an adaptor.

In some embodiments, each of the adaptors comprise unique tag sequences as further described herein and each further comprise cleavable groups flanking either end of the tag sequence in each adaptor. In some embodiments wherein unique taq sequences are employed, each generated target specific amplicon sequence includes at least 1 different sequence and up to 10⁷ different sequences. In certain embodiments each target specific pair of the plurality of adaptors includes up to 16,777,216 different adaptor combinations comprising different tag sequences.

In some embodiments, methods comprise contacting the plurality of gapped polynucleotide products with digestion and repair reagents simultaneously. In some embodiments, methods comprise contacting the plurality of gapped polynucleotide products sequentially with the digestion then repair reagents.

A digestion reagent useful in the methods provided herein comprises any reagent capable of cleaving the cleavable site present in adaptors, and in some embodiments includes, but is not limited to, one or a combination of uracil DNA glycosylase (UDG). apurinic endonuclease (e.g., APE1), RecJf, formamidopyrimidine [fapy]-DNA glycosylase (fpg), Nth endonuclease III, endonuclease VIII, polynucleotide kinase (PNK), Taq DNA polymerase, DNA polymerase I and/or human DNA polymerase beta.

A repair reagent useful in the methods provided herein comprises any reagent capable of repair of the gapped amplicons, and in some embodiments includes, but is not limited to, any one or a combination of Phusion DNA polymerase, Phusion U DNA polymerase, SuperFi DNA polymerase, Taq DNA polymerase, Human DNA polymerase beta, T4 DNA polymerase and/or T7 DNA polymerase, SuperFiU DNA polymerase, *E. coli* DNA ligase, T3 DNA ligase, T4 DNA ligase, T7 DNA ligase, Taq DNA ligase, and/or 9°N DNA ligase.

Thus, in certain embodiments, a digestion and repair reagent comprises any one or a combination of one or a combination of uracil DNA glycosylase (UDG) . apurinic endonuclease (e.g., APE1), RecJf, formamidopyrimidine [fapy]-DNA glycosylase (fpg), Nth endonuclease III, endonuclease VIII, polynucleotide kinase (PNK), Taq DNA polymerase, DNA polymerase I and/or human DNA polymerase beta; and any one or a combination of Phusion DNA polymerase, Phusion U DNA polymerase, SuperFi DNA polymerase, Taq DNA polymerase, Human DNA polymerase beta, T4 DNA polymerase and/or T7 DNA polymerase, SuperFiU DNA polymerase, E. *coli* DNA ligase, T3 DNA ligase, T4 DNA ligase, T7 DNA ligase, Taq DNA ligase, and/or 9°N DNA ligase. In certain embodiments, a digestion and repair reagent comprises any one or a combination of uracil DNA glycosylase (UDG) ,apurinic endonuclease (e.g., APE1), Taq DNA polymerase, Phusion U DNA polymerase, SuperFiU DNA polymerase, T7 DNA ligase. In certain embodiments, a digestion and repair reagent comprises any one or a combination of uracil DNA glycosylase (UDG), formamidopyrimidine [fapy]-DNA glycosylase (fpg), Phusion U DNA polymerase, Taq DNA polymerase, SuperFiU DNA polymerase, T4 PNK and T7 DNA ligase.

In some embodiments, methods comprise the digestion and repair steps carried out in a single step. In other embodiments, methods comprise the digestion and repair of steps carried out in a temporally separate manner at different temperatures.

In some embodiments methods of the invention are carried out wherein one or more of the method steps is conducted in manual mode. In particular embodiments, methods of the invention are carried out wherein each of the method steps is conducted manually. In some embodiments methods of the invention are carried out wherein one or more of the method steps is conducted in an automated mode. In particular embodiments, methods of the invention are carried wherein each of the method steps is automated. In some embodiments methods of the invention are carried out wherein one or more of the method steps is conducted in a combination of manual and automated modes.

In some embodiments, methods of the invention comprise at least one purification step. For example, in certain embodiments a purification step is carried out only after the second amplification of repaired amplicons. In some embodiments two purification steps are utilized, wherein a first purification step is carried out after the digestion and repair and a second purification step is carried out after the second amplification of repaired amplicons.

In some embodiments a purification step comprises conducting a solid phase adherence reaction, solid phase immobilization reaction or gel electrophoresis. In certain embodiments a purification step comprises separation conducted using Solid Phase Reversible Immobilization (SPRI) beads. In particular embodiments a purification step comprises separation conducted using SPRI beads wherein the SPRI beads comprise paramagnetic beads.

In some embodiments, methods comprise contacting a nucleic acid sample with a plurality of adaptors capable of amplification of one or more target nucleic acid sequences in the sample under conditions wherein the target nucleic acid(s) undergo a first amplification; digesting resulting first amplification products to reduce or eliminate resulting primer dimers and prepare partially digested target amplicons, thereby producing gapped, double stranded amplicons. The methods further comprise repairing the partially digested target amplicons, then purifying repaired amplicons; then amplifying the repaired target amplicons in a second amplification using universal primers, thereby producing a library of target nucleic acid sequences; and then purifying resulting library. Each of the plurality of adaptors used in the methods herein comprise a universal handle sequence and a target nucleic acid sequence and a cleavable moiety and optionally one or more tag sequences. At least two and up to one hundred thousand target specific adaptor pairs are included in the provided methods, wherein the target nucleic acid sequence of each adaptor includes at least one cleavable moiety and the universal handle sequence does not include the cleavable moiety. In some embodiments where an optional tag sequence is included in at least one adaptor, the cleavable moieties are included in the adaptor sequence flanking either end of the tag sequence.

In some embodiments, methods comprise contacting a nucleic acid sample with a plurality of adaptors capable of amplification of one or more target nucleic acid sequences in the sample under conditions wherein the target nucleic acid(s) undergo a first amplification; digesting resulting first amplification products to reduce or eliminate resulting primer dimers and prepare partially digested target amplicons, thereby producing gapped, double stranded amplicons. The methods further comprise repairing the partially digested target amplicons, and purifying repaired amplicons; then amplifying the repaired target amplicons in a second amplification using universal primers, thereby producing a library of target nucleic acid sequences; and then purifying resulting library. Each of the plurality of adaptors used in the methods herein comprise a universal handle sequence and a target nucleic acid sequence and a cleavable moiety and one or more tag sequences. At least two and up to one hundred thousand target specific adaptor pairs are included in the provided methods, wherein the target nucleic acid sequence of each adaptor includes at least one cleavable moiety, the universal handle sequence does not include the cleavable moiety, and cleavable moieties are included in the flanking either end of the tag sequence.

In some embodiments, methods comprise contacting a nucleic acid sample with a plurality of adaptors capable of amplification of one or more target nucleic acid sequences in the sample under conditions wherein the target nucleic acid(s) undergo a first amplification; digesting resulting first amplification products to reduce or eliminate resulting primer dimers and prepare partially digested target amplicons, thereby producing gapped, double stranded amplicons. The methods further comprise repairing the partially digested target amplicons, then purifying repaired amplicons; then amplifying the repaired target amplicons in a second amplification using universal primers, thereby producing a library of target nucleic acid sequences; and then purifying resulting library. Each of the plurality of adaptors used in the methods herein comprise a universal handle sequence and a target nucleic acid sequence and a cleavable moiety and optionally one or more tag sequences. At least two and up to one hundred thousand target specific adaptor pairs are included in the provided methods, wherein the target nucleic acid sequence of each adaptor includes at least one cleavable moiety and the universal handle sequence does not include the cleavable moiety. In some embodiments where an optional tag sequence is included in at least one adaptor, the cleavable moieties are included in the adaptor sequence flanking either end of the tag sequence. In some embodiments a digestion and repair reagent comprises any one or a combination of one or a combination of uracil DNA glycosylase (UDG). apurinic endonuclease (e.g., APE1), RecJf, formamidopyrimidine [fapy]-DNA glycosylase (fpg), Nth endonuclease III, endonuclease VIII, polynucleotide kinase (PNK), Taq DNA polymerase, DNA polymerase I and/or human DNA polymerase beta; and any one or a combination of Phusion DNA polymerase, Phusion U DNA polymerase, SuperFi DNA polymerase, Taq DNA polymerase, Human DNA polymerase beta, T4 DNA polymerase and/or T7 DNA polymerase, SuperFiU DNA polymerase, E. *coli* DNA ligase, T3 DNA ligase, T4 DNA ligase, T7 DNA ligase, Taq DNA ligase, and/or 9°N DNA ligase. In certain embodiments, a digestion and repair reagent comprises any one or a combination of uracil DNA glycosylase (UDG),apurinic endonuclease (e.g., APE1), Taq DNA polymerase, Phusion U DNA polymerase, SuperFiU DNA polymerase, T7 DNA ligase. In certain embodiments, a digestion and repair reagent comprises any one or a combination of uracil DNA glycosylase (UDG), formamidopyrimidine [fapy]-DNA glycosylase (fpg), Phusion U DNA polymerase, Taq DNA polymerase, SuperFiU DNA polymerase, T4 PNK and T7 DNA ligase.

In some embodiments, methods comprise contacting a nucleic acid sample with a plurality of adaptors capable of amplification of one or more target nucleic acid sequences in the sample under conditions wherein the target nucleic acid(s) undergo a first amplification; digesting resulting first amplification products to reduce or eliminate resulting primer dimers and prepare partially digested target amplicons, thereby producing gapped, double stranded amplicons. The methods further comprise repairing the partially digested target amplicons, and purifying repaired amplicons; then amplifying the repaired target amplicons in a second amplification using universal primers, thereby producing a library of target nucleic acid sequences; and then purifying resulting library. Each of the plurality of adaptors used in the methods herein comprise a universal handle sequence and a target nucleic acid sequence and a cleavable moiety and one or more tag sequences. At least two and up to one hundred thousand target specific adaptor pairs are included in the provided methods, wherein the target nucleic acid sequence of each adaptor includes at least one cleavable moiety, the universal handle sequence does not include the cleavable moiety, and cleavable moieties are included in the flanking either end of the tag sequence. In some embodiments a digestion and repair reagent comprises any one or a combination of one or a combination of uracil DNA glycosylase (UDG). apurinic endonuclease (e.g., APE1), RecJf, formamidopyrimidine [fapy]-DNA glycosylase (fpg), Nth endonuclease III, endonuclease VIII, polynucleotide kinase (PNK), Taq DNA polymerase, DNA polymerase I and/or human DNA polymerase beta; and any one or a combination of Phusion DNA polymerase, Phusion U DNA polymerase, SuperFi DNA polymerase, Taq DNA polymerase, Human DNA polymerase beta, T4 DNA polymerase and/or T7 DNA polymerase, SuperFiU DNA polymerase, E. *coli* DNA ligase, T3 DNA ligase, T4 DNA ligase, T7 DNA ligase, Taq DNA ligase, and/or 9°N DNA ligase. In certain embodiments, a digestion and repair reagent comprises any one or a combination of uracil DNA glycosylase (UDG) ,apurinic endonuclease (e.g., APE1), Taq DNA polymerase, Phusion U DNA polymerase, SuperFiU DNA polymerase, T7 DNA ligase. In certain embodiments, a digestion and repair reagent comprises any one or a combination of uracil DNA glycosylase (UDG), formamidopyrimidine [fapy]-DNA glycosylase (fpg), Phusion U DNA polymerase, Taq DNA polymerase, SuperFiU DNA polymerase, T4 PNK and T7 DNA ligase.

In certain embodiments methods of the invention are carried out in a single, addition only workflow reaction, allowing for rapid production of highly multiplexed targeted libraries. For example, in one embodiment, methods for preparing a library of target nucleic acid sequences comprise contacting a nucleic acid sample with a plurality of adaptors capable of amplification of one or more target nucleic acid sequences in the sample under conditions wherein the target nucleic acid(s) undergo a first amplification; digesting resulting first amplification products to reduce or eliminate resulting primer dimers and prepare partially digested target amplicons, thereby producing gapped, double stranded amplicons. The methods further comprise repairing the partially digested target amplicons; then amplifying the repaired target amplicons in a second amplification using universal primers, thereby producing a library of target nucleic acid sequences, and purifying the resulting library. In certain embodiments the purification comprises a single or repeated separating step that is carried out following production of the library following the second amplification; and wherein the other method steps are conducted in a single reaction vessel without requisite transferring of a portion (aliquot) of any of the products generated in steps to another reaction vessel. Each of the plurality of adaptors used in the methods herein comprise a universal handle sequence and a target nucleic acid sequence and a cleavable moiety and optionally one or more tag sequences. At least two and up to one hundred thousand target specific adaptor pairs are included in the provided methods, wherein the target nucleic acid sequence of each adaptor includes at least one cleavable moiety and the universal handle sequence does not include the cleavable moiety. In some embodiments where an optional tag sequence is included in at least one adaptor, the cleavable moieties are included in the adaptor sequence flanking either end of the tag sequence.

In another embodiment, methods for preparing a tagged library of target nucleic acid sequences are provided comprising contacting a nucleic acid sample with a plurality of adaptors capable of amplification of one or more target nucleic acid sequences in the sample under conditions wherein the target nucleic acid(s) undergo a first amplification; digesting resulting first amplification products to reduce or eliminate resulting primer dimers and prepare partially digested target amplicons, thereby producing gapped, double stranded amplicons. The methods further comprise repairing the partially digested target amplicons; then amplifying the repaired target amplicons in a second amplification using universal primers, thereby producing a library of target nucleic acid sequences, and purifying the resulting library. In certain embodiments the purification comprises a single or repeated separating step; and wherein the other method steps are optionally conducted in a single reaction vessel without requisite transferring of a portion of any of the products generated in steps to another reaction vessel. Each of the plurality of adaptors used in the methods herein comprise a universal handle sequence and a target nucleic acid sequence and a cleavable moiety and one or more tag sequences. At least two and up to one hundred thousand target specific adaptor pairs are included in the provided methods, wherein the target nucleic acid sequence of each adaptor includes at least one cleavable moiety, the universal handle sequence does not include the cleavable moiety, and the cleavable moieties are included flanking either end of the tag sequence.

, In one embodiment, methods for preparing a library of target nucleic acid sequences comprise contacting a nucleic acid sample with a plurality of adaptors capable of amplification of one or more target nucleic acid sequences in the sample under conditions wherein the target nucleic acid(s) undergo a first amplification; digesting resulting first amplification products to reduce or eliminate resulting primer dimers and prepare partially digested target amplicons, thereby producing gapped, double stranded amplicons. The methods further comprise repairing the partially digested target amplicon; then amplifying the repaired target amplicons in a second amplification using universal primers, thereby producing a library of target nucleic acid sequences, and purifying the resulting library.

In some embodiments a digestion reagent comprises any one or any combination of: uracil DNA glycosylase (UDG). AP endonuclease (APE1), RecJf, formamidopyrimidine [fapy]-DNA glycosylase (fpg), Nth endonuclease III, endonuclease VIII, polynucleotide kinase, Taq DNA polymerase, DNA polymerase I and/or human DNA polymerase beta. In certain embodiments a digestion reagent comprises any one or any combination of: uracil DNA glycosylase (UDG). AP endonuclease (APE1), RecJf, formamidopyrimidine [fapy]-DNA glycosylase (fpg), Nth endonuclease III, endonuclease VIII, polynucleotide kinase, Taq DNA polymerase, DNA polymerase I and/or human DNA polymerase beta wherein the digestion reagent lacks formamidopyrimidine [fapy]-DNA glycosylase (fpg).

In some embodiments a digestion reagent comprises a single-stranded DNA exonuclease that degrades in a 5'-3' direction. In some embodiments a cleavage reagent comprises a single-stranded DNA exonuclease that degrades abasic sites. In some embodiments herein the digestions reagent comprises an RecJf exonuclease. In particular embodiments a digestion reagent comprises APE1 and RecJf, wherein the cleavage reagent comprises an apurinic/apyrimidinic endonuclease. In certain embodiments the digestion reagent comprises an AP endonuclease (APE1).

In some embodiments a repair reagent comprises at least one DNA polymerase; wherein the gap-filling reagent comprises: any one or any combination of: Phusion DNA polymerase, Phusion U DNA polymerase, SuperFi DNA polymerase, Taq DNA polymerase, Human DNA polymerase beta, T4 DNA polymerase and/or T7 DNA polymerase and/or SuperFi U DNA polymerase. In some embodiments a repair reagent further comprises a plurality of nucleotides.

In some embodiment a repair reagent comprises an ATP-dependent or an ATP-independent ligase; wherein the repair reagent comprises any one or any combination of: *E. coli* DNA ligase, T3 DNA ligase, T4 DNA ligase, T7 DNA ligase, Taq DNA ligase. , 9°N DNA ligase

In certain embodiments a digestion and repair reagent comprises any one or a combination of one or a combination of uracil DNA glycosylase (UDG). apurinic endonuclease (e.g., APE1), RecJf, formamidopyrimidine [fapy]-DNA glycosylase (fpg), Nth endonuclease III, endonuclease VIII, polynucleotide kinase (PNK), Taq DNA polymerase, DNA polymerase I and/or human DNA polymerase beta; and any one or a combination of Phusion DNA polymerase, Phusion U DNA polymerase, SuperFi DNA polymerase, Taq DNA polymerase, Human DNA polymerase beta, T4 DNA polymerase and/or T7 DNA polymerase, SuperFiU DNA polymerase, *E. coli* DNA ligase, T3 DNA ligase, T4 DNA ligase, T7 DNA ligase, Taq DNA ligase, and/or 9°N DNA ligase. In particular embodiments, a digestion and repair reagent comprises any one or a combination of uracil DNA glycosylase (UDG), apurinic endonuclease (e.g., APE1), Taq DNA polymerase, Phusion U DNA polymerase, SuperFiU DNA polymerase, T7 DNA ligase. In certain embodiments a purification comprises a single or repeated separating step that is carried out following production of the library following the second amplification; and wherein method steps are conducted in a single reaction vessel without requisite transferring of a portion of any of the products generated in steps to another reaction vessel until a first purification. Each of the plurality of adaptors used in the methods herein comprise a universal handle sequence and a target nucleic acid sequence and a cleavable moiety and optionally one or more tag sequences. At least two and up to one hundred thousand target specific adaptor pairs are included in the provided methods, wherein the target nucleic acid sequence of each adaptor includes at least one cleavable moiety and the universal handle sequence does not include the cleavable moiety. In some embodiments where an optional tag sequence is included in at least one adaptor, the cleavable moieties are included in the adaptor sequence flanking either end of the tag sequence.

In another embodiment, methods for preparing a tagged library of target nucleic acid sequences are provided comprising contacting a nucleic acid sample with a plurality of adaptors capable of amplification of one or more target nucleic acid sequences in the sample under conditions wherein the target nucleic acid(s) undergo a first amplification; digesting resulting first amplification products to reduce or eliminate resulting primer dimers and prepare partially digested target amplicons, thereby producing gapped, double stranded amplicons. The methods further comprise repairing the partially digested target amplicons; then amplifying the repaired target amplicons in a second amplification using universal primers, thereby producing a library of target nucleic acid sequences, and purifying the resulting library. In certain embodiments a digestion and repair reagent comprises any one or a combination of one or a combination of uracil DNA glycosylase (UDG). apurinic endonuclease (e.g., APE1), RecJf, formamidopyrimidine [fapy]-DNA glycosylase (fpg), Nth endonuclease III, endonuclease VIII, polynucleotide kinase (PNK), Taq DNA polymerase, DNA polymerase I and/or human DNA polymerase beta; and any one or a combination of Phusion DNA polymerase, Phusion U DNA polymerase, SuperFi DNA polymerase, Taq DNA polymerase, Human DNA polymerase beta, T4 DNA polymerase and/or T7 DNA polymerase, SuperFiU DNA polymerase, E. *coli* DNA ligase, T3 DNA ligase, T4 DNA ligase, T7 DNA ligase, Taq DNA ligase, and/or 9°N DNA ligase. In particular embodiments, a digestion and repair reagent comprises any one or a combination of uracil DNA glycosylase (UDG),apurinic endonuclease (e.g., APE1), Taq DNA polymerase, Phusion U DNA polymerase, SuperFiU DNA polymerase, T7 DNA ligase. In certain embodiments the purification comprises a single or repeated separating step that is carried out following production of the library following the second amplification; and wherein steps the other method steps are conducted in a single reaction vessel without requisite transferring of a portion (aliquot) of any of the products generated in steps to another reaction vessel. Each of the plurality of adaptors used in the methods herein comprise a universal handle sequence and a target nucleic acid sequence and a cleavable moiety and one or more tag sequences. At least two and up to one hundred thousand target specific adaptor pairs are included in the provided methods, wherein the target nucleic acid sequence of each adaptor includes at least one cleavable moiety, the universal handle sequence does not include the cleavable moiety, and the cleavable moieties are included flanking either end of the tag sequence.

In some embodiments, adaptor-dimer byproducts resulting from the first amplification of step of the methods are largely removed from the resulting library. In certain embodiments the enriched population of amplified target nucleic acids contains a reduced amount of adaptor-dimer byproduct. In particular embodiments adaptor dimer byproducts are eliminated.

In some embodiments, the library is prepared in less than 4 hours. In some embodiments, the library is prepared, enriched and sequenced in less than 3 hours. In some embodiments, the library is prepared, enriched and sequenced in 2 to 3 hours. In some embodiments, the library is prepared in approximately 2.5 hours. In some embodiments, the library is prepared in approximately 2.75 hours. In some embodiments, the library is prepared in approximately 3 hours.

### Compositions

Additional aspects of the invention comprise composition comprising a plurality of nucleic acid adaptors, as well as library compositions prepared according to the methods of the invention. Provided compositions are useful in conjunction with the methods described herein as well as for additional analysis and applications known in the art.

Thus, provided are composition comprising a plurality of nucleic acid adaptors, wherein each of the plurality of adaptors comprises a 5' universal handle sequence, optionally one or more tag sequences, and a 3' target nucleic acid sequence wherein each adaptor comprises a cleavable moiety, wherein the target nucleic acid sequence of the adaptor includes at least one cleavable moiety, and when tag sequences are present cleavable moieties are included flanking either end of the tag sequence and wherein the universal handle sequence does not include the cleavable moiety. At least two and up to one hundred thousand target specific adaptor pairs are included in provided compositions. Provided composition allow for rapid production of highly multiplexed targeted libraries.

In some embodiments, provided compositions comprise plurality of nucleic acid adaptors, wherein each of the plurality of adaptors comprise a 5' universal handle sequence, one or more tag sequences, and a 3' target nucleic acid sequence wherein each adaptor comprises a cleavable moiety; wherein the target nucleic acid sequence of the adaptor includes at least one cleavable moiety, cleavable moieties are included flanking either end of the tag sequence and the universal handle sequence does not include the cleavable moiety. At least two and up to one hundred thousand target specific adaptor pairs are included in provided compositions. Provided composition allow for rapid production of highly multiplexed, tagged, targeted libraries.

Primer/adaptor compositions may be single stranded or double stranded. In some embodiments adaptor compositions comprise are single stranded adaptors. In some embodiments adaptor compositions comprise double stranded adaptors. In some embodiments adaptor compositions comprise a mixture of single stranded and double stranded adaptors.

In some embodiments, compositions include a plurality of adaptors capable of amplification of one or more target nucleic acid sequences comprising a multiplex of adaptor pairs capable of amplification of at least two different target nucleic acid sequences wherein the target-specific primer sequence is substantially non-complementary to other target specific primer sequences in the composition. In some embodiments, the composition comprises at least 25, 50, 75, 100, 150, 200, 250, 300, 350, 400, 450, 500, 750, 1000, 1250, 1500, 1750, 2000, 2250, 2500, 2750, 3000, 3250, 3500, 3750, 4000, 4500, 5000, 5500, 6000, 7000, 8000, 9000, 10000, 11000, or 12000, or more target-specific adaptor pairs. In some embodiments, target-specific adpator pairs comprise about 15 nucleotides to about 40 nucleotides in length, wherein at least one nucleotide is replaced with a cleavable group. In some embodiments the cleavable group is a uridine nucleotide. In some embodiments, the target-specific adaptor pairs are designed to amplify an exon, gene, exome or region of the genome associated with a clinical or pathological condition, e.g., amplification of one or more sites comprising one or more mutations (e.g., driver mutation) associated with a cancer, e.g., lung, colon, breast cancer, etc., or amplification of mutations associated with an inherited disease, e.g., cystic fibrosis, muscular dystrophies, etc. In some embodiments, the target-specific adaptor pairs when hybridized to a target sequence and amplified as provided herein generates a library of adaptor-ligated amplified target sequences that are about 100 to about 600 base pairs in length. In some embodiments, no one adaptor-ligated amplified target sequence is overexpressed in the library by more than 30% as compared to the remainder of other adaptor-ligated amplified target sequences in the library. In some embodiments, an adaptor-ligated amplified target sequence library is substantially homogenous with respect to GC content, amplified target sequence length or melting temperature (Tm) of the respective target sequences.

In some embodiments, the target-specific primer sequences of adaptor pairs in the compositions of the invention are target-specific sequences that can amplify specific regions of a nucleic acid molecule. In some embodiments, the target-specific adaptors can amplify genomic DNA or cDNA. In some embodiments, target-specific adaptors can amplify mammalian nucleic acid, such as, but not limited to human DNA or RNA, murine DNA or RNA, bovine DNA or RNA, canine DNA or RNA, equine DNA or RNA, or any other mammal of interest. In other embodiments, target specific adaptors include sequences directed to amplify plant nucleic acids of interest. In other embodiments, target specific adaptors include sequences directed to amplify infectious agents, e.g., bacterial and/or viral nucleic acids. In some embodiments, the amount of nucleic acid required for selective amplification is from about 1 ng to 1 microgram. In some embodiments, the amount of nucleic acid required for selective amplification of one or more target sequences is about 1 ng, about 5 ng or about 10 ng. In some embodiments, the amount of nucleic acid required for selective amplification of target sequence is about 10 ng to about 200 ng.

As described herein, each of the plurality of adaptors comprises a 5' universal handle sequence. In some embodiments a universal handle sequence comprises any one or any combination of an amplification primer binding sequence, a sequencing primer binding sequence and/or a capture primer binding sequence. In some embodiments the comparable maximal minimum melting temperatures of each adaptor universal handle sequence is higher than the comparable maximal minimum melting temperatures of each target nucleic acid sequence and each tag sequence present in the same adaptor. Preferably, the universal handle sequences of provided adaptors do not exhibit significant complementarity and/or hybridization to any portion of a unique tag sequence and/or target nucleic acid sequence of interest. In some embodiments a first universal handle sequence comprises any one or any combination of an amplification primer binding sequence, a sequencing primer binding sequence and/or a capture primer binding sequence. In some embodiments a second universal handle sequence comprises any one or any combination of an amplification primer binding sequence, a sequencing primer binding sequence and/or a capture primer binding sequence. In certain embodiments first and second universal handle sequences correspond to forward and reverse universal handle sequences and in certain embodiments the same first and second universal handle sequences are included for each of the plurality of target specific adaptor pairs. Such forward and reverse universal handle sequences are targeted in conjunction with universal primers to carry out a second amplification of repaired amplicons in production of libraries according to methods of the invention. In certain embodiments a first 5' universal handle sequence comprises two universal handle sequences(e.g., a combination of an amplification primer binding sequence, a sequencing primer binding sequence and/or a capture primer binding sequence); and a second 5' universal sequence comprises two universal handle sequences (e.g., a combination of an amplification primer binding sequence, a sequencing primer binding sequence and/or a capture primer binding sequence), wherein the 5' first and second universal handle sequences do not exhibit significant hybridization to any portion of a target nucleic acid sequence of interest.

The structure and properties of universal amplification primers or universal primers are well known to those skilled in the art and can be implemented for utilization in conjunction with provided methods and compositions to adapt to specific analysis platforms. Universal handle sequences of the adaptors provided herein are adapted accordingly to accommodate a preferred universal primer sequences. For example, e.g., as described herein universal PI and A primers with optional barcode sequences have been described in the art and utilized for sequencing on Ion Torrent sequencing platforms (Ion Xpress™ Adapters, Thermo Fisher Scientific). Similarly, additional and other universal adaptor/primer sequences described and known in the art (e.g., Illumina universal adaptor/primer sequences can be found,e.g., at https://support.illumina.com/content/dam/illuminasupport/documents/documentation/chemistry_documentation/experiment-design/illumina-adapter-sequences_1000000002694-01.pdf; PacBio universal adaptor/primer sequences, can be found, e.g., at https://s3.amazonaws.com/files.pacb.com/pdf/Guide_Pacific_Biosciences_Template_Preparation_an d_Sequencing.pdf; etc.) can be used in conjunction with the methods and compositions provided herein. Suitable universal primers of appropriate nucleotide sequence for use with adaptors of the invention are readily prepared using standard automated nucleic acid synthesis equipment and reagents in routine use in the art. One single type of universal primer or separate types (or even a mixture) of two different universal primers, for example a pair of universal amplification primers suitable for amplification of repaired amplicons in a second amplification are included for use in the methods of the invention. Universal primers optionally include a different tag (barcode) sequence, where the tag (barcode) sequence does not hybridize to the adaptor. Barcode sequences incorporated into amplicons in a second universal amplification can be utilized e.g., for effective identification of sample source.

In some embodiments adaptors further comprise a unique tag sequence located between the 5' first universal handle sequence and the 3' target-specific sequence, and wherein the unique tag sequence does not exhibit significant complementarity and/or hybridization to any portion of a unique tag sequence and/or target nucleic acid sequence of interest. In some embodiments the plurality of primer adaptor pairs has 10⁴-10⁹ different tag sequence combinations. Thus in certain embodiments each generated target specific adaptor pair comprises 10⁴-10⁹ different tag sequences. In some embodiments the plurality of primer adaptors comprise each target specific adaptor comprising at least 1 different unique tag sequence and up to 10⁵ different unique tag sequences. In some embodiments the plurality of primer adaptors comprise each target specific adaptor comprising at least 1 different unique tag sequence and up to 10⁵ different unique tag sequences. In certain embodiments each generated target specific amplicon generated comprises at least two and up to 10⁹ different adaptor combinations comprising different tag sequences, each having two different unique tag sequences. In some embodiments the plurality of primer adaptors comprise each target specific adaptor comprising 4096 different tag sequences. In certain embodiments each generated target specific amplicon generated comprises up to 16,777,216 different adaptor combinations comprising different tag sequences, each having two different unique tag sequences.

In some embodiments individual primer adaptors in the plurality of adaptors include a unique tag sequence (e.g., contained in a tag adaptor) comprising different random tag sequences alternating with fixed tag sequences. In some embodiments, the at least one unique tag sequence comprises a at least one random sequence and at least one fixed sequence, or comprises a random sequence flanked on both sides by a fixed sequence, or comprises a fixed sequence flanked on both sides by a random sequence. In some embodiments a unique tag sequence includes a fixed sequence that is 2-2000 nucleotides or base-pairs in length. In some embodiments a unique tag sequence includes a random sequence that is 2-2000 nucleotides or base-pairs in length.

In some embodiments, unique tag sequences include a sequence having at least one random sequence interspersed with fixed sequences. In some embodiments, individual tag sequences in a plurality of unique tags have the structure (N)ₙ(X)ₓ(M)ₘ(Y)_{y}, wherein "N" represents a random tag sequence that is generated from A, G, C, T, U or I, and wherein "n" is 2-10 which represents the nucleotide length of the "N" random tag sequence; wherein "X" represents a fixed tag sequence, and wherein "x" is 2-10 which represents the nucleotide length of the "X" random tag sequence; wherein "M" represents a random tag sequence that is generated from A, G, C, T, U or I, wherein the random tag sequence "M" differs or is the same as the random tag sequence "N", and wherein "m" is 2-10 which represents the nucleotide length of the "M" random tag sequence; and wherein "Y" represents a fixed tag sequence, wherein the fixed tag sequence of "Y" is the same or differs from the fixed tag sequence of "X", and wherein "y" is 2-10 which represents the nucleotide length of the "Y" random tag sequence. In some embodiments, the fixed tag sequence "X" is the same in a plurality of tags. In some embodiments, the fixed tag sequence "X" is different in a plurality of tags. In some embodiments, the fixed tag sequence "Y" is the same in a plurality of tags. In some embodiments, the fixed tag sequence "Y" is different in a plurality of tags. In some embodiments, the fixed tag sequences "(X)ₓ" and "(Y)_{y}" within the plurality of adaptors are sequence alignment anchors.

In some embodiments, the random sequence within a unique tag sequence is represented by "N", and the fixed sequence is represented by "X". Thus, a unique tag sequence is represented by N₁N₂N₃X₁X₂X₃ or by N₁N₂N₃X₁X₂X₃N₄N₅N₆X₄X₅X₆. Optionally, a unique tag sequence can have a random sequence in which some or all of the nucleotide positions are randomly selected from a group consisting of A, G, C, T, U and I. For example, a nucleotide for each position within a random sequence is independently selected from any one of A, G, C, T, U or I, or is selected from a subset of these six different types of nucleotides. Optionally, a nucleotide for each position within a random sequence is independently selected from any one of A, G, C or T. In some embodiments, the first fixed tag sequence "X₁X₂X₃" is the same or different sequence in a plurality of tags. In some embodiments, the second fixed tag sequence "X₄X₅X₆" is the same or different sequence in a plurality of tags. In some embodiments, the first fixed tag sequence "X₁X₂X₃" and the second fixed tag sequence "X₄X₅X₆" within the plurality of adaptors are sequence alignment anchors.

In some embodiments, a unique tag sequence comprises the sequence 5'-NNNACTNNNTGA-3', where "N" represents a position within the random sequence that is generated randomly from A, G, C or T, the number of possible distinct random tags is calculated to be 4⁶ (or 4^6) is about 4096, and the number of possible different combinations of two unique tags is 4¹² (or 4^12) is about 16.78 million. In some embodiments, the underlined portions of 5'-NNNACTNNNTGA-3' are a sequence alignment anchor.

In some embodiments, the fixed sequences within the unique tag sequence is a sequence alignment anchor that can be used to generate error-corrected sequencing data. In some embodiments fixed sequences within the unique tag sequence is a sequence alignment anchor that can be used to generate a family of error-corrected sequencing reads.

Adaptors provided herein comprise at least one cleavable moiety. In some embodiments a cleavable moiety is within the 3' target-specific sequence. In some embodiments a cleavable moiety is at or near the junction between the 5' first universal handle sequence and the 3' target-specific sequence. In some embodiments a cleavable moiety is at or near the junction between the 5' first universal handle sequence and the unique tag sequence, and at or near the junction between the unique tag sequence and the 3' target-specific sequence. The cleavable moiety can be present in a modified nucleotide, nucleoside or nucleobase. In some embodiments, the cleavable moiety can include a nucleobase not naturally occurring in the target sequence of interest.

In some embodiments the at least one cleavable moiety in the plurality of adaptors is a uracil base, uridine or a deoxyuridine nucleotide. In some embodiments a cleavable moiety is within the 3' target-specific sequence and the junctions between the 5' universal handle sequence and the unique tag sequence and/or the 3'target specific sequence wherein the at least one cleavable moiety in the plurality of adaptors is cleavable with uracil DNA glycosylase (UDG). In some embodiments, a cleavable moiety is cleaved, resulting in a susceptible abasic site, wherein at least one enzyme capable of reacting on the abasic site generates a gap comprising an extendible 3' end. In certain embodiments the resulting gap comprises a 5'-deoxyribose phosphate group. In certain embodiments the resulting gap comprises an extendible 3' end and a 5' ligatable phosphate group.

In another embodiment, inosine can be incorporated into a DNA-based nucleic acid as a cleavable group. In one exemplary embodiment, EndoV can be used to cleave near the inosine residue. In another exemplary embodiment, the enzyme hAAG can be used to cleave inosine residues from a nucleic acid creating abasic sites.

Where a cleavable moiety is present, the location of the at least one cleavable moiety in the adaptors does not significantly change the melting temperature (Tm) of any given double-stranded adaptor in the plurality of double-stranded adaptors. The melting temperatures (Tm) of any two given double-stranded adaptors from the plurality of double-stranded adaptors are substantially the same, wherein the melting temperatures (Tm) of any two given double-stranded adaptors does not differ by more than 10 °C of each other. However, within each of the plurality of adaptors, the melting temperatures of sequence regions differs, such that the comparable maximal minimum melting temperature of, for example, the universal handle sequence, is higher than the comparable maximal minimum melting temperatures of either the unique tag sequence and/or the target specific sequence of any adaptor. This localized differential in comparable maximal minimum melting temperatures can be adjusted to optimize digestion and repair of amplicons and ultimately improved effectiveness of the methods provided herein.

Further provided are compositions comprising a nucleic acid library generated by methods of the invention. Thus, provided are composition comprising a plurality of amplified target nucleic acid amplicons, wherein each of the plurality of amplicons comprises a 5' universal handle sequence, optionally a first unique tag sequences, an intermediate target nucleic acid sequence, optionally a second unique tag sequences and a 3' universal handle sequence . At least two and up to one hundred thousand target specific amplicons are included in provided compositions. Provided compositions include highly multiplexed targeted libraries. In some embodiments, provided compositions comprise a plurality of nucleic acid amplicons, wherein each of the plurality of amplicons comprise a a 5' universal handle sequence, a first unique tag sequences, an intermediate target nucleic acid sequence, a second unique tag sequences and a 3' universal handle sequence . At least two and up to one hundred thousand target specific tagged amplicons are included in provided compositions. Provided compositions include highly multiplexed tagged targeted libraries.

In some embodiments, library compositions include a plurality of target specific amplicons comprising a multiplex of at least two different target nucleic acid sequences. In some embodiments, the composition comprises at least 25, 50, 75, 100, 150, 200, 250, 300, 350, 400, 450, 500, 750, 1000, 1250, 1500, 1750, 2000, 2250, 2500, 2750, 3000, 3250, 3500, 3750, 4000, 4500, 5000, 5500, 6000, 7000, 8000, 9000, 10000, 11000, or 12000, or more target-specific amplicons. In some embodiments, the target-specific amplicons comprise one or more exon, gene, exome or region of the genome associated with a clinical or pathological condition, e.g., amplicons comprising one or more sites comprising one or more mutations (e.g., driver mutation) associated with a cancer, e.g., lung, colon, breast cancer, etc., or amplicons comprising mutations associated with an inherited disease, e.g., cystic fibrosis, muscular dystrophies, etc. In some embodiments, the target-specific amplicons comprise a library of adaptor-ligated amplicon target sequences that are about 100 to about 750 base pairs in length.

As described herein, each of the plurality of amplicons comprises a 5' universal handle sequence. In some embodiments a universal handle sequence comprises any one or any combination of an amplification primer binding sequence, a sequencing primer binding sequence and/or a capture primer binding sequence. Preferably, the universal handle sequences of provided adaptors do not exhibit significant complementarity and/or hybridization to any portion of a unique tag sequence and/or target nucleic acid sequence of interest. In some embodiments a first universal handle sequence comprises any one or any combination of an amplification primer binding sequence, a sequencing primer binding sequence and/or a capture primer binding sequence. In some embodiments a second universal handle sequence comprises any one or any combination of an amplification primer binding sequence, a sequencing primer binding sequence and/or a capture primer binding sequence. In certain embodiments first and second universal handle sequences correspond to forward and reverse universal handle sequences and in certain embodiments the same first and second universal handle sequences are included for each of the plurality of target specific amplicons. Such forward and reverse universal handle sequences are targeted in conjunction with universal primers to carry out a second amplification of a preliminary library composition in production of resulting amplified according to methods of the invention. In certain embodiments a first 5' universal handle sequence comprises two universal handle sequences(e.g., a combination of an amplification primer binding sequence, a sequencing primer binding sequence and/or a capture primer binding sequence); and a second 5' universal sequence comprises two universal handle sequences (e.g., a combination of an amplification primer binding sequence, a sequencing primer binding sequence and/or a capture primer binding sequence), wherein the 5' first and second universal handle sequences do not exhibit significant hybridization to any portion of a target nucleic acid sequence of interest.

The structure and properties of universal amplification primers or universal primers are well known to those skilled in the art and can be implemented for utilization in conjunction with provided methods and compositions to adapt to specific analysis platforms. Universal handle sequences of the adaptors and amplicons provided herein are adapted accordingly to accommodate a preferred universal primer sequences. For example, e.g., as described herein universal PI and A primers with optional barcode sequences have been described in the art and utilized for sequencing on Ion Torrent sequencing platforms (Ion Xpress™ Adapters, Thermo Fisher Scientific). Similarly, additional and other universal adaptor/primer sequences described and known in the art (e.g., Illumina universal adaptor/primer sequences can be found,e.g., at https://support.illumina.com/content/dam/illuminasupport/documents/documentation/chemistry_documentation/experiment-design/illumina-adapter-sequences_1000000002694-01.pdf; PacBio universal adaptor/primer sequences, can be found, e.g., at https://s3.amazonaws.com/files.pacb.com/pdf/Guide_Pacific_Biosciences_Template_Preparation_an d_Sequencing.pdf; etc.) can be used in conjunction with the methods and compositions provided herein. Suitable universal primers of appropriate nucleotide sequence for use with libraries of the invention are readily prepared using standard automated nucleic acid synthesis equipment and reagents in routine use in the art. One single type or separate types (or even a mixture) of two different universal primers, for example a pair of universal amplification primers suitable for amplification of a preliminary library may be used in production of the libraries of the invention. Universal primers optionally include a tag (barcode) sequence, where the tag (barcode) sequence does not hybridize to adaptor sequence or to target nucleic acid sequences. Barcode sequences incorporated into amplicons in a second universal amplification can be utilized e.g., for effective identification of sample source to thereby generate a barcoded library. Thus provided compositions include highly multiplexed barcoded targeted libraries. Provided compositions also include highly multiplexed barcoded tagged targeted libraries.

In some embodiments amplicon libraries comprise a unique tag sequence located between the 5' first universal handle sequence and the 3' target-specific sequence, and wherein the unique tag sequence does not exhibit significant complementarity and/or hybridization to any portion of a unique tag sequence and/or target nucleic acid sequence. In some embodiments the plurality of amplicons has 10⁴-10⁹ different tag sequence combinations. Thus in certain embodiments each of the plurality of amplicons in a library comprises 10⁴-10⁹ different tag sequences. In some embodiments each of the plurality of amplicons in a library comprises at least 1 different unique tag sequence and up to 10⁵ different unique tag sequences. In certain embodiments each target specific amplicon in a library comprises at least two and up to 10⁹ different combinations comprising different tag sequences, each having two different unique tag sequences. In some embodiments each of the plurality of amplicons in a library comprise a tag sequence comprising 4096 different tag sequences. In certain embodiments each target specific amplicon of a library comprises up to 16,777,216 different combinations comprising different tag sequences, each having two different unique tag sequences.

In some embodiments individual amplicons in the plurality of amplicons of a library include a unique tag sequence (e.g., contained in a tag adaptor sequence) comprising different random tag sequences alternating with fixed tag sequences. In some embodiments, the at least one unique tag sequence comprises a at least one random sequence and at least one fixed sequence, or comprises a random sequence flanked on both sides by a fixed sequence, or comprises a fixed sequence flanked on both sides by a random sequence. In some embodiments a unique tag sequence includes a fixed sequence that is 2-2000 nucleotides or base-pairs in length. In some embodiments a unique tag sequence includes a random sequence that is 2-2000 nucleotides or base-pairs in length.

In some embodiments, unique tag sequences include a sequence having at least one random sequence interspersed with fixed sequences. In some embodiments, individual tag sequences in a plurality of unique tags have the structure (N)ₙ(X)ₓ(M)ₘ(Y)_{y}, wherein "N" represents a random tag sequence that is generated from A, G, C, T, U or I, and wherein "n" is 2-10 which represents the nucleotide length of the "N" random tag sequence; wherein "X" represents a fixed tag sequence, and wherein "x" is 2-10 which represents the nucleotide length of the "X" random tag sequence; wherein "M" represents a random tag sequence that is generated from A, G, C, T, U or I, wherein the random tag sequence "M" differs or is the same as the random tag sequence "N", and wherein "m" is 2-10 which represents the nucleotide length of the "M" random tag sequence; and wherein "Y" represents a fixed tag sequence, wherein the fixed tag sequence of "Y" is the same or differs from the fixed tag sequence of "X", and wherein "y" is 2-10 which represents the nucleotide length of the "Y" random tag sequence. In some embodiments, the fixed tag sequence "X" is the same in a plurality of tags. In some embodiments, the fixed tag sequence "X" is different in a plurality of tags. In some embodiments, the fixed tag sequence "Y" is the same in a plurality of tags. In some embodiments, the fixed tag sequence "Y" is different in a plurality of tags. In some embodiments, the fixed tag sequences "(X)ₓ" and "(Y)_{y}" within the plurality of amplicons are sequence alignment anchors.

In some embodiments, the random sequence within a unique tag sequence is represented by "N", and the fixed sequence is represented by "X". Thus, a unique tag sequence is represented by N₁N₂N₃X₁X₂X₃ or by N₁N₂N₃X₁X₂X₃N₄N₅N₆X₄X₅X₆. Optionally, a unique tag sequence can have a random sequence in which some or all of the nucleotide positions are randomly selected from a group consisting of A, G, C, T, U and I. For example, a nucleotide for each position within a random sequence is independently selected from any one of A, G, C, T, U or I, or is selected from a subset of these six different types of nucleotides. Optionally, a nucleotide for each position within a random sequence is independently selected from any one of A, G, C or T. In some embodiments, the first fixed tag sequence "X₁X₂X₃" is the same or different sequence in a plurality of tags. In some embodiments, the second fixed tag sequence "X₄X₅X₆" is the same or different sequence in a plurality of tags. In some embodiments, the first fixed tag sequence "X₁X₂X₃" and the second fixed tag sequence "X₄X₅X₆" within the plurality of amplicons are sequence alignment anchors.

In some embodiments, a unique tag sequence comprises the sequence 5'-NNNACTNNNTGA-3', where "N" represents a position within the random sequence that is generated randomly from A, G, C or T, the number of possible distinct random tags is calculated to be 4⁶ (or 4^6) is about 4096, and the number of possible different combinations of two unique tags is 4¹² (or 4^12) is about 16.78 million. In some embodiments, the underlined portions of 5'-NNNACTNNNTGA-3' are a sequence alignment anchor.

In some embodiments, the fixed sequences within the unique tag sequence is a sequence alignment anchor that can be used to generate error-corrected sequencing data. In some embodiments fixed sequences within the unique tag sequence is a sequence alignment anchor that can be used to generate a family of error-corrected sequencing reads.

### Kits, Systems

Further provided herein are kits for use in preparing libraries of target nucleic acids using methods of the first or second aspects of the invention. Embodiments of a kit comprise a supply of at least a pair of target specific adaptors as defined herein which are capable of producing a first amplification product; as well as optionally a supply of at least one universal pair of amplification primers capable of annealing to the universal handle(s) of the adaptor and priming synthesis of an amplification product, which amplification product would include a target sequence of interest ligated to a universal sequence. Adaptors and/or primers may be supplied in kits ready for use, or more preferably as concentrates requiring dilution before use, or even in a lyophilized or dried form requiring reconstitution prior to use. In certain embodiments kits further include a supply of a suitable diluent for dilution or reconstitution of the components. Optionally, kits further comprise supplies of reagents, buffers, enzymes, dNTPs, etc., for use in carrying out amplification, digestion, repair, and/or purification in the generation of library as provided herein. Non-limiting examples of such reagents are as described in the Materials and Methods sections of the accompanying Exemplification. Further components which optionally are supplied in the kit include components suitable for purification of libraries prepared using the provided methods._In some embodiments, provided is a kit for generating a target-specific library comprising a plurality of target-specific adaptors having a 5' universal handle sequence, a 3' target specific sequence and a cleavable group, a DNA polymerase, an adaptor, dATP, dCTP, dGTP, dTTP, and a digestion reagent. In some embodiments, the kit further comprises one or more antibodies, a repair reagent, universal primers optionally comprising nucleic acid barcodes, purification solutions or columns.

Particular features of adaptors for inclusion in kits are as described elsewhere herein in relation to other aspects of the invention. The structure and properties of universal amplification primers are well known to those skilled in the art and can be implemented for utilization in conjunction with provided methods and compositions to adapt to specific analysis platforms (e.g., as described herein universal PI and A primers have been described in the art and utilized for sequencing on Ion Torrent sequencing platforms). Similarly, additional and other universal adaptor/primer sequences described and known in the art (e.g., Illumina universal adaptor/primer sequences, PacBio universal adaptor/primer sequences, etc.) can be used in conjunction with the methods and compositions provided herein. Suitable primers of appropriate nucleotide sequence for use with adaptors included in the kit is readily prepared using standard automated nucleic acid synthesis equipment and reagents in routine use in the art. A kit may include a supply of one single type of universal primer or separate types (or even a mixture) of two different universal primers, for example a pair of amplification primers suitable for amplification of templates modified with adaptors in a first amplification. A kit may comprise at least a pair of adaptors for first amplification of a sample of interest according to the methods of the invention, plus at least two different amplification primers that optionally carry a different tag (barcode) sequence, where the tag (barcode) sequence does not hybridize to the adaptor. A kit can be used to amplify at least two different samples where each sample is amplified according to methods of the invention separately and a second amplification comprises using a single universal primer having a barcode, and then pooling prepared sample libraries after library preparations. In some embodiments a kit includes different universal primer-pairs for use in second amplification step described herein. In this context the 'universal' primer-pairs may be of substantially identical nucleotide sequence but differ with respect to some other feature or modification.

Further provided are systems, e.g., systems used to practice methods provided herein, and/or comprising compositions provided herein. In some embodiments, systems facilitate methods carried out in automated mode. In certain embodiments, systems facilitate high throughput mode. In certain embodiments, systems include, e.g., a fluid handling element, a fluid containing element, a heat source and/or heat sink for achieving and maintaining a desired reaction temperature, and/or a robotic element capable of moving components of the system from place to place as needed (e.g., a multiwell plate handling element).

### Samples

As defined herein, "sample" and its derivatives, is used in its broadest sense and includes any specimen, culture and/or the like that is suspected of including a target nucleic acid. In some embodiments, a sample comprises DNA, RNA, chimeric nucleic acid, hybrid nucleic acid, multiplexforms of nucleic acids or any combination of two or more of the foregoing. In some embodiments a sample useful in conjunction with methods of the invention includes any biological, clinical, surgical, agricultural, atmospheric or aquatic-based specimen containing one or more target nucleic acid of interest. In some embodiments, a sample includes nucleic acid molecules obtained from an animal such as a human or mammalian source. In another embodiment, a sample includes nucleic acid molecules obtained from a non-mammalian source such as a plant, bacteria, virus or fungus. In some embodiments, the source of the nucleic acid molecules may be an archived or extinct sample or species. In some embodiments a sample includes isolated nucleic acid sample prepared, for example, from a source such as genomic DNA, RNA or a prepared sample such as, e.g., fresh-frozen or formalin-fixed paraffin-embedded (FFPE) nucleic acid specimen. It is also envisioned that a sample is from a single individual, a collection of nucleic acid samples from genetically related members, multiple nucleic acid samples from genetically unrelated members, multiple nucleic acid samples (matched) from a single individual such as a tumor sample and normal tissue sample, or genetic material from a single source that contains two distinct forms of genetic material such as maternal and fetal DNA obtained from a maternal subject, or the presence of contaminating bacteria DNA in a sample that contains plant or animal DNA. In some embodiments, a source of nucleic acid material includes nucleic acids obtained from a newborn (e.g., a blood sample for newborn screening). In some embodiments, provided methods comprise amplification of multiple target-specific sequences from a single nucleic acid sample. In some embodiments, provided methods comprise target-specific amplification of two or more target sequences from two or more nucleic acid samples or species. In certain embodiments, provided methods comprise amplification of highly multiplexed target nucleic acid sequences from a single sample. In particular embodiments, provided methods comprise amplification of highly multiplexed target nucleic acid sequences from more than one sample, each from the same source organism.

In some embodiments a sample comprises a mixture of target nucleic acids and non-target nucleic acids. In certain embodiments a sample comprises a plurality of initial polynucleotides which comprises a mixture of one or more target nucleic acids and may include one or more non-target nucleic acids. In some embodiments a sample comprising a plurality of polynucleotides comprises a portion or aliquot of an originating sample; in some embodiments, a sample comprises a plurality of polynucleotides which is the entire originating sample. In some embodiments a sample comprises a plurality of initial polynucleotides is isolated from the same source or from the same subject at different time points.

In some embodiments, a nucleic acid sample includes cell-free nucleic acids from a biological fluid, nucleic acids from a tissue, nucleic acids from a biopsied tissue, nucleic acids from a needle biopsy, nucleic acids from a single cell or nucleic acids from two or more cells. In certain embodiments, a single reaction mixture contains 1-100 ng of the plurality of initial polynucleotides. In some embodiments a plurality of initial polynucleotides comprises a formalin fixed paraffin-embedded (FFPE) sample; genomic DNA; RNA; cell free DNA or RNA; circulating tumor DNA or RNA; fresh frozen sample, or a mixture of two or more of the foregoing; and in some embodiments a the plurality of initial polynucleotides comprises a nucleic acid reference standard. In some embodiments, a sample includes nucleic acid molecules obtained from biopsies, tumors, scrapings, swabs, blood, mucus, urine, plasma, semen, hair, laser capture micro-dissections, surgical resections, and other clinical or laboratory obtained sample. In some embodiments, a sample is an epidemiological, agricultural, forensic or pathogenic sample. In certain embodiments, a sample includes a reference. In some embodiments a sample is a normal tissue or well documented tumor sample. In certain embodiments a reference is a standard nucleic acid sequence (e.g., Hg19).

### Target Nucleic Acid Sequence Analysis

Provided methods and compositions of the invention are particularly suitable for amplifying, optionally tagging, and preparing target sequences for subsequent analysis. Thus, in some embodiments, methods provided herein include analyzing resulting library preparations. For example, methods comprise analysis of a polynucleotide sequence of a target nucleic acid, and, where applicable, analysis of any tag sequence(s) added to a target nucleic acid. In some embodiments wherein multiple target nucleic acid regions are amplified, provided methods include determining polynucleotide sequences of multiple target nucleic acids. Provided methods further optionally include using a second tag sequence(s), e.g., barcode sequence, to identify the source of the target sequence (or to provide other information about the sample source). In certain embodiments, use of prepared library composition is provided for analysis of the sequences of the nucleic acid library.

In particular embodiments, use of prepared tagged library compositions is provided for further analyzing the sequences of the target nucleic acid library. In some embodiments determination of sequences comprises determining the abundance of at least one of the target sequences in the sample. In some embodiments determination of a low frequency allele in a sample is comprised in determination of sequences of a nucleic acid library. In certain embodiments, determination of the presence of a mutant target nucleic acid in the plurality of polynucleotides is comprised in determination of sequences of a nucleic acid library. In some embodiments, determination of the presence of a mutant target nucleic acid comprises detecting the abundance level of at least one mutant target nucleic acid in the plurality of polynucleotides. For example, such determination comprises detecting at least one mutant target nucleic acid is present at 0.05% to 1% of the original plurality of polynucleotides in the sample, detecting at least one mutant target nucleic acid is present at about 1% to about 5% of the polynucleotides in the sample, and/or detecting at least 85%-100% of target nucleic acids in sample. In some embodiments, determination of the presence of a mutant target nucleic acid comprises detecting and identification of copy number variation and/or genetic fusion sequences in a sample.

In some embodiments, nucleic acid sequencing of the amplified target sequences produced by the teachings of this disclosure include *de novo* sequencing or targeted re-sequencing. In some embodiments, nucleic acid sequencing further includes comparing the nucleic acid sequencing results of the amplified target sequences against a reference nucleic acid sequence. In some embodiments, nucleic acid sequencing of the target library sequences further includes determining the presence or absence of a mutation within a nucleic acid sequence. In some embodiments, nucleic acid sequencing includes the identification of genetic markers associated with disease (e.g., cancer and/or inherited disease).

In some embodiments, prepared library of target sequences of the disclosed methods is used in various downstream analysis or assays with, or without, further purification or manipulation. In some embodiments analysis comprises sequencing by traditional sequencing reactions, high throughput next generation sequencing, targeted multiplex array sequence detection, or any combination of two or more of the foregoing. In certain embodiments analysis is carried out by high throughput next generation sequencing. In particular embodiments sequencing is carried out in a bidirectional manner, thereby generating sequence reads in both forward and reverse strands for any given amplicon.

In some embodiments, library prepared according to the methods provided herein is then further manipulated for additional analysis. For example, \ prepared library sequences is used in downstream enrichment techniques known in the art, such a bridge amplification or emPCR to generate a template library that is then used in next generation sequencing. In some embodiments, the target nucleic acid library is used in an enrichment application and a sequencing application. For example, sequence determination of a provided target nucleic acid library is accomplished using any suitable DNA sequencing platform. In some embodiments, the library sequences of the disclosed methods or subsequently prepared template libraries is used for single nucleotide polymorphism (SNP) analysis, genotyping or epigenetic analysis, copy number variation analysis, gene expression analysis, analysis of gene mutations including but not limited to detection, prognosis and/or diagnosis, detection and analysis of rare or low frequency allele mutations, nucleic acid sequencing including but not limited to de novo sequencing, targeted resequencing and synthetic assembly analysis. In one embodiment, prepared library sequences are used to detect mutations at less than 5% allele frequency. In some embodiments, the methods disclosed herein is used to detect mutations in a population of nucleic acids at less than 4%, 3%, 2% or at about 1% allele frequency. In another embodiment, libraries prepared as described herein are sequenced to detect and/or identify germline or somatic mutations from a population of nucleic acid molecules. In certain embodiments, sequencing adaptors are ligated to the ends of the prepared libraries generate a plurality of libraries suitable for nucleic acid sequencing.

In some embodiments, methods for preparing a target-specific amplicon library are provided for use in a variety of downstream processes or assays such as nucleic acid sequencing or clonal amplification. In some embodiments, the library is amplified using bridge amplification or emPCR to generate a plurality of clonal templates suitable for nucleic acid sequencing. For example, optionally following target-specific amplification a secondary and/or tertiary amplification process including, but not limited to, a library amplification step and/or a clonal amplification step is performed. "Clonal amplification" refers to the generation of many copies of an individual molecule. Various methods known in the art is used for clonal amplification. For example, emulsion PCR is one method, and involves isolating individual DNA molecules along with primer-coated beads in aqueous bubbles within an oil phase. A polymerase chain reaction (PCR) then coats each bead with clonal copies of the isolated library molecule and these beads are subsequently immobilized for later sequencing. Emulsion PCR is used in the methods published by Marguilis *et al.* and Shendure and Porreca *et al.* (also known as "polony sequencing", commercialized by Agencourt and recently acquired by Applied Biosystems). Margulies, et al. (2005) Nature 437: 376-380; Shendure et al., Science 309 (5741): 1728-1732. Another method for clonal amplification is "bridge PCR," where fragments are amplified upon primers attached to a solid surface. These methods, as well as other methods of clonal amplification, both produce many physically isolated locations that each contain many copies derived from a single molecule polynucleotide fragment. Thus, in some embodiments, the one or more target specific amplicons are amplified using for example, bridge amplification or emPCR to generate a plurality of clonal templates suitable for nucleic acid sequencing.

In some embodiments, at least one of the library sequences to be clonally amplified are attached to a support or particle. A support can be comprised of any suitable material and have any suitable shape, including, for example, planar, spheroid or particulate. In some embodiments, the support is a scaffolded polymer particle as described in U.S. Published App. No. 20100304982. In certain embodiments methods comprise depositing at least a portion of an enriched population of library sequences onto a support (e.g., a sequencing support), wherein the support comprises an array of sequencing reaction sites. In some embodiments, an enriched population of library sequences are attached to the sequencing reaction sites on the support. wherein the support comprises an array of 10² - 10¹⁰ sequencing reaction sites.

Sequence determination means determination of information relating to the sequence of a nucleic acid and may include identification or determination of partial as well as full sequence information of the nucleic acid. Sequence information may be determined with varying degrees of statistical reliability or confidence. In some embodiments sequence analysis includes high throughput, low depth detection such as by qPCR, rtPCR, and/or array hybridization detection methodologies known in the art. In some embodiments, sequencing analysis includes the determination of the in depth sequence assessment, such as by Sanger sequencing or other high throughput next generation sequencing methods. Next-generation sequencing means sequence determination using methods that determine many (typically thousands to billions) nucleic acid sequences in an intrinsically massively parallel manner, e.g. where many sequences are read out, e.g., in parallel, or alternatively using an ultra-high throughput serial process that itself may be parallelized. Thus, in certain embodiments, methods of the invention include sequencing analysis comprising massively parallel sequencing. Such methods include but are not limited to pyrosequencing (for example, as commercialized by 454 Life Sciences, Inc., Branford, Conn.); sequencing by ligation (for example, as commercialized in the SOLiD™. technology, Life Technologies, Inc., Carlsbad, Calif.); sequencing by synthesis using modified nucleotides (such as commercialized in TruSeq™ and HiSeg™. technology by Illumina, Inc., San Diego, Calif.; HeliScope™ by Helicos Biosciences Corporation, Cambridge, Mass.; and PacBio Sequel® or RS systems by Pacific Biosciences of California, Inc., Menlo Park, Calif.), sequencing by ion detection technologies (e.g., Ion Torrent™ technology, Life Technologies, Carlsbad, Calif.); sequencing of DNA nanoballs (Complete Genomics, Inc., Mountain View, Calif.); nanopore-based sequencing technologies (for example, as developed by Oxford Nanopore Technologies, LTD, Oxford, UK), and like highly parallelized sequencing methods.

For example, in certain embodiments, libraries produced by the teachings of the present disclosure are sufficient in yield to be used in a variety of downstream applications including the Ion Xpress™ Template Kit using an Ion Torrent™ PGM system (e.g., PCR-mediated addition of the nucleic acid fragment library onto Ion Sphere™ Particles)(Life Technologies, Part No. 4467389) or Ion Torrent Proton™ system). For example, instructions to prepare a template library from the amplicon library can be found in the Ion Xpress Template Kit User Guide (Life Technologies, Part No. 4465884). Instructions for loading the subsequent template library onto the Ion Torrent™ Chip for nucleic acid sequencing are described in the Ion Sequencing User Guide (Part No. 4467391). Similarly, sequencing using other platforms (e.g., PacBio, Illumina, Helicos, Complete Genomics, Oxford Nanopore) may be carried out using adapted methodologies to incorporate the relevant template preparation according to the instructions and guidance provided with each of the respective platforms.

The initiation point for the sequencing reaction may be provided by annealing a sequencing primer to a product of a solid-phase amplification reaction. In this regard, one or both of the adaptors added during formation of template library may include a nucleotide sequence which permits annealing of a sequencing primer to amplified products derived by whole genome or solid-phase amplification of the template library. Depending on implementation of an embodiment of the invention, a tag sequence and/or target nucleic acid sequence may be determined in a single read from a single sequencing primer, or in multiple reads from two different sequencing primers. In the case of two reads from two sequencing primers, a 'tag read' and a 'target sequence read' are performed in either order, with a suitable denaturing step to remove an annealed primer after the first sequencing read is completed.

In some embodiments, a sequencer is coupled to server that applies parameters or software to determine the sequence of the amplified target nucleic acid molecules. In certain embodiments, the sequencer is coupled to a server that applies parameters or software to determine the presence of a low frequency mutation allele present in a sample.

### EMBODIMENTS

In one embodiment, a method for preparing a library of target nucleic acid sequences is provided comprising contacting a nucleic acid sample with a plurality of adaptors capable of amplification of one or more target nucleic acid sequences in the sample under conditions wherein the target nucleic acid(s) undergo a first amplification; digesting resulting first amplification products to reduce or eliminate resulting primer dimers and prepare partially digested target amplicons, producing gapped, double stranded amplicons, then repairing the partially digested target amplicons; and amplifying the repaired target amplicons in a second amplification using universal primers, wherein each of the plurality of adaptors comprise a universal handle sequence and a target nucleic acid sequence and a cleavable moiety, wherein at least two and up to one hundred thousand target specific adaptor pairs are included, and wherein the target nucleic acid sequence of the adaptor includes at least one cleavable moiety and the universal handle sequence does not include the cleavable moiety. Optionally one or more tag sequences are comprised in each of the plurality of adaptors. Such methods thereby produce a library of target nucleic acid sequence. In some embodiments, the digestion and repair is carried out in a single step. In particular embodiments the plurality of gapped polynucleotide products in digestion are contacted with the digestion and repair reagents simultaneously. In other embodiments the digestion and repair step is carried out in a temporally separate manner at different temperatures. In particular embodiments the plurality of gapped polynucleotide products in digestion are contacted sequentially with the digestion and repair reagents. In some embodiments one or more of the method steps is conducted in manual mode or in an automated mode or a combination thereof. In particular embodiments each of the method steps is carried out in automated mode. In some embodiments the foregoing methods further comprise at least one purification step. In particular embodiments a purification step is carried out only after the second universal amplification step. In other particular embodiments a purification is carried out after the digestion and repair step and an additional purification is carried out after the second universal amplification. In some of the embodiments adaptor-dimer by products resulting from the first amplification are removed from the resulting library, and in some embodiments an enriched population of amplified target nucleic acids contains a reduced amount of adaptor-dimer byproduct. In certain embodiments, adaptor-dimer byproducts are eliminated. In the foregoing methods the plurality of adaptors capable of amplification of one or more target nucleic acid sequences comprises a multiplex of adaptor pairs capable of amplification of at least two different target nucleic acid sequences. In some embodiments, each target specific pair of the plurality of adaptors includes up to 16,777,216 different adaptor combinations comprising different tag sequences. In certain embodiments each generated target specific amplicon sequence includes at least 1 different sequence and up to 10⁷ different sequences. In some embodiments, the foregoing methods further comprise analyzing the sequence of the resulting library of target nucleic acid sequences. Such analyzing comprises sequencing by traditional sequencing reactions, high throughput next generation sequencing, targeted multiplex array sequence detection, or any combination of two or more of the foregoing. In other embodiments, the foregoing methods further comprise determining the abundance of at least one of the target nucleic acid sequences in the sample. Such determining is carried out by high throughput throughput next generation sequencing in certain embodiments. In particular embodiments, such sequencing is carried out in a bidirectional manner, thereby generating sequence reads in both forward and reverse strands for any given amplicon. In some embodiments the foregoing methods comprise digestion reagent selected from any one or a combination of uracil DNA glycosylase (UDG),. apurinic endonuclease (e.g., APE1), RecJf, formamidopyrimidine [fapy]-DNA glycosylase (fpg), Nth endonuclease III, endonuclease VIII, polynucleotide kinase (PNK), Taq DNA polymerase, DNA polymerase I and/or human DNA polymerase beta. In some embodiments, the foregoing methods methods comprise repair reagent selected from any one or a combination of Phusion DNA polymerase, Phusion U DNA polymerase, SuperFi DNA polymerase, Taq DNA polymerase, Human DNA polymerase beta, T4 DNA polymerase and/or T7 DNA polymerase, SuperFiU DNA polymerase, E. *coli* DNA ligase, T3 DNA ligase, T4 DNA ligase, T7 DNA ligase, Taq DNA ligase, and/or 9°N DNA ligase. In particular embodiments the foregoing methods comprise digestion and repair reagent selected from any one or a combination of uracil DNA glycosylase (UDG),apurinic endonuclease (e.g., APE1), Taq DNA polymerase, Phusion U DNA polymerase, SuperFiU DNA polymerase, 7 DNA ligase. In more particular embodiments the foregoing methods comprise digestion and repair reagent selected from any one or a combination of uracil DNA glycosylase (UDG) ,. formamidopyrimidine [fapy]-DNA glycosylase (fpg), Phusion U DNA polymerase, Taq DNA polymerase, SuperFiU DNA polymerase, T4 PNK and T7 DNA ligase. In preferred embodiments, the foregoing methods generate compositions comprising nucleic acid library. In particularly preferred embodiments, generated compositions comprising nucleic acid library are useful for analysis of sequences.. In specific embodiments, use comprises determination of low frequency allele(s) in a sample.

In one embodiment, a method for preparing a library of target nucleic acid sequences is provided comprising contacting a nucleic acid sample with a plurality of adaptors capable of amplification of one or more target nucleic acid sequences in the sample under conditions wherein the target nucleic acid(s) undergo a first amplification; digesting resulting first amplification products to reduce or eliminate resulting primer dimers and prepare partially digested target amplicons, producing gapped, double stranded amplicons, then repairing the partially digested target amplicons; and amplifying the repaired target amplicons in a second amplification using universal primers, wherein each of the plurality of adaptors comprise a universal handle sequence and a target nucleic acid sequence and a cleavable moiety and a tag sequence is included in at least one adaptor, and the cleavable moieties are included flanking either end of the tag sequence, wherein at least two and up to one hundred thousand target specific adaptor pairs are included, and wherein the target nucleic acid sequence of the adaptor includes at least one cleavable moiety and the universal handle sequence does not include the cleavable moiety. Such methods thereby produce a library of target nucleic acid sequence. In some embodiments, the digestion and repair is carried out in a single step. In particular embodiments the plurality of gapped polynucleotide products in digestion are contacted with the digestion and repair reagents simultaneously. In other embodiments the digestion and repair step is carried out in a temporally separate manner at different temperatures. In particular embodiments the plurality of gapped polynucleotide products in digestion are contacted sequentially with the digestion and repair reagents. In some embodiments one or more of the method steps is conducted in manual mode or in an automated mode or a combination thereof. In particular embodiments each of the method steps is carried out in automated mode. In some embodiments the foregoing methods further comprise at least one purification step. In particular embodiments a purification step is carried out only after the second universal amplification step. In other particular embodiments a purification is carried out after the digestion and repair step and an additional purification is carried out after the second universal amplification. In some of the embodiments adaptor-dimer by products resulting from the first amplification are removed from the resulting library, and in some embodiments an enriched population of amplified target nucleic acids contains a reduced amount of adaptor-dimer byproduct. In certain embodiments, adaptor-dimer byproducts are eliminated. In the foregoing methods the plurality of adaptors capable of amplification of one or more target nucleic acid sequences comprises a multiplex of adaptor pairs capable of amplification of at least two different target nucleic acid sequences. In some embodiments, each target specific pair of the plurality of adaptors includes up to 16,777,216 different adaptor combinations comprising different tag sequences. In certain embodiments each generated target specific amplicon sequence includes at least 1 different sequence and up to 10⁷ different sequences. In some embodiments, the foregoing methods further comprise analyzing the sequence of the resulting library of target nucleic acid sequences. Such analyzing comprises sequencing by traditional sequencing reactions, high throughput next generation sequencing, targeted multiplex array sequence detection, or any combination of two or more of the foregoing. In other embodiments, the foregoing methods further comprise determining the abundance of at least one of the target nucleic acid sequences in the sample. Such determining is carried out by high throughput throughput next generation sequencing in certain embodiments. In particular embodiments, such sequencing is carried out in a bidirectional manner, thereby generating sequence reads in both forward and reverse strands for any given amplicon. In some embodiments the foregoing methods comprise digestion reagent selected from any one or a combination of uracil DNA glycosylase (UDG),. apurinic endonuclease (e.g., APE1), RecJf, formamidopyrimidine [fapy]-DNA glycosylase (fpg), Nth endonuclease III, endonuclease VIII, polynucleotide kinase (PNK), Taq DNA polymerase, DNA polymerase I and/or human DNA polymerase beta. In some embodiments, the foregoing methods methods comprise repair reagent selected from any one or a combination of Phusion DNA polymerase, Phusion U DNA polymerase, SuperFi DNA polymerase, Taq DNA polymerase, Human DNA polymerase beta, T4 DNA polymerase and/or T7 DNA polymerase, SuperFiU DNA polymerase, *E. coli* DNA ligase, T3 DNA ligase, T4 DNA ligase, T7 DNA ligase, Taq DNA ligase, and/or 9°N DNA ligase. In particular embodiments the foregoing methods comprise digestion and repair reagent selected from any one or a combination of uracil DNA glycosylase (UDG),apurinic endonuclease (e.g., APE1), Taq DNA polymerase, Phusion U DNA polymerase, SuperFiU DNA polymerase, 7 DNA ligase. In more particular embodiments the foregoing methods comprise digestion and repair reagent selected from any one or a combination of uracil DNA glycosylase (UDG),. formamidopyrimidine [fapy]-DNA glycosylase (fpg), Phusion U DNA polymerase, Taq DNA polymerase, SuperFiU DNA polymerase, T4 PNK and T7 DNA ligase. In preferred embodiments, the foregoing methods generate compositions comprising nucleic acid library. In particularly preferred embodiments, generated compositions comprising nucleic acid library are useful for analysis of sequences.. In specific embodiments, use comprises determination of low frequency allele(s) in a sample.

In one embodiment, a method for preparing a library of target nucleic acid sequences is provided comprising contacting a nucleic acid sample with a plurality of adaptors capable of amplification of one or more target nucleic acid sequences in the sample under conditions wherein the target nucleic acid(s) undergo a first amplification; digesting resulting first amplification products to reduce or eliminate resulting primer dimers and prepare partially digested target amplicons, producing gapped, double stranded amplicons, then repairing the partially digested target amplicons; and amplifying the repaired target amplicons in a second amplification using universal primers, wherein each of the plurality of adaptors comprise a universal handle sequence and a target nucleic acid sequence and a cleavable moiety, wherein at least two and up to one hundred thousand target specific adaptor pairs are included, and wherein the target nucleic acid sequence of the adaptor includes at least one cleavable moiety and the universal handle sequence does not include the cleavable moiety and the melting temperature of each universal sequence is higher than the melting temperature of each target nucleic acid sequence and each tag sequence present. Optionally one or more tag sequences are comprised in each of the plurality of adaptors. Such methods thereby produce a library of target nucleic acid sequence. In some embodiments, the digestion and repair is carried out in a single step. In particular embodiments the plurality of gapped polynucleotide products in digestion are contacted with the digestion and repair reagents simultaneously. In other embodiments the digestion and repair step is carried out in a temporally separate manner at different temperatures. In particular embodiments the plurality of gapped polynucleotide products in digestion are contacted sequentially with the digestion and repair reagents. In some embodiments one or more of the method steps is conducted in manual mode or in an automated mode or a combination thereof. In particular embodiments each of the method steps is carried out in automated mode. In some embodiments the foregoing methods further comprise at least one purification step. In particular embodiments a purification step is carried out only after the second universal amplification step. In other particular embodiments a purification is carried out after the digestion and repair step and an additional purification is carried out after the second universal amplification. In some of the embodiments adaptor-dimer by products resulting from the first amplification are removed from the resulting library, and in some embodiments an enriched population of amplified target nucleic acids contains a reduced amount of adaptor-dimer byproduct. In certain embodiments, adaptor-dimer byproducts are eliminated. In the foregoing methods the plurality of adaptors capable of amplification of one or more target nucleic acid sequences comprises a multiplex of adaptor pairs capable of amplification of at least two different target nucleic acid sequences. In some embodiments, each target specific pair of the plurality of adaptors includes up to 16,777,216 different adaptor combinations comprising different tag sequences. In certain embodiments each generated target specific amplicon sequence includes at least 1 different sequence and up to 10⁷ different sequences. In some embodiments, the foregoing methods further comprise analyzing the sequence of the resulting library of target nucleic acid sequences. Such analyzing comprises sequencing by traditional sequencing reactions, high throughput next generation sequencing, targeted multiplex array sequence detection, or any combination of two or more of the foregoing. In other embodiments, the foregoing methods further comprise determining the abundance of at least one of the target nucleic acid sequences in the sample. Such determining is carried out by high throughput throughput next generation sequencing in certain embodiments. In particular embodiments, such sequencing is carried out in a bidirectional manner, thereby generating sequence reads in both forward and reverse strands for any given amplicon. In some embodiments the foregoing methods comprise digestion reagent selected from any one or a combination of uracil DNA glycosylase (UDG),. apurinic endonuclease (e.g., APE1), RecJf, formamidopyrimidine [fapy]-DNA glycosylase (fpg), Nth endonuclease III, endonuclease VIII, polynucleotide kinase (PNK), Taq DNA polymerase, DNA polymerase I and/or human DNA polymerase beta. In some embodiments, the foregoing methods methods comprise repair reagent selected from any one or a combination of Phusion DNA polymerase, Phusion U DNA polymerase, SuperFi DNA polymerase, Taq DNA polymerase, Human DNA polymerase beta, T4 DNA polymerase and/or T7 DNA polymerase, SuperFiU DNA polymerase, E. *coli* DNA ligase, T3 DNA ligase, T4 DNA ligase, T7 DNA ligase, Taq DNA ligase, and/or 9°N DNA ligase. In particular embodiments the foregoing methods comprise digestion and repair reagent selected from any one or a combination of uracil DNA glycosylase (UDG) ,apurinic endonuclease (e.g., APE1), Taq DNA polymerase, Phusion U DNA polymerase, SuperFiU DNA polymerase, 7 DNA ligase. In more particular embodiments the foregoing methods comprise digestion and repair reagent selected from any one or a combination of uracil DNA glycosylase (UDG),. formamidopyrimidine [fapy]-DNA glycosylase (fpg), Phusion U DNA polymerase, Taq DNA polymerase, SuperFiU DNA polymerase, T4 PNK and T7 DNA ligase. In preferred embodiments, the foregoing methods generate compositions comprising nucleic acid library. In particularly preferred embodiments, generated compositions comprising nucleic acid library are useful for analysis of sequences.. In specific embodiments, use comprises determination of low frequency allele(s) in a sample.

In one embodiment, a method for preparing a library of target nucleic acid sequences is provided comprising contacting a nucleic acid sample with a plurality of adaptors capable of amplification of one or more target nucleic acid sequences in the sample under conditions wherein the target nucleic acid(s) undergo a first amplification; digesting resulting first amplification products to reduce or eliminate resulting primer dimers and prepare partially digested target amplicons, producing gapped, double stranded amplicons, then repairing the partially digested target amplicons; and amplifying the repaired target amplicons in a second amplification using universal primers, wherein each of the plurality of adaptors comprise a universal handle sequence and a target nucleic acid sequence and a cleavable moiety, wherein at least two and up to one hundred thousand target specific adaptor pairs are included, and wherein the target nucleic acid sequence of the adaptor includes at least one cleavable moiety and the universal handle sequence does not include the cleavable moiety. Optionally one or more tag sequences are comprised in each of the plurality of adaptors. Such methods are carried out in a single, addition only workflow reaction, allowing for rapid production of highly multiplexed targeted libraries thereby produce a library of target nucleic acid sequence. In some embodiments, the digestion and repair is carried out in a single step. In particular embodiments the plurality of gapped polynucleotide products in digestion are contacted with the digestion and repair reagents simultaneously. In other embodiments the digestion and repair step is carried out in a temporally separate manner at different temperatures. In particular embodiments the plurality of gapped polynucleotide products in digestion are contacted sequentially with the digestion and repair reagents. In some embodiments one or more of the method steps is conducted in manual mode or in an automated mode or a combination thereof. In particular embodiments each of the method steps is carried out in automated mode. In some embodiments the foregoing methods further comprise at least one purification step. In particular embodiments a purification step is carried out only after the second universal amplification step. In other particular embodiments a purification is carried out after the digestion and repair step and an additional purification is carried out after the second universal amplification. In some of the embodiments adaptor-dimer by products resulting from the first amplification are removed from the resulting library, and in some embodiments an enriched population of amplified target nucleic acids contains a reduced amount of adaptor-dimer byproduct. In certain embodiments, adaptor-dimer byproducts are eliminated. In the foregoing methods the plurality of adaptors capable of amplification of one or more target nucleic acid sequences comprises a multiplex of adaptor pairs capable of amplification of at least two different target nucleic acid sequences. In some embodiments, each target specific pair of the plurality of adaptors includes up to 16,777,216 different adaptor combinations comprising different tag sequences. In certain embodiments each generated target specific amplicon sequence includes at least 1 different sequence and up to 10⁷ different sequences. In some embodiments, the foregoing methods further comprise analyzing the sequence of the resulting library of target nucleic acid sequences. Such analyzing comprises sequencing by traditional sequencing reactions, high throughput next generation sequencing, targeted multiplex array sequence detection, or any combination of two or more of the foregoing. In other embodiments, the foregoing methods further comprise determining the abundance of at least one of the target nucleic acid sequences in the sample. Such determining is carried out by high throughput throughput next generation sequencing in certain embodiments. In particular embodiments, such sequencing is carried out in a bidirectional manner, thereby generating sequence reads in both forward and reverse strands for any given amplicon. In some embodiments the foregoing methods comprise digestion reagent selected from any one or a combination of uracil DNA glycosylase (UDG),. apurinic endonuclease (e.g., APE1), RecJf, formamidopyrimidine [fapy]-DNA glycosylase (fpg), Nth endonuclease III, endonuclease VIII, polynucleotide kinase (PNK), Taq DNA polymerase, DNA polymerase I and/or human DNA polymerase beta. In some embodiments, the foregoing methods methods comprise repair reagent selected from any one or a combination of Phusion DNA polymerase, Phusion U DNA polymerase, SuperFi DNA polymerase, Taq DNA polymerase, Human DNA polymerase beta, T4 DNA polymerase and/or T7 DNA polymerase, SuperFiU DNA polymerase, *E. coli* DNA ligase, T3 DNA ligase, T4 DNA ligase, T7 DNA ligase, Taq DNA ligase, and/or 9°N DNA ligase. In particular embodiments the foregoing methods comprise digestion and repair reagent selected from any one or a combination of uracil DNA glycosylase (UDG) ,apurinic endonuclease (e.g., APE1), Taq DNA polymerase, Phusion U DNA polymerase, SuperFiU DNA polymerase, 7 DNA ligase. In more particular embodiments the foregoing methods comprise digestion and repair reagent selected from any one or a combination of uracil DNA glycosylase (UDG),. formamidopyrimidine [fapy]-DNA glycosylase (fpg), Phusion U DNA polymerase, Taq DNA polymerase, SuperFiU DNA polymerase, T4 PNK and T7 DNA ligase. In preferred embodiments, the foregoing methods generate compositions comprising nucleic acid library. In particularly preferred embodiments, generated compositions comprising nucleic acid library are useful for analysis of sequences.. In specific embodiments, use comprises determination of low frequency allele(s) in a sample.

In one embodiment, a method for preparing a library of target nucleic acid sequences is provided comprising contacting a nucleic acid sample with a plurality of adaptors capable of amplification of one or more target nucleic acid sequences in the sample under conditions wherein the target nucleic acid(s) undergo a first amplification; digesting resulting first amplification products to reduce or eliminate resulting primer dimers and prepare partially digested target amplicons, producing gapped, double stranded amplicons, then repairing the partially digested target amplicons; and amplifying the repaired target amplicons in a second amplification using universal primers, wherein each of the plurality of adaptors comprise a universal handle sequence and a target nucleic acid sequence and a cleavable moiety and all of the adaptors comprise tag sequences having cleavable groups flanking either end of the tag sequence, wherein at least two and up to one hundred thousand target specific adaptor pairs are included, and wherein the target nucleic acid sequence of the adaptor includes at least one cleavable moiety and the universal handle sequence does not include the cleavable moiety. Such methods thereby produce a library of target nucleic acid sequence. In some embodiments, the digestion and repair is carried out in a single step. In particular embodiments the plurality of gapped polynucleotide products in digestion are contacted with the digestion and repair reagents simultaneously. In other embodiments the digestion and repair step is carried out in a temporally separate manner at different temperatures. In particular embodiments the plurality of gapped polynucleotide products in digestion are contacted sequentially with the digestion and repair reagents. In some embodiments one or more of the method steps is conducted in manual mode or in an automated mode or a combination thereof. In particular embodiments each of the method steps is carried out in automated mode. In some embodiments the foregoing methods further comprise at least one purification step. In particular embodiments a purification step is carried out only after the second universal amplification step. In other particular embodiments a purification is carried out after the digestion and repair step and an additional purification is carried out after the second universal amplification. In some of the embodiments adaptor-dimer by products resulting from the first amplification are removed from the resulting library, and in some embodiments an enriched population of amplified target nucleic acids contains a reduced amount of adaptor-dimer byproduct. In certain embodiments, adaptor-dimer byproducts are eliminated. In the foregoing methods the plurality of adaptors capable of amplification of one or more target nucleic acid sequences comprises a multiplex of adaptor pairs capable of amplification of at least two different target nucleic acid sequences. In some embodiments, each target specific pair of the plurality of adaptors includes up to 16,777,216 different adaptor combinations comprising different tag sequences. In certain embodiments each generated target specific amplicon sequence includes at least 1 different sequence and up to 10⁷ different sequences. In some embodiments, the foregoing methods further comprise analyzing the sequence of the resulting library of target nucleic acid sequences. Such analyzing comprises sequencing by traditional sequencing reactions, high throughput next generation sequencing, targeted multiplex array sequence detection, or any combination of two or more of the foregoing. In other embodiments, the foregoing methods further comprise determining the abundance of at least one of the target nucleic acid sequences in the sample. Such determining is carried out by high throughput throughput next generation sequencing in certain embodiments. In particular embodiments, such sequencing is carried out in a bidirectional manner, thereby generating sequence reads in both forward and reverse strands for any given amplicon. In some embodiments the foregoing methods comprise digestion reagent selected from any one or a combination of uracil DNA glycosylase (UDG),. apurinic endonuclease (e.g., APE1), RecJf, formamidopyrimidine [fapy]-DNA glycosylase (fpg), Nth endonuclease III, endonuclease VIII, polynucleotide kinase (PNK), Taq DNA polymerase, DNA polymerase I and/or human DNA polymerase beta. In some embodiments, the foregoing methods methods comprise repair reagent selected from any one or a combination of Phusion DNA polymerase, Phusion U DNA polymerase, SuperFi DNA polymerase, Taq DNA polymerase, Human DNA polymerase beta, T4 DNA polymerase and/or T7 DNA polymerase, SuperFiU DNA polymerase, E. *coli* DNA ligase, T3 DNA ligase, T4 DNA ligase, T7 DNA ligase, Taq DNA ligase, and/or 9°N DNA ligase. In particular embodiments the foregoing methods comprise digestion and repair reagent selected from any one or a combination of uracil DNA glycosylase (UDG),apurinic endonuclease (e.g., APE1), Taq DNA polymerase, Phusion U DNA polymerase, SuperFiU DNA polymerase, 7 DNA ligase. In more particular embodiments the foregoing methods comprise digestion and repair reagent selected from any one or a combination of uracil DNA glycosylase (UDG) ,. formamidopyrimidine [fapy]-DNA glycosylase (fpg), Phusion U DNA polymerase, Taq DNA polymerase, SuperFiU DNA polymerase, T4 PNK and T7 DNA ligase. In preferred embodiments, the foregoing methods generate compositions comprising nucleic acid library. In particularly preferred embodiments, generated compositions comprising nucleic acid library are useful for analysis of sequences.. In specific embodiments, use comprises determination of low frequency allele(s) in a sample.

In one embodiment, provided is a method for preparing a library of target nucleic acid sequences comprising contacting a nucleic acid sample with a plurality of adaptors capable of amplification of one or more target nucleic acid sequences in the sample under conditions wherein the target nucleic acid(s) undergo a first amplification, digesting resulting first amplification products to reduce or eliminate resulting primer dimers and prepare partially digested target amplicons, producing gapped, double stranded amplicons, then repairing the partially digested target amplicons, and amplifying the repaired target amplicons in a second amplification using universal primers; wherein each of the plurality of adaptors comprises a universal handle sequence, one or more tag sequences, a target nucleic acid sequence and a cleavable moiety; and wherein at least two and up to one hundred thousand target specific adaptor pairs are included and wherein the target nucleic acid sequence of the adaptor includes at least one cleavable moiety, cleavable moieties are included in the flanking either end of the tag sequence and the universal handle sequence does not include the cleavable moiety. In certain embodiments the melting temperature of each universal sequence is higher than the melting temperature of each target nucleic acid sequence and each tag sequence present. Such methods thereby produce a library of target nucleic acid sequence. In particular embodiments such methods are carried out in a single, addition only workflow reaction, allowing for rapid production of highly multiplexed targeted libraries. In some embodiments, the digestion and repair is carried out in a single step. In particular embodiments the plurality of gapped polynucleotide products in digestion are contacted with the digestion and repair reagents simultaneously. In other embodiments the digestion and repair step is carried out in a temporally separate manner at different temperatures. In particular embodiments the plurality of gapped polynucleotide products in digestion are contacted sequentially with the digestion and repair reagents. In some embodiments one or more of the method steps is conducted in manual mode or in an automated mode or a combination thereof. In particular embodiments each of the method steps is carried out in automated mode. In some embodiments the foregoing methods further comprise at least one purification step. In particular embodiments a purification step is carried out only after the second universal amplification step. In other particular embodiments a purification is carried out after the digestion and repair step and an additional purification is carried out after the second universal amplification. In some of the embodiments adaptor-dimer by products resulting from the first amplification are removed from the resulting library, and in some embodiments an enriched population of amplified target nucleic acids contains a reduced amount of adaptor-dimer byproduct. In certain embodiments, adaptor-dimer byproducts are eliminated. In the foregoing methods the plurality of adaptors capable of amplification of two or more target nucleic acid sequences comprises a multiplex of adaptor pairs capable of amplification of target nucleic acid sequences. In certain embodiments all of the adaptors comprise tag sequences having cleavable groups flanking either end of the tag sequences. In some embodiments, each target specific pair of the plurality of adaptors includes up to 16,777,216 different adaptor combinations comprising different tag sequences. In certain embodiments each generated target specific amplicon sequence includes at least 1 different sequence and up to 10⁷ different sequences. In some embodiments, the foregoing methods further comprise analyzing the sequence of the resulting library of target nucleic acid sequences. Such analyzing comprises sequencing by traditional sequencing reactions, high throughput next generation sequencing, targeted multiplex array sequence detection, or any combination of two or more of the foregoing. In other embodiments, the foregoing methods further comprise determining the abundance of at least one of the target nucleic acid sequences in the sample. Such determining is carried out by high throughput throughput next generation sequencing in certain embodiments. In particular embodiments, such sequencing is carried out in a bidirectional manner, thereby generating sequence reads in both forward and reverse strands for any given amplicon. In some embodiments the foregoing methods comprise digestion reagent selected from any one or a combination of uracil DNA glycosylase (UDG),. apurinic endonuclease (e.g., APE1), RecJf, formamidopyrimidine [fapy]-DNA glycosylase (fpg), Nth endonuclease III, endonuclease VIII, polynucleotide kinase (PNK), Taq DNA polymerase, DNA polymerase I and/or human DNA polymerase beta. In some embodiments, the foregoing methods methods comprise repair reagent selected from any one or a combination of Phusion DNA polymerase, Phusion U DNA polymerase, SuperFi DNA polymerase, Taq DNA polymerase, Human DNA polymerase beta, T4 DNA polymerase and/or T7 DNA polymerase, SuperFiU DNA polymerase, *E*. *coli* DNA ligase, T3 DNA ligase, T4 DNA ligase, T7 DNA ligase, Taq DNA ligase, and/or 9°N DNA ligase. In particular embodiments the foregoing methods comprise digestion and repair reagent selected from any one or a combination of uracil DNA glycosylase (UDG),apurinic endonuclease (e.g., APE1), Taq DNA polymerase, Phusion U DNA polymerase, SuperFiU DNA polymerase, 7 DNA ligase. In more particular embodiments the foregoing methods comprise digestion and repair reagent selected from any one or a combination of uracil DNA glycosylase (UDG) ,. formamidopyrimidine [fapy]-DNA glycosylase (fpg), Phusion U DNA polymerase, Taq DNA polymerase, SuperFiU DNA polymerase, T4 PNK and T7 DNA ligase. In preferred embodiments, the foregoing methods generate compositions comprising nucleic acid library. In particularly preferred embodiments, generated compositions comprising nucleic acid library are useful for analysis of sequences. In specific embodiments, use comprises determination of low frequency allele(s) in a sample.

In one embodiment provided is a composition comprising a plurality of nucleic acid adaptors, wherein each of the plurality of adaptors comprise a 5' universal handle sequence, one or more tag sequences, and a 3' target nucleic acid sequence wherein each adaptor comprises a cleavable moiety, the target nucleic acid sequence of the adaptor includes at least one cleavable moiety, cleavable moieties are included flanking either end of the tag sequence and the universal handle sequence does not include the cleavable moiety, and at least two and up to one hundred thousand target specific adaptor pairs are included. In some embodiments the melting temperature of each adaptor universal sequence is higher than the melting temperature of each target nucleic acid sequence and each tag sequence present in the same adaptor. The provided compositions allow for rapid production of highly multiplexed targeted libraries. In particular embodiments, the composition comprises multiplex of adaptor pairs capable of amplification of at least two different target nucleic acid sequences. In certain embodiments, each target specific pair of the plurality of adaptors includes up to 16,777,216 different adaptor combinations comprising different tag sequences In certain embodiments, compositions each generated target specific amplicon produced by target specific pairs of the plurality of adaptors produces at least 1 different sequence and up to 10⁷ different sequences. The foregoing compositions comprise adaptors wherein they are single stranded or double stranded. Yet additional embodiments provide kits comprising the adaptor compositions of any of the foregoing embodiments. In some embodiments such kits further comprise any one or more of an amplification reagent, a digestion reagent and a repair reagent. In certain embodiments such kits further comprise an amplification reagent, a digestion reagent and a repair reagent.

### EXEMPLIFICATION

### Example 1

Provided methods of the invention comprise streamlined procedures enabling rapid, highly multiplexed PCR. See FIG. 1. The invention optionally allows for the incorporation of one or more unique tag sequences, if so desired. Exemplary methods of the invention comprise the following protocols:

### Example 1A

### Materials and Method

Optional Reverse Transcription (RT) Reaction method (10uL reaction)_may be carried out in samples where RNA and DNA are analyzed:

### Materials

2uL 5x SuperScript™ VILO™ (Thermo Fisher Scientific) mix into a microtube or microwell, ≤ 8uL volume of DNA+RNA sample for ≤ 20ng total amount of DNA+RNA sample (∼1% RNA sample of the total nucleic acid (TNA));
nuclease-free H2O to the above tube/well to make 10uL total reaction volume;

### Method:

42C for 30 min
85C for 1 min
4C hold (indefinitely)

### Amplification:

### Materials

| | |
|---|---|
| ul | dH₂O (to 30ul final) |
| ul | 20 ng genomic DNA sample |
| 48 nM | Panel of Adaptors |
| 15ul | PhusionU multiplex PCR master mix |
| 2.4ul | 2u/ul Phusion U DNA polymerase |

### Amplification:

98C for 2min
3 cycles of the following:
98C for 30s
64C for 2min
62C for 2min
60C for 4min
58C for 2min
72C for 30s
72C for 2min
4C hold (indefinitely).

### Digestion, Fill-in, Ligation:

### Materials

| | |
|---|---|
| 2ul | (5u/ul) UDG, |
| 4ul | (10u/ul) FPG |
| 0.5ul | (10u/ul) T4 PNK |
| 1ul | (3000u/ul) T7 ligase |
| 1ul | (10mM) ATP. |

### Method

Mix the materials above, add to reaction mixture.
Incubate:
30C for 20min
55C for 20min
25C for 10min
98C for 2min
4C hold (indefinitely)

The resulting repaired sample is purified using 35ul Ampure® beads (Beckman Coulter, Inc.) according to the manufacturer instructions.

Amplification:

### Materials

1ul for each PI and A-universal primers, optionally containing barcode sequence (Ion Xpress™ Adapters, Thermo Fisher Scientific)

### Method

Incubate:
98C for 2min

22 cycles of
98C for 15s
64C for 15s
72C for 15s
72C for 5min
4C hold (indefinitely)

The resulting sample is purified using 35ul Ampure® beads (Beckman Coulter, Inc.) according to the manufacturer instructions. Optionally, the purification step is repeated IX to 2X.

### Example 1B

### Materials and Method

Optional Reverse Transcription (RT) Reaction method (10uL reaction)_may be carried out in samples where RNA and DNA are analyzed:

### Materials

2uL 5x SuperScript™ VILO™ (Thermo Fisher Scientific) mix into a microtube or microwell, ≤ 8uL volume of DNA+RNA sample for ≤ 20ng total amount of DNA+RNA sample (∼1% RNA sample of the total nucleic acid (TNA));
nuclease-free H2O to the above tube/well to make 10uL total reaction volume;

### Method:

42C for 30 min
85C for 1 min
4C hold (indefinitely)

### Amplification:

### Materials

| | |
|---|---|
| ul | dH₂O (to 30ul final) |
| ul | 20 ng genomic DNA sample |
| 48 nM | Panel of Adaptors |
| 15ul | PhusionU multiplex PCR master mix |
| 2.4ul | 2u/ul Phusion U DNA polymerase |

### Amplification:

98C for 2min
3 cycles of the following:
98C for 30s
   64C for 2min
   62C for 2min
   60C for 4min
   58C for 2min
   72C for 30s
72C for 2min
4C hold (indefinitely).

### Digestion, Fill-in, Ligation:

### Materials

| | |
|---|---|
| 2ul | (5u/ul) UDG, |
| 4ul | (10u/ul) APE1 |
| 0.5ul | (1u/ul) Taq polymerase |
| 1ul | (3000u/ul) T7 ligase |
| 1ul | (10mM) ATP. |

### Method

Mix the materials above, add to reaction mixture. Incubate:
30C for 20min
55C for 20min
25C for 10min
98C for 2min
4C hold (indefinitely)

### Amplification:

### Materials

1ul for each PI and A-universal primers, optionally containing barcode sequence (Ion Xpress™ Adapters, Thermo Fisher Scientific)

### Method

Incubate:
98C for 2min
22 cycles of
98C for 15s
64C for 15s
72C for 15s
72C for 5min
4C hold (indefinitely)

The resulting sample is purified using 35ul Ampure® beads (Beckman Coulter, Inc.) according to the manufacturer instructions. Optionally, purification step may be repeated IX to 2X.

### Example 1C

### Materials and Method

Optional Reverse Transcription (RT) Reaction method (10uL reaction)_may be carried out in samples where RNA and DNA are analyzed:

### Materials

2uL 5x SuperScript™ VILO™ (Thermo Fisher Scientific) mix into a microtube or microwell, ≤ 8uL volume of DNA+RNA sample for ≤ 20ng total amount of DNA+RNA sample (∼1% RNA sample of the total nucleic acid (TNA));
nuclease-free H2O to the above tube/well to make 10uL total reaction volume;

### Method:

42C for 30 min
85C for 1 min
4C hold (indefinitely)

### Amplification:

### Materials

| | |
|---|---|
| _ul | dH₂O (to 30ul final) |
| _ul | Genomic DNA sample (∼20ng) |
| 6ul | Adaptor Panel 250nM |
| 15ul | PhusionU multiplex PCR master mix (F-562) |
| 3.0ul | 2u/ul SuperFiU DNA Polymerase |

### Amplification

Assemble mixture of materials in reaction in 96-well plate wells, amplify using method:
99C for 2min
3 cycles of the following:
   99C for 30s
   64C for 2min
   62C for 2min
   60C for 4min
   58C for 2min
   72C for 30s
72C for 2min
4C hold (indefinitely)

### Digestion. Fill-in. Ligation:

### Materials

| | |
|---|---|
| O.lul | VIP Oligo 10 uM (P/N 4385451Thermo Fisher Scientific, Inc.) |
| 2ul | (5u/ul) UDG |
| 4ul | (10u/ul) APE1 (NEB, M0282L) |
| 0.5ul | (1u/ul) Taq polymerase (EP0404) |
| 1ul | (3000u/ul) T7 ligase (NEB M0318L) |
| 1ul | (10mM) ATP |

### Method

Mix the above materials, add into reaction mixture
Incubate:
30C for 15min
50C for 15min
55C for 15min
25C for 10min
98C for 2min
4C hold (indefinitely)

### Amplification

### Materials

1ul for each PI and A-Barcode-universal primers optionally containing barcode sequence (Ion Xpress™ Adapters, Thermo Fisher Scientific)

### Method

Add into the reaction wells the above materials, amplify:
99C for 2min
20 cycles:
   99C for 20s
   64C for 20s
   72C for 20s
72C for 5min
4C hold (indefinitely)

The resulting sample is purified using IX Ampure® beads (Beckman Coulter, Inc.) according to the manufacturer instructions. Optionally, purification step may be repeated IX to 2X.

### Example ID

### Materials and Method

Optional Reverse Transcription (RT) Reaction method (10uL reaction)_may be carried out in samples where RNA and DNA are analyzed:

### Materials

2uL 5x SuperScript™ VILO™ (Thermo Fisher Scientific) mix into a microtube or microwell, ≤ 8uL volume of DNA+RNA sample for ≤ 20ng total amount of DNA+RNA sample (∼1% RNA sample of the total nucleic acid (TNA));
nuclease-free H2O to the above tube/well to make 10uL total reaction volume;

### Method:

42C for 30 min
85C for 1 min
4C hold (indefinitely)

### Amplification:

### Materials

| | |
|---|---|
| _x_ul | nuclease free dH₂O (x to 30ul final) |
| _y_ul | Genomic DNA sample (y ∼20ng) or y 10uL of RT reaction for DNA+RNA sample |
| 12.5ul | Adaptor Panel for ∼50nM each primer concentration |
| 7.5ul | Platinum™ SuperFi™ PCR master mix , replacing SuperFi enzyme with 0.96 U/ µL SuperFiU™ DNA Polymerase |
| 3.0ul | 2U/ul SuperFiU™ DNA Polymerase |

optionally, an control may be included in reaction, (e.g, Acrometrix Oncology Hotspot Control (Thermo Fisher Scientific))

### Amplification

Assemble mixture of materials in reaction in 96-well plate wells, seal, vortex and centrifuge plate, amplify using method:
99C for 1 s
   3 cycles of the following:
   99C for 30s
   64C for 2min
   60C for 6min
   72C for 30s
then 72C for 2min
4C hold (indefinitely)

### Digestion, Fill-in, Ligation:

### Materials

| | |
|---|---|
| O.lul | VIP Oligo 0.2 uM (P/N 4385451Thermo Fisher Scientific, Inc.) |
| 2ul | (5u/ul) UDG |
| 4ul | (8U/ul) APE1 (NEB, M0282L) |
| 0.5ul | (0.1U/ul) Taq polymerase (EP0404) |
| 1ul | (6000U/ul) T7 ligase (NEB M0318L) |
| 1ul | (2mM) ATP |
| 0.5ul | mAB2A7 (0.6mg/mL) |
| 0.25ul | mAB5D3 (0.25mg/mL) |

### Method

Mix the above materials, add into reaction mixture, seal plate, vortex and centrifuge
Incubate:
30C for 15min
50C for 15min
55C for 15min
25C for 10min
98C for 2min
4C hold (indefinitely)

### Amplification

### Materials

1ul for each PI and A-Barcode-universal primers optionally barcoded sequence (Ion Xpress™ Adapters, Thermo Fisher Scientific); or 1uL each of 10uM BC1-Ah, and 1uL of 10uM P1-P1h (IonCode™ Barcode Adapters, Thermo Fisher Scientific), for uni-directional library

### Method

Add into the reaction wells the above materials, seal plate, vortex and centrifuge, then amplify:
99C for 15s
5 cycles:
   99C for 15s
   62C for 20s
   72C for 20s
15 cycles:
   99C for 15s
   70C for 40s
72C for 5min
4C hold (indefinitely)

The resulting sample is purified using IX Ampure® beads (Beckman Coulter, Inc.) according to the manufacturer instructions.

Optionally, purification may be repeated IX to 2X.

### Example IE

### Materials and Method

### Amplification:

### Materials

| | |
|---|---|
| x_ul | dH₂O (x to 30ul final) |
| y_ul | Genomic DNA sample (y∼20ng) or y 10uL of RT reaction for DNA+RNA sample |
| 12.5ul | Adaptor Panel for ∼50nM each primer concentration |
| 7.5ul | Platinum™ SuperFi™ PCR master mix , replacing SuperFi enzyme with 0.96 U/ µL SuperFiU™ DNA Polymerase |
| 3.0ul | 2u/ul SuperFiU™ DNA Polymerase |

### Amplification

Assemble mixture of materials in reaction in 96-well plate wells, seal plate, vortex and centrifuge, then amplify using method:
99C for 1 s
   3 cycles of the following:
   99C for 30s
   64C for 2min
   60C for 6min
   72C for 30s
72C for 2min
4C hold (indefinitely)

### Digestion, Fill-in, Ligation:

### Materials

| | |
|---|---|
| O.lul | VIP Oligo 0.2 uM (P/N 4385451Thermo Fisher Scientific, Inc.) |
| 2ul | (5u/ul) UDG |
| 4ul | (8U/ul) APE1 (NEB, M0282L) |
| 0.5ul | (0.1 U/ul) Taq polymerase (EP0404) |
| 1ul | (6000U/ul) T7 ligase (NEB M0318L) |
| 1ul | (2mM) ATP |
| 0.5ul | mAB2A7 (0.6mg/mL) |
| 0.25ul | mAB5D3 (0.25mg/mL) |

### Method

Mix the above materials, add into reaction mixture, seal plate, vortex and centrifuge
Incubate:
30C for 15min
50C for 15min
55C for 15min
25C for 10min
98C for 2min
4C hold (indefinitely)

### Amplification

### Materials

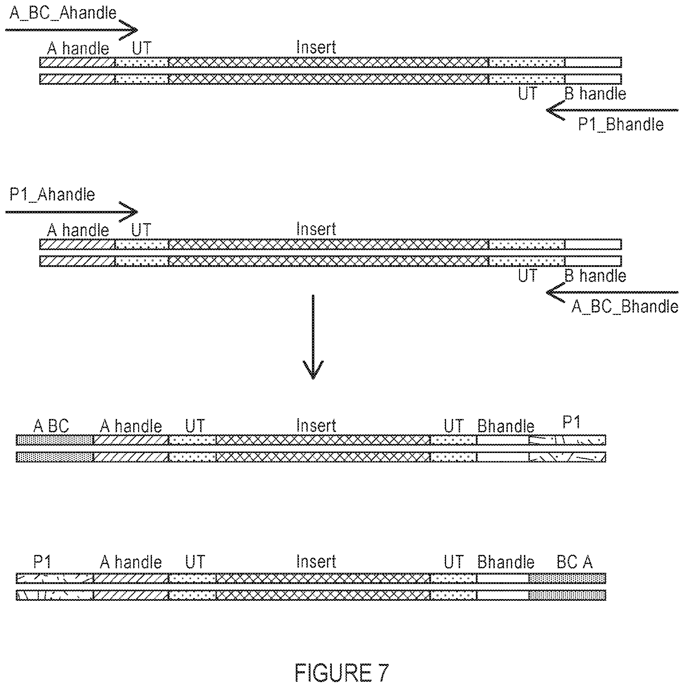

1uL of 10uM BC1-Ah, 1uL of 10uM P1-Uh, 1.5uL of 10uM BC1-Uh, and 1.5uL of 10uM PI-Ah. herein for bi-directional library preparation. BC1-Ah comprises barcode sequence and complementary sequence to universal A handle of forward adapters herein; BC1-Uh comprises barcode sequence and complementary sequence to universal handle of any of reverse adapters B, C, D, or E herein; P1-Uh comprises Ion adapter PI adapter sequence, barcode sequence, and complementary sequence to universal B, C, D, or E handle of any of reverse adapters B, C, D, or E herein; PI-Ah comprises Ion adapter PI adapter sequence, barcode sequence, and complementary sequence to universal handle of A handle of forward adapters herein. See FIG. 7.

### Method

Add into the reaction wells the above materials, seal plate, vortex, centrifuge then amplify:
99C for 15s
5 cycles:
   99C for 15s
   62C for 20s
   72C for 20s
15 cycles:
   99C for 15s
   70C for 40s
72C for 5min
4C hold (indefinitely)

The resulting sample is purified using IX Ampure® beads (Beckman Coulter, Inc.) according to the manufacturer instructions.

Optionally, purification may be repeated IX to 2X.

### Example IF

### Materials and Method

Optional Reverse Transcription (RT) Reaction method (10uL reaction)_may be carried out in samples where RNA and DNA are analyzed:

### Materials

2uL 5x SuperScript™ VILO™ (Thermo Fisher Scientific) mix into a microtube or microwell, ≤ 8uL volume of DNA+RNA sample for ≤ 20ng total amount of DNA+RNA sample (∼1% RNA sample of the total nucleic acid (TNA));
nuclease-free H2O to the above tube/well to make 10uL total reaction volume;

### Method:

42C for 30 min
85C for 1 min
4C hold (indefinitely)

### Amplification:

### Materials

| | |
|---|---|
| x_ul | nuclease free dH₂O (x to 30ul final) |
| _y_ul | Genomic DNA sample (y ∼20ng) or y 10uL of RT reaction for DNA+RNA sample |
| 12.5ul | Adaptor Panel for ∼50nM each primer concentration |
| 7.5ul | Platinum™ SuperFi™ PCR master mix, replacing SuperFi enzyme with 0.96 |
| U/ µL SuperFiU™ | DNA Polymerase |
| 3.0ul | 2U/ul SuperFiU™ DNA Polymerase |

optionally, a control may be included in reaction, (e.g, Acrometrix Oncology Hotspot Control (Thermo Fisher Scientific))

### Amplification

Assemble mixture of materials in reaction in 96-well plate wells, seal, vortex and centrifuge plate, amplify using method:
99C for 1 s
   3 cycles of the following:
   99C for 30s 64C for 2min 60C for 6min 72C for 30s
then 72C for 2min
4C hold (indefinitely)

### Digestion, Fill-in, Ligation:

### Materials

| | |
|---|---|
| 0.1ul | VIP Oligo 0.2 uM (P/N 4385451Thermo Fisher Scientific, Inc.) |
| 2ul | (5u/ul) UDG |
| 4ul | (8U/ul) APE1 (NEB, M0282L) |
| 0.5ul | (0.1U/ul) Taq polymerase (EP0404) |
| 1ul | (6000U/ul) T7 ligase (NEB M0318L) |
| 1ul | (2mM) ATP |
| 0.5ul | mAB2A7 (0.6mg/mL) |
| 0.25ul | mAB5D3 (0.25mg/mL) |

### Method

Mix the above materials, add into reaction mixture, seal plate, vortex and centrifuge
Incubate:
30C for 15min
50C for 15min
55C for 15min
25C for 10min
98C for 2min
4C hold (indefinitely)

### Amplification

### Materials

1ul for each of (1) P5-index-A-handle primer; (2) P5-index-I-handle primer; (3) P7-index-A-handle primer; and (4) P7-index-I-handle primer . See Table F.

### Method

Add into the reaction wells the above materials, seal plate, vortex and centrifuge, then amplify:
99C for 15s
5 cycles:
   99C for 15s
   62C for 20s
   72C for 20s
15 cycles:
   99C for 15s
   70C for 40s
72C for 5min
4C hold (indefinitely)

The resulting sample is purified using IX Ampure® beads (Beckman Coulter, Inc.) according to the manufacturer instructions.

Optionally, purification may be repeated IX to 2X.

### Example 2

The first step of provided methods comprises a few rounds of amplification, for example, three to six cycles of amplification, and in certain instances, three cycles of amplification using forward and reverse adaptors to each gene specific target sequence. Each adaptor contains a 5'universal sequence, and a 3' gene specific target sequence. In some embodiments adaptors optionally comprise a unique tag sequence located between the 5' universal and the 3' gene specific target sequences.

In specific embodiments wherein unique tag sequences are utilized, each gene specific target adaptor pair includes a multitude of different unique tag sequences in each adaptor. For example, each gene specific target adaptor comprises up to 4096 TAGs. Thus, each target specific adaptor pair comprises at least four and up to 16,777,216 possible combinations.

Each of the provided adaptors comprises a cleavable uracil in place of thymine at specific locations in the forward and reverse adaptor sequences. Positions of uracils (Us) are consistent for all forward and reverse adaptors having unique tag sequences, wherein uracils (Us) are present flanking the 5' and 3' ends of the unique tag sequence when present; and Us are present in each of the gene specific target sequence regions, though locations for each gene specific target sequence will inevitably vary. Uracils flanking each unique tag sequence (UT) and in gene-specific sequence regions are designed in conjunction with sequences and calculated Tm of such sequences, to promote fragment dissociation at a temperature lower than melting temperature of the universal handle sequences, which are designed to remain hybridized at a selected temperature. Variations in Us in the flanking sequences of the UT region are possible, however designs keep the melting temperature below that of the universal handle sequences on each of the forward and reverse adaptors. Exemplary adaptor sequence structures comprise:
Forward Adaptor:
Rev Adaptor B
Rev Adaptor C
Rev Adaptor D
Rev Adaptor E
Wherein each N is a base selected from A, C, G, or T and the constant sections of the UT region are used as anchor sequences to ensure correct identification of variable (N) portion. The constant and variable regions of the UT can be significantly modified (e.g., alternative constant sequence, >3Ns per section) as long as the Tm of the UT region remains below that of the universal handle regions. Importantly, cleavable uracils are absent from each forward (e.g., TCTGTACGGTGACAAGGCG (SEQ ID NO:6) and reverse (e.g., CTCTATGGGCAGTCGGTGAT(SEQ ID NO:7) universal handle sequence.

Enzymes used for amplification include (but are not limited to): Phusion U DNA polymerase; SuperFi U DNA polymerase; Taq DNA polymerase; Veraseq Ultra DNA polymerase. SuperFi U DNA Polymerase is a modified version of high fidelity SuperFi DNA Polymerase, available from Thermo Fisher Scientific. SuperFiU DNA comprises a modification in the uracilbinding pocket (e.g., AA 36) and a family B polymerase catalytic domain (e.g., AA 762). SuperFiU is described in US Provisional patent application no 62/524,730 filed June 26, 2017, and International Patent application no. PCT/EP2018/066896, filed June 25, 2018and published as WO/2019/002178. Polymerase enzymes may be limited in their ability to utilize uracil and/or any alternative cleavable residues (e.g., inosine, etc.) included into adaptor sequences. In certain embodiments, it may also be advantageous to use a mixture of polymerases to reduce enzyme specific PCR errors.

The second step of methods involves partial digestion of resulting amplicons, as well as any unused uracil-containing adaptors. For example, where uracil is incorporated as a cleavable site, digestion and repair includes enzymatic cleavage of the uridine monophosphate from resulting primers, primer dimers and amplicons, and melting DNA fragments, then repairing gapped amplicons by polymerase fill-in and ligation. This step reduces and potentially eliminates primerdimer products that occur in multiplex PCR. In some instances, digestion and repair are carried out in a single step. In certain instances, it may be desirable to separate digestion and repair- steps temporally. For example, thermolabile polymerase inhibitors may be utilized in conjunction with methods, such that digestion occurs at lower temperatures (25-40°C), then repair is activated by increasing temperature enough to disrupt a polymerase-inhibitor interaction (e.g., polymerase-Ab), though not high enough to melt the universal handle sequences.

Uracil-DNA Glycosylase (UDG) enzyme can be used to remove uracils, leaving abasic sites which can be acted upon by several enzymes or enzyme combinations including (but not limited to) : APE 1-Apurinic/apyrimidinic endonuclease; FPG-Formamidopyrimidine [fapy]-DNA glycosylase; Nth-Endonuclease III; Endo VIII-Endonuclease VIII; PNK-Polynucleotide Kinase; Taq- Thermus aquaticus DNA polymerase; DNA pol I-DNA polymerase I; Pol beta-Human DNA polymerase beta. In a particular implementation, the method uses Human apurinic/apyrimidinic endonuclease, APE1. APE1 activity leaves a 3'-OH and a 5'deoxyribose-phosphate (5'-dRP). Removal of the 5'-dRP can be accomplished by a number of enzymes including recJ, Polymerase beta, Taq, DNA pol I, or any DNA polymerase with 5'-3' exonuclease activity. Removal of the 5'-dRP by any of these enzymes creates a ligatable 5'-phosphate end. In another implementations, UDG activity removes the Uracil and leaves and abasic site which is removed by FPG, leaving a 3' and 5'-phosphate. The 3'-phosphate is then removed by T4 PNK, leaving a polymerase extendable 3'-OH. The 5'-deoxyribose phosphate can then be removed by Polymerase beta, fpg, Nth, Endo VIII, Taq, DNA pol I, or any other DNA polymerase with 5'-3' exonuclease activity. In a particular implementation Taq DNA polymerase is utilized.

Repair fill-in process can be accomplished by almost any polymerase, possibly the amplification polymerase used for amplification in step 1 or by any polymerase added in step 2 including (but not limited to) : Phusion DNA polymerase; Phusion U DNA polymerase; SuperFi DNA polymerase; SuperFi U DNA polymerase; TAQ; Pol beta; T4 DNA polymerase; and T7 DNA polymerase. Ligation repair of amplicons can be performed by many ligases including (but not limited to) : T4 DNA ligase; T7 DNA ligase; Taq DNA ligase. In a particular implementation of the methods, Taq DNA polymerase is utilized and ligation repaired in accomplished by T7 DNA ligase.

A last step of library preparation involves amplification of the repaired amplicons by standard PCR protocols using universal primers that contain sequences complementary to the universal handle sequences on the 5' and 3' ends of prepared amplicons. For example, an A-universal primer, and a PI universal primer, each part of the Ion Express Adaptor Kit (Thermo Fisher Scientific, Inc.) may optionally contain a sample specific barcode. The last library amplification step may be performed by many polymerases including, but not limited to : Phusion DNA polymerase; Phusion U DNA polymerase; SuperFi DNA polymerase; SuperFi U DNA polymerase; Taq DNA polymerase; Veraseq Ultra DNA polymerase.

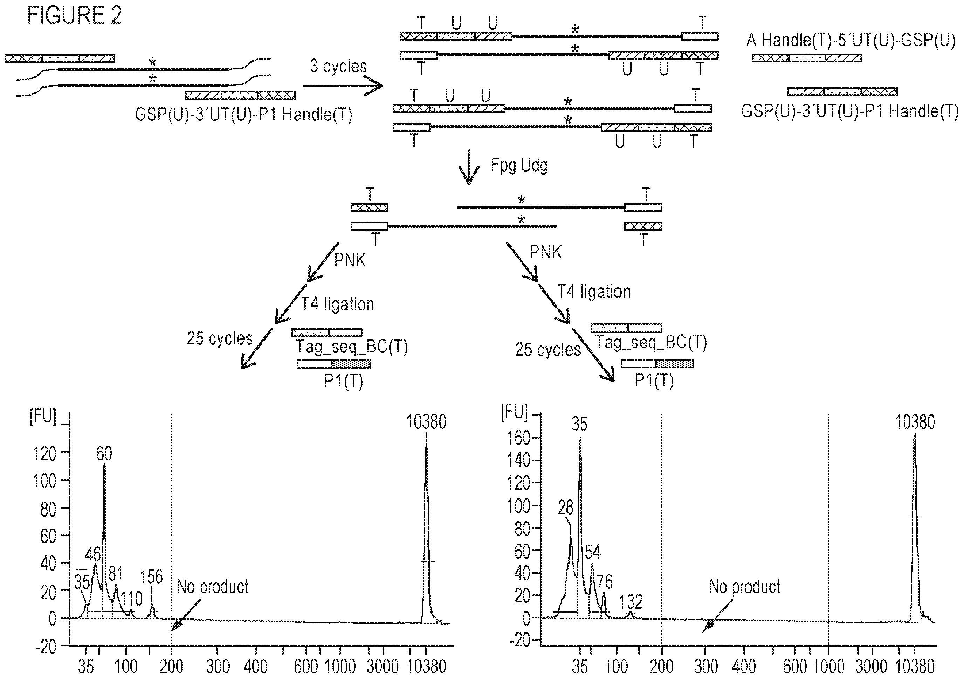

2A, In one specific implementation, adaptors were designed using the composition design approach provided herein, including universal handle-unique tag-gene specific target sequence described in Example 2 above, and targeted to genes using the ONCOMINE™ Focus Research Panel (Thermo Fisher Scientific, Inc.) target sequences and ION AMPLISEQ™ Designer (Thermo Fisher Scientific, Inc). Forward and reverse adaptors described above were utilized comprising
Forward Adaptor: Rev Adaptor B With target sequences specific to targets as in Table A, and adaptors each comprise 4096 unique tag sequences for each gene specific target sequence, resulting in an estimate of 16,777,216 different unique tag combinations for each gene specific target sequence pair. Preparation of library was carried out according to the method described above for Example 1A. Formamidopyrimidine [fapy]-DNA glycosylase (FPG) /UDG enzyme is utilized for digestion, which is expected to create abasic sites at all uracil positions, FPG is expected to cleave on the 5' and 3' side of the abasic site (leaving a 3'-phosphate and a 5' phosphate) and removal of the 3'phosphate (by T4 PNK for example) should produce an extendable 3'-OH and a ligatable 5'-phosphate. However, as shown by the BioAnalyzer trace (See FIGURE 2), this process consistently failed to generate recoverable product. The process can be rescued however by the addition of an additional purification step post-repair. The purification process can be anything inactivates and removes the repair enzymes prior to the next amplification step. Similar results were obtained if endoVIII was utilized.

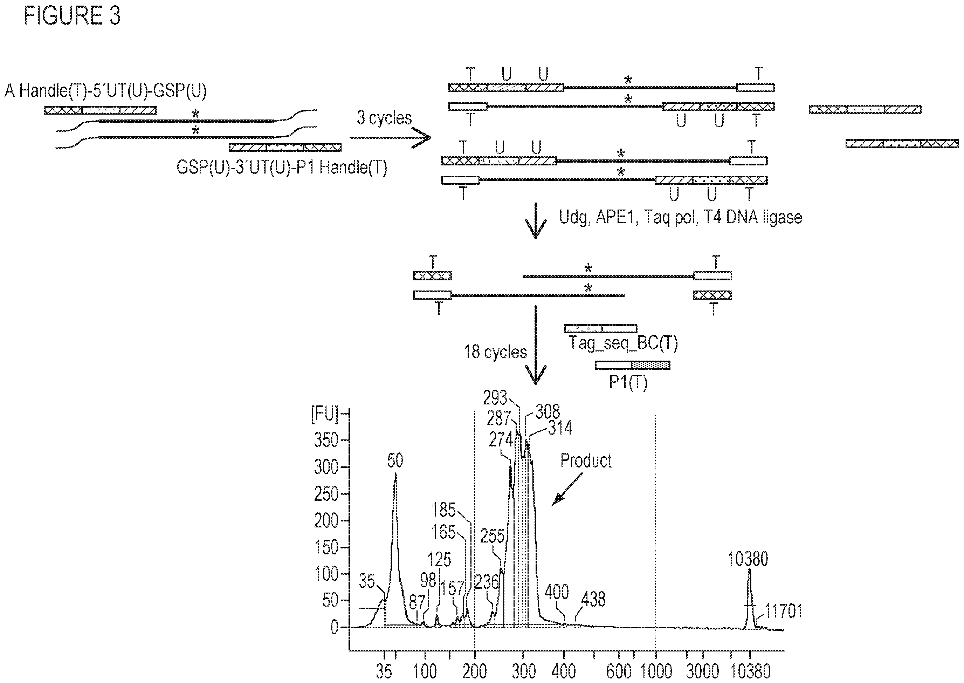

2B. In another specific implementation, adaptors were prepared as described in section 2A for targets of the ONCOMINE™ Focus Assay. See Table B. Forward and reverse adaptors described above were utilized comprising
Forward Adaptor: Reverse Adaptor was any of Rev Adaptor B, Rev Adaptor C, Rev Adaptor D, Rev Adaptor E: Rev Adaptor B Rev Adaptor C Rev Adaptor D Rev Adaptor E With target sequences specific to targets as in Table B, and adaptors each comprise 4096 unique tag sequences for each gene specific target sequence, resulting in an estimate of 16,777,216 different unique tag combinations for each gene specific target sequence pair. Preparation of library was carried out according to the method described above for 1C. See FIGURE 3, Table 1. Similar successful sequencing results were generated with each of the reverse adaptor pairings.

### Example 3

Prepared libraries are sequenced, and analyzed. Sequencing can be carried out by a variety of known methods, including, but not limited to sequencing by synthesis, sequencing by ligation, and/or sequencing by hybridization. Sequencing has been carried out in the examples herein using the Ion Torrent platform (Thermo Fisher Scientific, Inc.), however, libraries can be prepared and adapted for analysis, e.g., sequencing, using any other platforms, e.g., Illumina, PacBio, etc. Result may be analyzed using a number of metrics to assess performance, for example:
∘ # of families (with ng input DNA captured) The median # of families is a measure of the number of families that maps to an individual target. In this case, each unique molecular tag is a family.
∘ Uniformity is a measure of the percentage of target bases covered by at least 0.2x the average read depth. This metric is used to ensure that the technology does not selectively under-amplify certain targets.
∘ Positives/Negatives: When a control sample with known mutations is utilized is analyzed (e.g., Acrometrix Oncology Hotspot Control DNA, Thermo Fisher Scientific, Inc.), the number of True Positives can be tracked.
   ▪ True Positives: The number of True Positives informs on the number of mutations that were present and correctly identified.
   ▪ False positives(FP): (Hot spot and Whole Target) The number of False Positives informs on the number of mutations that are determined to be present, but known not to be in the sample.
   ▪ False negatives (FN) (if acrometrix spike-in is used) The number of False Negatives informs on the number of mutations that were present but not identified.
∘ On/Off Target is the percentage of mapped reads that were aligned/not aligned over a target region. This metric is used to ensure the technology amplifies predominantly the targets to which the panel was designed.
∘ Low quality is tracked to ensure the data is worth analyzing. This metric is a general system metric and isn't directly related to this technology.

### Example 4

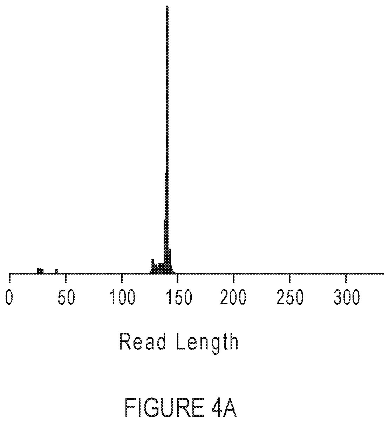

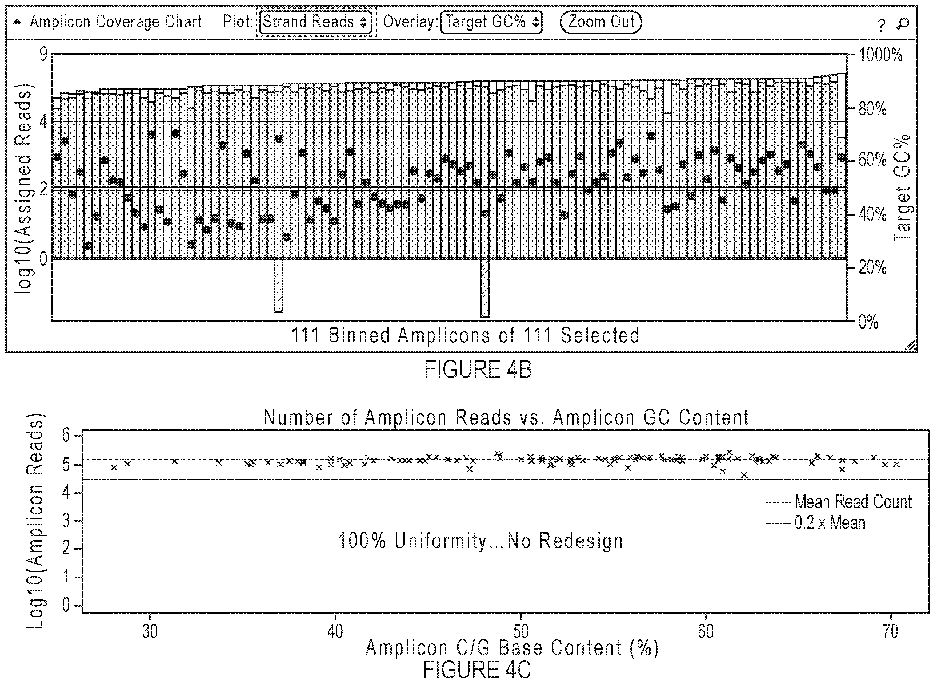

One benefit of the instant invention is the ability to use Ampliseq.com designer in conjunction with the provided methodology. Adaptors were designed using the composition design approach provided herein, including universal handle-unique tag-gene specific target sequence described in Example 2 above, and targeted to genes using the ONCOMINE™ Focus Research Panel (Thermo Fisher Scientific, Inc.) target sequences and ION AMPLISEQ™ Designer (Thermo Fisher Scientific, Inc). Forward and reverse adaptors described above were utilized comprising Forward Adaptor: Rev Adaptor B With target sequences specific to targets as in Table A, and adaptors each comprise 4096 unique tag sequences for each gene specific target sequence, resulting in an estimate of 16,777,216 different unique tag combinations for each gene specific target sequence pair. Library was prepared using 20ng of genomic DNA and ∼1% Acrometrix Oncomine™ Hotspot Control (AOHC) DNA (Thermo Fisher Scientific, Inc.), according to the protocol described above in Example 1C. Prepared library was sequenced using Ion 520/530 Templating/Sequencing kits and instrumentation (Thermo Fisher Scientific, Inc.). Performance with the panel (eg., yield, uniformity) indicates the technology is able to effectively make use of the designer pipeline. See Figure 4A-4C.

Results using the AOHC DNA (shown in Table 1) demonstrate that, using this protocol, we effectively identify most of the True Positives (71 or 75) present in the AOHC and importantly did not generate any False positives.

**TABLE 1**

| | Oncology Panel (Ex 4) | BRCA Panel (Ex 5) | Oncology HotSpot Panel (Ex 3) | Oncology HotSpot Bidirectional (ex 6) |
|---|---|---|---|---|
| True Positives | 75 | NA | NA | NA |
| TP in SNP, INDEL | 71;4 | NA | NA | NA |
| False Negatives | 3 | NA | NA | NA |
| False Positives | 0 | 0 | 0 | 0 |
| Uniformity | 98.60% | 100% | 100% | 100% |
| Low Quality | 15% | 28% | 31% | 26% |
| On Target | 98% | 95% | 96% | 95% |
| # of Families | 4398 | 5208 | 8755 | 6391 |

### Example 5

Adaptors were designed according to the composition design approach provided herein, including universal handle-unique tag-gene specific target sequence described in Example 2 above, and targeted to genes using the BRCA Research Panel (Thermo FisherScientific, Inc.) target sequences and ION AMPLISEQ™ Designer (Thermo Fisher Scientific, Inc). Forward and reverse adaptors described above were utilized comprising
Forward Adaptor: Rev Adaptor B

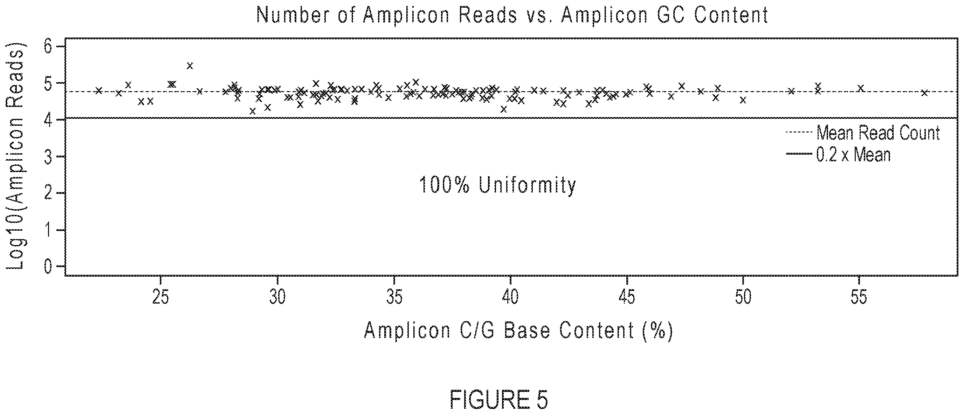

With target sequences specific to targets as in Table C, and adaptors each comprise 4096 unique tag sequences for each gene specific target sequence, resulting in an estimate of 16,777,216 different unique tag combinations for each gene specific target sequence pair. Library was prepared using 20ng genomic DNA according to the protocol described above in Example 1C Prepared library was sequenced using Ion 520/530 Templating/Sequencing kits and instrumentation (Thermo Fisher Scientific, Inc.).. Similar to Example 5, performance (e.g., yield, uniformity) with the panel indicates the technology is able to use the designer pipeline. See Figure 5 and Table 1.

### Example 6

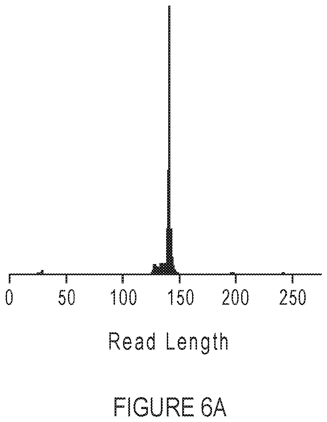

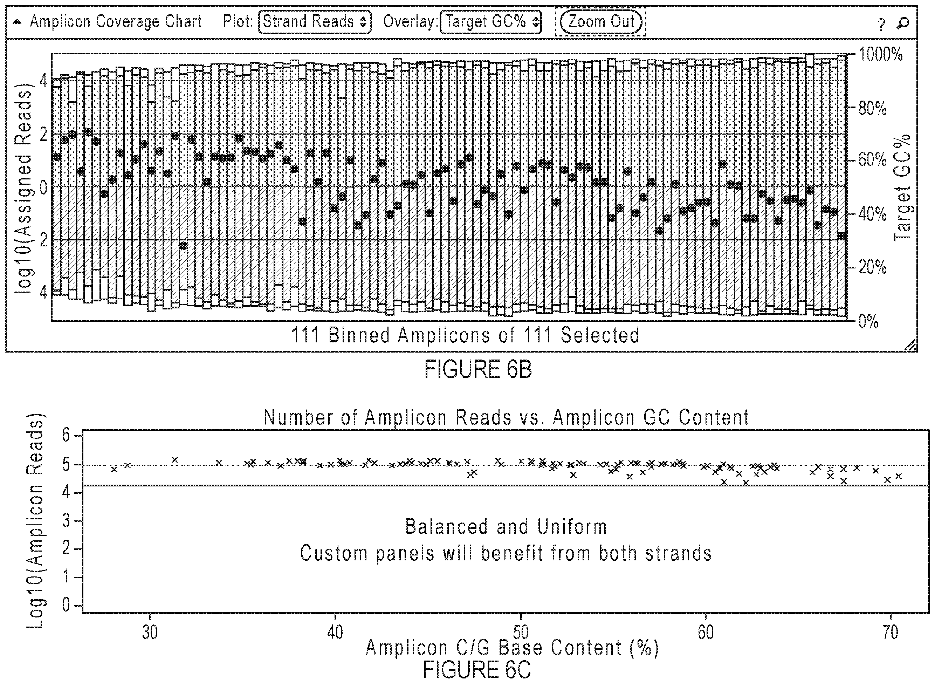

Primers were designed using the composition design approach provided herein and targeted to oncology genes using those of the panel target sequences as described above in Example 4, except that the library amplification step utilized two primer pairs (to put the two universal sequences on each end of amplicons, e.g., an A-universal handle and a P1-universal handle on each end) to enable bi-directional sequencing. Prepared library was sequenced using Ion 520/530 Templating/Sequencing kits and instrumentation (Thermo Fisher Scientific, Inc.). See Figure 7. Performance (e.g., yield, uniformity) with the instant panel indicates the technology is able to use the designer pipeline and effectively generate sequencing data for both strands of DNA. See Figure 6A-6C and Table 1.

### Example 7

Primers were designed using the composition design approach provided herein and targeted to a wide variety of oncology target sequences. Forward and reverse adaptors described above were utilized comprising
Forward Adaptor: Rev Adaptor C

**TABLE 2A: Family Generation, Coverage, and Uniformity**

| Sample | Input | AmpliSeq HD | | | | |
|---|---|---|---|---|---|---|
| | | Median Read Counts per Target | Uniformity (U50) | Median # Families Size>=3 | Molecular Conversion | Median # Families Size>=3 |
| cfDNA 2016B | 20 ng | 61,939 | 95.9% | 5794 | 48% | 5794 |
| | | 63,679 | 95.9% | 5879 | 49% | 5879 |
| cfDNA 416G | 20 ng | 79,004 | 98.6% | 7676 | 64% | 7676 |
| | | 61,694 | 98.6% | 7322 | 61% | 7322 |
| 0.5% fMM | 6000 copies | 61,458 | 98.6% | 5466 | 46% | 5466 |
| | | 62,019 | 98.6% | 5685 | 47% | 5685 |
| 0.1% fMM | 6000 copies | 70,397 | 98.6% | 6278 | 52% | 6278 |
| | | 60,879 | 98.6% | 5946 | 50% | 5946 |
| gDNA | 292 copies | 22,650 | 97.3% | 340 | 57% | 340 |
| | | 79,746 | 98.6% | 354 | 59% | 354 |

**TABLE 2B: Sensitivity, Specificity, and FPs/lib, Hot Spots Only**

| Sample | Input | AmpliSeq HD | | |
|---|---|---|---|---|
| | | Sensitivity (%) | Specificity (%) | FP |
| cfDNA 2016B | 20 ng | | 100.00 | 0 |
| | | | 99.70 | 1 |
| cfDNA 416G | 20 ng | | 100.00 | 0 |
| | | | 100.00 | 0 |
| 0.5% allelic Frequency | 6000 copies | 100.0 | 100.00 | 0 |
| | | 100.0 | 100.00 | 0 |
| 0.1% allelic Frequency | 6000 copies | 85.14 | 100.00 | 0 |
| | | 94.60 | 100.00 | 0 |
| gDNA | 292 copies | | 100.00 | 0 |
| | | | 100.00 | 0 |

With target sequences specific to targets as in Table D and adaptors each comprise 4096 unique tag sequences for each gene specific target sequence, resulting in an estimate of 16,777,216 different unique tag combinations for each gene specific target sequence pair. Samples containing 19.8ng of cell free DNA and 0.2 ng of total RNA were processed as described in example ID, starting with the optional reverse transcriptase step. Total RNA for some samples listed contained 5 spiked in fusion constructs. See Table D. Prepared library was sequenced using Ion 520/530 Templating/Sequencing kits and instrumentation (Thermo Fisher Scientific, Inc.). Performance (e.g., yield, uniformity, molecular conversion, sensitivity) with the instant panel indicates the technology can efficiently convert input DNA into library and detect mutations present at frequencies as low as 0.1% to 0.5% . See Table 2A-2B. Additionally, results confirm the technology can efficiently convert input DNA and cDNA into library and detect fusions present at frequencies of∼1%. See Table 3A-3B.

**TABLE 3A Fusions**

| | | |
|---|---|---|
| LRIG3-ROS1 | EZR-ROS1 | KLC1-ALK |
| CCDC6-RET | GOPC-ROS1 | SDC4-ROS1 |
| CD74-ROS1 | HIP1-ALK | SLC34A2-ROS1 |
| CUX1-RET | KIF5B-ALK | TPM3-ROS1 |
| EML4-ALK | KIF5B-RET | TPR-ALK |

**TABLE 3B: Family Generation, Coverage, and Uniformity (No Activation)**

| **Sample** | **Input** | **FP** | **U50** | **Conversion** |
|---|---|---|---|---|
| cfDNA 5022 | 10 ng | 0 (343) | 98.5 | 44% |
| | | 0 (323) | | |
| cfDNA 5022 +total RNA | 10 ng | 0 (343) | 99.25 | 51% |
| | | 2 (323) | | |
| cfDNA 5022 +Trifusion | 10 ng | 0 (343) | 98.5 | 50% |
| | | 1 (323) | | |
| gDNA | 10 ng | 0 (343) | 93.98 | 45% |
| | | 2(323) | | |
| gDNA +total RNA | 10 ng | 0 (343) | 93.98 | 54% |
| | | 0 (323) | | |
| gDNA +Trifusion | 10 ng | 1 (343) | 95.49 | 53% |
| | | 1 (323) | | |

### Example 8

Primers were designed using the composition design approach provided herein and targeted to genes using those of short tandem repeats (STRs), which are useful for high resolution genotyping and analysis of complex mixtures. Forward and reverse adaptors described above were utilized comprising
Forward Adaptor: Rev Adaptor E

With target sequences specific to targets as in Table E and adaptors each comprise 4096 unique tag sequences for each gene specific target sequence, resulting in an estimate of 16,777,216 different unique tag combinations for each gene specific target sequence pair. Samples containing 1 to 10 ng of genomic DNA were processed as described in example ID without the optional reverse transcriptase step. Prepared library was sequenced using Ion 520/530 Templating/Sequencing kits and instrumentation (Thermo Fisher Scientific, Inc.). Performance (e.g., yield, uniformity) with the instant panel indicates that even challenging STR targets (which are often shortened by 1 or more repeats during amplification) can be efficiently converted into a library. Results were consistent across titration levels of input DNA. See Table 4. When results were compared to standard operating procedure according to manufacturer instructions using Torrent Suite Molecular Diagnostics plugin to evaluate the same targets, results generated using compositions and methods provided herein yielded more consistent signal over each of the repeat regions, with less stutter (data not shown).

**TABLE 4:**

| Barcode Name | Input DNA | Median Read Counts per Target | Median # Families Size>=3 | Half-Double Uniformity (Families Size>=3) | 80% Uniformity (Families Size>=3) |
|---|---|---|---|---|---|
| BC_0102 | 1ng | 37,727 | 257 | 77.78% | 63.89% |
| BC_0105 | 2ng | 35,056 | 412 | 83.33% | 63.89% |
| BC_0108 | 5ng | 32,478 | 1021 | 80.56% | 69.44% |
| BC_0120 | 10ng | 30,915 | 1646 | 86.11% | 63.89% |

### Example 9

Primers were designed using the composition design approach provided herein and targeted to oncology genes target sequences as described above in Example 6, where two primer pairs were utilized in library amplification (to put the two universal sequences on each end of amplicons, e.g., an A-universal handle and a P1-universal handle on each end) to enable bi-directional sequencing. Library preparation was carried out on samples containing spiked in AOHC control as described according to methods of Example IE above without optional RT step. See Figure 7. Prepared library was sequenced using Ion 520/530 Templating/Sequencing kits and instrumentation (Thermo Fisher Scientific, Inc.), then analyzed separately for unidirectional sequence results as well as results analyzed from bidirectional sequencing. Performance (e.g., yield, uniformity, sensitivity) with the instant panel indicates the technology is able to use the designer pipeline and effectively generate sequencing data for both strands of DNA, and bidirectional sequence analysis results in reduction of indel False Positives measured. See Table 5.

**TABLE 5**

| | **Bidirectional, Analyzed Unidirectional** | **Bidirectional, Analyzed Bidirectional** |
|---|---|---|
| True Positives | 67 | 67 |
| Sensitivity | 91.8 | 91.8 |
| TP in SNP, INDEL | 65;2 | 65;2 |
| False Negatives | 6 | 6 |
| False Positives in SNP, INDEL | 1:2 | 1:0 |

### Example 10

For each of the Ion barcode adaptors, a single barcode is included in an A adapter. Addition of a second set of barcodes on the PI adapter can effectively reduce the level of contamination artifacts in results by filtering out identified contamination reads. Primers were designed using the composition design approach provided herein and targeted to a wide variety of oncology target sequences. Samples containing 20ng of genomic DNA were processed similarly to those described in Example 7 above and using the method of example ID, however, additionally barcoded PI adapters were also utilized, wherein a barcode 12mer sequence was inserted into the PI adapter sequence of the reverse adapator. Sample containing genomic DNA for library preparation was processed with barcode 8 in both A and PI adapters. Additional samples were also processed with barcodes 1,2 ,3, 4, 5, 6, 7 and 9 (each in both PI and A barcoded adapters), but without genomic DNA. Performance (e.g., yield, uniformity, Conversion) with the instant panel indicates that additional barcodes can effectively identify contamination. See Table 6.

**TABLE 6:**

| **Reverse Barcode** | **Reads Detected** | **% Total** |
|---|---|---|
| bc1 | 332 | 0.001 % |
| bc2 | 54 | 0.000% |
| bc3 | 261 | 0.001 % |
| bc4 | 481 | 0.001% |
| bc5 | 9,908 | 0.019% |
| bc6 | 8,532 | 0.016% |
| bc7 | 2,656 | 0.005% |
| bc8 | 52,089,480 | 99.941% |
| bc9 | 1,403 | 0.003% |
| bc10 | 7,131 | 0.014% |

### Example 11

In another specific implementation, adaptors were prepared as described in example 2A for targets of the ONCOMINE™ Focus Assay, as in Table B, as well as described in example 6 with target sequences specific to targets as in Table D and adaptors each comprise 4096 unique tag sequences for each gene specific target sequence, resulting in an estimate of 16,777,216 different unique tag combinations for each gene specific target sequence pair.. Forward and reverse adaptors utilized comprising
*Forward Adaptor:* *Rev Adaptor I:*

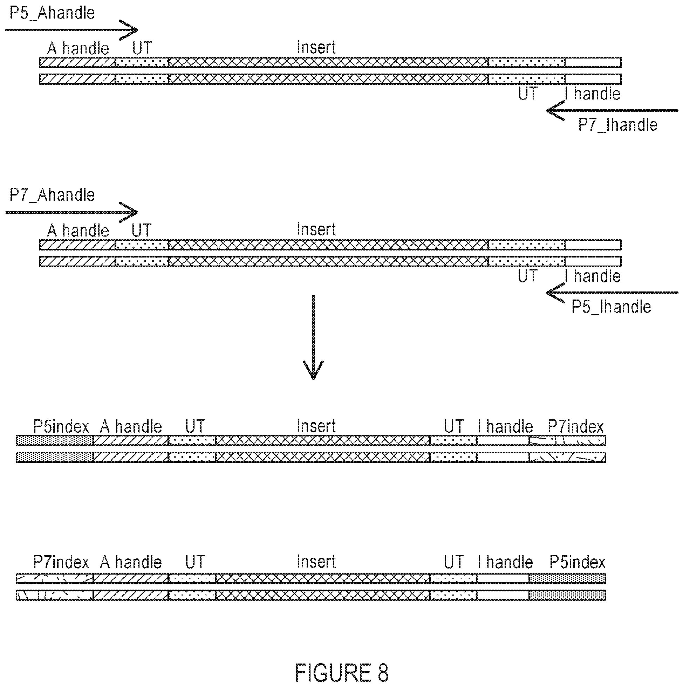

Preparation of library was carried out according to the method described above for IF. See also FIGURE 8. The workflow has been adapted to use amplification primers to enable libraries to carry out sequencing runs on the Illumina platform. The design (shown schematically in Figure 8) contains: (1) P5 grafting primer region; (2) P5 index(A-H) region; (3) P5 sequencing/index read primer region; (4) A-handle region; (5) UT region; (6) gene specific insert; (7) UT region; (8) I-handle region; (9) P7 sequencing/index read primer region; (10) P7 index (1-12) region; and (11) P7 grafting primer region. 3 libraries were made with an oncology panel comprising targets of Table D having idex5-01-idex7-5, idex5-02-index7-6 and idex5-7-idex7-7 respectively. 2 libraries were made with Focus panel comprising targets of Table B having idex5-01-idex7-5, and idex5-7-idex7-7 respectively. See Table F. All libraries are made with 19.6ng of g24385 with 0.4ng spike-in AOHC so we could detect 0.1% allele frequency.

**TABLE F**

| **Name** | **Sequence (5' to 3')** | **SEQ ID** |
|---|---|---|
| 5-01-Ah | | 1706 |
| 5-02-Ah | | 1707 |
| 5-07-Ah | | 1708 |
| 5-08-Ah | | 1709 |
| 5-09-Ah | | 1710 |
| 5-010-Ah | | 1711 |
| 5-013-Ah | | 1712 |
| 5-014-Ah | | 1713 |
| 01-lh | | 1714 |
| 02-lh | | 1715 |
| 5-07-lh | | 1716 |
| 5-08-lh | | 1717 |
| 5-09-lh | | 1718 |
| 5-010-lh | | 1719 |
| 5-013-lh | | 1720 |
| 5-014-lh | | 1721 |
| 7-1-Ah | | 1722 |
| 7-2-Ah | | 1723 |
| 7-3-Ah | | 1724 |
| 7-4-Ah | | 1725 |
| 7-5-Ah | | 1726 |
| 7-6-Ah | | 1727 |
| 7-7-Ah | | 1728 |
| 7-8-Ah | | 1729 |
| 7-9-Ah | | 1730 |
| 7-10-Ah | | 1731 |
| 7-1-Ah | | 1732 |
| 7-12-Ah | | 1733 |
| 7-1-lh | | 1734 |
| 7-2-lh | | 1735 |
| 7-3-lh | | 1736 |
| 7-4-lh | | 1737 |
| 7-5-lh | | 1738 |
| 7-6-lh | | 1739 |
| 7-7-lh | | 1740 |
| 7-8-lh | | 1741 |
| 7-9-lh | | 1742 |
| 7-10-lh | | 1743 |
| 7-11-lh | | 1744 |
| 7-12-lh | | 1745 |

To mimic low level of mutant variants (0.1%) presence in DNA samples, we used purified genomic DNA and spiked in small quantity of AcroMetrix Oncology Hotpot Control plasmid. These samples are used as our control samples for the purpose of demonstrating the library preparation method and assessing the sensitivity and specificity for low levels mutant variants detection by this assay method. Bioanalyzer results matched library structure designs, and yield and purity of libraries were on par with those prepared on other methods described above. Similar successful sequencing results were generated with each of the adaptor pairings.

A MiSeq sequencing run successfully generated clusters, and produced sequencing and indexing reads. Sequencing results of the panel run on the Illumina MiSeq indicate similar performance as compared to the standard AmpliSeq HD version run on the Ion S5 using a 540 chip. See Table 7.

**TABLE 7**

| | **MiSeq** | **S5 540** |
|---|---|---|
| Raw Read Accuracy (%) | 99.31 | 99.27 |
| Mapped Reads | 12,994,280 | 17,855,575 |
| Mean Depth | 46,674 | 62,429 |
| On-Target (%) | 98.91 | 98.64 |
| coverageAnalysis Uniformity (%) | 97.86 | 97.98 |
| Half-Double Uniformity (%) | 86.62 | 83.64 |
| 0.1% MegaMix TP | 140 | 138 |
| 0.1% MegaMix FN | 11 | 13 |
| 0.1% MegaMix FP | 58 | 38 |

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

**TABLE A**

| **Primer Name** | **SEQ ID** | **Primer Sequence (target of primer A)** | **Primer Name** | **SEQ ID** | **Primer Sequence (target seq of primer B)** |
|---|---|---|---|---|---|
| F1 | 8 | GCTCCCAGGCACUTGATGAUAC | R1 | 104 | ACAGAAUCACAUGCCACACAGT |
| F2 | 9 | TGTTGCCATTUCAGGGTTTCUGA | R2 | 105 | TTCCTTCUAAAAGGCCATGAAGATCUG |
| F3 | 10 | ACCAAUGCGAGGAAGAAAAACAAUC | R3 | 106 | TCTGAAGAACAUGTGUGAGCACA |
| F4 | 11 | TCTTCAGAAU CTTGTTGGCUGCAT | R4 | 107 | CCCAACCCACAUTTCCTTTATAGATGTUT |
| F5 | 12 | AGAGTTGCAUCCTTCCCTTCUCT | R5 | 108 | TGTTCCAACAGGAUCTGUCCAAAA |
| F6 | 13 | CTGGCAUGACGCAGTTTCTUC | R6 | 109 | CCAGTGTCTGUCCTTGCCTTUC |
| F7 | 14 | GATCAAAGAGACGAAGUCTCTUGCA | R7 | 110 | CUATGACAAGAAAAUGGACACCAACAAG |
| F8 | 15 | CTTGCCUAGACAGCACCGUAAT | R8 | 111 | AGGAGGAUAAAGACCTGGUCCAT |
| F9 | 16 | TGGTTTCTGGUGGGACCATTAUG | R9 | 112 | GTCCTCUGGATCTCTTCAUGCA |
| F10 | 17 | TTGAAAGAGAACACACUTACTCUCCAC | R10 | 113 | CTGAGACATTCCUATGTCCTGCUC |
| F11 | 18 | CTCTACCAGAGUTAATCAACTGAUGCA | R11 | 114 | TUGAUACAAAACAAGCCCACGAACT |
| F12 | 19 | TATCAACTGTCCUTGTTGGCAAAUCA | R12 | 115 | CCAGCCTAATCTUGTTTTTCTTATGTTCUG |
| F13 | 20 | GCAAATGACTU GCTATTATTGAU GGCA | R13 | 116 | GTTATAGATGGUGAAACCTGTTTGTUGG |
| F14 | 21 | GTGGGATCATAUTCATCTACAAAGUGGT | R14 | 117 | GATTACTGGTTUCCAACAGGTTCTUG |
| F15 | 22 | GGAGGTCAUGGCATCGAGUT | R15 | 118 | TATGGTCTUGGACATCCAGGAUCT |
| F16 | 23 | GTGAGGCAGUCTTTACUCACCT | R16 | 119 | TAGGAAATGCAUTTCCTTTCTUCCCA |
| F17 | 24 | GGGAAATGUGAGCCCTUGAGAT | R17 | 120 | CCTGTGGCUGTCAGTATTUGGA |
| F18 | 25 | ACTCTTGCUCCTTCCATCCTUG | R18 | 121 | GTTCATCCUGCTGGAGCUCAT |
| F19 | 26 | CCCAAUGCAGCGAACAATGTUC | R19 | 122 | GTAGCTGCTGAAAAUGTAACTTTGTAUCC |
| F20 | 27 | GATCAGGGCUTCCAUGAGGAAA | R20 | 123 | ACTCTGTAGGCUGCAGTTCUCA |
| F21 | 28 | CTCAAGAGUGAGCCACTTCTUACC | R21 | 124 | CTCCTCTTG UCTTCTCCTTUGCA |
| F22 | 29 | AGAATAAAACACAUACAAGTTGGAAATTTCUGG | R22 | 125 | CTTGTGAGTGGAUGGGTAAAACCUAT |
| F23 | 30 | ACCUGAGCCAAGGACTTTUACC | R23 | 126 | CGGACTGAAAGUATAACCTTCTTCTTUCC |
| F24 | 31 | TGTCAATTAGCUGGAACATCUGAAACT | R24 | 127 | GCATGTGAACAUTCTGCTTTTCAUGG |
| F25 | 32 | GTGCCCTATUACCTCAATCAUCCT | R25 | 128 | ACGCCTTCACCUTTAACACCUC |
| F26 | 33 | CCAGACAGAAAAGCGGCUGTUA | R26 | 129 | ACTTGGGAGGUATCCACAUCCT |
| F27 | 34 | CCTAGTAGAATGTTUACTACCAAATGGAAUGA | R27 | 130 | AGATTCATCTUGAAGAAGTTGAUGGAGG |
| F28 | 35 | TTTTTGAUGAAACAAGACGACTTTGUG | R28 | 131 | GAATAGGATATTGUATCATACCAATTTCUCGAT |
| F29 | 36 | CACAGCUACACCATATATGAAUGGAGA | R29 | 132 | CAGCATTTGACTTUACCTTATCAATGTCUC |
| F30 | 37 | GATCTATGTUCGAACAGGTATCUACCATG | R30 | 133 | ACTGCTAAACACTAAUATAACCTTTGGAAAUAT |
| F31 | 38 | GGGAAGAAAAGUGTTTTGAAATGTGTUT | R31 | 134 | CATTTTTCCAGATACUAGAGTGTCTGTGUA |
| F32 | 39 | TTTGAATCTTUGGCCAGTACCUCA | R32 | 135 | CATAAGAGAGAAGGUTTGACTGCCAUAAA |
| F33 | 40 | GACTAGCUAGAGACAATGAATUAAGGGAAAA | R33 | 136 | GAATCTCCATTTUAGCACTTACCTGUGA |
| F34 | 41 | CTGAGATGCACAAUAAAACAGTUAGCC | R34 | 137 | AGAATGTCAGTUAAGTTAATGAGCTTTUCCAT |
| F35 | 42 | GACAUTCUCAAACAGGAGAAGAAGGA | R35 | 138 | GCTTGATUCCAAGGACCATGATCUG |
| F36 | 43 | TCTTTTCTCAAGUTGGCCTGAAUCA | R36 | 139 | CAATTCCCAAAAUGAAGGTAGCUACAC |
| F37 | 44 | CAATCTTTTGAUGACATTGCATACATUCGA | R37 | 140 | GGAAGATCCAAUCCATTTTTGTTGUCC |
| F38 | 45 | GTATGCAGGCAUCCTCAGCUA | R38 | 141 | CGGGAAGCGGGAGAUCTUG |
| F39 | 46 | CUGGUGACCGAGGACAACGT | R39 | 142 | GGCGTCCUACTGGCAUGA |
| F40 | 47 | GTCCUGGGAGTCUCAGGACA | R40 | 143 | CCTUCAGCAGCTUGAAGAGCT |
| F41 | 48 | CCAGTTACCUGTCCTGGTCAUT | R41 | 144 | GGAAACTCCCAUCTTGAGTCAUAAGG |
| F42 | 49 | AGTGAAAAACAAGCUCTCATGTCUGA | R42 | 145 | CATGTGTCCAGUGAAAATCCUCACT |
| F43 | 50 | GGAAAAATTGUGAAGATCTGTGACTTUGG | R43 | 146 | CTGACTUTAGAGATTAAAGUGAAGGAGGAT |
| F44 | 51 | AGCACTCTGACAUATGGCCATUT | R44 | 147 | CCTGGACAAAAAUACCAATCTATTGUGG |
| F45 | 52 | GGCACGGTUGAATGTAAGGCTUA | R45 | 148 | ACTGATATGGUAGACAGAGCCUAAACAT |
| F46 | 53 | CCCACAGAAACCCAUGTAUGAAGT | R46 | 149 | ACTGACCAAAACUCAGCCTGUT |
| F47 | 54 | TUGACAGAACGGGAAGCCCUCAT | R47 | 150 | CCTGACAGACAAUAAAAGGCAGCUT |
| F48 | 55 | CCTTACTCAUGGTCGGAUCACAA | R48 | 151 | GTTGAAACUAAAAATCCTTUGCAGGACT |
| F49 | 56 | CCAATATTATGGAUCCCAACTGCCUA | R49 | 152 | ACATTCTGAAGCAGCUTGGAGTUT |
| F50 | 57 | GCTACTTTGAUTTCTCCACTUCCAAC | R50 | 153 | GAGGAGATTGAAAAUCTTCCTGCCUT |
| F51 | 58 | AAAGGCAUGGAGCATCTGUACA | R51 | 154 | TTGGTCCGUCTCCUCCACGG |
| F52 | 59 | GTTATGTCCUCATTGCCCUCAACA | R52 | 155 | CTTCAGTCCGGUTTTATTTGCATCAUAG |
| F53 | 60 | CGAGGGCAAAUACAGCTTUGGT | R53 | 156 | GACTCTCCAAGAUGGGATACUCCA |
| F54 | 61 | TGATGGAGAUGTGATAATTUCAGGAAACA | R54 | 157 | CGGTGACTUACTGCAGCTGTUT |
| F55 | 62 | GAGACATGCAUGAACATTTTTCUCCA | R55 | 158 | TCCAGACCAGGGUGTTGTTTUC |
| F56 | 63 | GCCTCTUACACCCAGUGGAGAA | R56 | 159 | TGTGCCAGGGACCUTACCTTAUA |
| F57 | 64 | CCTTCTCUCTCTGTCAUAGGGACT | R57 | 160 | CACAGCAAAGCAGAAACUCACAUC |
| F58 | 65 | CTCCAGGAAGCCUACGTGAUG | R58 | 161 | TGTGTUCCCGGACATAGUCCA |
| F59 | 66 | CAGGAACGUACTGGUGAAAACAC | R59 | 162 | GAAAATGCUGGCTGACCUAAAGC |
| F60 | 67 | GCTTGTAAGUGCCCGAAGTGUA | R60 | 163 | CACAACCCACUGAGGTATATGTATAGGUAT |
| F61 | 68 | CGCAGTGCUAACCAAGTTCTTUC | R61 | 164 | CCATGGTTAAATAAAAUGCCACTTACTGUT |
| F62 | 69 | CAGTCAAGGUTGCTGATTTUGGT | R62 | 165 | CTTGGTGGUAAACTTTTGAGTTUGCA |
| F63 | 70 | CGAATCGCUACCCTGCTGUT | R63 | 166 | CCAAGCCUCATGGUGCCAT |
| F64 | 71 | TACCGAUACCGUGCGGG | R64 | 167 | TACUGGCAGCAAGUGCCCAG |
| F65 | 72 | CTGTCCUCCACAGGCATTTTUG | R65 | 168 | CCCTCACUCACAGCACATAGUC |
| F66 | 73 | CCATCCCUGACTGTGAGAUCAA | R66 | 169 | CCAGGUACGCCTCCAGAUGA |
| F67 | 74 | TCAGTGGAAAAAUAGCCTCAATTCTUACC | R67 | 170 | CTTCATGAAGACCUCACAGTAAAAAUAGGT |
| F68 | 75 | TATTATGACTUGTCACAATGUCACCACAT | R68 | 171 | GACTCGAGTGAUGATTGGGAGATUC |
| F69 | 76 | TTCCUTAGTCTTTCTTUGAAGCAGCA | R69 | 172 | AGATGCTCUGAGAAAGGCATUAGAAAG |
| F70 | 77 | TCTGACUCCACGAGAACTTGAUCATA | R70 | 173 | TATTGTTAACCUTGCAGAATGGUCGA |
| F71 | 78 | CAAGGCAUAAAAGCTGGGAAAUAGG | R71 | 174 | CUACCTGCCUACGCAACAAGAT |
| F72 | 79 | GGCTAUGGCACCUGCAACT | R72 | 175 | GGGACCUCAGATGTGCTGTUG |
| F73 | 80 | CCACAGAUCCACTGUGCGAC | R73 | 176 | GTGGCTTGUGGGCAAACTUG |
| F74 | 81 | CCATCCTGACCUGGTATGGUCA | R74 | 177 | CCTGCTUCAGGACGTTGAACUC |
| F75 | 82 | CAGCTCGTUCATCGGGACUT | R75 | 178 | ACCTGGCUCCTCTTCACGUA |
| F76 | 83 | CCTCCTTCCUAGAGAGTTAGAGUAACT | R76 | 179 | CACCCACACUTACACATCACTTUG |
| F77 | 84 | CACTGTGTTACUGCCATCGACTUA | R77 | 180 | TCGAGATTUAGCAGCCAGAAATGTUT |
| F78 | 85 | GATTCAATCAAACUGCAGAGTATTUGGG | R78 | 181 | TGATCTGGUGTCAGAGAUGGAGAT |
| F79 | 86 | GGCTTCTTGG UCGTGTTCTUCA | R79 | 182 | CUAGCGCCUGGAAGAGAAAAGGAGAT |
| F80 | 87 | CCCAGCGUCCTCAAAAGTUACA | R80 | 183 | CCCTCCACAAUCATTCCTGUGT |
| F81 | 88 | CTCTACGUCTCCUCCGACCA | R81 | 184 | CTTATTTATTGGTCUCTCATTCTCCCAUCC |
| F82 | 89 | CCTGTACTGGUGGATGTCCUCA | R82 | 185 | GCCTGTUGGACATCCTGGAUAC |
| F83 | 90 | CGCCAGGCUCACCTCTAUAG | R83 | 186 | AGGAGCGAUGACGGAATAUAAGC |
| F84 | 91 | TGTCTTTGCUGATGTTTCAAUAAAAGGAA | R84 | 187 | AGTTAAGGACTCUGAAGATGTACCTAUGG |
| F85 | 92 | CATGTACTGGUCCCTCATUGCA | R85 | 188 | GTAATAATCCAGACUGTGTTTCTCCCUT |
| F86 | 93 | TACCTCTATTGTUGGATCATATTCGUCCA | R86 | 189 | TATTATAAGGCCU GCTGAAAATGACUGAAT |
| F87 | 94 | CAGACACTGUACAAGCTCUACGA | R87 | 190 | GAAUAAAGAGGAGCAGGTUGAGGAA |
| F88 | 95 | CTCTGUCACAGTGGATUCGAGA | R88 | 191 | CAACATGACGAAGAUGGCAAACTUC |
| F89 | 96 | TCCUTCCATAGUGACCAAGACCA | R89 | 192 | GGGTACAUACAAAGCAGTCTGUGT |
| F90 | 97 | GGTTCCATUGGTAGCTGGUGAT | R90 | 193 | GCCCATTTTTATCUACTTCCATCTTGUCA |
| F91 | 98 | TGTAAAGAGACAGCCUTTCCTCUGA | R91 | 194 | AGTTCACAAAUCCATCAATGTTGCUC |
| F92 | 99 | ACACUCTUGAGGGCCACAAAG | R92 | 195 | TGTGATTGUAGGGTCTCCCTUGAT |
| F93 | 100 | CCGCTCCUTGTAGCCAAUGA | R93 | 196 | GGGTCUGACGGGTAGAGUGT |
| F94 | 101 | CCACUTUGGAACAGGACCAAC | R94 | 197 | TCAAAGTCGTCAUCCTTCAGTTCUC |
| F95 | 102 | CTTTCTTCCACCUTTCTCCAGCUA | R95 | 198 | CATCGCTGUAGAACGCACCAUA |
| F96 | 103 | TCAAGCCCUCCAACATCCUAGT | R96 | 199 | GGAAACTTCTGTUCATACCGACATGUAG |

**TABLE B**

| **Primer Name** | **SEQ ID** | **Primer Sequence (target seq of Primer A)** | **Primer Name** | **SEQ ID** | **Primer Sequence (target seq of Primer B/C/D/E)** |
|---|---|---|---|---|---|
| F97 | 200 | GAGGTCGTATUCGTCCACAAAAUGGT | R97 | 469 | GAGGCAGGAGACCCUGUAGGAG |
| F98 | 201 | GAAACTGCTTAGUAACTAGCAGAAGTGTUC | R98 | 470 | GAGGATAUATGCCAUACCCCAGCAAA |
| F99 | 202 | GACAAAGTTGUGTGTTGTAAGUGGAACA | R99 | 471 | GACCAAGAAAGGCUTGTGTCTACATTTUT |
| F100 | 203 | GAGCACCAATCUTTCTTCTGCCTTTUG | R100 | 472 | GACCAAAUCAAGAAACCTGTTUGAGAGAA |
| F101 | 204 | GAGGGAUCCCAAGGAAGAGAAGUGA | R101 | 473 | GACACTTCCCUTGTGGGAATGUCAA |
| F102 | 205 | GAGCTACTCTCCUGAACTCTCTCACUC | R102 | 474 | GATGCATCAGAACCCUCCTTGAAUC |
| F103 | 206 | GACTCCCAGUTGCAACGTUAGGT | R103 | 475 | GAGGUCCACGGAUCCAGAAACAAG |
| F104 | 207 | GATCACGUGTCCCCCTUCCA | R104 | 476 | GAGGTTCCTCCUCTCCTGGTCUC |
| F105 | 208 | GAACATTTGGCUGTGACTTCUAAGAAGAAA | R105 | 477 | GAGCTCACTAACUAACGTGAAAGCCTUAC |
| F106 | 209 | GAGATGAUCCAGATGTUAGGGCAGT | R106 | 478 | GAGGTTTTGCACAAGUTAGGTTTGTTTUG |
| F107 | 210 | GAGCCCAAATUGATTTCGATGATCTUCA | R107 | 479 | GAACCTTTATATCGTUACTCTGAATCTTATCTUCC |
| F108 | 211 | GAGCTCAAGAGUGAGCCACTUCT | R108 | 480 | GATGACTCTGTCUCCTCTTGTCTTCUC |
| F109 | 212 | GAATTAACACACAUCAGTGGAACTTCUGT | R109 | 481 | GAAAACGTTTTUCACCTTAGCATTTUGT |
| F110 | 213 | GAGCAAUCCAAAAGAAUAGCAGCCAAA | R110 | 482 | GACACCATCUCCATATCATUGAGACCAAAT |
| F111 | 214 | GACTTTGTGGAAUAGCCCATGAAGAGUA | R111 | 483 | GACGACAGACUACTTTGGTTCTCTTTUGT |
| F112 | 215 | GAACAGGAAGAGCACAGUCACTTUG | R112 | 484 | GACTCACUGACAAGCTCCUCGT |
| F113 | 216 | GACCATGCAGAGUGAAAGGATAUCCC | R113 | 485 | GAGCCTTTTCTTUTGCTTCCCTTGUT |
| F114 | 217 | GATGGAGCCGCUGACACCUA | R114 | 486 | GACAGGACCUGGCCCUGAC |
| F115 | 218 | GAGGTGUCTAGCCCAUGGGAGAA | R115 | 487 | GACCCAUCACACACCATAACUCCAC |
| F116 | 219 | GAGTCTGGUCCACATTGCTCUCA | R116 | 488 | GAGCTGUCCCCTCACCATUCAG |
| F117 | 220 | GAGCAAGAGUACACACTCCTCATTUGG | R117 | 489 | GATCACAACCCACUGAGGTATATGTAUAGG |
| F118 | 221 | GACTGGTTTCUGGTGGGACCATUA | R118 | 490 | GACAUGCACCGGAAAAGCGAUG |
| F119 | 222 | GACTCCCAGGCACUTGATGATACUC | R119 | 491 | GACATTTCTAGGUTACAGGCCUGGAT |
| F120 | 223 | GAGGGCTUGGTAACGTCCUGT | R120 | 492 | GACTCCAUGCCCCTCACUCA |
| F121 | 224 | GATGTGTCAAGGAGUTCGAAGATUCAC | R121 | 493 | GAATTGAAAATCUTCCTGCCTUCCCT |
| F122 | 225 | GAGUACAGCCAGTGTGUCCGA | R122 | 494 | GAGCAAAAGTGGUCCTCTCTGAAUCT |
| F123 | 226 | GACCATCCGGGCUTTACGCAAAUA | R123 | 495 | GAATATCAUCCAGCCTGTGTCTTUCC |
| F124 | 227 | GACCCCACUGAACCTCTCTTACATUT | R124 | 496 | GAGAGGGAAGGCAGGAUCTCUAAC |
| F125 | 228 | GACCCUAACAGCCATGCTTTCUC | R125 | 497 | GACCAGGCAAUGGAAAGGGTACAUA |
| F126 | 229 | GACGGCGAUGCTGAGAACCAAUA | R126 | 498 | GAGAAUAAAGAGGAGCAGGTUGAGGAA |
| F127 | 230 | GACCGACGUTGACCGCAUC | R127 | 499 | GAGGGCAAAUGAGCCTCUCAGT |
| F128 | 231 | GAAAATATTUCAGTGTCCGTUCACACACAA | R128 | 500 | GATCCAGATTGAUCTTGGGAGTGUAAAAA |
| F129 | 232 | GACCACACUGACGTGCCTCUC | R129 | 501 | GAGTCTTTGTGUTCCCGGACAUAGT |
| F130 | 233 | GAGCGCCACAGAGAAGUTGTUGA | R130 | 502 | GAGGGTCUGACGGGTAGAGUGT |
| F131 | 234 | GACCACAAAAUGGATCCAGACAACUGT | R131 | 503 | GAGCTTGCTCTGAUAGGAAAATGAGATCUA |
| F132 | 235 | GAACTGTTTCGTAUTTATAGCTGATTTGAUGGA | R132 | 504 | GACCTCTTCCUCAGGATTGCCTUT |
| F133 | 236 | GACCTCAUTGCCCUCAACACAGT | R133 | 505 | GATCAGTCCGGTUTTATTTGCATCATAGUT |
| F134 | 237 | GACACCACGUACCAGATGGAUGT | R134 | 506 | GACCCAAAGACUCTCCAAGATGGGAUA |
| F135 | 238 | GAAGACATGCAUGAACATTTTTCUCCAC | R135 | 507 | GATCCAGACCAGGGUGTTGTTTUC |
| F136 | 239 | GATGUGGAGCCTCTUACACCCA | R136 | 508 | GAGTGCCAGGGACCUTACCTTAUAC |
| F137 | 240 | GAACGTCTTCCUTCTCTCTCTGUCA | R137 | 509 | GACTGAGGUTCAGAGCCAUGGA |
| F138 | 241 | GAGAATGTGAAAAUTCCAGTGGCCAUC | R138 | 510 | GAGUCATATCUCCCCAAACCCCAAT |
| F139 | 242 | GAGGGTGTGUGGTCTCCCAUAC | R139 | 511 | GAGCCATAGGGCAUAAGCTGTGUC |
| F140 | 243 | GAGGATGAGCUACCTGGAGGAUGT | R140 | 512 | GACCTTGGTCCUTCACCTAACCTUG |
| F141 | 244 | GAGGTCACTGUACACCTTACACAUGAA | R141 | 513 | GACCCTCUTTAGCCAUGGCAAGG |
| F142 | 245 | GACATCACUGTAAACCTUGCAGACAAAC | R142 | 514 | GATGGTCTCUCATTCTCCCAUCCC |
| F143 | 246 | GAGCTTCTTGGUCGTGTTCTTCAUT | R143 | 515 | GACTCCTCCUGTGATCTGCAAUCT |
| F144 | 247 | GAUGGAAGCCCAGCCATTUCTAAA | R144 | 516 | GAGATGAUGAAGATGATUGGGAAACACAAG |
| F145 | 248 | GAGCCCCUGAGCGTCAUCT | R145 | 517 | GAGGGCTGUGCGTCACTGUA |
| F146 | 249 | GAGAGCTGGUGGAGGCUGA | R146 | 518 | GAGGAGCCCAGGCCUTTCUT |
| F147 | 250 | GAGUGACCGAGGACAACGUGAT | R147 | 519 | GAGCGTCCUACTGGCAUGACC |
| F148 | 251 | GACTCUGGGAGATCTUCACGCT | R148 | 520 | GACCACUCACAGGTCGTGUGT |
| F149 | 252 | GAGGATTGCAGAUTGGGCCTUG | R149 | 521 | GAACATGATGGAUGTCACGTTCUCAAA |
| F150 | 253 | GAATAATCCAUTGCCTGTCUAAAGAACACT | R150 | 522 | GATGTTAACCUTGCAGAATGGUCGAT |
| F151 | 254 | GAGACTTGGUGTTGTTGAUGGCAAA | R151 | 523 | GACUGCAGGATTCCUACCGGAA |
| F152 | 255 | GACCAACAUGACTTACTTGAUCCCCAT | R152 | 524 | GAATCACCAAAUGGCACCAUACGA |
| F153 | 256 | GAACCCUGGCCTACCTGGUC | R153 | 525 | GAAGTTCAAGCUGAAGAAGATGUGGAA |
| F154 | 257 | GATGAAGCAGCAAGUATGAUGAGCAA | R154 | 526 | GACTGACACCUAGCTGTGATCCUG |
| F155 | 258 | GAGGCACGGTUGAATGTAAGGCTUA | R155 | 527 | GAACTGATATGGUAGACAGAGCCUAAACAT |
| F156 | 259 | GACCACACCCUGTTCACTCCTUT | R156 | 528 | GAGTCTCAGTCAUTAGAGCACTCUGG |
| F157 | 260 | GAAAGGTGATCUATTTTTCCCTTTCUCC | R157 | 529 | GATTTCATACUGACCAAAACUCAGCCT |
| F158 | 261 | GAGCTTTTTGCUAAAATGCATGTTUCCAA | R158 | 530 | GAGACACGGCUTTACCTCCAAUG |
| F159 | 262 | GACAAAGAATGGUCCTGCACCAGTAAUAT | R159 | 531 | GAAGGCCTGCUGAAAATGACTGAATAUAA |
| F160 | 263 | GATCCTCATGUACTGGTCCCTCAUT | R160 | 532 | GAGTAAAAGGUGCACTGTAATAAUCCAGACT |
| F161 | 264 | GACAGATCTGTATUTATTTCAGTGTTACTUACCT | R161 | 533 | GAGACTCTGAAGAUGTACCTATGGTCCUA |
| F162 | 265 | GACATGTCAACAUCGCTCTAATUCAGAGA | R162 | 534 | GAGCTTTUCAAAAGGCTUAAACACAGGAT |
| F163 | 266 | GATGTTACGCAGUGCTAACCAAGUT | R163 | 535 | GAGCAAACCACAAAAGUATACTCCAUGGT |
| F164 | 267 | GAGCTGAUTTTGGTCTUGCCAGAG | R164 | 536 | GATCTGACTTGGUGGTAAACTTTTGAGUT |
| F165 | 268 | GACCTCACCTCUATGGTGGGATCAUAT | R165 | 537 | GAGTTCTTGCUGGTGTGAAATGACUG |
| F166 | 269 | GATTCGCCTGUCCTCATGTATUGG | R166 | 538 | GACACCCCCAGGAUTCTUACAGAAAA |
| F167 | 270 | GAGCACUGGGACTTTGGTAATUCAC | R167 | 539 | GACATCTCTUGGAAACTCCCATCTUGA |
| F168 | 271 | GACAGTGAAAAACAAGCUCTCATGTCUG | R168 | 540 | GACCACATGUGTCCAGTGAAAAUCCT |
| F169 | 272 | GACAGTGTGUCCACCGTGAUCT | R169 | 541 | GAAGTGAAGGAGGAUGAGCCUGA |
| F170 | 273 | GATGGAATGCCAGAACUACAATCTTTUGAT | R170 | 542 | GAGTGGAAGATCCAAUCCATTTTTGTTGUC |
| F171 | 274 | GAGACGCAUTTCCACAGCUACAC | R171 | 543 | GAAGCATCAGCAUTTGACTTTACCTTAUCA |
| F172 | 275 | GAGCTTTGAAUCTTTGGCCAGUACCT | R172 | 544 | GACATAAGAGAGAAGGUTTGACTGCCAUA |
| F173 | 276 | GAGATGCAGCCAUTGACCTGTTUAC | R173 | 545 | GAAGAAAACCATUACTTGTCCATCGUCT |
| F174 | 277 | GAGGGATUAAAGCTGGCTAUGGCA | R174 | 546 | GACCTTGTUGGGACCTCAGAUGT |
| F175 | 278 | GAAGCAUACGCAGCCTGUACC | R175 | 547 | GAGTGGUAGCAGTGGAUGCAGAA |
| F176 | 279 | GAGCTUCCAGGAGCGATCGTUT | R176 | 548 | GAAGGCCCCAUACAATTTGAUGACA |
| F177 | 280 | GAAGCTCGTUCATCGGGACTUG | R177 | 549 | GACCATGGUGCACCUGGGAT |
| F178 | 281 | GACTGGTUACTGAAAGCUCAGGGAT | R178 | 550 | GAACTTTGCGUGGTGTAGATATGAUCAA |
| F179 | 282 | GAGGACTCTGUGAGTGGGATTTGTTUT | R179 | 551 | GAGTCTTCACUCACCTCGGAUGA |
| F180 | 283 | GACATCCCUGACTGTGAGAUCAAGAA | R180 | 552 | GACAGGUACGCCTCCAGAUGAG |
| F181 | 284 | GAATCAACCUGCTTGGTGTCUG | R181 | 553 | GAAACUCCCGCAGGTTUCCCT |
| F182 | 285 | GAACGAGGACCUGTGGGACUC | R182 | 554 | GAGTGCCTUGCCCTTTTTGUGG |
| F183 | 286 | GACATUCCCCAACAGCTGUGGT | R183 | 555 | GACCGGGAUGCCAGGAUACG |
| F184 | 287 | GACCTTCCUCCTGAAGGCCUGA | R184 | 556 | GAGGGCUGTACCTCCUCAGAGA |
| F185 | 288 | GAAAGTGCUTGTGCCCUGCAT | R185 | 557 | GAACAGGCUGCCCAAGGGCUA |
| F186 | 289 | GACCCCTCTUGGACCTTAGAUGC | R186 | 558 | GACAGUGATCAGAUGAGCAGCAG |
| F187 | 290 | GACGCAUGGAGAAGAAACTGCAUG | R187 | 559 | GAACGGUCTUGGAACCCAGAGA |
| F188 | 291 | GATCCCCTAUGTGCAAGTCCUAAAG | R188 | 560 | GAGCTATTGAUGTCTGCAGTCUGG |
| F189 | 292 | GACCACTGUGCAGAAGCTCUCC | R189 | 561 | GATUGACTUGCCGGAAGAGCCT |
| F190 | 293 | GACGCCGGCCUCGTGAGUC | R190 | 562 | GAAGACCUCCGAGTCACTCCUG |
| F191 | 294 | GACAAATGCUGAAAGCTGTACCAUACC | R191 | 563 | GAAAAAGACTCGGAUGATGTACCTAUGG |
| F192 | 295 | GATGAGGCAGUCTTTACTCACCUG | R192 | 564 | GATTCCTTTCTUCCCAGAGACATUGC |
| F193 | 296 | GAGCAAAGACUGGTTCTCACUCACC | R193 | 565 | GAACATCCCUCTCTGCTCUGCA |
| F194 | 297 | GACGAUCTGTTCUACACGGAACCC | R194 | 566 | GAGGCTGGTTAUTGAAACCTTGTTTUACAT |
| F195 | 298 | GACCAGACAAGCCUACAGTAGGAAUC | R195 | 567 | GACTACCCCCGUACCAAGUACAAAC |
| F196 | 299 | GACTCCACAGACCCUCTCCTUGC | R196 | 568 | GATCGUCGAAGCGGCUGAC |
| F197 | 300 | GAAGGGTGTCUCTCTGTGGCTTUA | R197 | 569 | GAGACTCTGUAGGCTGCAGTTCUC |
| F198 | 301 | GAGTTTCUGCAGATTGACTUGCACA | R198 | 570 | GACTTCTTCCUACCTGTTTCCCAUGAC |
| F199 | 302 | GATCAGGAAACAAAAAUTTGTGCTAUGCAA | R199 | 571 | GAGGACCCAUTAGAACCAACUCCAT |
| F200 | 303 | GAGCTGGAGGAGCUAGAGCTUGAT | R200 | 572 | GAGGCTTGUGGGAGACCTUGAAC |
| F201 | 304 | GAGGGCTGUCGTGGTAGACTUAGA | R201 | 573 | GACCTGGTAGTCUCAAGCAGATGTTAAUG |
| F202 | 305 | GAAAGACTTCUCAAATTGTTGCCATTUCAG | R202 | 574 | GAAACGGACATGAGUTTGTTTTCCTTCUA |
| F203 | 306 | GACGAGGAAGAAAAACAAUCCCACTUG | R203 | 575 | GACAGCCAACAAGAUTCTGAAGAACAUG |
| F204 | 307 | GAGAGATCCTTUCGAAGTCATCGTCUT | R204 | 576 | GATCCCTAGGUAGCTAACCCCUAC |
| F205 | 308 | GAGGGCAAUGTCAATTAGCUGGAAC | R205 | 577 | GAAAAACACGGCAUGTGAACATTCUG |
| F206 | 309 | GACACTGTGTTACUGCCATCGACTUAC | R206 | 578 | GAGTATTCAUCGAGATTUAGCAGCCAGA |
| F207 | 310 | GACTTTTACCCUCTTCAGCTCAGTTUCT | R207 | 579 | GAGAGAGAGGACUGACTATCGGACUG |
| F208 | 311 | GATCTCCTCCAACCUAATAGTGTATUCACA | R208 | 580 | GAGACTGUCAAGCAGAGAATGGGUAC |
| F209 | 312 | GATTTCGUAAGTGTTACUCAAGAAGCAGAA | R209 | 581 | GAGAATAGGATATTGUATCATACCAATTTCUCGAT |
| F210 | 313 | GAATGCCCCCAAGAAUCCTAGUAGAA | R210 | 582 | GAACGAAAATGUAAGAAGATTCATCTUGAAGAAG |
| F211 | 314 | GAAGAGATGATTGUTGAATTTTCCTTTUGGG | R211 | 583 | GAAAAGCCATTTTUCCAGATACTAGAGUGT |
| F212 | 315 | GACGGAACUCGAATCGCUACCCT | R212 | 584 | GAGGGUCCCCAAGACACCUACG |
| F213 | 316 | GACTCGATGCUGTTCCCAGGUAC | R213 | 585 | GACCGAGAACUGAGGGTGGUACA |
| F214 | 317 | GATCATACAGACACUTCATTTGGAGUACC | R214 | 586 | GATACTAGAACUCAAAACACTGGCTGUT |
| F215 | 318 | GAAAAAATAAAGCTUGGCTTCAAGTTGUCT | R215 | 587 | GAGTAAGTCTTCACUTTCAGATTTTAGTUGGG |
| F216 | 319 | GAACATTGTGACCUTAATTTTGTGATCTCTUG | R216 | 588 | GATGCTTCCTGGUCTTTAGGATTTCUT |
| F217 | 320 | GACCCCACTCAUGTTTAGCAGATGUA | R217 | 589 | GATTTTACTTCUGCTTGGTGGCAUG |
| F218 | 321 | GAGGACAGGUTTTGTTGTUGAGGAAG | R218 | 590 | GATTTTACCCTCAUGGCTTAGTAGCATTATUT |
| F219 | 322 | GAAGCAAGGTCAUAAATTATTCTCCATATTTUCCA | R219 | 591 | GAAAAAATATUCATCCAGCTUCAGGAAAAGG |
| F220 | 323 | GATCTTTTTACCTUATAGATGGGAAACAUGAGAG | R220 | 592 | GAATCAGTCUGGTGGATGGGUAACA |
| F221 | 324 | GAATGTGTCTTTCAUGAGAAAAACAAGATCAUT | R221 | 593 | GACTAATAATGAATAAUTGGGTATGAGGCUACAGT |
| F222 | 325 | GATAGTAGCTGAUCCACAGAAGTTCAGUA | R222 | 594 | GATGTGAGAGAGCAAUCAAGGAGUG |
| F223 | 326 | GAAAGACTCTGAAUACCACCATCAAGAATAAUAAA | R223 | 595 | GAGTCTGAGAGUAGAAGGCAGATTCTGUA |
| F224 | 327 | GAATCTACAGGCCAAUGGTTCCTUC | R224 | 596 | GAAACTTUGCGGAGATCUGAAAACCA |
| F225 | 328 | GAGCCAGTAGUCACAAAGATTTCTUACCA | R225 | 597 | GACTCTAAAGAAGGAAGUGAGAACTTCUCC |
| F226 | 329 | GAAGAAGAUTGGGUGGGCAGAC | R226 | 598 | GACTTTCTTCACUCAAAGTGCCTATTTUGAC |
| F227 | 330 | GAAACCACTGATACAUTTTTCTACTTTCCUGAA | R227 | 599 | GATTCTTTTGAGAACUGAGTGATTTAUGACCT |
| F228 | 331 | GAAGACTTCTTUGAGATATTTCCATAGCUCAC | R228 | 600 | GAAGAAGTUAGAAACAGAACTGTATGUAAGCAT |
| F229 | 332 | GACATTTTTGTTTATGUTATTCTCTCTACCUCAGC | R229 | 601 | GAGCTATACGAACTUAGAAGTGAGAAATAATCUT |
| F230 | 333 | GAAGTTATAGGTAAUCGATGCATATAGCTCAUCT | R230 | 602 | GATTTCTCCAGGUCCAAAATGAATAACTATTUGA |
| F231 | 334 | GAAATTGTTTGTAGGGUTGGTTATTAGTGACUAT | R231 | 603 | GAATCCAGGAUAGGAAGCACACAUG |
| F232 | 335 | GACCACTATGUAAGACAAAGGCUGGT | R232 | 604 | GATTTTATAACTAGATUTTCCTTCTCTCCATUCC |
| F233 | 336 | GAGTTTCTGUAGCCCATACTTTGGAUGA | R233 | 605 | GAAATTCATACAUTTTTCTCTAACUGCAAACAT |
| F234 | 337 | GACACTGUGAAGGCCCTTTCTTCUG | R234 | 606 | GAGCAGTTGUGAGATTATCTTTTCAUGGC |
| F235 | 338 | GAGCATAGGAGATAAUCATAGGAATCCCAAAUT | R235 | 607 | GATGTTTTTCTAAUGTGTTAAAGTTCATUGGAAC |
| F236 | 339 | GAAGTCACTGGAAUTGTTGGGCUAC | R236 | 608 | GACCATGACTGUCACAGTGACCUT |
| F237 | 340 | GACTUCCAGGAGCCGUAGAGTTT | R237 | 609 | GATTGUGGCCCAAACAAAGCUC |
| F238 | 341 | GAACTCTTCCTATTUTTGTAGTGACCTGTUT | R238 | 610 | GAGTGCTTGGAAAUGGAATGGTTTUAGAAT |
| F239 | 342 | GATTCCTGAUAAAGCACAGCTGTAGUG | R239 | 611 | GACTACTGUAACCAAGAGGTGACTUCAG |
| F240 | 343 | GATTGTUCAAGCAGCGAGUCC | R240 | 612 | GAAGCCGAUATCCCUGCAGAC |
| F241 | 344 | GACATGAACUACCTGGACCGCUT | R241 | 613 | GAGTCGGUGTAGAUGCACAGCT |
| F242 | 345 | GAGGTGCTGUCTGGGAAGAUGT | R242 | 614 | GATGCCCAAGGUACTGCAUGGT |
| F243 | 346 | GAAAAGAGGCAGUAGCATCTTCUCC | R243 | 615 | GACUCACGCCUAAACCAGAACC |
| F244 | 347 | GAAATCCTGGAGCUTTGGTGTCTAATUC | R244 | 616 | GATAGCTGGCUCCGCACCUT |
| F245 | 348 | GATGCAGAAGCGGUTTCTGUG | R245 | 617 | GACTGCUGGGCGCCGUAAC |
| F246 | 349 | GAGCCTCAGAGAUAAAGGCAAAGATUG | R246 | 618 | GAACCCACACAAGCGAAUCTCUG |
| F247 | 350 | GAACCATTATTTCTTUGTTTTGTTTTTCCTGUAT | R247 | 619 | GAACTTTGCUGCCTTAATGACATUCC |
| F248 | 351 | GATACCAACCAAGUTTCATUAACCACAGT | R248 | 620 | GAGGTACCUGAGATGGAGGAGUC |
| F249 | 352 | GACCATGTTGGUCACTTACTCAAAGATTUT | R249 | 621 | GATTGAGCCACUAAGCAGTAACCATUC |
| F250 | 353 | GAAATGGGCAAGGUATGGATGUGG | R250 | 622 | GACCCGAAGUTCTTCTGCAGUCC |
| F251 | 354 | GATGGCGCAUCAGATCCTAGTUT | R251 | 623 | GAGCCGAAACGAUCAAGGUGAGT |
| F252 | 355 | GATGTAACAACCUAAAGGGAATAGGAAGAAUG | R252 | 624 | GATCAGTAGAAAGAUGGTACCAAAAUGGGT |
| F253 | 356 | GAGGCACTGGUTCTCATTCCUG | R253 | 625 | GAGACCGAGCUCGGGTGUAT |
| F254 | 357 | GAATGACTCAAUACCAACCCCUCCA | R254 | 626 | GAGCCTTTGTGGUCATGGGAAAGTAUA |
| F255 | 358 | GACATAACCATGAUATTAATAGGACTCCUGCT | R255 | 627 | GAATTTGCCTGAAATUACACATAGAACTTTCUG |
| F256 | 359 | GATGAACGCAAAACCUGTTGAAGTUAAAA | R256 | 628 | GAGGGATGGGUGACTGAGAUGGT |
| F257 | 360 | GACTTAAAAATGTCAAUATCTGGCCTCAAAUACG | R257 | 629 | GAGTGGACAUGCGAAUGGAGGA |
| F258 | 361 | GAGGGAAGCAATUTGCTACACTTTAATTUAAAC | R258 | 630 | GATTTACTCTGACAGCUAAATGAACTCAAATGUA |
| F259 | 362 | GAACAGAAGCUTCTAATCCUCAACGT | R259 | 631 | GAAGGCTCAGAACACUTTACTGAATTTUG |
| F260 | 363 | GAAGTTGAUGCCAATTCACAAUCACCA | R260 | 632 | GATTACTTAGAAGAAAAUTGCTCCTTGUCAGA |
| F261 | 364 | GACTGAGGTCTATUCACTTTCTTTTCATCTTUG | R261 | 633 | GAGTGAAGGAAACCAUTCGTGAUAAAGC |
| F262 | 365 | GACTATTTGTTTCTUCCCCATGGAATTGUC | R262 | 634 | GAGAAAATUGGACCCAGTTCTCUGCT |
| F263 | 366 | GATAATCTTUGAACTGCCTGUGCACT | R263 | 635 | GAATCTTTCAACUGTAAAATTCACTGUGGGT |
| F264 | 367 | GAAAGCAAUGGCTTGGGAAGUAAGA | R264 | 636 | GACCATTCTCAUATCCTAGGTCUGCCT |
| F265 | 368 | GATCTAAAGGTTTTTCUGATTTCCTCATTAGGAUT | R265 | 637 | GAGCACUCCATTTUGGACAGCAA |
| F266 | 369 | GATCCAGTCATTTUGAGAAAGACAACTUACT | R266 | 638 | GAGTTTATTTTCTGGUTTCAATAGAACAAGTUGA |
| F267 | 370 | GATTCATTAATATTTUCAGATCACCAGTTGATUG | R267 | 639 | GATTTGAAAGGTAGAUTGCCATAATGTATCATUG |
| F268 | 371 | GAATTAACTGTACCUCCAACTTTCTTACTATAUGC | R268 | 640 | GAATGTGGAATCTUTGTTTAGTTTTACTCUGGT |
| F269 | 372 | GATTAAGAAACUAGAAACTGTTTAGACUGCCT | R269 | 641 | GAATGGTTTAGTCUGACACATATTTAACACUT |
| F270 | 373 | GATGGTTCTGUCGACTAAACUGC | R270 | 642 | GATTTTCAAGTTAUAGAAACATGTCATGTTGUCA |
| F271 | 374 | GAACGGACACUATGTCCTTAAGCUGA | R271 | 643 | GACTAGACTTUGAGACCTGCTAAATAATUAGATG |
| F272 | 375 | GATTGGCAUGGCTTCTCUAGCT | R272 | 644 | GACAGGUCCAAGUGAACCAGGGA |
| F273 | 376 | GACACCTTCUACCGCTCACUGC | R273 | 645 | GAAGGGCACUGACCCTGGUA |
| F274 | 377 | GAATTGACUCTGAATGUCGGCCAA | R274 | 646 | GATCTGCAGGAGGGUGCTCTUA |
| F275 | 378 | GACTCTGCCCCUAAGAAACCUGGA | R275 | 647 | GAGGGCAACUACACCTGCATUGT |
| F276 | 379 | GAATTACTCUAACTTTCGCAUGCACAC | R276 | 648 | GATGCCAAGACAGUGAAGTTCAAAUG |
| F277 | 380 | GAAGUGGGCAGCAGTTTCUGA | R277 | 649 | GAGACACCACCUACTTCTCCGUCA |
| F278 | 381 | GAAACCAACTGCUTGTATGCTTTCUGG | R278 | 650 | GAAAGACCAAAAGAGAAUGGAAAGTACUGAC |
| F279 | 382 | GAGCAGCCUTACCTGGTUGGA | R279 | 651 | GAAGAGUATCCATCUCCAGGAGACG |
| F280 | 383 | GATTACAGCUCGTTGGUGCAGT | R280 | 652 | GATGTGGCUCTCCGCCCAUT |
| F281 | 384 | GAATGGAAACCCUGACAGAGTCTTUG | R281 | 653 | GAGCTACACCATUAGCTTCACTGATTUT |
| F282 | 385 | GATGTTATGAGCUTAGCACCTUGCAG | R282 | 654 | GACTGGATGGUAAGAGGAGTTTCTTCAUC |
| F283 | 386 | GACCAGTTCUGCAGTTAGAGGTUG | R283 | 655 | GACCTTTCCCCUCCCCTACCUAG |
| F284 | 387 | GATTTGATTCTTAAUCACCTAAGGAUGGCT | R284 | 656 | GACAAAACAAAGUCAAAGAGAATTATGAAATGUG |
| F285 | 388 | GAATCCGTACCUTCCACCAATCUG | R285 | 657 | GACAGGGATTTUGGTTACTACTTTGCUAAGA |
| F286 | 389 | GATATAACAATGAAUGACCAAAAGGAAATUAACAA | R286 | 658 | GATTGTCTTCUGGACACGTTCUGAAA |
| F287 | 390 | GATAACTTTCCATAUGCAAACCTACTGGCUA | R287 | 659 | GATATTAAGCTTTCUTGGAAAATTCTCTTUCCCT |
| F288 | 391 | GACCUGGACGTCTUGGAAAAGGG | R288 | 660 | GATTCAGAAGTUAGGAAAGGAGUCCAG |
| F289 | 392 | GATCTGGGUCAAGGAUGGCACA | R289 | 661 | GACACCUGUCACCCGCACAC |
| F290 | 393 | GAGCCCACUTCCCATCUGGGT | R290 | 662 | GACAGTCAGUAACGCCAGUGAGT |
| F291 | 394 | GATCCCCGCUGCTGUGCAAC | R291 | 663 | GAGUCCCGTGGUGCAAAGGC |
| F292 | 395 | GACCCACATGUCCAGCACCUT | R292 | 664 | GAATGTACACTAGTTUCCGGAATAAACCTTUT |
| F293 | 396 | GAGCTGGUGAAACAGGTAGUGAGT | R293 | 665 | GACTUGACCCCUGCGAGCCA |
| F294 | 397 | GACTCCAGGUCCTTGTGUGAGC | R294 | 666 | GACCCACUCAAGCTCAGCTGUAA |
| F295 | 398 | GACCTCACGAACUGTGCTGAUGG | R295 | 667 | GACTGAGAATGGCUACCTCTCGATAUG |
| F296 | 399 | GAGGATTCGAGAAGUGACAGGCTAUG | R296 | 668 | GAGGCTGGAGUTGGTGTTATAGTUCAA |
| F297 | 400 | GAATTGGTAGCUGGTGATGTTCCUC | R297 | 669 | GACTCACACATCUTGAGCCCATTTTTAUC |
| F298 | 401 | GACAGCTAATUCATCTGGAGAUCAAACCC | R298 | 670 | GACTGAGAGGGUGTCACATACCAUG |
| F299 | 402 | GATGTCAGTTCCCUCCTTTTCTATTTTCUC | R299 | 671 | GATTCATACCGACAUGTAGGACCTUGT |
| F300 | 403 | GATGGGCACGGUAATGCUGCT | R300 | 672 | GAACTTCUCACACCGCTGTGUT |
| F301 | 404 | GACTCUGCGGTGGTUGGCAT | R301 | 673 | GACCTCACCUCCGTTTCCUGCA |
| F302 | 405 | GACTCCACCUCCAGGAACTTACUC | R302 | 674 | GATCUGGCCCCCTUAGGAGGA |
| F303 | 406 | GAGGGATCTTGUGAAATGTCATCTGACUC | R303 | 675 | GATCATCCTCUCCCCATAGAAAAGUCC |
| F304 | 407 | GATCAACCCTGTUTTTCTCCCTCTTATUG | R304 | 676 | GATCTCUGCCATCATTUCCGGAAAG |
| F305 | 408 | GACTGUACAGCATGAAGUGCAAGAAC | R305 | 677 | GATGCAAGGAAUGCGATGAAGUAGA |
| F306 | 409 | GACCATTAACAUGGCCTACCAGAGUT | R306 | 678 | GAGTCGCUAACACGTGTGTGTUC |
| F307 | 410 | GATTGCCUAGACAGCACCGTAAUG | R307 | 679 | GATGGCTAAACTUGACCTTTTTACTCUGC |
| F308 | 411 | GAGACGCAGUTTCTTCTTCTCAUCG | R308 | 680 | GATTCCTCAGCAUCGACCTUGC |
| F309 | 412 | GATATCGAGTGTGUGCATATGTGTATGTUG | R309 | 681 | GAAATCTATATACTUCCTTACCTGGGATUGGA |
| F310 | 413 | GAAGGGAAAAUGACAAAGAACAGCUCA | R310 | 682 | GAACATGCTGAGAUCAGCCAAATUC |
| F311 | 414 | GAGGCCTGCUTTTGGAGTCCUAT | R311 | 683 | GAGCAGUGAAAAGAGTCUCAAACACAA |
| F312 | 415 | GAGAAGAGCCUCCACCATCUCCA | R312 | 684 | GACCCACAGGCCUTCTUCGAG |
| F313 | 416 | GACACACAUGCCATCATTCUAGGAAG | R313 | 685 | GACTGGUATGAGAAACUGCACGAGT |
| F314 | 417 | GAGAGGTTTUCCAGCACTCTGACAUAT | R314 | 686 | GAAATACCAATCUATTGTGGGCTCUGG |
| F315 | 418 | GATCTTCTCTG UTTCAGGGCAUGAAC | R315 | 687 | GACCTCCTTCUGCATGGTATTCTTUCT |
| F316 | 419 | GAAGATTCUGCCGAACCAATGGAUC | R316 | 688 | GAATTAAAGCAGUGCTCATGATUGGG |
| F317 | 420 | GATATGACTUGTCACAATGUCACCACAT | R317 | 689 | GACGGGACUCGAGTGATGATUGG |
| F318 | 421 | GAGTGCCCTATUACCTCAATCATCCUG | R318 | 690 | GACTTCACCTTUAACACCTCCAGUCC |
| F319 | 422 | GATCAGTTACTACCUGAAAATGACACTTUGT | R319 | 691 | GACTCCTCTAGCUATCTTAATGACTUGGAC |
| F320 | 423 | GATAAAGACCTUCTTCCGTGTGUCCT | R320 | 692 | GACTGCTTTCATUCATAGGGAAATACAUAAGAAA |
| F321 | 424 | GATACATTTATTTUGAGAAACTTGAGAGAACUTCA | R321 | 693 | GATTCAATATTTTAAAUAGTCTGGCCUAAACGGT |
| F322 | 425 | GAAGATGGTGATAGAUCTTTAAGAGAATTGCUT | R322 | 694 | GATGATTTCCAGUATTAATTGGCAAUAAGAGAAT |
| F323 | 426 | GAAGCTTTTGATAAGAGUTAGGAAATCACTAGUC | R323 | 695 | GAATGAAAGCUAAAACATAAGATGAAUGGGAAAA |
| F324 | 427 | GAAGGAUAAAAACCAGCATTATTTATTUGAGCA | R324 | 696 | GAATTATTTCTTACCACUTTTCCTTTCTCCUGT |
| F325 | 428 | GACTGACCCAUAATCTTGCACCATTUACC | R325 | 697 | GAATTGTGAGATUAACAGCAGGGAUACC |
| F326 | 429 | GATTTGAAATGAAUGTTCACGACAAAUGC | R326 | 698 | GAGCTTCATTGTCTUGATAAAATTTATGGTATCUT |
| F327 | 430 | GAACAACCAAAACAAUACACACAGAGATTUT | R327 | 699 | GACCAGCTCTTUCATATCTTAACATTUAGCAACA |
| F328 | 431 | GAACGGAGGGUCATGTGTATATTAAGUAAG | R328 | 700 | GAGCCAAAACATTTUGTCCCTTTCTATAATTUG |
| F329 | 432 | GAGAATTAAGUGTGTACTACUCCCAAGAGAAAA | R329 | 701 | GATGGACTTCAAGUGATCACTTGUG |
| F330 | 433 | GATATAGGATGAGUAGCTCCAAATTAATGAAUGT | R330 | 702 | GAAGCCTGUGGTGCTTTTUGCG |
| F331 | 434 | GAGCTGTAGAAUAGTCAAGAGGAATUGCA | R331 | 703 | GAAAGTCAAACUACACTCAGAACCUGAAT |
| F332 | 435 | GACTCAGTGCUCTAAATCCAGAGCUG | R332 | 704 | GAGCAAAGGCCAAAGAUAAAATGCTTACUG |
| F333 | 436 | GAATGAAATATUGTCAACTCTCTUAGGCAAAAT | R333 | 705 | GAAAGCTACAGAAUGTGAACAGTCTTCTUAAA |
| F334 | 437 | GATACTTUGCAAAGCTGAATUAGACAGCA | R334 | 706 | GAGAGGTAGAUGCTGTAATTGCTGAUACAA |
| F335 | 438 | GAACATGAGCAUCACATTTTCCTUGG | R335 | 707 | GACAAACACCTCCUGATAAATTGGCTTUG |
| F336 | 439 | GATGCCTTATGAAUATATTCACGCUGACT | R336 | 708 | GACCCTACUCCAAGGAGCUCAGG |
| F337 | 440 | GAACCAGGUAAGCACCGAAGUC | R337 | 709 | GACCCAGTUACCATAACTACTCUGAGAAAA |
| F338 | 441 | GAACCAAGCCGCUGGTUCA | R338 | 710 | GACTTGCAGAGCUATCCCCUAAAGC |
| F339 | 442 | GAAGACCCCUTTAACTCAAGACUGC | R339 | 711 | GAGCTGCACCGAGUCGTAGUC |
| F340 | 443 | GAGCGAGGAUATCTGGAAGAAATUCGA | R340 | 712 | GAGTCGUTGTCUCCCCGAAGG |
| F341 | 444 | GAUTCUCCACGGCCGACCA | R341 | 713 | GAATACAGTCCUGGATGATGATGTTTTUGA |
| F342 | 445 | GAAAGTCCCUCAAAAATAGGAGGUGCT | R342 | 714 | GAGGACAAGAAAAGUGCAACTUCCCA |
| F343 | 446 | GAGACAGAUCAGCAACAACCGAAAAUG | R343 | 715 | GATTTCATTGUTTTCCAACUCCGGGAT |
| F344 | 447 | GATTTGTCCAGAGACCUTTCTAACGTAUT | R344 | 716 | GATTUCCACAGAAACAACAUCGATTTCTTC |
| F345 | 448 | GATTTCUGAAGAGGACTTGTUGCG | R345 | 717 | GATGCATTUGATCATGCATTUGAAACAAGT |
| F346 | 449 | GAGATTTTUCAGTTAATAATAUCCCCCGAGCT | R346 | 718 | GAAGTCTGUGCGCGCTUGC |
| F347 | 450 | GAAATCGCCUCCGGAUCCC | R347 | 719 | GAGTGCGCACGUCGCAAUC |
| F348 | 451 | GAGTCATTCCTTCUTTTTAAAATGGTGCTUAAGT | R348 | 720 | GAGGATGTAUACAAAAGGCGGATGUG |
| F349 | 452 | GAGGTCCCCCACCUCTCTTTUG | R349 | 721 | GAGCCAGAGAGUCCCTTUCACC |
| F350 | 453 | GACTCUCCAGGAAGGCTCACAUC | R350 | 722 | GATGCCACTCUTTGGGTTGAGUT |
| F351 | 454 | GATGGCATUGCCTTGTCCTUG | R351 | 723 | GATTTCAAACUGGAGGCTTAUCACCAA |
| F352 | 455 | GAGGCAGAAAACCAAAACAUTGGCTUA | R352 | 724 | GAATACAAGCATGAAAAUCAAAACATATCTTCUGC |
| F353 | 456 | GAAATTGTTCCTCAAGUTTGTTTAAGGACTUAAAA | R353 | 725 | GAGTAAATGGTAGCTUTTATCATAATCACCAGUC |
| F354 | 457 | GAAGTGGTATCAUCCCCATTTAATAGCUG | R354 | 726 | GATCCATTCAAGACUTTAGCAGGTGGUA |
| F355 | 458 | GAACAAATACAAAACUGTCCACATCTATGTUG | R355 | 727 | GAGTTACTCTCATGUGAGAACCATTTGAAUGA |
| F356 | 459 | GAAACAAACCATAGCUATAATGAAGAACTTGCUA | R356 | 728 | GAGTTTTTCTTATCUCTTAAAATGTTTCTGCUACA |
| F257 | 460 | GAAATAGTTGATCAUACTTTGTAACAGAAUCACA | R257 | 729 | GAAAGGUACAAGTUAAGGCACACAGAAG |
| F358 | 461 | GACTCATCTCCCTUTAATTTTGGCACATTAUT | R358 | 730 | GATGGATCTUGGCACAATGAUAACAGG |
| F359 | 462 | GAAACTATCTUCTTTGGACTTCUGAAGAGAC | R359 | 731 | GATGCTATAGTACCAGUACCTTTTAAGGTUCA |
| F360 | 463 | GAAGTAAATAATGGTTUCTCCTTCTCTTACTTUG | R360 | 732 | GACCGTAAGGUGGCCTACTTUGC |
| F361 | 464 | GATTCCTGGUGGCATTCAAUAAAGCA | R361 | 733 | GACAGCGTTTTCUTGTATTCCTGTATTUAGC |
| F362 | 465 | GAAGGAGCAACUTAGGGATCUGGT | R362 | 734 | GAGGAACTGTGAAUGAACTTGTAGGUG |
| F363 | 466 | GAACCCCTAAUCTGGTCAACCUG | R363 | 735 | GACCUGACCAGGGCGUCAAA |
| F364 | 467 | GAAGAAATAGAAAACUACAGGACGTTAUCCAG | R364 | 736 | GAAGTTCATCTUCGAAGCTCAAATTUCAG |
| F365 | 468 | GATCCGCTTTCUAAAATGTCAGTTGUC | R365 | 737 | GATACAACAAAAUGTTTGACTTCAUGCAGGT |

**TABLE C**

| **Primer Name** | **SEQ ID** | **Primer Sequence** | **Primer Name** | **SEQ ID** | **Primer Sequence** |
|---|---|---|---|---|---|
| F366 | 738 | AAAACTCAGTAUCAACAACTACCGGUAC | R366 | 870 | TCCCGTGGCUGGTAAATCTGAAAUA |
| F367 | 739 | CTCAGAAAUGGAAAAAACCTGCAGUAAA | R367 | 871 | CCAAAACATGAAUGTTCTCAACAAGUG |
| F368 | 740 | GTTCCCTCUGCGTGTTCTCAUAA | R368 | 872 | ATTCCTGCACUAATGTGTTCATUCT |
| F369 | 741 | AAGAACCTGUGTGAAAGTATCUAGCAC | R369 | 873 | GUCCAAAGCGAGCAAGAGAAUCC |
| F370 | 742 | AUAAACCAAACCCAUGCAAAAGGAC | R370 | 874 | AGTTCCAGUAGTCCTACTTUGACACT |
| F371 | 743 | GCATTGAUGGAAGGAAGCAAAUACA | R371 | 875 | AGAGCACGTUCTTCTGCTGTAUG |
| F372 | 744 | CCAGCTTCAUAGACAAAGGTTCUCT | R372 | 876 | AGTTGAATATCTGTTUTTCAACAAGTACATTUT |
| F373 | 745 | GTGGTTTCTUCCATTGACCACAUC | R373 | 877 | GCCTGGCCUGAATGCCTUAAA |
| F374 | 746 | CAAAUGGGCAGGACTCTUAGGT | R374 | 878 | CAATTTCAACACAAGCUAAACTAGUAGGAT |
| F375 | 747 | GUGAGGAAACTUCTGCAGAGGT | R375 | 879 | TCAACAAAAGUGCCAGTAGUCATTTC |
| F376 | 748 | GGAAGCAGGGAAGCUCTTCAUC | R376 | 880 | CTGTTTTUAGCAAAAGCGUCCAGA |
| F377 | 749 | TGGTTUGAAGAACTTTCTUCAGAAGCT | R377 | 881 | AGTCAGCCCUTGCTCTTUGAAT |
| F378 | 750 | AGGGAGACUGUGTGTAATATTTGCG | R378 | 882 | TTGGCCAUACAAAGTGAUAAAGGACTT |
| F379 | 751 | GCCAGTATUGAAGAATGTTGAAGAUCAAAA | R379 | 883 | TTTGCAGGGUGAAGAGCTAGUC |
| F380 | 752 | GCCAAAAGGAAGUCTGTTUCCAC | R380 | 884 | TGTACAAAUGGGACTAACAGGUGGA |
| F381 | 753 | CATGCCACACAUTCTCTTTTTACAUGT | R381 | 885 | AGCATACCAAGTCUACTGAATAAACACTUT |
| F382 | 754 | GTAGAGUGCTACACTGUCCAACA | R382 | 886 | CCTGGAGTCGAUTGATTAGAGCCUA |
| F383 | 755 | CTCTGAGAAAGAAUGAAATGGAGTUGGA | R383 | 887 | AATGTGTTATGUGGCTCCATTATUAGCT |
| F384 | 756 | AAACAAATTTUCCAGCGCTTCUGA | R384 | 888 | GCATTTTTACCUACGATATTCCTCCAAUG |
| F385 | 757 | AGCAATAAAAGTGUATAAATGCCTGTAUGC | R385 | 889 | ACCAGTAAAAAUAAAGAACCAGGAGUGG |
| F386 | 758 | TCAACAAGTTGACUAAATCTCGTACTTUCT | R386 | 890 | TTATAGAGGTTTUCTACTGTTGCUGCAT |
| F387 | 759 | CATTCTTACAUAAAGGACACTGUGAAGG | R387 | 891 | GCAGTTGTGAGAUTATCTTTTCAUGGC |
| F388 | 760 | CCCTTACAGAUGGAGTCTTTUGGC | R388 | 892 | CATCATTCACCCUTGGCACAGUAA |
| F389 | 761 | AAAGACCTTTUGGTAACTCAGACUCAG | R389 | 893 | AAAUATTTTCTAGGAATUGCGGGAGGA |
| F390 | 762 | ACATTCACUGAAAATUGTAAAGCCTATAATTG | R390 | 894 | CAGGUAAUCGGCTCTAAAGAAACATG |
| F391 | 763 | GGTTGTGCTTTTUAAATTTCAATTTTATTTTUGCT | R391 | 895 | CAGAGAGATUCGAGGCAGAGUG |
| F392 | 764 | GGATGUCACAACCGUGTGG | R392 | 896 | AGTAGUGGATTTUGCTTCTCTGATATAAACT |
| F393 | 765 | AGTGAAAACUAAAATGGAUCAAGCAGATG | R393 | 897 | GCTCTUAGCCAAAATATUAGCATAAAAATCAG |
| F394 | 766 | AAACTAGTTTTUGCCAGTTTTTUAAAATAACCT | R394 | 898 | AAAAAGCATUGTTTTTAATCAUACCTGACTT |
| F395 | 767 | TTTTTACCCCCAGUGGTATGUGG | R395 | 899 | GGTACAGAUTTGTAAATCUCAGGGCAA |
| F396 | 768 | GAAAACACAAAUCAAAGAGAAGCUGCA | R396 | 900 | GAGAUCACGGGUGACAGAGC |
| F397 | 769 | ATATTTAGUAGCCAGGACAGUAGAAGGA | R397 | 901 | ACCTACCTGAUACCCCAGAUCCC |
| F398 | 770 | AAATATTTCAGUGTCCGTUCACACACAA | R398 | 902 | TCCAGATTGAUCTTGGGAGTGUAAAAA |
| F399 | 771 | GCAGAUGCAAGGTATTCTGUAAAGG | R399 | 903 | GTGTGCTAGAGGUAACTCATGATAAUGG |
| F400 | 772 | ACCTACATAAAACUCTTTCCAGAATGTUGT | R400 | 904 | GAAAGGGUCAACAAAAGAATGUCCAT |
| F401 | 773 | CCCTTTCTGTUGAAGCTGTCAATUC | R401 | 905 | GAAAGTTCCCCAAUTGAAAGTUGCAG |
| F402 | 774 | AGAUGGTATGTUGCCAACACGAG | R402 | 906 | AACTTTGTAATUCAACATTCATCGTTGUGT |
| F403 | 775 | GATGTTTCCGUCAAATCGTGUGG | R403 | 907 | TAGATGATAGGUGGTACATGCACAGUT |
| F404 | 776 | GTAGAACTATCUGCAGACACCUCAAAC | R404 | 908 | ACCUGAATTATCACTAUCAGAACAAAGCA |
| F405 | 777 | CCAGAACCACCAUCTTTCAGTAATTUG | R405 | 909 | GAACAGUACCCGTTCCCTUGA |
| F406 | 778 | ATCATAAAATGTUGGAGCTAGGTCCUT | R406 | 910 | CTTGAGGACCUGCGAAAUCCAG |
| F407 | 779 | TATGATGGAAGGGUAGCTGTUAGAAGG | R407 | 911 | TGGAAAGCTTCUCAAAGTATTTCATTTUCT |
| F408 | 780 | GGTTAAAATGTCACUCTGAGAGGAUAGC | R408 | 912 | GCAGCGTTTAUAGTCTGCTTTTACAUC |
| F409 | 781 | GGAAATTTGUAAAATGTGCUCCCCAAA | R409 | 913 | AACGGGCTUGGAAGAAAATAAUCAAG |
| F410 | 782 | AATTCCTTGTCACUCAGACCAACUC | R410 | 914 | TCTGCTAGCUTGTTTTCTUCACAGT |
| F411 | 783 | ACTAAGGTGAUGTTCCTGAGAUGC | R411 | 915 | AACAATATACCTUCTCAGTCTACUAGGCAT |
| F412 | 784 | ACTTTCCUTAATGTCATTTUCAGCAAAACT | R412 | 916 | CAGATAACTUAGAACAGCCTAUGGGAAG |
| F413 | 785 | CAGTCTGAACUACTTCTTCATATTCTUGCT | R413 | 917 | GGCCAAAATUGAATGCTATGCTUAGAT |
| F414 | 786 | CTAGTTCTGCUTGAATGTTTTCAUCACT | R414 | 918 | AGCACAATUAGCCGTAATAACATUAGAGAA |
| F415 | 787 | TGGAATGTTCTCAUTTCCCATTTCTCUT | R415 | 919 | TGGACTCATTACUCCAAATAAACAUGGA |
| F416 | 788 | GTTTCGTUGCCTCTGAACUGAGAT | R416 | 920 | GTCTAATATCAAGCCUGTACAGACAGUT |
| F417 | 789 | CCTTGATTTTCTUCCTTTTGTTCACATUCA | R417 | 921 | TGCAGAATACAUTCAAGGTTUCAAAGC |
| F418 | 790 | TTTCTATGCTUGTTTCCCGACUGT | R418 | 922 | AATAAATGTGUGAGTCAGTGUGCAG |
| F419 | 791 | GAUGAAAGCTCCTUCACCACAGAA | R419 | 923 | AAGCCTTCAUCCGGAGAGTGUA |
| F420 | 792 | CCTAGAGTGCUAACTTCCAGUAACG | R420 | 924 | TAATGCUGAAGACCCCAAAGATCUC |
| F421 | 793 | CTTGGAAGGCUAGGATTGACAAATUCT | R421 | 925 | GCCAAAUGAACAGACAAGUAAAAGACA |
| F422 | 794 | TTGTTACTCTTCUTGGCTCCAGTUG | R422 | 926 | GCAAATTGATAGUTGTTCTAGCAGUGAA |
| F423 | 795 | TTAGGTGGGCUTAGATTTCTACUGACT | R423 | 927 | CAGCAGTAUAAGCAATATGGAACUCGAA |
| F424 | 796 | TGCTTATAGGTUCAGCTTTCGTTTUG | R424 | 928 | GGAGCAGAATGGUCAAGTGATGAAUA |
| F425 | 797 | CCACTATGUAAGACAAAGGCUGGT | R425 | 929 | TTTTATAACTAGATTTUCCTTCTCTCCATUCC |
| F426 | 798 | TCCGTTTGGTUAGTTCCCTGATTTAUC | R426 | 930 | AGAGCGTCCCCUCACAAATAAAUT |
| F427 | 799 | GTATTATCTGUGGCTCAGTAACAAAUGC | R427 | 931 | GAAAGAGTTCACUCCAAATCAGUAGAGA |
| F428 | 800 | TTAAAGCCTCAUGAGGATCACUGG | R428 | 932 | GGTTCTGAUGACTCACATGAUGGG |
| F429 | 801 | AGTTCATCACTUCTGGAAAACCACUC | R429 | 933 | CCCTGTGUGAGAGAAAAGAATGGAAUAA |
| F430 | 802 | GGGATCAGCATUCAGATCTACCTTTUT | R430 | 934 | AGGCUGAATTCTGTAAUAAAAGCAAACA |
| F431 | 803 | TTCAGCCTTTTCUACATTCATTCTGUCT | R431 | 935 | AGGGTAGTTC UGTTTCAAACTUG CAT |
| F432 | 804 | TACCCTGAUACTTTTCTGGAUGCCT | R432 | 936 | TGTATATTTTCAGCUGCTTGTGAATTTUCT |
| F433 | 805 | GAATCCAAACUGATTTCATCCCUGGT | R433 | 937 | GACAGTTCTGCAUACATGTAACTAGUGT |
| F434 | 806 | AGCTGCCUACCACAAATACAAATTAUG | R434 | 938 | GCGGAUACAACCUCAAAAGACG |
| F435 | 807 | CAGAGTTCUCACAGTTCCAAGGTUAG | R435 | 939 | TGUCAAGTTTCTCTUCAGGAGGAAAAG |
| F436 | 808 | GAAGAAGAAGAAAACAAAUGGTTTUACCAAG | R436 | 940 | AAGGAAAATAACUCTCCTGAACATCUAAAAGA |
| F437 | 809 | ATCACCACGTCAUAGAAAGTAATTGUGC | R437 | 941 | TGTTGAAGAGCUATTGAAAATCATTTGUGC |
| F438 | 810 | CATTCAAACTTACTUGCAAAATATGTGGUC | R438 | 942 | ACAGCTCAAAGUTGAACTTATTCACUAAGA |
| F439 | 811 | GCATAGGAGATAAUCATAGGAATCCCAAAUT | R439 | 943 | ATGTTTTTCTAATGUGTTAAAGTTCATUGGA |
| F440 | 812 | AGTTGTAGTTGTUGAATTCAGTATCAUCCT | R440 | 944 | GCCAGTTTCCAUATGATCCATCTAUAGT |
| F441 | 813 | TGTGCCTTTCCUAAGGAATTTGCTAAUA | R441 | 945 | AGAAACCTTAACCAUACTGCCGTATAUG |
| F442 | 814 | AAAAGATAAUGGAAAGGGAUGACACAGC | R442 | 946 | GCCACTTTTUGGGTATCTGCACUA |
| F443 | 815 | CTGTTAAGGCCCAGUTAGATCCUC | R443 | 947 | TTCAAGAGGUGTACAGGCAUCAG |
| F444 | 816 | AGGCAGTTCUAGAAGAATGAAAACTCUT | R444 | 948 | GGTCAGGAAAGAAUCCAAGTTTGGTAUA |
| F445 | 817 | TGTACCTAGCAUTCTGCCTCAUAC | R445 | 949 | CCTCAGCTCCUAGACTTTCAGAAATAUG |
| F446 | 818 | TAGACCTTTTCCUCTGCCCTTAUCA | R446 | 950 | AAACTCCATCUCAAACAAACAAACAAATUAAT |
| F447 | 819 | CACATUATTACAGTGGAUGGAGAAGACA | R447 | 951 | CCTCCTGAATTTUAGTGAATAAGGCTUCT |
| F448 | 820 | CTTCTTTGGGUGTTTTATGCTTGGUT | R448 | 952 | GCAAAGCACGAACUTGCUGT |
| F449 | 821 | GCAGAGCTUTATGAAGCAGUGAAGA | R449 | 953 | GTGAUGGCCAGAGAGTCUAAAACAG |
| F450 | 822 | TCTTAAATGGUCACAGGGTTATTUCAGT | R450 | 954 | TGACATCCCTUGATAAACCTTGTUCC |
| F451 | 823 | TTCCATTGCATCUTTCTCATCTTTCUC | R451 | 955 | TTTTTGTCGCUGCTAACTGTATGTUA |
| F452 | 824 | TTCACTUCAGCAAATTTTTAGAUCCAGAC | R452 | 956 | GCTCCAACTAAUCATAAGAGATTTUAAAAGAC |
| F453 | 825 | TGCCCCTTUCGTCTATTTGUCAG | R453 | 957 | AAGTAAGAAGGCCUGATTTGGATUCT |
| F454 | 826 | GGAGATTTTTCTGUGTTTTCTGCTAGUC | R454 | 958 | GCTATTTCCTUGATACTGGACTGUCAAA |
| F455 | 827 | TGACAUACTTTGCAAUGAAGCAGAAAA | R455 | 959 | ATTCCTTGAGUTTACATTAACTUACCAGAAG |
| F456 | 828 | GGATCCTGATAUGTCTTGGTCAAGTUC | R456 | 960 | ATGACAATTATCAACCUCATCTGCTCUT |
| F457 | 829 | GGCACCAAAUACGAAACACCCAUA | R457 | 961 | TAAATTGUTTTTCTCCTGTUGAACCAGACA |
| F458 | 830 | ATATCTGTCAGTGAAUCCACTAGGACUG | R458 | 962 | CCTGCTTATTTUTCTCACATTCTUCCG |
| F459 | 831 | TGAAGAAGCAUCUGAAACTGTATTTCCT | R459 | 963 | GGTTUAGAGACTTTCUCAAAGGCTTAGAT |
| F460 | 832 | GGACTACTACTATAUGTGCATTGAGAGTTUT | R460 | 964 | GTGTTTUCACTGTCTGUCACAGAAG |
| F461 | 833 | TGGCTTATAAAATATUAATGTGCTTCTGTTUT | R461 | 965 | AAAACTATCTTCUTCAGAGGTATCUACAACT |
| F462 | 834 | GGTAAAAAUGCCTATTGGAUCCAAAGAG | R462 | 966 | GTGACGUACTGGGTTTTUAGCAAG |
| F463 | 835 | AATCTACAAAAAGUAAGAACUAGCAAGACT | R463 | 967 | GGCTTCTGATTUGCTACATTTGAAUCT |
| F464 | 836 | AAGTGACAAAATCUCCAAGGAAGTTGUA | R464 | 968 | AGGTCTTTTTCTGAAAUATTTTGGTCACAUG |
| F465 | 837 | GAATTCTTUGCCACGTATTTCUAGCC | R465 | 969 | AGATATTGCCUGCTTTACUGCAAGAA |
| F466 | 838 | GGCTTCTTCAUTTCAGGGTAUCAAAAA | R466 | 970 | TGTATTTCCAGUCCACTTUCAGAGG |
| F467 | 839 | AATACAUACTGTTTGCUCACAGAAGGAG | R467 | 971 | TTGTTTTCTTTTUCAAAGTGGATATUAAACCT |
| F468 | 840 | ACCGAAAGACCAAAAAUCAGAACTAATUAAC | R468 | 972 | CAGAAGGAATCGUCATCTATAAAACTATAUGT |
| F469 | 841 | TCACAGAAUGATTCUGAAGAACCAACT | R469 | 973 | CTGTAGTTTTTCCTUATTACATTTTGCTTCUT |
| F470 | 842 | ATTACCCCAGAAGCUGATTCTCUGT | R470 | 974 | TGGGATTGAAAGUCAGTATCACTGTAUT |
| F471 | 843 | TATATGATCATGAAAAUGCCAGCACUCT | R471 | 975 | GTTACCTTTGAGCUTGTCTGACATTTUG |
| F472 | 844 | TTCCCATGGAAAAGAAUCAAGATGTAUG | R472 | 976 | TTGGATTACTCTUAGATTTGTGTTTTGGTUG |
| F473 | 845 | ACTGTCAATCCAGACUCTGAAGAACUT | R473 | 977 | ATGGTAGAGTTCUTGAAAATGGGTUC |
| F474 | 846 | CAGGUGAUAAACAAGCAACCCAAGT | R474 | 978 | GTATTTTATCTATATUCAAGGAGATGTCCGAUT |
| F475 | 847 | TGGCATTAGAUAATCAAAAGAAACUGAGC | R475 | 979 | GCCTTTTGGCUAGGTGTTAAATTAUGG |
| F476 | 848 | GAATCAGGAAGUCAGTTTGAATTTACUCAG | R476 | 980 | GTCTACCUGACCAATCGAUGGG |
| F477 | 849 | GCCTGTUGAAAAATGACTGUAACAAAAG | R477 | 981 | AGCTTTTUGCAGAGCTTCAGUAGA |
| F478 | 850 | TGAAGATAACAAAUATACTGCUGCCAGT | R478 | 982 | GGCCAGATAATTUAAGACATATGTTGUGC |
| F479 | 851 | AGGAGGGAAACACUCAGATUAAAGAAGA | R479 | 983 | GCTCCGTTTUAGTAGCAGTTAACUGT |
| F480 | 852 | TTTCAGACTGCAAGUGGGAAAAATAUT | R480 | 984 | GTCTGTTTCCUCATAACTTAGAATGUCCAT |
| F481 | 853 | TCTTCTTACAACUCCCTATACATTCTCAUT | R481 | 985 | TCACTGTGCGAAGACUTTTATGTCUA |
| F482 | 854 | CCAGTTGGTACUGGAAATCAACTAGUG | R482 | 986 | TTTCACTTTGUCCAAAGATTCCTTUGC |
| F483 | 855 | AAAAGAGCAAGGUACTAGTGAAAUCACC | R483 | 987 | AGAATTCTGCAUTTCTTTACACTTUGGG |
| F484 | 856 | AAAAACCTTGTTUCTATTGAGACTGUGG | R484 | 988 | GGACTGATTUGTGTAACAAGTUGCAG |
| F485 | 857 | AATTCAGCCTUAGCTTTTTACACAAGUT | R485 | 989 | TCATACAAATAATUTCCTACATAATCUGCAGT |
| F486 | 858 | TGACAAAAAUCATCTCUCCGAAAAACAA | R486 | 990 | CAATACTGGCUCAATACCAGAAUCAAGT |
| F487 | 859 | AATAATTTTGAGGUAGGGCCACCUG | R487 | 991 | AACCTGCCAUAATTTTCGTTUGGC |
| F488 | 860 | TCATAACTCTCUAGATAATGATGAATGUAGCA | R488 | 992 | GAAGTTTCCAAACUAACATCACAAGGUG |
| F489 | 861 | GTATAGGGAAGCUTCATAAGTCAGTCUC | R489 | 993 | ATTTCAGAAAACACUTGTCTTGCGUT |
| F490 | 862 | AGAAGATAGUACCAAGCAAGTCTTTUCC | R490 | 994 | ACCACATTATAUGAAAAGCCTTTTUGGG |
| F491 | 863 | TAGTACAGCAAGUGGAAAGCAAGUT | R491 | 995 | GGUTTCTCTTAUCAACACGAGGAAGT |
| F492 | 864 | CAGGCTTCACCUAAAAACGTAAAAAUG | R492 | 996 | CTGTCAGTTCAUCATCTTCCAUAAAAGC |
| F493 | 865 | ATGAAATAUTTCTTTTUAGGAGAACCCTCAAT | R493 | 997 | TATACCAUACCTAUAGAGGGAGAACAGATAT |
| F494 | 866 | ATATATTUTCTCCCCATUGCAGCACAA | R494 | 998 | GCTTGAAGATTTUTCCAAAGTCAGAUGT |
| F495 | 867 | AGGACATCCAUTTTATCAAGTTTCUGCT | R495 | 999 | GTTTTGCTTTUGTCTGTTTTCCUCCAA |
| F496 | 868 | TGGCTCTGATGAUAGTAAAAATAAGATTAAUGA | R496 | 1000 | AGGCAAAAATTCAUCACACAAATTGUCA |
| F497 | 869 | GCTGTATACGUATGGCGTTTCUAAACAT | R497 | 1001 | TCATTGGAGGGUATGAGCCAUCC |

**TABLE D**

| **Primer Name** | **SEQ ID** | **Primer Sequence** | **Primer Name** | **SEQ ID** | **Primer Sequence (target of PrimerC)** |
|---|---|---|---|---|---|
| F498 | 1746 | GAGUGUGCGUGGCUCUCA | R498 | 1275 | UGCCAUCAUUCUUGAGGAGGAAG |
| F499 | 1002 | ACAACUGCAGCAAAGACUGGT | R499 | 1276 | UGCAAUCCCUGCCCCGGUT |
| F500 | 1003 | AGUUAAUUUUGGUUACAUCCCUCUCUGC | R500 | 1277 | GGAUUGCAGGCUCACCCCAAT |
| F501 | 1004 | AUCGAUCUGUUAGAAACCUCUCCAG | R501 | 1278 | CUGGAUUUCCUCAUGGAAGCC |
| F502 | 1005 | GGACUCUGUAGGCUGCAGT | R502 | 1279 | AAAUCCAGUUCGUCCUGUUCA |
| F503 | 1006 | UGAGGCAGUCUUUACUCACCUG | R503 | 1280 | GAAACUGCCUCUUGACCUGUCC |
| F504 | 1007 | ACAAGCAAAGUCUCUAUGGUGAUUAUGT | R504 | 1281 | AGGACAGUCAUGUUGCCAGUAUUAAAAT |
| F505 | 1008 | CAACUACCAUCCAGCAACAGAAAAT | R505 | 1282 | CUUCCAUGACUUUGGCAAUCUGG |
| F506 | 1009 | GACAGAUGAGAGAAAUGCACUUAGAAGA | R506 | 1283 | GAACAUGUCCUAUUUGAAUUUUCCGACUT |
| F507 | 1010 | AGGAAUGUGUUUCUCCAUACAGGUC | R507 | 1284 | GACACAAAGACUGGCUUACAUUUUGAT |
| F508 | 1011 | CUUCAAGCAGUGAGAAUACGUCCA | R508 | 1285 | AGGCUGACCACUUCUACUCUGT |
| F509 | 1012 | AGGGUCCAGGUUCUUCCAGA | R509 | 1286 | GCACUCAGGCUGGAUGAACAA |
| F510 | 1013 | GAUAGUUUUGAGAGUCGUUCGAUUGC | R510 | 1287 | UGUCCAGGGCUAUCUGGAAGAUC |
| F511 | 1014 | CUCCACCACCUCCUCAAACAG | R511 | 1288 | GCAGCAUUUACUGCAGCUUG |
| F512 | 1015 | AUCAGCCAGGCACAAAGC | R512 | 1289 | UGACAGAAGUACAUCUGCUAAACAUGA |
| F513 | 1016 | CAUCUUUGUCAUCAGCUGAAGAUGAAAT | R513 | 1290 | CUCACAGGAUCUUCAGCUGACC |
| F514 | 1017 | GCCUAAAGAAUCAAAUGAAAACCAAGAGA | R514 | 1291 | ACUUUGUUGGCAUGGCAGAAAT |
| F515 | 1018 | GUGACCCGGAGCACUUCC | R515 | 1292 | GCCGUGGUGCUGACCAT |
| F516 | 1019 | CCACAUUACAUACUUACCAUGCCACT | R516 | 1293 | GUGAUGAUUGGGAGAUUCCUGAUG |
| F517 | 1020 | AUGGGACCCACUCCAUCG | R517 | 1294 | GCUCUGAUAGGAAAAUGAGAUCUACUGUT |
| F518 | 1021 | CCCUUCUAAGGACCCCCUCUUC | R518 | 1295 | CUCCAGCAGGGCUUCGAT |
| F519 | 1022 | CUCUGCCGGGCUUUGAUCUT | R519 | 1296 | GGACUUUGCAACUUCAACAAAACUC |
| F520 | 1023 | UACUACCGCCUCACACGCT | R520 | 1297 | CUAGGUGUCUCCCCCUGUAAG |
| F521 | 1024 | UUCCCUCUCUCCUUCUGCCUC | R521 | 1298 | AGGUUCAGGCCUUGCACT |
| F522 | 1025 | CCAGCAGAAGACAAAAAGACAAACA | R522 | 1299 | CCAGCCCAGGAAGCAAAGAG |
| F523 | 1026 | GGAGAGGGAGGAGAGCUAACT | R523 | 1300 | UUAAAACUGGUCUCGCUCUCCC |
| F524 | 1027 | AGAAGCUGUGCAUUUACACCGA | R524 | 1301 | GAAAGCGGGAAUCGCAGAAA |
| F525 | 1028 | UUUUGCUGAUGCUAUGCUCUCCAC | R525 | 1302 | GGAAGACCUCUUCUUCGCACUT |
| F526 | 1029 | CUGAUCCGCAAGCAUGCUC | R526 | 1303 | CAAAAGAGCUCCCCCAUCUCC |
| F527 | 1030 | UGAGCUCGCUCACUUGUGAUG | R527 | 1304 | AGAAGAGACAUCUGGACUUAGCCAA |
| F528 | 1031 | CAGGAUCCAAAUUCGUUCUGUGC | R528 | 1305 | AUCAUCGACGGUGGGUACAUG |
| F529 | 1032 | UACUAACAACUCUGGUCUGGACCAT | R529 | 1306 | CGUUGGUCCUGACGGUACUG |
| F530 | 1033 | AUGCUAUUUGGACAAUAAACUCACCUUG | R530 | 1307 | CCAUUCUGAGGACUGCUGGUUUAUA |
| F531 | 1034 | CUCCUUACCUCAUACAGUGCAGAAA | R531 | 1308 | CCAGGACCAUCAUCCUACUGUAA |
| F532 | 1035 | CCCUGAGUGCAGCUUCGAUC | R532 | 1309 | GAGUGUUUGCUCCUCACUCUUC |
| F533 | 1036 | CGAUCAUGGAUGGCGGGUAC | R533 | 1310 | CUUCAGGUUUCCUUCUCUCAUGGUT |
| F534 | 1037 | UUUAUGAAUGGAGAGGCUGCUG | R534 | 1311 | GGAUGAGCUCACAGAGCUGC |
| F535 | 1038 | ACCUCUCACCCUUAUAAGUCUUCUGA | R535 | 1312 | UCAAAAAGCAUGCCCAGACCUUT |
| F536 | 1039 | ACUUGAGCUUCCCUAGGACCA | R536 | 1313 | ACUUUGAGACUUCUGCUUUGCUC |
| F537 | 1040 | GGGAGACAAUACGUGUCGGG | R537 | 1314 | AUCGAAAAACUGUGCAUCUACACC |
| F538 | 1041 | AAGACAGGUAGCGAUCCAGGUAG | R538 | 1315 | AGACCCAGCAGUGACUGT |
| F539 | 1042 | CUAGGUGCCCAUGUCCAUCUG | R539 | 1316 | ACAAGUAUAAUGAGCACCCCUUCT |
| F540 | 1043 | GAUGUCCAAUGUACCUGAGGCAA | R540 | 1317 | GUGGACACACCUGUAUUCCUGAG |
| F541 | 1044 | CAGCAGAAAGAGGACUCAGAAUAGAAAAUC | R541 | 1318 | CCGACCAGUUGGGCAAAAUC |
| F542 | 1045 | AGAGAUUCGCUUGUGUGGGUUAAA | R542 | 1319 | GCAGAAGUCUGUUUUCUUCAUGGUT |
| F543 | 1046 | ACCAGGGUUACCUUGAUCUCC | R543 | 1320 | AGGCCAUGUUGGGUUAAAGG |
| F544 | 1047 | ACUUCUCAAUUGCUACGGGCAAUC | R544 | 1321 | AGAAUCUACAGCUACCAGAUGGCA |
| F545 | 1048 | AGAUCUCGGUGAACGAUGCAAT | R545 | 1322 | CCUAGUUUCCAGUGCAUCUGUACC |
| F546 | 1049 | UAUGUGGACUGCAGAAGAACUUCG | R546 | 1323 | GGUCCCCAUCCAUUCUUCCUAUUC |
| F547 | 1050 | UGUGGUUUAUGAACAAGCGAUUUGG | R547 | 1324 | UGUGGAGUGUUGGCUGUAUCUUUG |
| F548 | 1051 | GCUCAUAUCGAGAGGUAGCCAUUC | R548 | 1325 | CUCUGUAAGCGACUUUUGGUGAUAG |
| F549 | 1052 | GCAGACGAGCUUGACAUCAGAAA | R549 | 1326 | GCCCAACCAAUUGAGAAGUUUGUAA |
| F550 | 1053 | AAGACUUCGGGUGCUCUGUAC | R550 | 1327 | UUAGUCAGGAGUCUAAGCCAACAG |
| F551 | 1054 | GGGACAGACUGUCAUUCAAAAUAGGA | R551 | 1328 | CUUGCCCGCAUCUAUAGUUUCCA |
| F552 | 1055 | UGAAGAAAGUCCAGACCUCGGA | R552 | 1329 | AACUUCCGUUUUGAGUGUUUACUGAUUT |
| F553 | 1056 | GUCGACUGCCUGAUAAGACAUGA | R553 | 1330 | UUACUUGGAUAAAGUUCCAGAGCCT |
| F554 | 1057 | UAGUUUGGUUUCUCUGUCUGUUCGUG | R554 | 1331 | GCUUAUUGCCACCUACUUAACCUCT |
| F555 | 1058 | AAGCCUCCAUCGCUACCCT | R555 | 1332 | CUUCAGCCCUGCAGGGAAA |
| F556 | 1059 | UCAGGCGCCAAGUAGGT | R556 | 1333 | GGCAAGUUCAACAUUAUUCCCUUUUGUA |
| F557 | 1060 | AAAGCGGCUGUUAGUCACUGG | R557 | 1334 | UCUUCCUCAGGAUUGCCUUUACC |
| F558 | 1061 | UAAAGAUCAUGUCUCGGCUCAAGGA | R558 | 1335 | CAUACAGAGAGGGUCAUCAGUGAUAC |
| F559 | 1062 | GUGCACAGGUUAUUCUGAUUUCCC | R559 | 1336 | GAAAGUCUCCCACAAAGUAACCC |
| F560 | 1063 | AGAAGGGCUAGGCCAAUUGAC | R560 | 1337 | AUAGUCAUAGCCGGGCCACA |
| F561 | 1064 | GUCAGCCUGAACAUAACAUCCUUG | R561 | 1338 | CCAGUUUAUUGUAUUUGCAUAGCACA |
| F562 | 1065 | GGGACCUCCGGUCAGAAAAC | R562 | 1339 | GGACCCAUUAGAACCAACUCCAUAAA |
| F563 | 1066 | CUCCCAACCAAGCUCUCUUGA | R563 | 1340 | UACCUUAUACACCGUGCCGAA |
| F564 | 1067 | CCCAGAAGGUGAGAAAGUUAAAAUUCC | R564 | 1341 | CCACACAGCAAAGCAGAAACUC |
| F565 | 1068 | AGGGCAUGAACUACUUGGAGG | R565 | 1342 | UUCUUUCUCUUCCGCACCCA |
| F566 | 1069 | GCCUCUCCCUCCCUCCAGGAA | R566 | 1343 | GUGAGGCAGAUGCCCAGCA |
| F567 | 1070 | UGCCUCACCUCCACCGT | R567 | 1344 | CCAAUAUUGUCUUUGUGUUCCCGGACA |
| F568 | 1071 | AAGUGUAAGAAGUGCGAAGGG | R568 | 1345 | UGUGUUCCUUUGGAGGUGGC |
| F569 | 1072 | GCCUUUUUAACUGGUAGAGAUUGGUG | R569 | 1346 | GAUCCAGAGGAGGAGUAUGUGUGA |
| F570 | 1073 | UCAUCACCUUCCUUUCAUGCUCUC | R570 | 1347 | UCUUCCUCCAUCUCAUAGCUGUCG |
| F571 | 1074 | UCCUACGUGGUGUGUGUCUGAA | R571 | 1348 | CGUCCUGUUUUCAGGCCAAG |
| F572 | 1075 | UGAUCAUCGAAUUCUCCAAAAUGGC | R572 | 1349 | AUUAGAGGGACUCUUCCCAAUGGA |
| F573 | 1076 | GAUGAGAUGUGGUACAAGCAUUCCA | R573 | 1350 | CCACGGUGGAAUUGUUGCUG |
| F574 | 1077 | CCCCUACAGCAUUGUUAAGAAAGUAUUT | R574 | 1351 | AUACCAGGCUAGUAUAGAUGCUUAGGG |
| F575 | 1078 | CUGGGACUAGCAUGCUGACC | R575 | 1352 | CAGACACCAACUCCCGGAAUC |
| F576 | 1079 | CUGCCUGUCUCUGGUUCUGT | R576 | 1353 | CAGAACUCUCUCCCCAGCAG |
| F577 | 1080 | ACUUGGAGUGAGUUUGGAUGGG | R577 | 1354 | CAGCUUCAUGUCUGUGCCG |
| F578 | 1081 | UCCUGAUCUCCUUAGACAACUACCUT | R578 | 1355 | UCACACCGCUGUGUUCCAUC |
| F579 | 1082 | UGUUCCUAUUUCAGCCCCACUC | R579 | 1356 | GUUGUGAGCGAUGAGCACGUA |
| F580 | 1083 | GGAAAGGGUCCUCUGAUCAUUGC | R580 | 1357 | AAAAUCGUGUCCUGGUAGCAGAG |
| F581 | 1084 | CGAGGGCCGGUAUACAUUCG | R581 | 1358 | CCCACCAAAAUGAGAAAACUGUGUT |
| F582 | 1085 | GAAUGUGAAAAUUCCAGUGGCCAT | R582 | 1359 | UGUCCUCCUAGCAGGAGAGG |
| F583 | 1086 | AUACCCUCUCAGCGUACCCUT | R583 | 1360 | CCGUGGAUGUCAGGCAGAUG |
| F584 | 1087 | AGCGCUUUGUGGUCAUCCA | R584 | 1361 | AUACUGGACUCAUCUCUCCUUCCC |
| F585 | 1088 | AAACUAGCCCUCAAUCCCUGAC | R585 | 1362 | AAAGACCACCCCCAAGACC |
| F586 | 1089 | CACAGUUGGAGGACUUCCUCUUC | R586 | 1363 | AUAACUCCACACAUCACUCUGGT |
| F587 | 1090 | CUACAUGGGUGCUUCCCAUUCC | R587 | 1364 | UUGACAUGGUUGGGACUCUUGAC |
| F588 | 1091 | GUCCUCGUGGCCAUGAAUGAA | R588 | 1365 | UGGCAAACUUCCCAUCGUAGAC |
| F589 | 1092 | CCCAAUCCCCACACCAAGUAUC | R589 | 1366 | GUUGAUCAUUGUUCCUUCCCCUCA |
| F590 | 1093 | AUGUUCCUCCCUCAUCUCUAAUGGT | R590 | 1367 | CCAUCUUGUCAGGAGGACAGG |
| F591 | 1094 | UGGACUCGAGCAACAUUGAUGG | R591 | 1368 | GGCAGGAUCUCUAACCCAUUGAG |
| F592 | 1095 | GCUGAAGUACCAGACCUGCUA | R592 | 1369 | CUCAGCAGGUAACUCACACUUG |
| F593 | 1096 | GGAUUUGACCCUCCAUGAUCAGG | R593 | 1370 | CUUCCCUGGGUGCUCCAT |
| F594 | 1097 | UCACUCUCUCUCUGCGCAUUC | R594 | 1371 | GUGGAUAUGGUCCUUCUCUUCC |
| F595 | 1098 | CAUGAAGUGCAAGAACGUGGT | R595 | 1372 | GGCUAGUGGGCGCAUGUAG |
| F596 | 1099 | GCGGAUCAGAGCCUCAAAC | R596 | 1373 | AUCAAAGUCCAGCACCAGCA |
| F597 | 1100 | ACUGUCCUGUUUUGAUAUCCCAGAUUUT | R597 | 1374 | GGGAAUUGCAUUCACACGUUAACA |
| F598 | 1101 | CUGUCUCAAUAUCCCAAACCCUAAG | R598 | 1375 | UUUGUUUUGUUUUUCUGUUUCUCCCUCUG |
| F599 | 1102 | UAUUAGUAUGCCCCUGCAACGUG | R599 | 1376 | GAGGGUUGUUAGUGGAGCAUAUGA |
| F600 | 1103 | CAACCCUCCUGCCAUCAUAUUGA | R600 | 1377 | UGAGACAGGCCAGUGUUUACAUG |
| F601 | 1104 | CAACCAUGACAAGAUUUUCCCUUACC | R601 | 1378 | GAGACUGGAGAAUGUAUACACACCUT |
| F602 | 1105 | UGCCUGUGGAGGAACUUUUCA | R602 | 1379 | CGACAUCUCCUCGGGCUT |
| F603 | 1106 | CUUCCUCUCGCCCAUCACA | R603 | 1380 | CGUAGAGCUCCGGGUGUC |
| F604 | 1107 | AGUGCCUCCUCUCCCAUCUT | R604 | 1381 | CUACCCAGGGCCACUGUUUT |
| F605 | 1108 | CACUCCUUGCUUCUCAGAUGAAACC | R605 | 1382 | GGGACAUUCACCACAUCGACUA |
| F606 | 1109 | CAGGUACUCCCGCAGGUUG | R606 | 1383 | UGGCCUCUUCUCCUGUGC |
| F607 | 1110 | CACGCAUACGGUUUGGUUUGG | R607 | 1384 | CUUCUUCUUCCCAUAGAUGCUCUCC |
| F608 | 1111 | CUAGAAGCUCUCUAUCCCACACCT | R608 | 1385 | GAGGCAUUAUUUGACCGGAUCUAC |
| F609 | 1112 | CAAGGAAUGCCUUCAAAAAGUUGGG | R609 | 1386 | CUGAGUAUGAGCUUCCCGAAGAC |
| F610 | 1113 | AGAUGAUGAUCUCCAGGUACAGG | R610 | 1387 | CCUGCUAACACCCUGUUCG |
| F611 | 1114 | CGGCACUGCAUGCAAUUUCUT | R611 | 1388 | CUGCCUGUCUCUCUUGGCUUT |
| F612 | 1115 | CCAUUUAUAGCUGAGUCUCCAUCCUG | R612 | 1389 | UAUGAACUUCCAGAGGACCCAAAAUG |
| F613 | 1116 | CCCAGUUGUGGGUACCUUUAGAT | R613 | 1390 | GGAAAAGAACGGCAGUAAAUACGG |
| F614 | 1117 | CUUUCAAACGAGUCAAGCAAGAAUGG | R614 | 1391 | AAUACGGGUCCAUCAAUCACACG |
| F615 | 1118 | ACCACACUUUCCAUAAUGAGGCT | R615 | 1392 | CAGUACUUGGUAUUCUGUGCUAGGA |
| F616 | 1119 | CUUUUCCAUCUUUUCUGUGUUGGUC | R616 | 1393 | GGAAGCUGUCCAUCAGUAUACAUUC |
| F617 | 1120 | CAGACAAAUCCCAAAACAAACCUGA | R617 | 1394 | GGCCCUCCUUCAGUUUAGUUGAG |
| F618 | 1121 | GUAGCUACAGGACUCAGAUACGUG | R618 | 1395 | GGUGGAGGCGAUAGUGGAUAG |
| F619 | 1122 | GUAUUUGGGCGAAUGCAGUUUUUC | R619 | 1396 | AGAUGGAGAUGAUGAAGAUGAUUGGG |
| F620 | 1123 | CCAGAGAAAAGAGAGUUACUCACACA | R620 | 1397 | GUCAAGUGGAUGGCUCCAGAAG |
| F621 | 1124 | ACUGUGUUACUGCCAUCGACUUAC | R621 | 1398 | CCAGAAAUGUUUUGGUAACAGAAAACAA |
| F622 | 1125 | GGUAUUCUCGGAGGUUGCCUUT | R622 | 1399 | AUUCUCUCUUUAGGGAGCUUCUCUUC |
| F623 | 1126 | CUUGGUCGUGUUCUUCAUUCGG | R623 | 1400 | UGGAAGAGAAAAGGAGAUUACAGCUUC |
| F624 | 1127 | ACCACUGUGGAGGCAUUUG | R624 | 1401 | AUUGGUCUCUCAUUCUCCCAUCC |
| F625 | 1128 | AGUGAAGAUCUCCCACAUUAACACC | R625 | 1402 | GUUUAGGUUUUGGCAACGUGGAT |
| F626 | 1129 | CUUGCCCAAAGCAACCUUCUC | R626 | 1403 | UCACCAGAUGCUAUGUGCUAAUCC |
| F627 | 1130 | AUUGGUUGCGGCCAUCUCT | R627 | 1404 | UCCUACCUGUGUCCACACC |
| F628 | 1131 | ACCAAUUUCAUAGGCGUGGC | R628 | 1405 | GGCAUGGGACAGAGUCGUT |
| F629 | 1132 | GCCUAUCGCUCUGCUCUCUC | R629 | 1406 | UUGUGCAAGGAGAGAACCUCUA |
| F630 | 1133 | UAACCCAGCGACGAACUUUCC | R630 | 1407 | CCUAUCCCAGAACUGGAGACAGAAA |
| F631 | 1134 | GCCCCUGAGCGUCAUCUG | R631 | 1408 | UGUACACCUUGCAGUGGAACT |
| F632 | 1135 | CUGGUGGAGGCUGACGA | R632 | 1409 | AGCCCAGGCCUUUCUUGG |
| F633 | 1136 | ACAACGUGAUGAAGAUCGCAGA | R633 | 1410 | ACUGGCAUGACCCCCAC |
| F634 | 1137 | GGGAGAUCUUCACGCUGGG | R634 | 1411 | UGCCACUCACAGGUCGT |
| F635 | 1138 | GUCUGAGGAGCCCGUGT | R635 | 1412 | GCAGAAACUCCCGCAGGT |
| F636 | 1139 | UCCUCGGAGCAGUGAGGG | R636 | 1413 | ACUCCAGAUACUGCAUGCCT |
| F637 | 1140 | AGCCUCUCCACGCUCCCUC | R637 | 1414 | ACUCCCGCAGGUUUCCC |
| F638 | 1141 | CUCACAUUGCCCCUGACAACAUA | R638 | 1415 | ACGGGAAAGUGGUGAAGAUAUGUG |
| F639 | 1142 | GUGUCCUUUCAGGAUGGUGGAUG | R639 | 1416 | AGAAACAUGAUGGAUGUCACGUUCUC |
| F640 | 1143 | GGUGACAUUUUCAAAGCAGUGUAUCC | R640 | 1417 | UGUUAACCUUGCAGAAUGGUCGAT |
| F641 | 1144 | GGGUAUUCGAUGAUCCCUGUGG | R641 | 1418 | AUGACUUGGACCGCGUAGC |
| F642 | 1145 | CCUCCCCACCAGCAUGUUT | R642 | 1419 | GCAUCCUACCGUUGAAGCACT |
| F643 | 1146 | GGCUUUGGUGAGAUCCAUUGAC | R643 | 1420 | CACCUGGAACUUGGUCUCAAAGAUT |
| F644 | 1147 | GCAUGUACUGGUCCCGCAT | R644 | 1421 | AUUCCUACCGGAAGCAGGT |
| F645 | 1148 | UGGUUCUGGAUCAGCUGGAUG | R645 | 1422 | AUGACGGAAUAUAAGCUGGUGGT |
| R646 | 1149 | UGCCAACAUGACUUACUUGAUCC | R646 | 1423 | AAAAUAUCCCCCGGCUUGUGAG |
| F647 | 1150 | GGACUAGGCGUGGGAUGUUUUT | R647 | 1424 | GAAGAAGAUGUGGAAAAGUCCCAAUG |
| F648 | 1151 | AGUGGAUCCCCUCUCCACC | R648 | 1425 | GUCCCUGGCUGGACCAA |
| F649 | 1152 | GAGGUUUUCCAGCACUCUGACAUAT | R649 | 1426 | CACACAUUGGAGCAUGCCAUUC |
| F650 | 1153 | CGGUUGAAUGUAAGGCUUACAACG | R650 | 1427 | AGCCUAAACAUCCCCUUAAAUUGGAUT |
| F651 | 1154 | GAACGGGAAGCCCUCAUGUC | R651 | 1428 | CGGCUUUACCUCCAAUGGUG |
| F652 | 1155 | CCUUACUCAUGGUCGGAUCACAAAG | R652 | 1429 | GCAGAGAAUGGGUACUCACGUUUC |
| F653 | 1156 | CCCUUUCUCCCCACAGAAAC | R653 | 1430 | UCAGCCUGUUUCUGGGAAACT |
| F654 | 1157 | GUAGAGCAAAUCCAUCCCCACA | R654 | 1431 | UGGAGAGAGAACAAAUAAAUGGUUACCUG |
| F655 | 1158 | UGUGCUUUUAGGGCCCACC | R655 | 1432 | GAUUCUUAUAAAGUGCAGCUUCUGCAT |
| F656 | 1159 | UCUGUUCAAUUUUGUUGAGCUUCUGAAUT | R656 | 1433 | CAGACGUCACUUUCAAACGUGUAT |
| F657 | 1160 | UCAGUGUUACUUACCUGUCUUGUCUUT | R657 | 1434 | CAGGCUCAGGACUUAGCAAGAA |
| F658 | 1161 | UGAAUUAGCUGUAUCGUCAAGGCA | R658 | 1435 | UAAGGCCUGCUGAAAAUGACUGAA |
| F659 | 1162 | UGUUUCUCCCUUCUCAGGAUUCCUA | R659 | 1436 | AGUCCUCAUGUACUGGUCCCT |
| F660 | 1163 | AAACCCGCAAUCCGGAAC | R660 | 1437 | CUGAUCUCGCCAUCGCUGUA |
| F661 | 1164 | CCCUCCAACAUCCUAGUCAACUC | R661 | 1438 | GUUCAUACCGACAUGUAGGACCUT |
| F662 | 1165 | GCUAGAGCUUGAUGAGCAGCAG | R662 | 1439 | UCAAAGUCGUCAUCCUUCAGUUC |
| F663 | 1166 | CCAUGGAGUCGAUGAGCUGG | R663 | 1440 | CCUCCAGAUGUGAAGCCCT |
| F664 | 1167 | GCCCAGCUCUGAGAUCCUUUC | R664 | 1441 | GCUGGAGGAGCUGGAACUT |
| F665 | 1168 | CAUUUCUGACAACUGAACUGCUCUC | R665 | 1442 | UAAACAGGAGCACGAGGAUGC |
| F666 | 1169 | UUACCAGCUUGUUCAUGUCUGGAUUC | R666 | 1443 | UAUUCAUCACGGCGCGCUT |
| F667 | 1170 | ACUGAGCUUGUUGGAAUAAGGAUGUT | R667 | 1444 | GAGUCCAGGAGAAAAUUCACAUGAGG |
| F668 | 1171 | UUGUAAGUGCCCGAAGUGUAAG | R668 | 1445 | ACAACCCACUGAGGUAUAUGUAUAGGUAUT |
| F669 | 1172 | UACGCAGUGCUAACCAAGUUCUUUC | R669 | 1446 | AGCACAGUGAAUUUUCUUGCCAUC |
| F670 | 1173 | CAGUCAAGGUUGCUGAUUUUGGUC | R670 | 1447 | GGUGGUAAACUUUUGAGUUUGCAGA |
| F671 | 1174 | UAUGGAUGUUGCCAAGCUGUAUUCUG | R671 | 1448 | GGGAAGGAGUGGUACAACAGAT |
| F672 | 1175 | GGUGGUCCUACCAUACAUGAAACAT | R672 | 1449 | ACAGCUAGUUUGCCAGUUAGUAAGC |
| F673 | 1176 | GCAAGCAAAAAGUUUGUCCACAGAG | R673 | 1450 | CACUUAAUUUGGAUUGUGGCACAGA |
| F674 | 1177 | ACAUCUCUCACCUCAUCUGUCCT | R674 | 1451 | CUCUUGUCAUCAGCUCCCAGA |
| F675 | 1178 | UCCCUGUAGUCCCGGAUGAG | R675 | 1452 | GCGCCAGCAUCCAGAGAUAC |
| F676 | 1179 | AAUUGUUGCCAUUUCAGGGUUUCUG | R676 | 1453 | GAGCGUGUGAUGCAGCUCUT |
| F677 | 1180 | CUCACCUAUCUCCCAGGCCUAAAAUA | R677 | 1454 | GUUUGACCGAAGAACCAAUUAUACCC |
| F678 | 1181 | ACAAACGAGAUGCCUCUUCCAG | R678 | 1455 | GAUGCUUCUCUCCUUCUUCUCUUGG |
| F679 | 1182 | GGCUGUCGUGGUAGACUUAGA | R679 | 1456 | UUCCCCAACCCACAUUUCCUUUAUAG |
| F680 | 1183 | CUGAGUGUAUCCUGGAGGUUGUUG | R680 | 1457 | CCAAAACCCUCCUGAUGUACACG |
| F681 | 1184 | GCUUGGUUCUGAUGUUUGUAGUGUAG | R681 | 1458 | GUCACAGCUCCAGUGUCUGUC |
| F682 | 1185 | UCCUUGUUGGUGUCCAUUUUCUUGT | R682 | 1459 | GAGAUCCAGGCUACCUGGUAUGAG |
| F683 | 1186 | ACAUGCCAUCAUUCUAGGAAGCUC | R683 | 1460 | AAGGACGACCCAGAGCUGAT |
| F684 | 1187 | CAGGACCCGCUUCUCUGAAAG | R684 | 1461 | AAAUUAAAAGGCAAGUGGACUUCGG |
| F685 | 1188 | AAGACCCCUUUAACUCAAGACUGC | R685 | 1462 | CUGUUGGUGAAGCUAACGUUGAG |
| F686 | 1189 | UGCUCCAUGAGGAGACACC | R686 | 1463 | AAAAUGGGAAAGGUAUCCAGCC |
| F687 | 1190 | AAUGUAACCUUGCUAAAGGAGUGAUUUCT | R687 | 1464 | CCACAGAAACAACAUCGAUUUCUUCC |
| F688 | 1191 | AACUGGCAAAUAUAUCAUUGAGCCAAAUC | R688 | 1465 | ACAGGGAUGGUGGUGGUT |
| F689 | 1192 | GGUGUGAAAUGACUGAGUACAAACUG | R689 | 1466 | UUCUGGAUUAGCUGGAUUGUCAGUG |
| F690 | 1193 | AUGGUGAAACCUGUUUGUUGGACAT | R690 | 1467 | GGCAAAUACACAGAGGAAGCCUT |
| F691 | 1194 | CCUGCUCAUGGUCUUUGAGUAUAUG | R691 | 1468 | AUGUCUAUAGGGAAGGGAAGACG |
| F692 | 1195 | GCCACACGCAACUGUCUAG | R692 | 1469 | GUCGGUGCUGUAGAUAUCCCT |
| F693 | 1196 | GACAAUCCUUGCUUACCUGAGGAAC | R693 | 1470 | ACAUUGUCAAGUUCUAUGGAGUGUGC |
| F694 | 1197 | GCUCGGGAUCCAUAUGUGGUAAT | R694 | 1471 | CUGGCUGAAGGUGGGUUUGAUT |
| F695 | 1198 | GGCCCUAUACUUAGGCCCUUUT | R695 | 1472 | AAGUCACACGGCCCUCC |
| F696 | 1199 | AACUCACGGUGGCUGCT | R696 | 1473 | UUGUUCUCAUUGGCUUCAAAGAUCUUUA |
| F697 | 1200 | UGUCCUGGUCAUUUAUAGAAACCGA | R697 | 1474 | UCUCUUGGAAACUCCCAUCUUGAG |
| F698 | 1201 | UCUCAUGUCUGAACUGAAGAUAAUGACT | R698 | 1475 | UGAGCCCACCUGACUUGG |
| F699 | 1202 | UUGGUAGCUCAGCUGGACUGAUAT | R699 | 1476 | ACAUGAGAGCUUGUUUUUCACUGG |
| F700 | 1203 | AUGAAGCAGGCUGAUACUACACAG | R700 | 1477 | AGAGUGAUCUCUGGAUGUCGGAAUA |
| F701 | 1204 | UUGUGAAGAUCUGUGACUUUGGC | R701 | 1478 | ACCAGUGAGGGAAGUGAGGAC |
| F702 | 1205 | CCUUUGGGUUAUAAAUAGUGCACUCAGA | R702 | 1479 | UAAGCAUCAGCAUUUGACUUUACCUUAT |
| F703 | 1206 | GGGAAGAAAAGUGUUUUGAAAUGUGUT | R703 | 1480 | CAAACAAGUUUAUAUUUCCCCAUGCCA |
| F704 | 1207 | UGGCUUUGAAUCUUUGGCCAGUA | R704 | 1481 | GAUUUGAUCCAGUAACACCAAUAGGGUT |
| F705 | 1208 | GUCGAGGCAAUGGAAAAGCUC | R705 | 1482 | AAACACAAACUAGAGUCACACACCUT |
| F706 | 1209 | AGAACAGCUCAAAGCAAUUUCUACA | R706 | 1483 | AGCACUUACCUGUGACUCCAUAG |
| F707 | 1210 | AGCAAGAGGCUUUGGAGUAUUUCAUG | R707 | 1484 | UUGUGUGGAAGAUCCAAUCCAUUUUUG |
| F708 | 1211 | UGUUCAUGCUGUGUAUGUAAUAGAAUGUT | R708 | 1485 | AACCAUAUCAAAUUCACACACUGGC |
| F709 | 1212 | CUGGAAUGCCAGAACUACAAUCUUUUGA | R709 | 1486 | CUCUUGCUCAGUUUUAUCUAAGGCUAG |
| F710 | 1213 | CUCAAGAAGCAGAAAGGGAAGAAUUUUT | R710 | 1487 | CAUACCAAUUUCUCGAUUGAGGAUCUUUUC |
| F711 | 1214 | UGACAGCCAUCAUCAAAGAGAUCG | R711 | 1488 | CCGCAGAAAUGGAUACAGGUC |
| F712 | 1215 | GGGAUUUCCUGCAGAAAGACUUGA | R712 | 1489 | AGAAAAUCAAAGCAUUCUUACCUUACUACA |
| F713 | 1216 | AAGGCACAAGAGGCCCUAG | R713 | 1490 | UCCAGGAAGAGGAAAGGAAAAACAT |
| F714 | 1217 | ACCAAUGGCUAAGUGAAGAUGACAAT | R714 | 1491 | AUUUGCCCCGAUGUAAUAAAUAUGCAC |
| F715 | 1218 | AGGUUAUCUUUUUACCACAGUUGCAC | R715 | 1492 | GUCAAGAUCUUCACAAAAGGGUUUGA |
| F716 | 1219 | UUUUCUGUCCACCAGGGAGUAACUA | R716 | 1493 | GCCACUGGUCUAUAAUCCAGAUGA |
| F717 | 1220 | GACAAGUUCAUGUACUUUGAGUUCCC | R717 | 1494 | GCAUCUUGUUCUGUUUGUGGAAGAA |
| F718 | 1221 | AGCAAAUAAAGACAAAGCCAACCG | R718 | 1495 | UCAACAACCCCCACAAAAUGUUT |
| F719 | 1222 | AGUUUAAGAUGAGUCAUAUUUGUGGGUUUT | R719 | 1496 | UGGAUUUGACGGCUCCUCUAC |
| F720 | 1223 | CUGACCAUGUGGACAUUAGGUGUG | R720 | 1497 | UUAACACCUCCAGUCCCUCAUCUG |
| F721 | 1224 | CCUUCCCUCGGGAAAAACUGAC | R721 | 1498 | UAAGAUGUCCACUGCUGUUCCUUCAUA |
| F722 | 1225 | GUUUGGUUUUGUAGGUCUUGUGGAUG | R722 | 1499 | CUUCAGCCAAGGCAGCAAUG |
| F723 | 1226 | GAGGUGGCCUGAUCUUCACAA | R723 | 1500 | GAUAUGGAUUCACACAGACACUAUCACA |
| F724 | 1227 | CGCUUAUGCAUACUCAGGAUGAGUT | R724 | 1501 | CAAGGUGUUUCUUUGAUGCUCUGT |
| F725 | 1228 | UAAGGUUCCUUCAAGCUGCCCUA | R725 | 1502 | CCUGUGGACAUUGGAGAGUUGAC |
| F726 | 1229 | CAUGGGAGGAUGUUCUUUCCCAUUT | R726 | 1503 | GAACCUUAAAUGUCUCUCCUACCUGA |
| F727 | 1230 | UUUUCUUCCUAAGGUUGCACAUAGG | R727 | 1504 | AAGGCACCUGACCCAAACA |
| F728 | 1231 | AUUUUUGGCUUCCUGGCCUUT | R728 | 1505 | GCACAUAGUCCCGGAAGCUG |
| F729 | 1232 | GGAAAGCCUCACCUGUCUACG | R729 | 1506 | UUCUUGAUCUCACAGUCAGGGAUG |
| F730 | 1233 | UCAAGAAUCGCCCGAGCC | R730 | 1507 | AUGAGCAGCGUGGCCUT |
| F731 | 1234 | UUGGUUCGGACAGACAACCC | R731 | 1508 | UAGCUGUGCAUGUCCUGGUG |
| F732 | 1235 | CUCUGCACAGCUCCAAUGAGAC | R732 | 1509 | UAGGUGAGGACCACAAACCAAAC |
| F733 | 1236 | GCUACAAGAACUACCGAUACCGT | R733 | 1510 | UGGUCUUCACUCACCUCGGAT |
| F734 | 1237 | CUCGGAGAGGAGCCAUACUG | R734 | 1511 | UUCCUCCAGAAGCUUGAACUCT |
| F735 | 1238 | UAUAAUGACAGUUAACCCUGCCAGGA | R735 | 1512 | CCCAAGCCUGGGACCUCUAUUAT |
| F736 | 1239 | AGGAAGAGCACAGUCACUUUGA | R736 | 1513 | CAUGCUGGACCUUCUGCAC |
| F737 | 1240 | CAGUGGAGCGAAUUCCUUUGGA | R737 | 1514 | AGACUGCUAAGGCAUAGGAAUUUUCG |
| F738 | 1241 | UUGGGUCGUUGGGCAUUCC | R738 | 1515 | UUUGACUCUGUCUCCUCUUGUCUUCT |
| F739 | 1242 | CAGUUCACAGUGCAGCGAAAA | R739 | 1516 | GAGAUGAAGCAAACAACAGUGGAG |
| F740 | 1243 | AAAUAUCUACACACAGGUCUACAAGGUC | R740 | 1517 | AUUUCAUGCAAACUAGAUAACUACCUGUAA |
| F741 | 1244 | CAUCCGGGCUUUACGCAAAUAA | R741 | 1518 | UGGAGUUUGUCUGCUGAAUGAACC |
| F742 | 1245 | GCCUCCUUCAGGAAUUCAAUCUUCT | R742 | 1519 | AGCUCACAGAAAUGUCUGCUAUACUG |
| F743 | 1246 | AUGAGUUCUGGGCACUGGG | R743 | 1520 | AUGAGGAGUGUGUACUCUUGCAUC |
| F744 | 1247 | GAUGCAAACUCUUGCACAAAUGCT | R744 | 1521 | GCCAAGAGUUACGGGAUUCCAT |
| F745 | 1248 | GAACCCCGAGGGCAAAUACAG | R745 | 1522 | AGGAUGCCUGACCAGUUAGAGG |
| F746 | 1249 | CAGUUCGUGGGCUUGUUUUGUAUC | R746 | 1523 | AAAAGACUCGGAUGAUGUACCUAUGG |
| F747 | 1250 | UUAAAGCUGGCUAUGGCACCUG | R747 | 1524 | CACUCACCCUGGAUGUCUUCG |
| F748 | 1251 | CAUCUCUCACCAUCCCAAGG | R748 | 1525 | CACCGUAGCUCCAGACAUCA |
| F749 | 1252 | AUACGCAGCCUGUACCCA | R749 | 1526 | AAGGAGAAGAGGACAGCGG |
| F750 | 1253 | CACCUCUCUCAAGAGUUUGGAUGG | R750 | 1527 | CCUGCACUUCUAGGCACUUACUAA |
| F751 | 1254 | AGAUUGCGAGAGAGCUGCAT | R751 | 1528 | GGCACUUGCACAGAGAUGAT |
| F752 | 1255 | CUGUGCUGCAUUUCAGAGAACG | R752 | 1529 | AUUUGAUGACAUGUGGGUGGUUG |
| F753 | 1256 | AAGACCCAAGCUGCCUGAC | R753 | 1530 | GGAGCCGUAUUUGGCGT |
| F754 | 1257 | GCUAUUUUUCCUCACAGCUCGUUC | R754 | 1531 | CCUCUUCACGUAGGAAUCCUCUUC |
| F755 | 1258 | CUCCUUCCUAGAGAGUUAGAGUAACUUC | R755 | 1532 | AUCACUUUGCGUGGUGUAGAUAUGAT |
| F756 | 1259 | GAGCCUGUUUUGUGUCUACUGUUCUA | R756 | 1533 | AGGACUCUGAAGAUGUACCUAUGGT |
| F757 | 1260 | CUCUUGCAGCAGCCAGACT | R757 | 1534 | ACAGUUUCCAUAGGUCUGAAAAUGUUT |
| F758 | 1261 | CCAUGGGACUGACUUUCUGC | R758 | 1535 | AGCCCAACCCUUGUCCUUAC |
| F759 | 1262 | GCUGAGGACCUGGUCCUCT | R759 | 1536 | GGGACAGCAUCAAAUCAUCCAUUG |
| F760 | 1263 | CUGCACCAGCAGCUCCUA | R760 | 1537 | CCAGACGGAAACCGUAGCUG |
| F761 | 1264 | CCCGGACGAUAUUGAACAAUGGT | R761 | 1538 | GGAGCAGCCUCUGGCAUT |
| F762 | 1265 | AGCCUCACCACGAGCUG | R762 | 1539 | GGCAAGGAAAGGUGAUAAAAGUGAAUCT |
| F763 | 1266 | CACCUUUCCUUGCCUCUUUCCUA | R763 | 1540 | ACUUGAUAAGAGGUCCCAAGACUUAGT |
| F764 | 1267 | CUCAAGGAUGCCCAGGCT | R764 | 1541 | CCUAUGGCUUUCCAACCUAGGA |
| F765 | 1268 | CCUCCCUGCUUCUGUCUCCUA | R765 | 1542 | CCCUUCUGUCUUGAACAUGAGUUUT |
| F766 | 1269 | CCAGUUGCAAACCAGACCUC | R766 | 1543 | UGUGGAGUAUUUGGAUGACAGAAACA |
| F767 | 1270 | ACUCCACACGCAAAUUUCCUUC | R767 | 1544 | AGGCCUCUGAUUCCUCACUGAT |
| F768 | 1271 | AGGGUGGCAAGUGGCUC | R768 | 1545 | CCUAGGUUGGCUCUGACUGT |
| F769 | 1272 | GAGGCUCCCCUUUCUUGC | R769 | 1546 | UGCCUCUUGCUUCUCUUUUCCT |
| F770 | 1273 | CUGUGACUGCUUGUAGAUGGC | R770 | 1547 | UUCCUACAGUACUCCCCUGC |
| F771 | 1274 | CUGUCGUCUCUCCAGCCC | R771 | 1548 | AGUCACAGCACAUGACGGA |
| | | | | | |
| F772 | 1549 | GGAGGAGGCGAUGGCUACUA | R772a | 1574 | CUGCAGUUAGAGGUUGGUGACA |
| | | | R772b | 1575 | CCCGCCAAGCACGUAUACU |
| F773 | 1550 | GGAGACCUACAAACUGAAGUGCAA | R773a | 1576 | CCGGAAGAGGAGUAGCUGAC |
| | | | R773b | 1577 | CUCCUAGAGUUUUUCCAAGAACCAAGU |
| F774 | 1551 | CCAUGCAGAAUGCCACCAAGUA | R774 | 1578 | AUUUGCAGCUACUACUCUGAACUGAA |
| F775 | 1552 | CAGGCACUCCUUGGAGCAA | R775 | 1579 | UCAGUGGGAUUGUAACAACCAGAAAU |
| F776 | 1553 | CUGUUUGAAAUGAGCAGGCACU | R776 | 1580 | GCACUGUCACCCCUUCCUUG |
| F777 | 1554 | ACUGGAGGACCCGUCUUCU | | | |
| F778 | 1555 | AGACCUUAAGGGAACAGCUCUCAU | | | |
| F779 | 1556 | GUGGAGUCAUGCUUAUAUGGAGCAAA | R779 | 1581 | GCUCCAUCUGCAUGGCUUG |
| F780 | 1557 | GACAGAAAAAUAAUUCUGUGGGAUCAU | | | |
| F781 | 1558 | UCCUGAAAGAGAAAUAGAGGUUCCUGAU | | | |
| F782 | 1559 | GGTGGCCATAGGAACGCA | | | |
| F783 | 1560 | UGGAUGCAGAAACCAGAGAUCUAGU | | | |
| F784 | 1561 | CUGGUCCCCAGACAACAAGUAU | | | |
| F785 | 1562 | GAAGAUCAUGUGGCCUCAGUGAA | R780 | 1582 | GGGUUGUAGUCGGUCAUGAUGG |
| F786 | 1563 | GUCGAAAAUACCUUCAACACCCAAAUU | R781 | 1583 | CCUGGCCCUUGAAGCACUA |
| | | | R782 | 1584 | ACCCCAUCUUCCCCAUCCAU |
| F787 | 1564 | CCAAAACUGCAGACAAGCAUAAAGAUG | R786 | 1585 | CUACCUCACAGUGACUGCAGUUUA |
| | | | R787 | 1586 | AGAGAGGAUCAGCGAGAGUGG |
| F788 | 1565 | CAGGCAGAAGUUGAUCGACUCU | R788 | 1587 | GUCUCGUUGCCCAAAUUGAU |
| F789 | 1566 | AAAGAAGAGUGCACAAAUGUUAGAGGA | R789 | 1588 | AGUGUUUUCAUUCGAUUCCUGUCUUCU |
| F790 | 1567 | CCAGCUUCCUAUAACUUGGACGAU | | | |
| F791 | 1568 | GAACCACAUCAUGGUCUCUGUCU | R791 | 1589 | GGUGAUGCCGUGGUUGAUGU |
| F792 | 1569 | UCAUCGGGAAGACCUGGCUUA | R792 | 1590 | AGUUCUCGCUUCAGCACGAU |
| F793 | 1570 | GCUGCAGGACUAUGAGGAGAAGA | | | |
| F794 | 1571 | CUCCCAGAGACCAACGUUCA | R794 | 1591 | UGGCCAAGCAAUCUGCGUAU |
| F795 | 1572 | GGACCUGGACCGUGUCCUUA | R795 | 1592 | UGCCAGGAUCAUAGCGUUUACAG |
| F796 | 1573 | GGACCUGGACCGUGUCCUUA | R796a | 1593 | CUGGAGCAGGUCCACUAUAGGU |
| | | | R796b | 1594 | UCCUCACACCUGCUCCUCA |
| | | | R796c | 1595 | GCUGAUGGGUGGGCACUG |
| | | | R796d | 1596 | GGUCUACCAGGACUGUCCCU |
| F797 | 1760 | CCCUUCGUAGACAUAUAGCUGUUCUC | R797 | 1597 | GGAAGGCAGGAAGAUUUUCAAUCUC |
| F798 | 1761 | UGGUGCUAGUUGCAAAGACACAA | R798 | 1598 | CGUUUAUAAGCACUGUCACCCCUU |
| F799 | 1762 | AGCGACGCCAUUGCUCAU | | | |
| F800 | 1763 | CCUCAACCAUUUCCGGCAAAU | | | |
| F801 | 1764 | CCAGCUCCCUGCGAAGAG | R801 | 1599 | AGGAUGAUGGCACUGAACUCC |
| F802 | 1765 | AAUCCCUGCAGUAGAUACGAAGACUA | R802 | 1600 | CACGUUAGUUAGUGAGCCAGGUAAU |
| F803 | 1766 | AGACCUUGCAGAAAUAGGAAUUGCU | R803 | 1601 | CUCAGGGCUCUGCAGCUCC |
| F804 | | | R804 | 1602 | CCUCCGGAAGGUCAUCUCA |
| F805 | 1767 | AAAGAAAAGACAGUUGGAGGAAUCUGU | R805 | | |
| F806 | 1768 | GAAGAAAAUGAAAAGGAGUUAGCAGCAU | R806b | 1603 | CUCCUAGAGUUUUUCCAAGAACCAAGU |
| | | | R807 | 1604 | GAACCAAGUUCUUCCGAGGGAAU |
| F807 | 1769 | AGUGGCAAAAGAACUUCAGACUUUACA | | | |
| F808 | 1770 | GCGCUGCUCAGAAGCAAAA | | | |
| F809 | 1771 | GUAGAUCGCAUAAAGGAAGCAGUCA | R809 | 1605 | ACAGCGGCUGCGAUCACC |
| F810 | 1772 | GGAAGCAGUCAGGUCAAAGAAUAUGG | | | |
| F811 | | | R811 | 1606 | GCUGACUGCACAGGACAGG |
| F812 | 1773 | CAAGCAGAAACACUGUACAAAGAGAUU | | | |
| F813 | 1774 | GAGGGCGAGCUGCAUGAU | R813a | 1607 | CGAGACCCCAAAAGGUGUUUC |
| | | | R813b | 1608 | UCCACAUUUGUUGAGCACAAGGA |
| F814 | 1775 | CACAUCUUCAGGUGCUGGAUUUUUC | | | |
| F815 | 1776 | CUUUUGAAAAGCCAGUGAUGAUCUCAA | R815 | 1609 | CACCUUUAACUGCUUCAGGGUCAAUAU |
| F816 | 1777 | GCACCUUGACUUUAAGUGAGAGCA | R816 | 1610 | UGUUGUCCCGUGGCCAUU |
| F817 | 1778 | ACAGCACUGUUAUUACUACUUGGGUUUU | R817 | 1611 | GGCAUGAACCGUUCUGAGAUG |
| F818 | 1779 | CAAGCUCCUUACAUACCCAGCA | R818 | 1612 | CCAAAUUCGCCUUCUCCUAGAGU |
| F819 | 1780 | GCGUUUCCUCGCUUGCAUU | R819 | 1613 | CUCCUCUGCACCAAGGUAAACA |
| F820 | 1781 | CGGGCAGGAATCTGATGACTTT | R820 | 1614 | UCCCUUCUAGUAAUUUGGGAAUGCC |
| F821 | 1782 | GCAGGGCAGCAACAUCUUUG | | | |
| F822 | 1783 | GGCUCCUGAGACCUUUGAUAACAUAAC | | | |
| F823 | 1784 | CGUGUGCUCCCUGGAUAUUCUUAGUA | R823 | 1615 | UCAGCUUUCUCCCACUGUAUUGAAUUUU |
| F824 | 1785 | CUGGCUCCGGGUGACAGC | R824 | 1616 | UCGGAAGGGCUGUGGAAUUG |
| | | | R824b | 1617 | CGUAGGCACACUCAAACAACGA |
| F825 | 1747 | CUGGCUCCGGGUGACAGC | R825 | 1618 | CUGAUUUCUGAACAUGGACUGUGG |
| F826 | 1748 | GACUCCCAUGACCCCCAUC | R826 | 1619 | ACGAAGUGCAAUGGUCUUUAGGU |
| F827 | 1749 | AAAAAUGUUAUGUCAGCGUUUGGCUUAA | | | |
| F828 | 1750 | GUAGGCGCGAGCUAAGCA | R828 | 1620 | GUGAGUCAUUUGUCUUGCUUUUGGU |
| F829 | 1751 | CAGGUCAUAUUGAACAUUCCAGAUACCU | | | |
| F830 | 1752 | GGUCCUGACGCAGGCUUC | | | |
| | | | F829 | 1621 | GACAGUCUGAAUCAUGUCCUUCAGU |
| | | | F830 | 1622 | GGGCUGCCCACCAUCUUC |
| | | | F831 | 1623 | UCAGCCUGAUAGUCUGGUACAAACU |
| F831 | 1753 | GUACCUGCAUCAACCCCUCUAA | | | |
| F832 | 1754 | CAGAGACCCGUGCUGAGUUU | | | |
| F833 | 1755 | GGAGAGAAGAGUGCACAAUACCA | R833 | 1624 | CCUCCACCUUGGGCUACUCA |
| F834 | 1756 | CCUGUAAUCCCUGCACUUUAGGA | R834 | 1625 | GGGUGAGCCUUGACACACA |
| F835 | 1757 | ACUUUCCAGUUGAGCAUCCCAAAUU | R835 | 1626 | CAGGGAUCAGUUCAGCUGUACC |
| F836 | 1758 | CGUCAGCGUGAUAUGUACCGUAUUUUAU | | | |
| F837 | 1759 | CACCUCAGUAAUAUGGAAGUCCAAGUU | | | |

**TABLE E**

| **Primer Name** | **SEQ ID** | **Primer Sequence** | **Primer Name** | **SEQ ID** | **Primer Sequence** |
|---|---|---|---|---|---|
| F838 | 1627 | UGGGCUCUGUAAAGAAUAGUG | R838a | 1665 | AUCAGAGCUUAAACUGGGAAG |
| | | | R838b | 1666 | AUCAGAGCUUAAACUGGGAAA |
| F839 | 1628 | UGCACACUUGGACAGCAUUUC | R839 | 1667 | GUCUCAGUUUUCCUACCUGUAAAAUGAAG |
| F840 | 1629 | CCAGGACCAAUCUGGUCACAAACAUA | R840 | 1668 | ACUUAUUCUGACAGUUCUCUUUUUCCCT |
| F841 | 1630 | GGUGGGAGGAAAAGACAUAGGAT | R841 | 1669 | GGUGGCAGUGAGCUGUAACAGUA |
| F842 | 1631 | CUCCAGAGAGAAAGAAUCAACAGG | R842 | 1670 | UCAGCCUCCAUAUCACUUGAGC |
| F843 | 1632 | GCAUCCGUGACUCUCUGGAC | R843 | 1671 | AACUUGGGUUGAGCCAUAGGC |
| F844 | 1633 | UCAGUGAGCCAAUUCCUUGUAAUAACUC | R844 | 1672 | CCUGGUUCCAUGGAUUCCACAUUAAGA |
| F845 | 1634 | CAGAUCCCAAGCUCUUCCUCUT | R845 | 1673 | GCGUUUGUGUGUGCAUCUGT |
| F846 | 1635 | GUUCAUGCCACUGCACUUCACT | R846 | 1674 | UCUGGUGUGUGGAGAUGUCUUAC |
| F847 | 1636 | GGUGCACCCAUUACCCGAAT | R847a | 1675 | GGCUGCAAAAAGCUAUAAUUGUACC |
| | | | R847b | 1676 | GGCUGCAAAAAGCUAUAACUGUACC |
| F848 | 1637 | UCCCCAUAUAAGUUCAAGCCUGUGT | R848 | 1677 | UGUGUUAGUCAGGAUUCUUCAGAGA |
| F849 | 1638 | UUGUAUAGCUACAGUUUUUCUGUUGGT | R849 | 1678 | UUCAGUUAUAUGUGUAUAAAUGUGUGCAUUG |
| F850 | 1639 | UAAAUAUGUGAGUCAAUUCCCCAAGUG | R850 | 1679 | CUCCAGAGACAGACUAAUAGGAGGUA |
| F851 | 1640 | GGCUAGAUUUUCCCCGAUGAUAGUAGT | R851 | 1680 | CCUGUGCCCAAGUUGAGAGAAT |
| F852 | 1641 | GGCUAGAUUUUCCCCUAUGAUAGUAGT | | | |
| F853 | 1642 | CAGUAAGUUAAAGGAUUGCAGGAG | R853 | 1681 | UAAUCCAGCUGUGGGAGGGA |
| F854 | 1643 | UGUGUAUAUGCAUUUACCUGUGAGUAUG | R854 | 1682 | GGUGCUAGGUGUGCUCAGGA |
| F855 | 1644 | UGUAACAAGGGCUACAGGAAUCAT | R855 | 1683 | CUUCACUCUCCUUCCCAAAUGUUUAUG |
| F856 | 1645 | GGGCAUCUCUUAUACUCAUGAAAUCAA | R856 | 1684 | CUAUGAUUCCCCCACUGCAGUC |
| F857 | 1646 | CUAUGCAGAAGAAUGAACCAGGGAT | R857 | 1685 | AGACCCCAAAAUUACUUGAGCCAAUUUA |
| F858 | 1647 | UGAUUCAUUUCCAUAGGGUAAGUGAAAA | R858 | 1686 | ACUUCAACUUCAAUUCAUCCACUGAAA |
| F859 | 1648 | GACAUUAUCACCAAUUUUUCUAGACG | R859 | 1687 | UGCUUGCCUGUAUGAAAAUAUCUC |
| F860 | 1649 | GACAUUCUCACCAAUUUUUCUAGACG | | | |
| F861 | 1650 | UGUGACAAGGGUGAUUUUCCUC | R861 | 1688 | UCCAAUCAUAGCCACAGUUUACAA |
| F862 | 1651 | CAUAAUUGUAUGAGCCACUUCCCAT | R862 | 1689 | GCACUCUUAUUCAUCUAGUUGCCUGT |
| F863 | 1652 | AGACUCACAAUGUACAAAAGCCUAUT | R863a | 1690 | CAUCAUGUGAGCCAAUUCCUCUC |
| | | | R863b | 1691 | CAUCAUGUGAGCCAAGUCCUCUC |
| F864 | 1653 | AAUAUAUAUAAAGGGUAUGAUAGAACACUUGUC | R864a | 1692 | UUGCACCAAAUAUUGGUAAUUAAAUGUUUACT |
| | | | R864b | 1693 | UUGCACCACAUAUUGGUAAUUAAAUGUUUACT |
| F865 | 1654 | GGCCUGGCAACUUAUAUGUAUUUUUGUAUT | R865 | 1694 | CACUGUAUCGUAUCCCAUUGCG |
| F866 | 1655 | GGCCUGACAACUUAUAUGUAUUUUUGUAUT | | | |
| F867 | 1656 | CCAUCCUUAUCUCUUGUGUAUCUAUUCAUUCAA | R867 | 1695 | UUGCAAGCAAUUGCCAUAGAGGGA |
| F868 | 1657 | GAUUUGUCUGUAAUUGCCAGCAAAA | R868 | 1696 | ACAGAUUAAACUGUAACCAAAAUAAAAUUAGGC |
| F869 | 1658 | GAGCAAGACACCAUCUCAAGAA | R869a | 1697 | UGCCUAACCUAUGGUCAUAACG |
| | | | R869b | 1698 | UGCCUAACCUAUGGUCAUACCG |
| F870 | 1659 | CAUGAUUGAUACAUGGAAAGAAUUCUCT | R870 | 1699 | CCCAGGAGGUGGAGAUUGAA |
| F871 | 1660 | ACCCAAAUCAACUCAACUCCAGUG | R871 | 1700 | UCCAUGUACUUUGUCCAAUGCUGA |
| F872 | 1661 | UUAGAGCAUUUAAAGUAAGCCACAGUGT | R872 | 1701 | UGUCAACACGAUUAACAUGCAAAGA |
| F873 | 1662 | CUGUACACAGGGCUUCCGAGT | R873 | 1702 | CAAAAUUCAAAGGGUAUCUGGGCUCT |
| F874 | 1663 | UUUCAGGGCUGUGAUCACUAGCAC | R874 | 1703 | UGUGCGCUGGUCUUACUCCUGUT |
| F875 | 1664 | AGAUACAUAGGUUAGAUAGAGAUAGGACAGA | R875 | 1704 | GCCCUAGUGGAUGAUAAGAAUAAUCAG |

## Claims

1. A method for preparing a library of target nucleic acid sequences comprising
(a) contacting a nucleic acid sample with a plurality of adaptors capable of amplification of one or more target nucleic acid sequences in the sample under conditions wherein the target nucleic acid(s) undergo a first amplification;
(b) digesting resulting first amplification products to reduce or eliminate resulting primer dimers and prepare partially digested target amplicons, producing gapped, double stranded amplicons, then repairing the partially digested target amplicons; and
(c) amplifying the repaired target amplicons in a second amplification using universal primers,
thereby producing a library of target nucleic acid sequences;
wherein each of the plurality of adaptors comprise a universal handle sequence and a target nucleic acid sequence and a cleavable moiety and optionally one or more tag sequences; and wherein at least two and up to one hundred thousand target specific adaptor pairs are included
wherein the target nucleic acid sequence of the adaptor includes at least one cleavable moiety and the universal handle sequence does not include the cleavable moiety.

2. The method of claim 1 wherein an optional tag sequence is included in at least one adaptor, and the cleavable moieties are included in the adaptor sequence flanking either end of the tag sequence.

3. The method of claim 1 or claim 2, wherein the melting temperature of each universal sequence is higher than the melting temperature of each target nucleic acid sequence and each tag sequence present.

4. The method of claim 1 or claim 2, carried out in a single, addition only workflow reaction, allowing for rapid production of highly multiplexed targeted libraries.

5. The method of claim 2 wherein all of the adaptors comprise tag sequences having cleavable groups flanking either end of the tag sequences.

6. The method of claim 1 or claim 2 wherein the digestion and repair of step (b) is carried out in a single step or wherein the digestion and repair of step (b) is carried out in a temporally separate manner at different temperatures.

7. The method of any one of the foregoing claims wherein one or more of the method steps is conducted in manual mode or in an automated mode or a combination thereofor wherein each of the method steps is carried out in automated mode.

8. The method of any one of the foregoing claims further comprising at least one purification step, optionally wherein a purification step is carried out only after step (c); or wherein a first purification step is carried out after step (b) and a second purification step is carried out after step (c).

9. The method of claim 8, wherein adaptor-dimer byproducts resulting from the first amplification of step (a) are removed from the resulting library or wherein the enriched population of amplied target nucleic acids contains a reduced amount of adaptor-dimer byproduct or wherein adaptor-dimer byproducts are eliminated.

10. The method of any one of the foregoing claims, wherein the plurality of adaptors capable of amplification of one or more target nucleic acid sequences comprise a multiplex of adaptor pairs capable of amplification of at least two different target nucleic acid sequences.

11. The method of claim 2 wherein each target specific pair of the plurality of adaptors includes up to 16,777,216 different adaptor combinations comprising different tag sequences.

12. The method of claim 11, wherein each generated target specific amplicon sequence includes at least 1 different sequence and up to 10⁷ different sequences.

13. The method of any one of the foregoing claims, further comprising analyzing the sequence of the resulting library of target nucleic acid sequences.

14. The method of claim 13, wherein analyzing comprises sequencing by traditional sequencing reactions, high throughput next generation sequencing, targeted multiplex array sequence detection, or any combination of two or more of the foregoing.

15. The method of claim 13 or claim 14, further comprising determining the abundance of at least one of the target nucleic sequences in the sample.

16. The method of claim 14, wherein analyzing is carried out by high throughput next generation sequencing and optionally wherein sequencing is carried out in a bidirectional manner, thereby generating sequence reads in both forward and reverse strands for any given amplicon.

17. The method of any of the foregoing claims, wherein the digestion reagent comprises any one or a combination of uracil DNA glycosylase (UDG) ,. apurinic endonuclease (e.g., APE1), RecJf, formamidopyrimidine [fapy]-DNA glycosylase (fpg), Nth endonuclease III, endonuclease VIII, polynucleotide kinase (PNK), Taq DNA polymerase, DNA polymerase I and/or human DNA polymerase beta.

18. The method of any of the foregoing claims wherein the repair reagent comprises any one or a combination of Phusion DNA polymerase, Phusion U DNA polymerase, SuperFi DNA polymerase, Taq DNA polymerase, Human DNA polymerase beta, T4 DNA polymerase and/or T7 DNA polymerase, SuperFiU DNA polymerase, *E. coli* DNA ligase, T3 DNA ligase, T4 DNA ligase, T7 DNA ligase, Taq DNA ligase, and/or 9°N DNA ligase.

## Patentansprüche

1. Verfahren zum Herstellen einer Bibliothek von Zielnukleinsäuresequenzen, das Folgendes umfasst:
(a) Inberührungbringen einer Nukleinsäureprobe mit mehreren Adaptoren, die zu einer Amplifikation einer oder mehrerer Zielnukleinsäuresequenzen in der Probe imstande sind, unter Bedingungenbei denen die Zielnukleinsäure(n) eine erste Amplifikation durchläuft/durchlaufen;
(b) Verdauen resultierender erster Amplifikationserzeugnisse, um resultierende Primerdimere zu reduzieren oder zu eliminieren und teilweise verdaute Zielamplifikats herzustellen, die beabstandete, doppelsträngige Amplifikate erzeugen, anschließend Reparieren der teilweise verdauten Zielamplifikate; und
(c) Amplifizieren der reparierten Zielamplifikate in einer zweiten Amplifikation unter Verwendung von Universalprimern,
wobei dadurch eine Bibliothek von Zielnukleinsäuresequenzen erzeugt wird;
wobei jeder der mehreren Adaptoren eine universelle Handle-Sequenz und eine Zielnukleinsäuresequenz und ein spaltbares Molekülteil und optional eine oder mehrere Markierungssequenzen umfasst; und wobei wenigstens zwei und bis zu einhunderttausend zielspezifische Adaptorenpaare eingeschlossen sind,
wobei die Zielnukleinsäuresequenz des Adaptors wenigstens ein spaltbares Molekülteil einschließt und die universelle Handle-Sequenz das spaltbare Molekülteil nicht einschließt.

2. Verfahren nach Anspruch 1, wobei eine optionale Markierungssequenz in wenigstens einem Adaptor eingeschlossen ist und die spaltbaren Molekülteile in der Adaptorsequenz eingeschlossen sind, die jedes Ende der Markierungssequenz flankiert.

3. Verfahren nach Anspruch 1 oder 2, wobei die Schmelztemperatur jeder Universalsequenz höher als die Schmelztemperatur jeder Zielnukleinsäuresequenz und jeder vorhandenen Markierungssequenz ist.

4. Verfahren nach Anspruch 1 oder 2, das in einer einzigen Arbeitsablaufreaktion nur durch Addition durchgeführt wird, die eine schnelle Erzeugung von stark gemultiplexten Zielbibliotheken ermöglicht.

5. Verfahren nach Anspruch 2, wobei die gesamten Adaptoren Markierungssequenzen umfassen, die spaltbare Gruppen aufweisen, die jedes Ende der Markierungssequenzen flankieren.

6. Verfahren nach Anspruch 1 oder 2, wobei die Verdauung und die Reparatur von Schritt (b) in einem einzigen Schritt durchgeführt werden oder wobei die Verdauung und die Reparatur von Schritt (b) in einer zeitlich getrennten Weise bei unterschiedlichen Temperaturen durchgeführt werden.

7. Verfahren nach einem der vorhergehenden Ansprüche, wobei einer oder mehrere der Verfahrensschritte in einem manuellen Modus oder in einem automatisierten Modus oder einer Kombination davon durchgeführt werden, oder wobei jeder der Verfahrensschritte in dem automatisierten Modus vorgenommen wird.

8. Verfahren nach einem der vorhergehenden Ansprüche, das ferner wenigstens einen Reinigungsschritt umfasst, optional wobei ein Reinigungsschritt nur nach Schritt (c) durchgeführt wird; oder wobei ein erster Reinigungsschritt nach Schritt (b) durchgeführt wird und ein zweiter Reinigungsschritt nach Schritt (c) durchgeführt wird.

9. Verfahren nach Anspruch 8, wobei Adaptor-Dimer-Nebenprodukte, die aus der ersten Amplifikation von Schritt (a) resultieren, aus der resultierenden Bibliothek entfernt werden oder wobei die angereicherte Population amplifizierter Zielnukleinsäuren eine reduzierte Menge von Adaptor-Dimer-Nebenprodukt enthält oder wobei Adaptor-Dimer-Nebenprodukte eliminiert werden.

10. Verfahren nach einem der vorhergehenden Ansprüche, wobei die mehreren Adaptoren, die zu der Amplifikation einer oder mehrerer Zielnukleinsäuresequenzen imstande sind, ein Multiplex von Adaptorenpaaren umfasst, die zu der Amplifikation von wenigstens zwei unterschiedlichen Zielnukleinsäuresequenzen imstande sind.

11. Verfahren nach Anspruch 2, wobei jedes zielspezifische Paar der mehreren Adaptoren bis zu 16.777.216 unterschiedliche Adaptorenkombinationen einschließt, die unterschiedliche Markierungssequenzen umfassen.

12. Verfahren nach Anspruch 11, wobei jede generierte zielspezifische Amplifikatsequenz wenigstens 1 unterschiedliche Sequenz und bis zu 10⁷ unterschiedliche Sequenzen einschließt.

13. Verfahren nach einem der vorhergehenden Ansprüche, das ferner ein Analysieren der Sequenz der resultierenden Bibliothek von Zielnukleinsäuresequenzen umfasst.

14. Verfahren nach Anspruch 13, wobei das Analysieren ein Sequenzieren durch traditionelle Sequenzierungsreaktionen, Hochdurchsatz-Sequenzieren der nächsten Generation, gezielte Multiplex-Array-Sequenzdetektion oder eine beliebige Kombination von zwei oder mehr der Vorhergehenden umfasst.

15. Verfahren nach Anspruch 13 oder 14, das ferner ein Bestimmen der Häufigkeit von wenigstens einer der Zielnukleinsäuresequenzen in der Probe umfasst.

16. Verfahren nach Anspruch 14, wobei das Analysieren durch Hochdurchsatz-Sequenzierung der nächsten Generation durchgeführt wird und optional wobei die Sequenzierung in einer bidirektionalen Weise durchgeführt wird, wobei dadurch Sequenz-Reads sowohl in Vorwärts- als auch in Rückwärtssträngen für jedes beliebige gegebene Amplifikat generiert werden.

17. Verfahren nach einem der vorhergehenden Ansprüche, wobei das Verdauungsreagens eine beliebige oder eine Kombination von Uracil-DNA-Glykosylase (UDG), apurinischer Endonuklease (z. B. APE1), RecJf, Formamidopyrimidin[fapy]-DNA-Glykosylase (fpg), Nth-Endonuklease III, Endonuklease VIII, Polynukleotidkinase (PNK), Taq-DNA-Polymerase, DNA-Polymerase I und/oder menschlicher DNA-Polymerase beta umfasst.

18. Verfahren nach einem der vorhergehenden Ansprüche, wobei das Reparaturreagens eine beliebige oder eine Kombination von Phusion-DNA-Polymerase, Phusion-U-DNA-Polymerase, SuperFi-DNA-Polymerase, Taq-DNA-Polymerase, menschlicher DNA-Polymerase beta, T4-DNA-Polymerase und/oder T7-DNA-Polymerase, SuperFiU-DNA-Polymerase, *E*.-*coli*-DNA-Ligase, T3-DNA-Ligase, T4-DNA-Ligase, T7-DNA-Ligase, Taq-DNA-Ligase und/oder 9°N-DNA-Ligase umfasst.

## Revendications

1. Procédé destiné à préparer une bibliothèque de séquences d'acide nucléique cibles comprenant
(a) la mise en contact d'un échantillon d'acide nucléique avec une pluralité d'adaptateurs capables d'amplifier une ou plusieurs séquences d'acide nucléique cibles dans l'échantillon dans des conditions dans lesquelles l'un ou les acides nucléiques cibles subissent une première amplification ;
(b) la digestion des premiers produits d'amplification obtenus pour réduire ou éliminer les dimères d'amorces obtenus et la préparation d'amplicons cibles partiellement digérés, la production d'amplicons double brin séparés, puis la réparation des amplicons cibles partiellement digérés ; et
(c) l'amplification des amplicons cibles réparés dans une seconde amplification à l'aide d'amorces universelles,
produisant ainsi une bibliothèque de séquences d'acides nucléiques cibles ;
chacun de la pluralité d'adaptateurs comprenant une séquence de poignée universelle et une séquence d'acide nucléique cible et un fragment clivable et éventuellement une ou plusieurs séquences de marqueurs ; et au moins deux et jusqu'à cent mille paires d'adaptateurs spécifiques à la cible étant incluses
la séquence d'acide nucléique cible de l'adaptateur comportant au moins un fragment clivable et la séquence à poignée universelle ne comportant pas le fragment clivable.

2. Procédé selon la revendication 1, une séquence de marqueur éventuelle étant incluse dans au moins un adaptateur, et les fragments clivables étant inclus dans la séquence d'adaptateur flanquant l'une ou l'autre extrémité de la séquence de marqueur.

3. Procédé selon la revendication 1 ou la revendication 2, la température de fusion de chaque séquence universelle étant supérieure à la température de fusion de chaque séquence d'acide nucléique cible et de chaque séquence de marqueur présente.

4. Procédé selon la revendication 1 ou 2, mis en œuvre en une seule réaction de flux de travail d'addition uniquement, permettant une production rapide de bibliothèques ciblées hautement multiplexées.

5. Procédé selon la revendication 2, tous les adaptateurs comprenant des séquences de marqueurs ayant des groupes clivables flanquant l'une ou l'autre extrémité des séquences de marqueurs.

6. Procédé selon la revendication 1 ou la revendication 2, la digestion et la réparation de l'étape (b) étant effectuées en une seule étape ou la digestion et la réparation de l'étape (b) étant effectuées de manière temporellement séparée à différentes températures.

7. Procédé selon l'une quelconque des revendications précédentes, une ou plusieurs des étapes du procédé étant effectuée en mode manuel ou dans un mode automatisé ou une combinaison de celles-ci, dans lequel chacune des étapes du procédé est effectuée en mode automatisé.

8. Procédé selon l'une quelconque des revendications précédentes, comprenant en outre au moins une étape de purification, éventuellement une étape de purification n'étant effectuée qu'après l'étape (c) ; ou une première étape de purification étant effectuée après l'étape (b) et une seconde étape de purification étant effectuée après l'étape (c).

9. Procédé selon la revendication 8, des sous-produits adaptateurs-dimères résultant de la première amplification de l'étape (a) étant éliminés de la bibliothèque résultante ou la population enrichie d'acides nucléiques cibles amplifiés contenant une quantité réduite de sous-produit adaptateurs-dimères ou les sous-produits adaptateurs-dimères étant éliminés.

10. Procédé selon l'une quelconque des revendications précédentes, la pluralité d'adaptateurs capables d'amplifier une ou plusieurs séquences d'acides nucléiques cibles comprenant un multiplex de paires d'adaptateurs capables d'amplifier au moins deux séquences d'acides nucléiques cibles différentes.

11. Procédé selon la revendication 2, chaque paire spécifique de cible de la pluralité d'adaptateurs comportant jusqu'à 16 777 216 combinaisons d'adaptateurs différentes comprenant différentes séquences de marqueurs.

12. Procédé selon la revendication 11, chaque séquence d'amplicon spécifique à une cible générée comportant au moins 1 séquence différente et jusqu'à 10⁷ séquences différentes.

13. Procédé selon l'une quelconque des revendications précédentes, comprenant en outre l'analyse de la séquence de la bibliothèque résultant de séquences d'acide nucléique cibles.

14. Procédé selon la revendication 13, l'analyse comprenant le séquençage par des réactions de séquençage traditionnelles, le séquençage de nouvelle génération à haut débit, la détection de séquence de multiplexage ciblé ou toute combinaison d'au moins deux éléments précédents.

15. Procédé selon la revendication 13 ou la revendication 14, comprenant en outre la détermination de l'abondance d'au moins une des séquences nucléiques cibles dans l'échantillon.

16. Procédé selon la revendication 14, dans lequel l'analyse est effectuée par séquençage de prochaine génération à haut débit et éventuellement dans lequel le séquençage est effectué de manière bidirectionnelle, générant ainsi des lectures de séquence à la fois en brins directs et inverses pour un amplicon donné.

17. Procédé selon l'une quelconque des revendications précédentes, le réactif de digestion comprenant l'une quelconque ou une combinaison d'uracile ADN glycosylase (UDG), endonucléase apurinique (par exemple, APE1), RecJf, formamidopyrimidine [fapy]-ADN glycosylase (fpg), Nth endonucléase III, endonucléase VIII, polynucléotide kinase (PNK), Taq ADN polymérase, ADN polymérase I et/ou ADN polymérase bêta humaine.

18. Procédé selon l'une quelconque des revendications précédentes, dans lequel le réactif de réparation comprend l'une quelconque ou une combinaison d'ADN polymérase Phusion, ADN polymérase Phusion U, ADN polymérase SuperFi, d'ADN polymérase Taq, d'ADN polymérase bêta, d'ADN polymérase T4 et/ou de T7 ADN polymérase, ADN polymérase SuperFiU, ADN ligase *E. coli,* ADN ligase T3, ADN ligase T4, ADN ligase T7, ADN ligase Taq et/ou ADN ligase 9°N.


### SEQUENCE LISTING

<110> LIFE TECHNOLOGIES CORPORATION
   ANDERSEN, Mark
   MAZUR, Daniel
<120> LIBRARY PREPARATION METHODS AND COMPOSITIONS AND USES THEREFOR
<130> LT01273
<150> US 62/527,893
   <151> 2017-06-30
<150> US 62/614,362
   <151> 2018-01-06
<150> US 62/685,424
   <151> 2018-06-15
<160> 1785
<170> PatentIn version 3.5
<210> 1
   <211> 50
   <212> DNA
   <213> Artificial Sequence
<220>
   <223> Synthetic DNA/RNA, Forward Adaptor
<220>
   <221> misc_feature
   <222> (21) .. (23)
   <223> n is a, c, g, t or u
<220>
   <221> misc_feature
   <222> (27) .. (29)
   <223> n is a, c, g, t or u
<400> 1
   tctgtacggt gacaaggcgu nnnactnnnt gaugaggacc gucgctuggt 50
<210> 2
   <211> 61
   <212> DNA
   <213> Artificial Sequence
<220>
   <223> Synthetic DNA/RNA, Rev Adaptor B
<220>
   <221> misc_feature
   <222> (22) .. (24)
   <223> n is a, c, g, t or u
<220>
   <221> misc_feature
   <222> (28) .. (30)
   <223> n is a, c, g, t or u
<400> 2
   ctctatgggc agtcggtgat unnnactnnn tgauccttct gcauggtatt ctttctctuc 60
   c 61
<210> 3
   <211> 60
   <212> DNA
   <213> Artificial Sequence
<220>
   <223> Synthetic DNA/RNA, Rev Adaptor C
<220>
   <221> misc_feature
   <222> (21) .. (23)
   <223> n is a, c, g, t or u
<220>
   <221> misc_feature
   <222> (27) .. (29)
   <223> n is a, c, g, t or u
<400> 3
   tctagtcggt cagtcacggu nnnactnnnt gauccttctg cauggtattc tttctctucc 60
<210> 4
   <211> 60
   <212> DNA
   <213> Artificial Sequence
<220>
   <223> Synthetic DNA/RNA, Rev Adaptor D
<220>
   <221> misc_feature
   <222> (21) .. (23)
   <223> n is a, c, g, t or u
<220>
   <221> misc_feature
   <222> (27) .. (29)
   <223> n is a, c, g, t or u
<400> 4
   tctagtgctg cagtcacggu nnnactnnnt gauccttctg cauggtattc tttctctucc 60
<210> 5
   <211> 60
   <212> DNA
   <213> Artificial Sequence
<220>
   <223> Synthetic DNA/RNA, Rev Adaptor E
<220>
   <221> misc_feature
   <222> (21) .. (23)
   <223> n is a, c, g, t or u
<220>
   <221> misc_feature
   <222> (27) .. (29)
   <223> n is a, c, g, t or u
<400> 5
   tgacaaggcg tagtcacggu nnnactnnnt gauccttctg cauggtattc tttctctucc 60
<210> 6
   <211> 19
   <212> DNA
   <213> Artificial Sequence
<220>
   <223> Synthetic Primer, Forward
<400> 6
   tctgtacggt gacaaggcg 19
<210> 7
   <211> 20
   <212> DNA
   <213> Artificial Sequence
<220>
   <223> Synthetic Primer, Reverse
<400> 7
   ctctatgggc agtcggtgat 20
<210> 8
   <211> 22
   <212> DNA
   <213> Artificial Sequence
<220>
   <223> Synthetic DNA/RNA primer, F1
<400> 8
   gctcccaggc acutgatgau ac 22
<210> 9
   <211> 23
   <212> DNA
   <213> Artificial Sequence
<220>
   <223> Synthetic DNA/RNA primer, F2
<400> 9
   tgttgccatt ucagggtttc uga 23
<210> 10
   <211> 25
   <212> RNA
   <213> Artificial Sequence
<220>
   <223> Synthetic Primer, F3
<400> 10
   accaaugcga ggaagaaaaa caauc 25
<210> 11
   <211> 24
   <212> DNA
   <213> Artificial Sequence
<220>
   <223> Synthetic DNA/RNA primer, F4
<400> 11
   tcttcagaau cttgttggcu gcat 24
<210> 12
   <211> 23
   <212> DNA
   <213> Artificial Sequence
<220>
   <223> Synthetic DNA/RNA primer, F5
<400> 12
   agagttgcau ccttcccttc uct 23
<210> 13
   <211> 21
   <212> DNA
   <213> Artificial Sequence
<220>
   <223> Synthetic DNA/RNA primer, F6
<400> 13
   ctggcaugac gcagtttctu c 21
<210> 14
   <211> 25
   <212> DNA
   <213> Artificial Sequence
<220>
   <223> Synthetic DNA/RNA primer, F7
<400> 14
   gatcaaagag acgaaguctc tugca 25
<210> 15
   <211> 22
   <212> DNA
   <213> Artificial Sequence
<220>
   <223> Synthetic DNA/RNA primer, F8
<400> 15
   cttgccuaga cagcaccgua at 22
<210> 16
   <211> 23
   <212> DNA
   <213> Artificial Sequence
<220>
   <223> Synthetic DNA/RNA primer, F9
<400> 16
   tggtttctgg ugggaccatt aug 23
<210> 17
   <211> 27
   <212> DNA
   <213> Artificial Sequence
<220>
   <223> Synthetic DNA/RNA primer, F10
<400> 17
   ttgaaagaga acacacutac tcuccac 27
<210> 18
   <211> 27
   <212> DNA
   <213> Artificial Sequence
<220>
   <223> Synthetic DNA/RNA primer, F11
<400> 18
   ctctaccaga gutaatcaac tgaugca 27
<210> 19
   <211> 26
   <212> DNA
   <213> Artificial Sequence
<220>
   <223> Synthetic DNA/RNA primer, F12
<400> 19
   tatcaactgt ccutgttggc aaauca 26
<210> 20
   <211> 27
   <212> DNA
   <213> Artificial Sequence
<220>
   <223> Synthetic DNA/RNA primer, F13
<400> 20
   gcaaatgact ugctattatt gauggca 27
<210> 21
   <211> 28
   <212> DNA
   <213> Artificial Sequence
<220>
   <223> Synthetic DNA/RNA primer, F14
<400> 21
   gtgggatcat autcatctac aaaguggt 28
<210> 22
   <211> 20
   <212> DNA
   <213> Artificial Sequence
<220>
   <223> Synthetic DNA/RNA primer, F15
<400> 22
   ggaggtcaug gcatcgagut 20
<210> 23
   <211> 22
   <212> DNA
   <213> Artificial Sequence
<220>
   <223> Synthetic DNA/RNA primer, F16
<400> 23
   gtgaggcagu ctttacucac ct 22
<210> 24
   <211> 22
   <212> DNA
   <213> Artificial Sequence
<220>
   <223> Synthetic DNA/RNA primer, F17
<400> 24
   gggaaatgug agccctugag at 22
<210> 25
   <211> 22
   <212> DNA
   <213> Artificial Sequence
<220>
   <223> Synthetic DNA/RNA primer, F18
<400> 25
   actcttgcuc cttccatcct ug 22
<210> 26
   <211> 22
   <212> DNA
   <213> Artificial Sequence
<220>
   <223> Synthetic DNA/RNA primer, F19
<400> 26
   cccaaugcag cgaacaatgt uc 22
<210> 27
   <211> 22
   <212> DNA
   <213> Artificial Sequence
<220>
   <223> Synthetic DNA/RNA primer, F20
<400> 27
   gatcagggcu tccaugagga aa 22
<210> 28
   <211> 24
   <212> DNA
   <213> Artificial Sequence
<220>
   <223> Synthetic DNA/RNA primer, F21
<400> 28
   ctcaagagug agccacttct uacc 24
<210> 29
   <211> 33
   <212> DNA
   <213> Artificial Sequence
<220>
   <223> Synthetic DNA/RNA primer, F22
<400> 29
   agaataaaac acauacaagt tggaaatttc ugg 33
<210> 30
   <211> 22
   <212> DNA
   <213> Artificial Sequence
<220>
   <223> Synthetic DNA/RNA primer, F23
<400> 30
   accugagcca aggactttua cc 22
<210> 31
   <211> 27
   <212> DNA
   <213> Artificial Sequence
<220>
   <223> Synthetic DNA/RNA primer, F24
<400> 31
   tgtcaattag cuggaacatc ugaaact 27
<210> 32
   <211> 24
   <212> DNA
   <213> Artificial Sequence
<220>
   <223> Synthetic DNA/RNA primer, F25
<400> 32
   gtgccctatu acctcaatca ucct 24
<210> 33
   <211> 22
   <212> DNA
   <213> Artificial Sequence
<220>
   <223> Synthetic DNA/RNA primer, F26
<400> 33
   ccagacagaa aagcggcugt ua 22
<210> 34
   <211> 32
   <212> DNA
   <213> Artificial Sequence
<220>
   <223> Synthetic DNA/RNA primer, F27
<400> 34
   cctagtagaa tgttuactac caaatggaau ga 32
<210> 35
   <211> 27
   <212> DNA
   <213> Artificial Sequence
<220>
   <223> Synthetic DNA/RNA primer, F28
<400> 35
   tttttgauga aacaagacga ctttgug 27
<210> 36
   <211> 27
   <212> DNA
   <213> Artificial Sequence
<220>
   <223> Synthetic DNA/RNA primer, F29
<400> 36
   cacagcuaca ccatatatga auggaga 27
<210> 37
   <211> 29
   <212> DNA
   <213> Artificial Sequence
<220>
   <223> Synthetic DNA/RNA primer, F30
<400> 37
   gatctatgtu cgaacaggta tcuaccatg 29
<210> 38
   <211> 28
   <212> DNA
   <213> Artificial Sequence
<220>
   <223> Synthetic DNA/RNA primer, F31
<400> 38
   gggaagaaaa gugttttgaa atgtgtut 28
<210> 39
   <211> 24
   <212> DNA
   <213> Artificial Sequence
<220>
   <223> Synthetic DNA/RNA primer, F32
<400> 39
   tttgaatctt uggccagtac cuca 24
<210> 40
   <211> 31
   <212> DNA
   <213> Artificial Sequence
<220>
   <223> Synthetic DNA/RNA primer, F33
<400> 40
   gactagcuag agacaatgaa tuaagggaaa a 31
<210> 41
   <211> 27
   <212> DNA
   <213> Artificial Sequence
<220>
   <223> Synthetic DNA/RNA primer, F34
<400> 41
   ctgagatgca caauaaaaca gtuagcc 27
<210> 42
   <211> 26
   <212> DNA
   <213> Artificial Sequence
<220>
   <223> Synthetic DNA/RNA primer, F35
<400> 42
   gacautcuca aacaggagaa gaagga 26
<210> 43
   <211> 25
   <212> DNA
   <213> Artificial Sequence
<220>
   <223> Synthetic DNA/RNA primer, F36
<400> 43
   tcttttctca agutggcctg aauca 25
<210> 44
   <211> 30
   <212> DNA
   <213> Artificial Sequence
<220>
   <223> Synthetic DNA/RNA primer, F37
<400> 44
   caatcttttg augacattgc atacatucga 30
<210> 45
   <211> 21
   <212> DNA
   <213> Artificial Sequence
<220>
   <223> Synthetic DNA/RNA primer, F38
<400> 45
   gtatgcaggc aucctcagcu a 21
<210> 46
   <211> 20
   <212> DNA
   <213> Artificial Sequence
<220>
   <223> Synthetic DNA/RNA primer, F39
<400> 46
   cuggugaccg aggacaacgt 20
<210> 47
   <211> 20
   <212> DNA
   <213> Artificial Sequence
<220>
   <223> Synthetic DNA/RNA primer, F40
<400> 47
   gtccugggag tcucaggaca 20
<210> 48
   <211> 22
   <212> DNA
   <213> Artificial Sequence
<220>
   <223> Synthetic DNA/RNA primer, F41
<400> 48
   ccagttaccu gtcctggtca ut 22
<210> 49
   <211> 26
   <212> DNA
   <213> Artificial Sequence
<220>
   <223> Synthetic DNA/RNA primer, F42
<400> 49
   agtgaaaaac aagcuctcat gtcuga 26
<210> 50
   <211> 29
   <212> DNA
   <213> Artificial Sequence
<220>
   <223> Synthetic DNA/RNA primer, F43
<400> 50
   ggaaaaattg ugaagatctg tgacttugg 29
<210> 51
   <211> 23
   <212> DNA
   <213> Artificial Sequence
<220>
   <223> Synthetic DNA/RNA primer, F44
<400> 51
   agcactctga cauatggcca tut 23
<210> 52
   <211> 23
   <212> DNA
   <213> Artificial Sequence
<220>
   <223> Synthetic DNA/RNA primer, F45
<400> 52
   ggcacggtug aatgtaaggc tua 23
<210> 53
   <211> 24
   <212> DNA
   <213> Artificial Sequence
<220>
   <223> Synthetic DNA/RNA primer, F46
<400> 53
   cccacagaaa cccaugtaug aagt 24
<210> 54
   <211> 23
   <212> DNA
   <213> Artificial Sequence
<220>
   <223> Synthetic DNA/RNA primer, F47
<400> 54
   tugacagaac gggaagcccu cat 23
<210> 55
   <211> 23
   <212> DNA
   <213> Artificial Sequence
<220>
   <223> Synthetic DNA/RNA primer, F48
<400> 55
   ccttactcau ggtcggauca caa 23
<210> 56
   <211> 26
   <212> DNA
   <213> Artificial Sequence
<220>
   <223> Synthetic DNA/RNA primer, F49
<400> 56
   ccaatattat ggaucccaac tgccua 26
<210> 57
   <211> 26
   <212> DNA
   <213> Artificial Sequence
<220>
   <223> Synthetic DNA/RNA primer, F50
<400> 57
   gctactttga uttctccact uccaac 26
<210> 58
   <211> 22
   <212> DNA
   <213> Artificial Sequence
<220>
   <223> Synthetic DNA/RNA primer, F51
<400> 58
   aaaggcaugg agcatctgua ca 22
<210> 59
   <211> 24
   <212> DNA
   <213> Artificial Sequence
<220>
   <223> Synthetic DNA/RNA primer, F52
<400> 59
   gttatgtccu cattgcccuc aaca 24
<210> 60
   <211> 22
   <212> DNA
   <213> Artificial Sequence
<220>
   <223> Synthetic DNA/RNA primer, F53
<400> 60
   cgagggcaaa uacagcttug gt 22
<210> 61
   <211> 29
   <212> DNA
   <213> Artificial Sequence
<220>
   <223> Synthetic DNA/RNA primer, F54
<400> 61
   tgatggagau gtgataattu caggaaaca 29
<210> 62
   <211> 26
   <212> DNA
   <213> Artificial Sequence
<220>
   <223> Synthetic DNA/RNA primer, F55
<400> 62
   gagacatgca ugaacatttt tcucca 26
<210> 63
   <211> 22
   <212> DNA
   <213> Artificial Sequence
<220>
   <223> Synthetic DNA/RNA primer, F56
<400> 63
   gcctctuaca cccaguggag aa 22
<210> 64
   <211> 24
   <212> DNA
   <213> Artificial Sequence
<220>
   <223> Synthetic DNA/RNA primer, F57
<400> 64
   ccttctcuct ctgtcauagg gact 24
<210> 65
   <211> 21
   <212> DNA
   <213> Artificial Sequence
<220>
   <223> Synthetic DNA/RNA primer, F58
<400> 65
   ctccaggaag ccuacgtgau g 21
<210> 66
   <211> 23
   <212> DNA
   <213> Artificial Sequence
<220>
   <223> Synthetic DNA/RNA primer, F59
<400> 66
   caggaacgua ctggugaaaa cac 23
<210> 67
   <211> 22
   <212> DNA
   <213> Artificial Sequence
<220>
   <223> Synthetic DNA/RNA primer, F60
<400> 67
   gcttgtaagu gcccgaagtg ua 22
<210> 68
   <211> 23
   <212> DNA
   <213> Artificial Sequence
<220>
   <223> Synthetic DNA/RNA primer, F61
<400> 68
   cgcagtgcua accaagttct tuc 23
<210> 69
   <211> 23
   <212> DNA
   <213> Artificial Sequence
<220>
   <223> Synthetic DNA/RNA primer, F62
<400> 69
   cagtcaaggu tgctgatttu ggt 23
<210> 70
   <211> 20
   <212> DNA
   <213> Artificial Sequence
<220>
   <223> Synthetic DNA/RNA primer, F63
<400> 70
   cgaatcgcua ccctgctgut 20
<210> 71
   <211> 17
   <212> DNA
   <213> Artificial Sequence
<220>
   <223> Synthetic DNA/RNA primer, F64
<400> 71
   taccgauacc gugcggg 17
<210> 72
   <211> 22
   <212> DNA
   <213> Artificial Sequence
<220>
   <223> Synthetic DNA/RNA primer, F65
<400> 72
   ctgtccucca caggcatttt ug 22
<210> 73
   <211> 22
   <212> DNA
   <213> Artificial Sequence
<220>
   <223> Synthetic DNA/RNA primer, F66
<400> 73
   ccatcccuga ctgtgagauc aa 22
<210> 74
   <211> 29
   <212> DNA
   <213> Artificial Sequence
<220>
   <223> Synthetic DNA/RNA primer, F67
<400> 74
   tcagtggaaa aauagcctca attctuacc 29
<210> 75
   <211> 29
   <212> DNA
   <213> Artificial Sequence
<220>
   <223> Synthetic DNA/RNA primer, F68
<400> 75
   tattatgact ugtcacaatg ucaccacat 29
<210> 76
   <211> 26
   <212> DNA
   <213> Artificial Sequence
<220>
   <223> Synthetic DNA/RNA primer, F69
<400> 76
   ttccutagtc tttcttugaa gcagca 26
<210> 77
   <211> 26
   <212> DNA
   <213> Artificial Sequence
<220>
   <223> Synthetic DNA/RNA primer, F70
<400> 77
   tctgacucca cgagaacttg aucata 26
<210> 78
   <211> 25
   <212> DNA
   <213> Artificial Sequence
<220>
   <223> Synthetic DNA/RNA primer, F71
<400> 78
   caaggcauaa aagctgggaa auagg 25
<210> 79
   <211> 19
   <212> DNA
   <213> Artificial Sequence
<220>
   <223> Synthetic DNA/RNA primer, F72
<400> 79
   ggctauggca ccugcaact 19
<210> 80
   <211> 20
   <212> DNA
   <213> Artificial Sequence
<220>
   <223> Synthetic DNA/RNA primer, F73
<400> 80
   ccacagaucc actgugcgac 20
<210> 81
   <211> 22
   <212> DNA
   <213> Artificial Sequence
<220>
   <223> Synthetic DNA/RNA primer, F74
<400> 81
   ccatcctgac cuggtatggu ca 22
<210> 82
   <211> 20
   <212> DNA
   <213> Artificial Sequence
<220>
   <223> Synthetic DNA/RNA primer, F75
<400> 82
   cagctcgtuc atcgggacut 20
<210> 83
   <211> 27
   <212> DNA
   <213> Artificial Sequence
<220>
   <223> Synthetic DNA/RNA primer, F76
<400> 83
   cctccttccu agagagttag aguaact 27
<210> 84
   <211> 24
   <212> DNA
   <213> Artificial Sequence
<220>
   <223> Synthetic DNA/RNA primer, F77
<400> 84
   cactgtgtta cugccatcga ctua 24
<210> 85
   <211> 28
   <212> DNA
   <213> Artificial Sequence
<220>
   <223> Synthetic DNA/RNA primer, F78
<400> 85
   gattcaatca aacugcagag tattuggg 28
<210> 86
   <211> 22
   <212> DNA
   <213> Artificial Sequence
<220>
   <223> Synthetic DNA/RNA primer, F79
<400> 86
   ggcttcttgg ucgtgttctu ca 22
<210> 87
   <211> 22
   <212> DNA
   <213> Artificial Sequence
<220>
   <223> Synthetic DNA/RNA primer, F80
<400> 87
   cccagcgucc tcaaaagtua ca 22
<210> 88
   <211> 20
   <212> DNA
   <213> Artificial Sequence
<220>
   <223> Synthetic DNA/RNA primer, F81
<400> 88
   ctctacguct ccuccgacca 20
<210> 89
   <211> 22
   <212> DNA
   <213> Artificial Sequence
<220>
   <223> Synthetic DNA/RNA primer, F82
<400> 89
   cctgtactgg uggatgtccu ca 22
<210> 90
   <211> 20
   <212> DNA
   <213> Artificial Sequence
<220>
   <223> Synthetic DNA/RNA primer, F83
<400> 90
   cgccaggcuc acctctauag 20
<210> 91
   <211> 29
   <212> DNA
   <213> Artificial Sequence
<220>
   <223> Synthetic DNA/RNA primer, F84
<400> 91
   tgtctttgcu gatgtttcaa uaaaaggaa 29
<210> 92
   <211> 22
   <212> DNA
   <213> Artificial Sequence
<220>
   <223> Synthetic DNA/RNA primer, F85
<400> 92
   catgtactgg uccctcatug ca 22
<210> 93
   <211> 29
   <212> DNA
   <213> Artificial Sequence
<220>
   <223> Synthetic DNA/RNA primer, F86
<400> 93
   tacctctatt gtuggatcat attcgucca 29
<210> 94
   <211> 23
   <212> DNA
   <213> Artificial Sequence
<220>
   <223> Synthetic DNA/RNA primer, F87
<400> 94
   cagacactgu acaagctcua cga 23
<210> 95
   <211> 22
   <212> DNA
   <213> Artificial Sequence
<220>
   <223> Synthetic DNA/RNA primer, F88
<400> 95
   ctctgucaca gtggatucga ga 22
<210> 96
   <211> 23
   <212> DNA
   <213> Artificial Sequence
<220>
   <223> Synthetic DNA/RNA primer, F89
<400> 96
   tccutccata gugaccaaga cca 23
<210> 97
   <211> 22
   <212> DNA
   <213> Artificial Sequence
<220>
   <223> Synthetic DNA/RNA primer, F90
<400> 97
   ggttccatug gtagctggug at 22
<210> 98
   <211> 25
   <212> DNA
   <213> Artificial Sequence
<220>
   <223> Synthetic DNA/RNA primer, F91
<400> 98
   tgtaaagaga cagccuttcc tcuga 25
<210> 99
   <211> 21
   <212> DNA
   <213> Artificial Sequence
<220>
   <223> Synthetic DNA/RNA primer, F92
<400> 99
   acacuctuga gggccacaaa g 21
<210> 100
   <211> 20
   <212> DNA
   <213> Artificial Sequence
<220>
   <223> Synthetic DNA/RNA primer, F93
<400> 100
   ccgctccutg tagccaauga 20
<210> 101
   <211> 21
   <212> DNA
   <213> Artificial Sequence
<220>
   <223> Synthetic DNA/RNA primer, F94
<400> 101
   ccacutugga acaggaccaa c 21
<210> 102
   <211> 24
   <212> DNA
   <213> Artificial Sequence
<220>
   <223> Synthetic DNA/RNA primer, F95
<400> 102
   ctttcttcca ccuttctcca gcua 24
<210> 103
   <211> 22
   <212> DNA
   <213> Artificial Sequence
<220>
   <223> Synthetic DNA/RNA primer, F96
<400> 103
   tcaagcccuc caacatccua gt 22
<210> 104
   <211> 22
   <212> DNA
   <213> Artificial Sequence
<220>
   <223> Synthetic DNA/RNA primer, R1
<400> 104
   acagaaucac augccacaca gt 22
<210> 105
   <211> 27
   <212> DNA
   <213> Artificial Sequence
<220>
   <223> Synthetic DNA/RNA primer, R2
<400> 105
   ttccttcuaa aaggccatga agatcug 27
<210> 106
   <211> 23
   <212> DNA
   <213> Artificial Sequence
<220>
   <223> Synthetic DNA/RNA primer, R3
<400> 106
   tctgaagaac augtgugagc aca 23
<210> 107
   <211> 29
   <212> DNA
   <213> Artificial Sequence
<220>
   <223> Synthetic DNA/RNA primer, R4
<400> 107
   cccaacccac auttccttta tagatgtut 29
<210> 108
   <211> 24
   <212> DNA
   <213> Artificial Sequence
<220>
   <223> Synthetic DNA/RNA primer, R5
<400> 108
   tgttccaaca ggauctgucc aaaa 24
<210> 109
   <211> 22
   <212> DNA
   <213> Artificial Sequence
<220>
   <223> Synthetic DNA/RNA primer, R6
<400> 109
   ccagtgtctg uccttgcctt uc 22
<210> 110
   <211> 28
   <212> DNA
   <213> Artificial Sequence
<220>
   <223> Synthetic DNA/RNA primer, R7
<400> 110
   cuatgacaag aaaauggaca ccaacaag 28
<210> 111
   <211> 23
   <212> DNA
   <213> Artificial Sequence
<220>
   <223> Synthetic DNA/RNA primer, R8
<400> 111
   aggaggauaa agacctgguc cat 23
<210> 112
   <211> 22
   <212> DNA
   <213> Artificial Sequence
<220>
   <223> Synthetic DNA/RNA primer, R9
<400> 112
   gtcctcugga tctcttcaug ca 22
<210> 113
   <211> 24
   <212> DNA
   <213> Artificial Sequence
<220>
   <223> Synthetic DNA/RNA primer, R10
<400> 113
   ctgagacatt ccuatgtcct gcuc 24
<210> 114
   <211> 25
   <212> DNA
   <213> Artificial Sequence
<220>
   <223> Synthetic DNA/RNA primer, R11
<400> 114
   tugauacaaa acaagcccac gaact 25
<210> 115
   <211> 30
   <212> DNA
   <213> Artificial Sequence
<220>
   <223> Synthetic DNA/RNA primer, R12
<400> 115
   ccagcctaat ctugtttttc ttatgttcug 30
<210> 116
   <211> 28
   <212> DNA
   <213> Artificial Sequence
<220>
   <223> Synthetic DNA/RNA primer, R13
<400> 116
   gttatagatg gugaaacctg tttgtugg 28
<210> 117
   <211> 26
   <212> DNA
   <213> Artificial Sequence
<220>
   <223> Synthetic DNA/RNA primer, R14
<400> 117
   gattactggt tuccaacagg ttctug 26
<210> 118
   <211> 24
   <212> DNA
   <213> Artificial Sequence
<220>
   <223> Synthetic DNA/RNA primer, R15
<400> 118
   tatggtctug gacatccagg auct 24
<210> 119
   <211> 26
   <212> DNA
   <213> Artificial Sequence
<220>
   <223> Synthetic DNA/RNA primer, R16
<400> 119
   taggaaatgc auttcctttc tuccca 26
<210> 120
   <211> 22
   <212> DNA
   <213> Artificial Sequence
<220>
   <223> Synthetic DNA/RNA primer, R17
<400> 120
   cctgtggcug tcagtattug ga 22
<210> 121
   <211> 21
   <212> DNA
   <213> Artificial Sequence
<220>
   <223> Synthetic DNA/RNA primer, R18
<400> 121
   gttcatccug ctggagcuca t 21
<210> 122
   <211> 29
   <212> DNA
   <213> Artificial Sequence
<220>
   <223> Synthetic DNA/RNA primer, R19
<400> 122
   gtagctgctg aaaaugtaac tttgtaucc 29
<210> 123
   <211> 22
   <212> DNA
   <213> Artificial Sequence
<220>
   <223> Synthetic DNA/RNA primer, R20
<400> 123
   actctgtagg cugcagttcu ca 22
<210> 124
   <211> 23
   <212> DNA
   <213> Artificial Sequence
<220>
   <223> Synthetic DNA/RNA primer, R21
<400> 124
   ctcctcttgu cttctccttu gca 23
<210> 125
   <211> 26
   <212> DNA
   <213> Artificial Sequence
<220>
   <223> Synthetic DNA/RNA primer, R22
<400> 125
   cttgtgagtg gaugggtaaa accuat 26
<210> 126
   <211> 29
   <212> DNA
   <213> Artificial Sequence
<220>
   <223> Synthetic DNA/RNA primer, R23
<400> 126
   cggactgaaa guataacctt cttcttucc 29
<210> 127
   <211> 26
   <212> DNA
   <213> Artificial Sequence
<220>
   <223> Synthetic DNA/RNA primer, R24
<400> 127
   gcatgtgaac autctgcttt tcaugg 26
<210> 128
   <211> 22
   <212> DNA
   <213> Artificial Sequence
<220>
   <223> Synthetic DNA/RNA primer, R25
<400> 128
   acgccttcac cuttaacacc uc 22
<210> 129
   <211> 22
   <212> DNA
   <213> Artificial Sequence
<220>
   <223> Synthetic DNA/RNA primer, R26
<400> 129
   acttgggagg uatccacauc ct 22
<210> 130
   <211> 28
   <212> DNA
   <213> Artificial Sequence
<220>
   <223> Synthetic DNA/RNA primer, R27
<400> 130
   agattcatct ugaagaagtt gauggagg 28
<210> 131
   <211> 33
   <212> DNA
   <213> Artificial Sequence
<220>
   <223> Synthetic DNA/RNA primer, R28
<400> 131
   gaataggata ttguatcata ccaatttcuc gat 33
<210> 132
   <211> 30
   <212> DNA
   <213> Artificial Sequence
<220>
   <223> Synthetic DNA/RNA primer, R29
<400> 132
   cagcatttga cttuacctta tcaatgtcuc 30
<210> 133
   <211> 33
   <212> DNA
   <213> Artificial Sequence
<220>
   <223> Synthetic DNA/RNA primer, R30
<400> 133
   actgctaaac actaauataa cctttggaaa uat 33
<210> 134
   <211> 30
   <212> DNA
   <213> Artificial Sequence
<220>
   <223> Synthetic DNA/RNA primer, R31
<400> 134
   catttttcca gatacuagag tgtctgtgua 30
<210> 135
   <211> 29
   <212> DNA
   <213> Artificial Sequence
<220>
   <223> Synthetic DNA/RNA primer, R32
<400> 135
   cataagagag aagguttgac tgccauaaa 29
<210> 136
   <211> 28
   <212> DNA
   <213> Artificial Sequence
<220>
   <223> Synthetic DNA/RNA primer, R33
<400> 136
   gaatctccat ttuagcactt acctguga 28
<210> 137
   <211> 32
   <212> DNA
   <213> Artificial Sequence
<220>
   <223> Synthetic DNA/RNA primer, R34
<400> 137
   agaatgtcag tuaagttaat gagctttucc at 32
<210> 138
   <211> 25
   <212> DNA
   <213> Artificial Sequence
<220>
   <223> Synthetic DNA/RNA primer, R35
<400> 138
   gcttgatucc aaggaccatg atcug 25
<210> 139
   <211> 27
   <212> DNA
   <213> Artificial Sequence
<220>
   <223> Synthetic DNA/RNA primer, R36
<400> 139
   caattcccaa aaugaaggta gcuacac 27
<210> 140
   <211> 27
   <212> DNA
   <213> Artificial Sequence
<220>
   <223> Synthetic DNA/RNA primer, R37
<400> 140
   ggaagatcca auccattttt gttgucc 27
<210> 141
   <211> 19
   <212> DNA
   <213> Artificial Sequence
<220>
   <223> Synthetic DNA/RNA primer, R38
<400> 141
   cgggaagcgg gagauctug 19
<210> 142
   <211> 18
   <212> DNA
   <213> Artificial Sequence
<220>
   <223> Synthetic DNA/RNA primer, R39
<400> 142
   ggcgtccuac tggcauga 18
<210> 143
   <211> 21
   <212> DNA
   <213> Artificial Sequence
<220>
   <223> Synthetic DNA/RNA primer, R40
<400> 143
   cctucagcag ctugaagagc t 21
<210> 144
   <211> 26
   <212> DNA
   <213> Artificial Sequence
<220>
   <223> Synthetic DNA/RNA primer, R41
<400> 144
   ggaaactccc aucttgagtc auaagg 26
<210> 145
   <211> 25
   <212> DNA
   <213> Artificial Sequence
<220>
   <223> Synthetic DNA/RNA primer, R42
<400> 145
   catgtgtcca gugaaaatcc ucact 25
<210> 146
   <211> 30
   <212> DNA
   <213> Artificial Sequence
<220>
   <223> Synthetic DNA/RNA primer, R43
<400> 146
   ctgactutag agattaaagu gaaggaggat 30
<210> 147
   <211> 28
   <212> DNA
   <213> Artificial Sequence
<220>
   <223> Synthetic DNA/RNA primer, R44
<400> 147
   cctggacaaa aauaccaatc tattgugg 28
<210> 148
   <211> 28
   <212> DNA
   <213> Artificial Sequence
<220>
   <223> Synthetic DNA/RNA primer, R45
<400> 148
   actgatatgg uagacagagc cuaaacat 28
<210> 149
   <211> 22
   <212> DNA
   <213> Artificial Sequence
<220>
   <223> Synthetic DNA/RNA primer, R46
<400> 149
   actgaccaaa acucagcctg ut 22
<210> 150
   <211> 25
   <212> DNA
   <213> Artificial Sequence
<220>
   <223> Synthetic DNA/RNA primer, R47
<400> 150
   cctgacagac aauaaaaggc agcut 25
<210> 151
   <211> 28
   <212> DNA
   <213> Artificial Sequence
<220>
   <223> Synthetic DNA/RNA primer, R48
<400> 151
   gttgaaacua aaaatccttu gcaggact 28
<210> 152
   <211> 24
   <212> DNA
   <213> Artificial Sequence
<220>
   <223> Synthetic DNA/RNA primer, R49
<400> 152
   acattctgaa gcagcutgga gtut 24
<210> 153
   <211> 26
   <212> DNA
   <213> Artificial Sequence
<220>
   <223> Synthetic DNA/RNA primer, R50
<400> 153
   gaggagattg aaaaucttcc tgccut 26
<210> 154
   <211> 20
   <212> DNA
   <213> Artificial Sequence
<220>
   <223> Synthetic DNA/RNA primer, R51
<400> 154
   ttggtccguc tccuccacgg 20
<210> 155
   <211> 28
   <212> DNA
   <213> Artificial Sequence
<220>
   <223> Synthetic DNA/RNA primer, R52
<400> 155
   cttcagtccg gutttatttg catcauag 28
<210> 156
   <211> 24
   <212> DNA
   <213> Artificial Sequence
<220>
   <223> Synthetic DNA/RNA primer, R53
<400> 156
   gactctccaa gaugggatac ucca 24
<210> 157
   <211> 22
   <212> DNA
   <213> Artificial Sequence
<220>
   <223> Synthetic DNA/RNA primer, R54
<400> 157
   cggtgactua ctgcagctgt ut 22
<210> 158
   <211> 22
   <212> DNA
   <213> Artificial Sequence
<220>
   <223> Synthetic DNA/RNA primer, R55
<400> 158
   tccagaccag ggugttgttt uc 22
<210> 159
   <211> 23
   <212> DNA
   <213> Artificial Sequence
<220>
   <223> Synthetic DNA/RNA primer, R56
<400> 159
   tgtgccaggg accutacctt aua 23
<210> 160
   <211> 24
   <212> RNA
   <213> Artificial Sequence
<220>
   <223> Synthetic Primer, R57
<400> 160
   cacagcaaag cagaaacuca cauc 24
<210> 161
   <211> 21
   <212> DNA
   <213> Artificial Sequence
<220>
   <223> Synthetic DNA/RNA primer, R58
<400> 161
   tgtgtucccg gacatagucc a 21
<210> 162
   <211> 23
   <212> DNA
   <213> Artificial Sequence
<220>
   <223> Synthetic DNA/RNA primer, R59
<400> 162
   gaaaatgcug gctgaccuaa agc 23
<210> 163
   <211> 30
   <212> DNA
   <213> Artificial Sequence
<220>
   <223> Synthetic DNA/RNA primer, R60
<400> 163
   cacaacccac ugaggtatat gtatagguat 30
<210> 164
   <211> 30
   <212> DNA
   <213> Artificial Sequence
<220>
   <223> Synthetic DNA/RNA primer, R61
<400> 164
   ccatggttaa ataaaaugcc acttactgut 30
<210> 165
   <211> 26
   <212> DNA
   <213> Artificial Sequence
<220>
   <223> Synthetic DNA/RNA primer, R62
<400> 165
   cttggtggua aacttttgag ttugca 26
<210> 166
   <211> 19
   <212> DNA
   <213> Artificial Sequence
<220>
   <223> Synthetic DNA/RNA primer, R63
<400> 166
   ccaagccuca tggugccat 19
<210> 167
   <211> 20
   <212> DNA
   <213> Artificial Sequence
<220>
   <223> Synthetic DNA/RNA primer, R64
<400> 167
   tacuggcagc aagugcccag 20
<210> 168
   <211> 22
   <212> DNA
   <213> Artificial Sequence
<220>
   <223> Synthetic DNA/RNA primer, R65
<400> 168
   ccctcacuca cagcacatag uc 22
<210> 169
   <211> 20
   <212> DNA
   <213> Artificial Sequence
<220>
   <223> Synthetic DNA/RNA primer, R66
<400> 169
   ccagguacgc ctccagauga 20
<210> 170
   <211> 30
   <212> DNA
   <213> Artificial Sequence
<220>
   <223> Synthetic DNA/RNA primer, R67
<400> 170
   cttcatgaag accucacagt aaaaauaggt 30
<210> 171
   <211> 25
   <212> DNA
   <213> Artificial Sequence
<220>
   <223> Synthetic DNA/RNA primer, R68
<400> 171
   gactcgagtg augattggga gatuc 25
<210> 172
   <211> 27
   <212> DNA
   <213> Artificial Sequence
<220>
   <223> Synthetic DNA/RNA primer, R69
<400> 172
   agatgctcug agaaaggcat uagaaag 27
<210> 173
   <211> 26
   <212> DNA
   <213> Artificial Sequence
<220>
   <223> Synthetic DNA/RNA primer, R70
<400> 173
   tattgttaac cutgcagaat ggucga 26
<210> 174
   <211> 22
   <212> DNA
   <213> Artificial Sequence
<220>
   <223> Synthetic DNA/RNA primer, R71
<400> 174
   cuacctgccu acgcaacaag at 22
<210> 175
   <211> 21
   <212> DNA
   <213> Artificial Sequence
<220>
   <223> Synthetic DNA/RNA primer, R72
<400> 175
   gggaccucag atgtgctgtu g 21
<210> 176
   <211> 20
   <212> DNA
   <213> Artificial Sequence
<220>
   <223> Synthetic DNA/RNA primer, R73
<400> 176
   gtggcttgug ggcaaactug 20
<210> 177
   <211> 22
   <212> DNA
   <213> Artificial Sequence
<220>
   <223> Synthetic DNA/RNA primer, R74
<400> 177
   cctgctucag gacgttgaac uc 22
<210> 178
   <211> 20
   <212> DNA
   <213> Artificial Sequence
<220>
   <223> Synthetic DNA/RNA primer, R75
<400> 178
   acctggcucc tcttcacgua 20
<210> 179
   <211> 24
   <212> DNA
   <213> Artificial Sequence
<220>
   <223> Synthetic DNA/RNA primer, R76
<400> 179
   cacccacacu tacacatcac ttug 24
<210> 180
   <211> 26
   <212> DNA
   <213> Artificial Sequence
<220>
   <223> Synthetic DNA/RNA primer, R77
<400> 180
   tcgagattua gcagccagaa atgtut 26
<210> 181
   <211> 24
   <212> DNA
   <213> Artificial Sequence
<220>
   <223> Synthetic DNA/RNA primer, R78
<400> 181
   tgatctggug tcagagaugg agat 24
<210> 182
   <211> 26
   <212> DNA
   <213> Artificial Sequence
<220>
   <223> Synthetic DNA/RNA primer, R79
<400> 182
   cuagcgccug gaagagaaaa ggagat 26
<210> 183
   <211> 22
   <212> DNA
   <213> Artificial Sequence
<220>
   <223> Synthetic DNA/RNA primer, R80
<400> 183
   ccctccacaa ucattcctgu gt 22
<210> 184
   <211> 30
   <212> DNA
   <213> Artificial Sequence
<220>
   <223> Synthetic DNA/RNA primer, R81
<400> 184
   cttatttatt ggtcuctcat tctcccaucc 30
<210> 185
   <211> 22
   <212> DNA
   <213> Artificial Sequence
<220>
   <223> Synthetic DNA/RNA primer, R82
<400> 185
   gcctgtugga catcctggau ac 22
<210> 186
   <211> 23
   <212> DNA
   <213> Artificial Sequence
<220>
   <223> Synthetic DNA/RNA primer, R83
<400> 186
   aggagcgaug acggaataua agc 23
<210> 187
   <211> 29
   <212> DNA
   <213> Artificial Sequence
<220>
   <223> Synthetic DNA/RNA primer, R84
<400> 187
   agttaaggac tcugaagatg tacctaugg 29
<210> 188
   <211> 28
   <212> DNA
   <213> Artificial Sequence
<220>
   <223> Synthetic DNA/RNA primer, R85
<400> 188
   gtaataatcc agacugtgtt tctcccut 28
<210> 189
   <211> 30
   <212> DNA
   <213> Artificial Sequence
<220>
   <223> Synthetic DNA/RNA primer, R86
<400> 189
   tattataagg ccugctgaaa atgacugaat 30
<210> 190
   <211> 25
   <212> DNA
   <213> Artificial Sequence
<220>
   <223> Synthetic DNA/RNA primer, R87
<400> 190
   gaauaaagag gagcaggtug aggaa 25
<210> 191
   <211> 25
   <212> DNA
   <213> Artificial Sequence
<220>
   <223> Synthetic DNA/RNA primer, R88
<400> 191
   caacatgacg aagauggcaa actuc 25
<210> 192
   <211> 24
   <212> DNA
   <213> Artificial Sequence
<220>
   <223> Synthetic DNA/RNA primer, R89
<400> 192
   gggtacauac aaagcagtct gugt 24
<210> 193
   <211> 29
   <212> DNA
   <213> Artificial Sequence
<220>
   <223> Synthetic DNA/RNA primer, R90
<400> 193
   gcccattttt atcuacttcc atcttguca 29
<210> 194
   <211> 26
   <212> DNA
   <213> Artificial Sequence
<220>
   <223> Synthetic DNA/RNA primer, R91
<400> 194
   agttcacaaa uccatcaatg ttgcuc 26
<210> 195
   <211> 24
   <212> DNA
   <213> Artificial Sequence
<220>
   <223> Synthetic DNA/RNA primer, R92
<400> 195
   tgtgattgua gggtctccct ugat 24
<210> 196
   <211> 20
   <212> DNA
   <213> Artificial Sequence
<220>
   <223> Synthetic DNA/RNA primer, R93
<400> 196
   gggtcugacg ggtagagugt 20
<210> 197
   <211> 25
   <212> DNA
   <213> Artificial Sequence
<220>
   <223> Synthetic DNA/RNA primer, R94
<400> 197
   tcaaagtcgt cauccttcag ttcuc 25
<210> 198
   <211> 22
   <212> DNA
   <213> Artificial Sequence
<220>
   <223> Synthetic DNA/RNA primer, R95
<400> 198
   catcgctgua gaacgcacca ua 22
<210> 199
   <211> 28
   <212> DNA
   <213> Artificial Sequence
<220>
   <223> Synthetic DNA/RNA primer, R96
<400> 199
   ggaaacttct gtucataccg acatguag 28
<210> 200
   <211> 26
   <212> DNA
   <213> Artificial Sequence
<220>
   <223> Synthetic DNA/RNA primer, F97
<400> 200
   gaggtcgtat ucgtccacaa aauggt 26
<210> 201
   <211> 30
   <212> DNA
   <213> Artificial Sequence
<220>
   <223> Synthetic DNA/RNA primer, F98
<400> 201
   gaaactgctt aguaactagc agaagtgtuc 30
<210> 202
   <211> 28
   <212> DNA
   <213> Artificial Sequence
<220>
   <223> Synthetic DNA/RNA primer, F99
<400> 202
   gacaaagttg ugtgttgtaa guggaaca 28
<210> 203
   <211> 27
   <212> DNA
   <213> Artificial Sequence
<220>
   <223> Synthetic DNA/RNA primer, F100
<400> 203
   gagcaccaat cuttcttctg cctttug 27
<210> 204
   <211> 25
   <212> RNA
   <213> Artificial Sequence
<220>
   <223> Synthetic Primer, F101
<400> 204
   gagggauccc aaggaagaga aguga 25
<210> 205
   <211> 27
   <212> DNA
   <213> Artificial Sequence
<220>
   <223> Synthetic DNA/RNA primer, F102
<400> 205
   gagctactct ccugaactct ctcacuc 27
<210> 206
   <211> 23
   <212> DNA
   <213> Artificial Sequence
<220>
   <223> Synthetic DNA/RNA primer, F103
<400> 206
   gactcccagu tgcaacgtua ggt 23
<210> 207
   <211> 20
   <212> DNA
   <213> Artificial Sequence
<220>
   <223> Synthetic DNA/RNA primer, F104
<400> 207
   gatcacgugt ccccctucca 20
<210> 208
   <211> 30
   <212> DNA
   <213> Artificial Sequence
<220>
   <223> Synthetic DNA/RNA primer, F105
<400> 208
   gaacatttgg cugtgacttc uaagaagaaa 30
<210> 209
   <211> 25
   <212> DNA
   <213> Artificial Sequence
<220>
   <223> Synthetic DNA/RNA primer, F106
<400> 209
   gagatgaucc agatgtuagg gcagt 25
<210> 210
   <211> 28
   <212> DNA
   <213> Artificial Sequence
<220>
   <223> Synthetic DNA/RNA primer, F107
<400> 210
   gagcccaaat ugatttcgat gatctuca 28
<210> 211
   <211> 23
   <212> DNA
   <213> Artificial Sequence
<220>
   <223> Synthetic DNA/RNA primer, F108
<400> 211
   gagctcaaga gugagccact uct 23
<210> 212
   <211> 29
   <212> DNA
   <213> Artificial Sequence
<220>
   <223> Synthetic DNA/RNA primer, F109
<400> 212
   gaattaacac acaucagtgg aacttcugt 29
<210> 213
   <211> 27
   <212> RNA
   <213> Artificial Sequence
<220>
   <223> Synthetic Primer, F110
<400> 213
   gagcaaucca aaagaauagc agccaaa 27
<210> 214
   <211> 28
   <212> DNA
   <213> Artificial Sequence
<220>
   <223> Synthetic DNA/RNA primer, F111
<400> 214
   gactttgtgg aauagcccat gaagagua 28
<210> 215
   <211> 25
   <212> DNA
   <213> Artificial Sequence
<220>
   <223> Synthetic DNA/RNA primer, F112
<400> 215
   gaacaggaag agcacaguca cttug 25
<210> 216
   <211> 26
   <212> DNA
   <213> Artificial Sequence
<220>
   <223> Synthetic DNA/RNA primer, F113
<400> 216
   gaccatgcag agugaaagga tauccc 26
<210> 217
   <211> 20
   <212> DNA
   <213> Artificial Sequence
<220>
   <223> Synthetic DNA/RNA primer, F114
<400> 217
   gatggagccg cugacaccua 20
<210> 218
   <211> 23
   <212> DNA
   <213> Artificial Sequence
<220>
   <223> Synthetic DNA/RNA primer, F115
<400> 218
   gaggtgucta gcccauggga gaa 23
<210> 219
   <211> 23
   <212> DNA
   <213> Artificial Sequence
<220>
   <223> Synthetic DNA/RNA primer, F116
<400> 219
   gagtctgguc cacattgctc uca 23
<210> 220
   <211> 27
   <212> DNA
   <213> Artificial Sequence
<220>
   <223> Synthetic DNA/RNA primer, F117
<400> 220
   gagcaagagu acacactcct cattugg 27
<210> 221
   <211> 24
   <212> DNA
   <213> Artificial Sequence
<220>
   <223> Synthetic DNA/RNA primer, F118
<400> 221
   gactggtttc uggtgggacc atua 24
<210> 222
   <211> 25
   <212> DNA
   <213> Artificial Sequence
<220>
   <223> Synthetic DNA/RNA primer, F119
<400> 222
   gactcccagg cacutgatga tacuc 25
<210> 223
   <211> 21
   <212> DNA
   <213> Artificial Sequence
<220>
   <223> Synthetic DNA/RNA primer, F120
<400> 223
   gagggctugg taacgtccug t 21
<210> 224
   <211> 27
   <212> DNA
   <213> Artificial Sequence
<220>
   <223> Synthetic DNA/RNA primer, F121
<400> 224
   gatgtgtcaa ggagutcgaa gatucac 27
<210> 225
   <211> 21
   <212> DNA
   <213> Artificial Sequence
<220>
   <223> Synthetic DNA/RNA primer, F122
<400> 225
   gaguacagcc agtgtguccg a 21
<210> 226
   <211> 24
   <212> DNA
   <213> Artificial Sequence
<220>
   <223> Synthetic DNA/RNA primer, F123
<400> 226
   gaccatccgg gcuttacgca aaua 24
<210> 227
   <211> 26
   <212> DNA
   <213> Artificial Sequence
<220>
   <223> Synthetic DNA/RNA primer, F124
<400> 227
   gaccccacug aacctctctt acatut 26
<210> 228
   <211> 23
   <212> DNA
   <213> Artificial Sequence
<220>
   <223> Synthetic DNA/RNA primer, F125
<400> 228
   gacccuaaca gccatgcttt cuc 23
<210> 229
   <211> 23
   <212> DNA
   <213> Artificial Sequence
<220>
   <223> Synthetic DNA/RNA primer, F126
<400> 229
   gacggcgaug ctgagaacca aua 23
<210> 230
   <211> 19
   <212> DNA
   <213> Artificial Sequence
<220>
   <223> Synthetic DNA/RNA primer, F127
<400> 230
   gaccgacgut gaccgcauc 19
<210> 231
   <211> 30
   <212> DNA
   <213> Artificial Sequence
<220>
   <223> Synthetic DNA/RNA primer, F128
<400> 231
   gaaaatattu cagtgtccgt ucacacacaa 30
<210> 232
   <211> 21
   <212> DNA
   <213> Artificial Sequence
<220>
   <223> Synthetic DNA/RNA primer, F129
<400> 232
   gaccacacug acgtgcctcu c 21
<210> 233
   <211> 23
   <212> DNA
   <213> Artificial Sequence
<220>
   <223> Synthetic DNA/RNA primer, F130
<400> 233
   gagcgccaca gagaagutgt uga 23
<210> 234
   <211> 27
   <212> DNA
   <213> Artificial Sequence
<220>
   <223> Synthetic DNA/RNA primer, F131
<400> 234
   gaccacaaaa uggatccaga caacugt 27
<210> 235
   <211> 33
   <212> DNA
   <213> Artificial Sequence
<220>
   <223> Synthetic DNA/RNA primer, F132
<400> 235
   gaactgtttc gtauttatag ctgatttgau gga 33
<210> 236
   <211> 23
   <212> DNA
   <213> Artificial Sequence
<220>
   <223> Synthetic DNA/RNA primer, F133
<400> 236
   gacctcautg cccucaacac agt 23
<210> 237
   <211> 23
   <212> DNA
   <213> Artificial Sequence
<220>
   <223> Synthetic DNA/RNA primer, F134
<400> 237
   gacaccacgu accagatgga ugt 23
<210> 238
   <211> 28
   <212> DNA
   <213> Artificial Sequence
<220>
   <223> Synthetic DNA/RNA primer, F135
<400> 238
   gaagacatgc augaacattt ttcuccac 28
<210> 239
   <211> 22
   <212> DNA
   <213> Artificial Sequence
<220>
   <223> Synthetic DNA/RNA primer, F136
<400> 239
   gatguggagc ctctuacacc ca 22
<210> 240
   <211> 25
   <212> DNA
   <213> Artificial Sequence
<220>
   <223> Synthetic DNA/RNA primer, F137
<400> 240
   gaacgtcttc cutctctctc tguca 25
<210> 241
   <211> 27
   <212> DNA
   <213> Artificial Sequence
<220>
   <223> Synthetic DNA/RNA primer, F138
<400> 241
   gagaatgtga aaautccagt ggccauc 27
<210> 242
   <211> 22
   <212> DNA
   <213> Artificial Sequence
<220>
   <223> Synthetic DNA/RNA primer, F139
<400> 242
   gagggtgtgu ggtctcccau ac 22
<210> 243
   <211> 24
   <212> DNA
   <213> Artificial Sequence
<220>
   <223> Synthetic DNA/RNA primer, F140
<400> 243
   gaggatgagc uacctggagg augt 24
<210> 244
   <211> 27
   <212> DNA
   <213> Artificial Sequence
<220>
   <223> Synthetic DNA/RNA primer, F141
<400> 244
   gaggtcactg uacaccttac acaugaa 27
<210> 245
   <211> 28
   <212> DNA
   <213> Artificial Sequence
<220>
   <223> Synthetic DNA/RNA primer, F142
<400> 245
   gacatcacug taaacctugc agacaaac 28
<210> 246
   <211> 25
   <212> DNA
   <213> Artificial Sequence
<220>
   <223> Synthetic DNA/RNA primer, F143
<400> 246
   gagcttcttg gucgtgttct tcaut 25
<210> 247
   <211> 24
   <212> DNA
   <213> Artificial Sequence
<220>
   <223> Synthetic DNA/RNA primer, F144
<400> 247
   gauggaagcc cagccattuc taaa 24
<210> 248
   <211> 19
   <212> DNA
   <213> Artificial Sequence
<220>
   <223> Synthetic DNA/RNA primer, F145
<400> 248
   gagccccuga gcgtcauct 19
<210> 249
   <211> 19
   <212> DNA
   <213> Artificial Sequence
<220>
   <223> Synthetic DNA/RNA primer, F146
<400> 249
   gagagctggu ggaggcuga 19
<210> 250
   <211> 22
   <212> DNA
   <213> Artificial Sequence
<220>
   <223> Synthetic DNA/RNA primer, F147
<400> 250
   gagugaccga ggacaacgug at 22
<210> 251
   <211> 22
   <212> DNA
   <213> Artificial Sequence
<220>
   <223> Synthetic DNA/RNA primer, F148
<400> 251
   gactcuggga gatctucacg ct 22
<210> 252
   <211> 22
   <212> DNA
   <213> Artificial Sequence
<220>
   <223> Synthetic DNA/RNA primer, F149
<400> 252
   gaggattgca gautgggcct ug 22
<210> 253
   <211> 30
   <212> DNA
   <213> Artificial Sequence
<220>
   <223> Synthetic DNA/RNA primer, F150
<400> 253
   gaataatcca utgcctgtcu aaagaacact 30
<210> 254
   <211> 25
   <212> DNA
   <213> Artificial Sequence
<220>
   <223> Synthetic DNA/RNA primer, F151
<400> 254
   gagacttggu gttgttgaug gcaaa 25
<210> 255
   <211> 27
   <212> DNA
   <213> Artificial Sequence
<220>
   <223> Synthetic DNA/RNA primer, F152
<400> 255
   gaccaacaug acttacttga uccccat 27
<210> 256
   <211> 20
   <212> DNA
   <213> Artificial Sequence
<220>
   <223> Synthetic DNA/RNA primer, F153
<400> 256
   gaacccuggc ctacctgguc 20
<210> 257
   <211> 26
   <212> DNA
   <213> Artificial Sequence

<220>
   <223> Synthetic DNA/RNA primer, F154
<400> 257
   gatgaagcag caaguatgau gagcaa 26
<210> 258
   <211> 25
   <212> DNA
   <213> Artificial Sequence
<220>
   <223> Synthetic DNA/RNA primer, F155
<400> 258
   gaggcacggt ugaatgtaag gctua 25
<210> 259
   <211> 23
   <212> DNA
   <213> Artificial Sequence
<220>
   <223> Synthetic DNA/RNA primer, F156
<400> 259
   gaccacaccc ugttcactcc tut 23
<210> 260
   <211> 28
   <212> DNA
   <213> Artificial Sequence
<220>
   <223> Synthetic DNA/RNA primer, F157
<400> 260
   gaaaggtgat cuatttttcc ctttcucc 28
<210> 261
   <211> 29
   <212> DNA
   <213> Artificial Sequence
<220>
   <223> Synthetic DNA/RNA primer, F158
<400> 261
   gagctttttg cuaaaatgca tgttuccaa 29
<210> 262
   <211> 29
   <212> DNA
   <213> Artificial Sequence
<220>
   <223> Synthetic DNA/RNA primer, F159
<400> 262
   gacaaagaat ggucctgcac cagtaauat 29
<210> 263
   <211> 25
   <212> DNA
   <213> Artificial Sequence
<220>
   <223> Synthetic DNA/RNA primer, F160
<400> 263
   gatcctcatg uactggtccc tcaut 25
<210> 264
   <211> 34
   <212> DNA
   <213> Artificial Sequence
<220>
   <223> Synthetic DNA/RNA primer, F161
<400> 264
   gacagatctg tatutatttc agtgttactu acct 34
<210> 265
   <211> 29
   <212> DNA
   <213> Artificial Sequence
<220>
   <223> Synthetic DNA/RNA primer, F162
<400> 265
   gacatgtcaa caucgctcta atucagaga 29
<210> 266
   <211> 25
   <212> DNA
   <213> Artificial Sequence
<220>
   <223> Synthetic DNA/RNA primer, F163
<400> 266
   gatgttacgc agugctaacc aagut 25
<210> 267
   <211> 24
   <212> DNA
   <213> Artificial Sequence
<220>
   <223> Synthetic DNA/RNA primer, F164
<400> 267
   gagctgautt tggtctugcc agag 24
<210> 268
   <211> 27
   <212> DNA
   <213> Artificial Sequence
<220>
   <223> Synthetic DNA/RNA primer, F165
<400> 268
   gacctcacct cuatggtggg atcauat 27
<210> 269
   <211> 24
   <212> DNA
   <213> Artificial Sequence
<220>
   <223> Synthetic DNA/RNA primer, F166
<400> 269
   gattcgcctg ucctcatgta tugg 24
<210> 270
   <211> 25
   <212> DNA
   <213> Artificial Sequence
<220>
   <223> Synthetic DNA/RNA primer, F167
<400> 270
   gagcacuggg actttggtaa tucac 25
<210> 271
   <211> 28
   <212> DNA
   <213> Artificial Sequence
<220>
   <223> Synthetic DNA/RNA primer, F168
<400> 271
   gacagtgaaa aacaagcuct catgtcug 28
<210> 272
   <211> 22
   <212> DNA
   <213> Artificial Sequence
<220>
   <223> Synthetic DNA/RNA primer, F169
<400> 272
   gacagtgtgu ccaccgtgau ct 22
<210> 273
   <211> 30
   <212> DNA
   <213> Artificial Sequence
<220>
   <223> Synthetic DNA/RNA primer, F170
<400> 273
   gatggaatgc cagaacuaca atctttugat 30
<210> 274
   <211> 23
   <212> DNA
   <213> Artificial Sequence
<220>
   <223> Synthetic DNA/RNA primer, F171
<400> 274
   gagacgcaut tccacagcua cac 23
<210> 275
   <211> 26
   <212> DNA
   <213> Artificial Sequence
<220>
   <223> Synthetic DNA/RNA primer, F172
<400> 275
   gagctttgaa uctttggcca guacct 26
<210> 276
   <211> 25
   <212> DNA
   <213> Artificial Sequence
<220>
   <223> Synthetic DNA/RNA primer, F173
<400> 276
   gagatgcagc cautgacctg ttuac 25
<210> 277
   <211> 24
   <212> DNA
   <213> Artificial Sequence
<220>
   <223> Synthetic DNA/RNA primer, F174
<400> 277
   gagggatuaa agctggctau ggca 24
<210> 278
   <211> 21
   <212> DNA
   <213> Artificial Sequence
<220>
   <223> Synthetic DNA/RNA primer, F175
<400> 278
   gaagcauacg cagcctguac c 21
<210> 279
   <211> 22
   <212> DNA
   <213> Artificial Sequence
<220>
   <223> Synthetic DNA/RNA primer, F176
<400> 279
   gagctuccag gagcgatcgt ut 22
<210> 280
   <211> 22
   <212> DNA
   <213> Artificial Sequence
<220>
   <223> Synthetic DNA/RNA primer, F177
<400> 280
   gaagctcgtu catcgggact ug 22
<210> 281
   <211> 25
   <212> DNA
   <213> Artificial Sequence
<220>
   <223> Synthetic DNA/RNA primer, F178
<400> 281
   gactggtuac tgaaagcuca gggat 25
<210> 282
   <211> 27
   <212> DNA
   <213> Artificial Sequence
<220>
   <223> Synthetic DNA/RNA primer, F179
<400> 282
   gaggactctg ugagtgggat ttgttut 27
<210> 283
   <211> 26
   <212> DNA
   <213> Artificial Sequence
<220>
   <223> Synthetic DNA/RNA primer, F180
<400> 283
   gacatcccug actgtgagau caagaa 26
<210> 284
   <211> 22
   <212> DNA
   <213> Artificial Sequence
<220>
   <223> Synthetic DNA/RNA primer, F181
<400> 284
   gaatcaaccu gcttggtgtc ug 22
<210> 285
   <211> 21
   <212> DNA
   <213> Artificial Sequence
<220>
   <223> Synthetic DNA/RNA primer, F182
<400> 285
   gaacgaggac cugtgggacu c 21
<210> 286
   <211> 22
   <212> DNA
   <213> Artificial Sequence
<220>
   <223> Synthetic DNA/RNA primer, F183
<400> 286
   gacatucccc aacagctgug gt 22
<210> 287
   <211> 22
   <212> DNA
   <213> Artificial Sequence
<220>
   <223> Synthetic DNA/RNA primer, F184
<400> 287
   gaccttccuc ctgaaggccu ga 22
<210> 288
   <211> 21
   <212> DNA
   <213> Artificial Sequence
<220>
   <223> Synthetic DNA/RNA primer, F185
<400> 288
   gaaagtgcut gtgcccugca t 21
<210> 289
   <211> 23
   <212> DNA
   <213> Artificial Sequence
<220>
   <223> Synthetic DNA/RNA primer, F186
<400> 289
   gacccctctu ggaccttaga ugc 23
<210> 290
   <211> 24
   <212> DNA
   <213> Artificial Sequence
<220>
   <223> Synthetic DNA/RNA primer, F187
<400> 290
   gacgcaugga gaagaaactg caug 24
<210> 291
   <211> 25
   <212> DNA
   <213> Artificial Sequence
<220>
   <223> Synthetic DNA/RNA primer, F188
<400> 291
   gatcccctau gtgcaagtcc uaaag 25
<210> 292
   <211> 22
   <212> DNA
   <213> Artificial Sequence
<220>
   <223> Synthetic DNA/RNA primer, F189
<400> 292
   gaccactgug cagaagctcu cc 22
<210> 293
   <211> 19
   <212> DNA
   <213> Artificial Sequence
<220>
   <223> Synthetic DNA/RNA primer, F190
<400> 293
   gacgccggcc ucgtgaguc 19
<210> 294
   <211> 27
   <212> DNA
   <213> Artificial Sequence
<220>
   <223> Synthetic DNA/RNA primer, F191
<400> 294
   gacaaatgcu gaaagctgta ccauacc 27
<210> 295
   <211> 24
   <212> DNA
   <213> Artificial Sequence
<220>
   <223> Synthetic DNA/RNA primer, F192
<400> 295
   gatgaggcag uctttactca ccug 24
<210> 296
   <211> 25
   <212> DNA
   <213> Artificial Sequence
<220>
   <223> Synthetic DNA/RNA primer, F193
<400> 296
   gagcaaagac uggttctcac ucacc 25
<210> 297
   <211> 24
   <212> DNA
   <213> Artificial Sequence
<220>
   <223> Synthetic DNA/RNA primer, F194
<400> 297
   gacgauctgt tcuacacgga accc 24
<210> 298
   <211> 26
   <212> DNA
   <213> Artificial Sequence
<220>
   <223> Synthetic DNA/RNA primer, F195
<400> 298
   gaccagacaa gccuacagta ggaauc 26
<210> 299
   <211> 23
   <212> DNA
   <213> Artificial Sequence
<220>
   <223> Synthetic DNA/RNA primer, F196
<400> 299
   gactccacag acccuctcct ugc 23
<210> 300
   <211> 24
   <212> DNA
   <213> Artificial Sequence
<220>
   <223> Synthetic DNA/RNA primer, F197
<400> 300
   gaagggtgtc uctctgtggc ttua 24
<210> 301
   <211> 25
   <212> DNA
   <213> Artificial Sequence
<220>
   <223> Synthetic DNA/RNA primer, F198
<400> 301
   gagtttcugc agattgactu gcaca 25
<210> 302
   <211> 30
   <212> DNA
   <213> Artificial Sequence
<220>
   <223> Synthetic DNA/RNA primer, F199
<400> 302
   gatcaggaaa caaaaauttg tgctaugcaa 30
<210> 303
   <211> 24
   <212> DNA
   <213> Artificial Sequence
<220>
   <223> Synthetic DNA/RNA primer, F200
<400> 303
   gagctggagg agcuagagct ugat 24
<210> 304
   <211> 24
   <212> DNA
   <213> Artificial Sequence
<220>
   <223> Synthetic DNA/RNA primer, F201
<400> 304
   gagggctguc gtggtagact uaga 24
<210> 305
   <211> 30
   <212> DNA
   <213> Artificial Sequence
<220>
   <223> Synthetic DNA/RNA primer, F202
<400> 305
   gaaagacttc ucaaattgtt gccattucag 30
<210> 306
   <211> 27
   <212> DNA
   <213> Artificial Sequence
<220>
   <223> Synthetic DNA/RNA primer, F203
<400> 306
   gacgaggaag aaaaacaauc ccactug 27
<210> 307
   <211> 27
   <212> DNA
   <213> Artificial Sequence
<220>
   <223> Synthetic DNA/RNA primer, F204
<400> 307
   gagagatcct tucgaagtca tcgtcut 27
<210> 308
   <211> 25
   <212> DNA
   <213> Artificial Sequence
<220>
   <223> Synthetic DNA/RNA primer, F205
<400> 308
   gagggcaaug tcaattagcu ggaac 25
<210> 309
   <211> 27
   <212> DNA
   <213> Artificial Sequence
<220>
   <223> Synthetic DNA/RNA primer, F206
<400> 309
   gacactgtgt tacugccatc gactuac 27
<210> 310
   <211> 28
   <212> DNA
   <213> Artificial Sequence
<220>
   <223> Synthetic DNA/RNA primer, F207
<400> 310
   gacttttacc cucttcagct cagttuct 28
<210> 311
   <211> 30
   <212> DNA
   <213> Artificial Sequence
<220>
   <223> Synthetic DNA/RNA primer, F208
<400> 311
   gatctcctcc aaccuaatag tgtatucaca 30
<210> 312
   <211> 30
   <212> DNA
   <213> Artificial Sequence
<220>
   <223> Synthetic DNA/RNA primer, F209
<400> 312
   gatttcguaa gtgttacuca agaagcagaa 30
<210> 313
   <211> 26
   <212> DNA
   <213> Artificial Sequence
<220>
   <223> Synthetic DNA/RNA primer, F210
<400> 313
   gaatgccccc aagaauccta guagaa 26
<210> 314
   <211> 31
   <212> DNA
   <213> Artificial Sequence
<220>
   <223> Synthetic DNA/RNA primer, F211
<400> 314
   gaagagatga ttgutgaatt ttcctttugg g 31
<210> 315
   <211> 23
   <212> DNA
   <213> Artificial Sequence
<220>
   <223> Synthetic DNA/RNA primer, F212
<400> 315
   gacggaacuc gaatcgcuac cct 23
<210> 316
   <211> 23
   <212> DNA
   <213> Artificial Sequence
<220>
   <223> Synthetic DNA/RNA primer, F213
<400> 316
   gactcgatgc ugttcccagg uac 23
<210> 317
   <211> 29
   <212> DNA
   <213> Artificial Sequence
<220>
   <223> Synthetic DNA/RNA primer, F214
<400> 317
   gatcatacag acacutcatt tggaguacc 29
<210> 318
   <211> 30
   <212> DNA
   <213> Artificial Sequence
<220>
   <223> Synthetic DNA/RNA primer, F215
<400> 318
   gaaaaaataa agctuggctt caagttguct 30
<210> 319
   <211> 32
   <212> DNA
   <213> Artificial Sequence
<220>
   <223> Synthetic DNA/RNA primer, F216
<400> 319
   gaacattgtg accutaattt tgtgatctct ug 32
<210> 320
   <211> 26
   <212> DNA
   <213> Artificial Sequence
<220>
   <223> Synthetic DNA/RNA primer, F217
<400> 320
   gaccccactc augtttagca gatgua 26
<210> 321
   <211> 26
   <212> DNA
   <213> Artificial Sequence
<220>
   <223> Synthetic DNA/RNA primer, F218
<400> 321
   gaggacaggu tttgttgtug aggaag 26
<210> 322
   <211> 35
   <212> DNA
   <213> Artificial Sequence
<220>
   <223> Synthetic DNA/RNA primer, F219
<400> 322
   gaagcaaggt cauaaattat tctccatatt tucca 35
<210> 323
   <211> 34
   <212> DNA
   <213> Artificial Sequence
<220>
   <223> Synthetic DNA/RNA primer, F220
<400> 323
   gatcttttta cctuatagat gggaaacaug agag 34
<210> 324
   <211> 33
   <212> DNA
   <213> Artificial Sequence
<220>
   <223> Synthetic DNA/RNA primer, F221
<400> 324
   gaatgtgtct ttcaugagaa aaacaagatc aut 33
<210> 325
   <211> 29
   <212> DNA
   <213> Artificial Sequence
<220>
   <223> Synthetic DNA/RNA primer, F222
<400> 325
   gatagtagct gauccacaga agttcagua 29
<210> 326
   <211> 35
   <212> DNA
   <213> Artificial Sequence
<220>
   <223> Synthetic DNA/RNA primer, F223
<400> 326
   gaaagactct gaauaccacc atcaagaata auaaa 35
<210> 327
   <211> 25
   <212> DNA
   <213> Artificial Sequence
<220>
   <223> Synthetic DNA/RNA primer, F224
<400> 327
   gaatctacag gccaauggtt cctuc 25
<210> 328
   <211> 29
   <212> DNA
   <213> Artificial Sequence
<220>
   <223> Synthetic DNA/RNA primer, F225
<400> 328
   gagccagtag ucacaaagat ttctuacca 29
<210> 329
   <211> 22
   <212> DNA
   <213> Artificial Sequence
<220>
   <223> Synthetic DNA/RNA primer, F226
<400> 329
   gaagaagaut gggugggcag ac 22
<210> 330
   <211> 33
   <212> DNA
   <213> Artificial Sequence
<220>
   <223> Synthetic DNA/RNA primer, F227
<400> 330
   gaaaccactg atacautttt ctactttccu gaa 33
<210> 331
   <211> 32
   <212> DNA
   <213> Artificial Sequence
<220>
   <223> Synthetic DNA/RNA primer, F228
<400> 331
   gaagacttct tugagatatt tccatagcuc ac 32
<210> 332
   <211> 35
   <212> DNA
   <213> Artificial Sequence
<220>
   <223> Synthetic DNA/RNA primer, F229
<400> 332
   gacatttttg tttatgutat tctctctacc ucagc 35
<210> 333
   <211> 34
   <212> DNA
   <213> Artificial Sequence
<220>
   <223> Synthetic DNA/RNA primer, F230
<400> 333
   gaagttatag gtaaucgatg catatagctc auct 34
<210> 334
   <211> 34
   <212> DNA
   <213> Artificial Sequence
<220>
   <223> Synthetic DNA/RNA primer, F231
<400> 334
   gaaattgttt gtagggutgg ttattagtga cuat 34
<210> 335
   <211> 26
   <212> DNA
   <213> Artificial Sequence
<220>
   <223> Synthetic DNA/RNA primer, F232
<400> 335
   gaccactatg uaagacaaag gcuggt 26
<210> 336
   <211> 28
   <212> DNA
   <213> Artificial Sequence
<220>
   <223> Synthetic DNA/RNA primer, F233
<400> 336
   gagtttctgu agcccatact ttggauga 28
<210> 337
   <211> 25
   <212> DNA
   <213> Artificial Sequence
<220>
   <223> Synthetic DNA/RNA primer, F234
<400> 337
   gacactguga aggccctttc ttcug 25
<210> 338
   <211> 33
   <212> DNA
   <213> Artificial Sequence
<220>
   <223> Synthetic DNA/RNA primer, F235
<400> 338
   gagcatagga gataaucata ggaatcccaa aut 33
<210> 339
   <211> 25
   <212> DNA
   <213> Artificial Sequence
<220>
   <223> Synthetic DNA/RNA primer, F236
<400> 339
   gaagtcactg gaautgttgg gcuac 25
<210> 340
   <211> 23
   <212> DNA
   <213> Artificial Sequence
<220>
   <223> Synthetic DNA/RNA primer, F237
<400> 340
   gactuccagg agccguagag ttt 23
<210> 341
   <211> 31
   <212> DNA
   <213> Artificial Sequence
<220>
   <223> Synthetic DNA/RNA primer, F238
<400> 341
   gaactcttcc tattuttgta gtgacctgtu t 31
<210> 342
   <211> 27
   <212> DNA
   <213> Artificial Sequence
<220>
   <223> Synthetic DNA/RNA primer, F239
<400> 342
   gattcctgau aaagcacagc tgtagug 27
<210> 343
   <211> 21
   <212> DNA
   <213> Artificial Sequence
<220>
   <223> Synthetic DNA/RNA primer, F240
<400> 343
   gattgtucaa gcagcgaguc c 21
<210> 344
   <211> 23
   <212> DNA
   <213> Artificial Sequence
<220>
   <223> Synthetic DNA/RNA primer, F241
<400> 344
   gacatgaacu acctggaccg cut 23
<210> 345
   <211> 22
   <212> DNA
   <213> Artificial Sequence
<220>
   <223> Synthetic DNA/RNA primer, F242
<400> 345
   gaggtgctgu ctgggaagau gt 22
<210> 346
   <211> 25
   <212> DNA
   <213> Artificial Sequence
<220>
   <223> Synthetic DNA/RNA primer, F243
<400> 346
   gaaaagaggc aguagcatct tcucc 25
<210> 347
   <211> 28
   <212> DNA
   <213> Artificial Sequence
<220>
   <223> Synthetic DNA/RNA primer, F244
<400> 347
   gaaatcctgg agcuttggtg tctaatuc 28
<210> 348
   <211> 21
   <212> DNA
   <213> Artificial Sequence
<220>
   <223> Synthetic DNA/RNA primer, F245
<400> 348
   gatgcagaag cgguttctgu g 21
<210> 349
   <211> 27
   <212> DNA
   <213> Artificial Sequence
<220>
   <223> Synthetic DNA/RNA primer, F246
<400> 349
   gagcctcaga gauaaaggca aagatug 27
<210> 350
   <211> 34
   <212> DNA
   <213> Artificial Sequence
<220>
   <223> Synthetic DNA/RNA primer, F247
<400> 350
   gaaccattat ttcttugttt tgtttttcct guat 34
<210> 351
   <211> 29
   <212> DNA
   <213> Artificial Sequence
<220>
   <223> Synthetic DNA/RNA primer, F248
<400> 351
   gataccaacc aaguttcatu aaccacagt 29
<210> 352
   <211> 30
   <212> DNA
   <213> Artificial Sequence
<220>
   <223> Synthetic DNA/RNA primer, F249
<400> 352
   gaccatgttg gucacttact caaagattut 30
<210> 353
   <211> 24
   <212> DNA
   <213> Artificial Sequence
<220>
   <223> Synthetic DNA/RNA primer, F250
<400> 353
   gaaatgggca agguatggat gugg 24
<210> 354
   <211> 23
   <212> DNA
   <213> Artificial Sequence
<220>
   <223> Synthetic DNA/RNA primer, F251
<400> 354
   gatggcgcau cagatcctag tut 23
<210> 355
   <211> 32
   <212> DNA
   <213> Artificial Sequence
<220>
   <223> Synthetic DNA/RNA primer, F252
<400> 355
   gatgtaacaa ccuaaaggga ataggaagaa ug 32
<210> 356
   <211> 22
   <212> DNA
   <213> Artificial Sequence
<220>
   <223> Synthetic DNA/RNA primer, F253
<400> 356
   gaggcactgg utctcattcc ug 22
<210> 357
   <211> 25
   <212> DNA
   <213> Artificial Sequence
<220>
   <223> Synthetic DNA/RNA primer, F254
<400> 357
   gaatgactca auaccaaccc cucca 25
<210> 358
   <211> 32
   <212> DNA
   <213> Artificial Sequence
<220>
   <223> Synthetic DNA/RNA primer, F255
<400> 358
   gacataacca tgauattaat aggactccug ct 32
<210> 359
   <211> 29
   <212> DNA
   <213> Artificial Sequence
<220>
   <223> Synthetic DNA/RNA primer, F256
<400> 359
   gatgaacgca aaaccugttg aagtuaaaa 29
<210> 360
   <211> 34
   <212> DNA
   <213> Artificial Sequence
<220>
   <223> Synthetic DNA/RNA primer, F257
<400> 360
   gacttaaaaa tgtcaauatc tggcctcaaa uacg 34
<210> 361
   <211> 33
   <212> DNA
   <213> Artificial Sequence
<220>
   <223> Synthetic DNA/RNA primer, F258
<400> 361
   gagggaagca atutgctaca ctttaattua aac 33
<210> 362
   <211> 26
   <212> DNA
   <213> Artificial Sequence
<220>
   <223> Synthetic DNA/RNA primer, F259
<400> 362
   gaacagaagc utctaatccu caacgt 26
<210> 363
   <211> 27
   <212> DNA
   <213> Artificial Sequence
<220>
   <223> Synthetic DNA/RNA primer, F260
<400> 363
   gaagttgaug ccaattcaca aucacca 27
<210> 364
   <211> 33
   <212> DNA
   <213> Artificial Sequence
<220>
   <223> Synthetic DNA/RNA primer, F261
<400> 364
   gactgaggtc tatucacttt cttttcatct tug 33
<210> 365
   <211> 30
   <212> DNA
   <213> Artificial Sequence
<220>
   <223> Synthetic DNA/RNA primer, F262
<400> 365
   gactatttgt ttctucccca tggaattguc 30
<210> 366
   <211> 26
   <212> DNA
   <213> Artificial Sequence
<220>
   <223> Synthetic DNA/RNA primer, F263
<400> 366
   gataatcttu gaactgcctg ugcact 26
<210> 367
   <211> 25
   <212> DNA
   <213> Artificial Sequence
<220>
   <223> Synthetic DNA/RNA primer, F264
<400> 367
   gaaagcaaug gcttgggaag uaaga 25
<210> 368
   <211> 35
   <212> DNA
   <213> Artificial Sequence
<220>
   <223> Synthetic DNA/RNA primer, F265
<400> 368
   gatctaaagg tttttcugat ttcctcatta ggaut 35
<210> 369
   <211> 31
   <212> DNA
   <213> Artificial Sequence
<220>
   <223> Synthetic DNA/RNA primer, F266
<400> 369
   gatccagtca tttugagaaa gacaactuac t 31
<210> 370
   <211> 34
   <212> DNA
   <213> Artificial Sequence
<220>
   <223> Synthetic DNA/RNA primer, F267
<400> 370
   gattcattaa tatttucaga tcaccagttg atug 34
<210> 371
   <211> 35
   <212> DNA
   <213> Artificial Sequence
<220>
   <223> Synthetic DNA/RNA primer, F268
<400> 371
   gaattaactg taccuccaac tttcttacta taugc 35
<210> 372
   <211> 32
   <212> DNA
   <213> Artificial Sequence
<220>
   <223> Synthetic DNA/RNA primer, F269
<400> 372
   gattaagaaa cuagaaactg tttagacugc ct 32
<210> 373
   <211> 23
   <212> DNA
   <213> Artificial Sequence
<220>
   <223> Synthetic DNA/RNA primer, F270
<400> 373
   gatggttctg ucgactaaac ugc 23
<210> 374
   <211> 26
   <212> DNA
   <213> Artificial Sequence
<220>
   <223> Synthetic DNA/RNA primer, F271
<400> 374
   gaacggacac uatgtcctta agcuga 26
<210> 375
   <211> 22
   <212> DNA
   <213> Artificial Sequence
<220>
   <223> Synthetic DNA/RNA primer, F272
<400> 375
   gattggcaug gcttctcuag ct 22
<210> 376
   <211> 22
   <212> DNA
   <213> Artificial Sequence
<220>
   <223> Synthetic DNA/RNA primer, F273
<400> 376
   gacaccttcu accgctcacu gc 22
<210> 377
   <211> 24
   <212> DNA
   <213> Artificial Sequence
<220>
   <223> Synthetic DNA/RNA primer, F274
<400> 377
   gaattgacuc tgaatgucgg ccaa 24
<210> 378
   <211> 24
   <212> DNA
   <213> Artificial Sequence
<220>
   <223> Synthetic DNA/RNA primer, F275
<400> 378
   gactctgccc cuaagaaacc ugga 24
<210> 379
   <211> 27
   <212> DNA
   <213> Artificial Sequence
<220>
   <223> Synthetic DNA/RNA primer, F276
<400> 379
   gaattactcu aactttcgca ugcacac 27
<210> 380
   <211> 21
   <212> DNA
   <213> Artificial Sequence
<220>
   <223> Synthetic DNA/RNA primer, F277
<400> 380
   gaagugggca gcagtttcug a 21
<210> 381
   <211> 27
   <212> DNA
   <213> Artificial Sequence
<220>
   <223> Synthetic DNA/RNA primer, F278
<400> 381
   gaaaccaact gcutgtatgc tttcugg 27
<210> 382
   <211> 21
   <212> DNA
   <213> Artificial Sequence
<220>
   <223> Synthetic DNA/RNA primer, F279
<400> 382
   gagcagccut acctggtugg a 21
<210> 383
   <211> 22
   <212> DNA
   <213> Artificial Sequence
<220>
   <223> Synthetic DNA/RNA primer, F280
<400> 383
   gattacagcu cgttggugca gt 22
<210> 384
   <211> 26
   <212> DNA
   <213> Artificial Sequence
<220>
   <223> Synthetic DNA/RNA primer, F281
<400> 384
   gaatggaaac ccugacagag tcttug 26
<210> 385
   <211> 26
   <212> DNA
   <213> Artificial Sequence
<220>
   <223> Synthetic DNA/RNA primer, F282
<400> 385
   gatgttatga gcutagcacc tugcag 26
<210> 386
   <211> 24
   <212> DNA
   <213> Artificial Sequence
<220>
   <223> Synthetic DNA/RNA primer, F283
<400> 386
   gaccagttcu gcagttagag gtug 24
<210> 387
   <211> 30
   <212> DNA
   <213> Artificial Sequence
<220>
   <223> Synthetic DNA/RNA primer, F284
<400> 387
   gatttgattc ttaaucacct aaggauggct 30
<210> 388
   <211> 24
   <212> DNA
   <213> Artificial Sequence
<220>
   <223> Synthetic DNA/RNA primer, F285
<400> 388
   gaatccgtac cutccaccaa tcug 24
<210> 389
   <211> 35
   <212> DNA
   <213> Artificial Sequence
<220>
   <223> Synthetic DNA/RNA primer, F286
<400> 389
   gatataacaa tgaaugacca aaaggaaatu aacaa 35
<210> 390
   <211> 31
   <212> DNA
   <213> Artificial Sequence
<220>
   <223> Synthetic DNA/RNA primer, F287
<400> 390
   gataactttc cataugcaaa cctactggcu a 31
<210> 391
   <211> 23
   <212> DNA
   <213> Artificial Sequence
<220>
   <223> Synthetic DNA/RNA primer, F288
<400> 391
   gaccuggacg tctuggaaaa ggg 23
<210> 392
   <211> 22
   <212> DNA
   <213> Artificial Sequence
<220>
   <223> Synthetic DNA/RNA primer, F289
<400> 392
   gatctggguc aaggauggca ca 22
<210> 393
   <211> 21
   <212> DNA
   <213> Artificial Sequence
<220>
   <223> Synthetic DNA/RNA primer, F290
<400> 393
   gagcccacut cccatcuggg t 21
<210> 394
   <211> 20
   <212> DNA
   <213> Artificial Sequence
<220>
   <223> Synthetic DNA/RNA primer, F291
<400> 394
   gatccccgcu gctgugcaac 20
<210> 395
   <211> 21
   <212> DNA
   <213> Artificial Sequence
<220>
   <223> Synthetic DNA/RNA primer, F292
<400> 395
   gacccacatg uccagcaccu t 21
<210> 396
   <211> 24
   <212> DNA
   <213> Artificial Sequence
<220>
   <223> Synthetic DNA/RNA primer, F293
<400> 396
   gagctgguga aacaggtagu gagt 24
<210> 397
   <211> 22
   <212> DNA
   <213> Artificial Sequence
<220>
   <223> Synthetic DNA/RNA primer, F294
<400> 397
   gactccaggu ccttgtguga gc 22
<210> 398
   <211> 23
   <212> DNA
   <213> Artificial Sequence
<220>
   <223> Synthetic DNA/RNA primer, F295
<400> 398
   gacctcacga acugtgctga ugg 23
<210> 399
   <211> 26
   <212> DNA
   <213> Artificial Sequence
<220>
   <223> Synthetic DNA/RNA primer, F296
<400> 399
   gaggattcga gaagugacag gctaug 26
<210> 400
   <211> 25
   <212> DNA
   <213> Artificial Sequence
<220>
   <223> Synthetic DNA/RNA primer, F297
<400> 400
   gaattggtag cuggtgatgt tccuc 25
<210> 401
   <211> 29
   <212> DNA
   <213> Artificial Sequence
<220>
   <223> Synthetic DNA/RNA primer, F298
<400> 401
   gacagctaat ucatctggag aucaaaccc 29
<210> 402
   <211> 30
   <212> DNA
   <213> Artificial Sequence
<220>
   <223> Synthetic DNA/RNA primer, F299
<400> 402
   gatgtcagtt cccucctttt ctattttcuc 30
<210> 403
   <211> 21
   <212> DNA
   <213> Artificial Sequence
<220>
   <223> Synthetic DNA/RNA primer, F300
<400> 403
   gatgggcacg guaatgcugc t 21
<210> 404
   <211> 20
   <212> DNA
   <213> Artificial Sequence
<220>
   <223> Synthetic DNA/RNA primer, F301
<400> 404
   gactcugcgg tggtuggcat 20
<210> 405
   <211> 24
   <212> DNA
   <213> Artificial Sequence
<220>
   <223> Synthetic DNA/RNA primer, F302
<400> 405
   gactccaccu ccaggaactt acuc 24
<210> 406
   <211> 29
   <212> DNA
   <213> Artificial Sequence
<220>
   <223> Synthetic DNA/RNA primer, F303
<400> 406
   gagggatctt gugaaatgtc atctgacuc 29
<210> 407
   <211> 29
   <212> DNA
   <213> Artificial Sequence
<220>
   <223> Synthetic DNA/RNA primer, F304
<400> 407
   gatcaaccct gtutttctcc ctcttatug 29
<210> 408
   <211> 26
   <212> DNA
   <213> Artificial Sequence
<220>
   <223> Synthetic DNA/RNA primer, F305
<400> 408
   gactguacag catgaagugc aagaac 26
<210> 409
   <211> 26
   <212> DNA
   <213> Artificial Sequence
<220>
   <223> Synthetic DNA/RNA primer, F306
<400> 409
   gaccattaac auggcctacc agagut 26
<210> 410
   <211> 24
   <212> DNA
   <213> Artificial Sequence
<220>
   <223> Synthetic DNA/RNA primer, F307
<400> 410
   gattgccuag acagcaccgt aaug 24
<210> 411
   <211> 25
   <212> DNA
   <213> Artificial Sequence
<220>
   <223> Synthetic DNA/RNA primer, F308
<400> 411
   gagacgcagu ttcttcttct caucg 25
<210> 412
   <211> 30
   <212> DNA
   <213> Artificial Sequence
<220>
   <223> Synthetic DNA/RNA primer, F309
<400> 412
   gatatcgagt gtgugcatat gtgtatgtug 30
<210> 413
   <211> 27
   <212> RNA
   <213> Artificial Sequence
<220>
   <223> Synthetic Primer, F310
<400> 413
   gaagggaaaa ugacaaagaa cagcuca 27
<210> 414
   <211> 23
   <212> DNA
   <213> Artificial Sequence
<220>
   <223> Synthetic DNA/RNA primer, F311
<400> 414
   gaggcctgcu tttggagtcc uat 23
<210> 415
   <211> 23
   <212> DNA
   <213> Artificial Sequence
<220>
   <223> Synthetic DNA/RNA primer, F312
<400> 415
   gagaagagcc uccaccatcu cca 23
<210> 416
   <211> 26
   <212> DNA
   <213> Artificial Sequence
<220>
   <223> Synthetic DNA/RNA primer, F313
<400> 416
   gacacacaug ccatcattcu aggaag 26
<210> 417
   <211> 27
   <212> DNA
   <213> Artificial Sequence
<220>
   <223> Synthetic DNA/RNA primer, F314
<400> 417
   gagaggtttu ccagcactct gacauat 27
<210> 418
   <211> 26
   <212> DNA
   <213> Artificial Sequence
<220>
   <223> Synthetic DNA/RNA primer, F315
<400> 418
   gatcttctct guttcagggc augaac 26
<210> 419
   <211> 25
   <212> DNA
   <213> Artificial Sequence
<220>
   <223> Synthetic DNA/RNA primer, F316
<400> 419
   gaagattcug ccgaaccaat ggauc 25
<210> 420
   <211> 28
   <212> DNA
   <213> Artificial Sequence
<220>
   <223> Synthetic DNA/RNA primer, F317
<400> 420
   gatatgactu gtcacaatgu caccacat 28
<210> 421
   <211> 27
   <212> DNA
   <213> Artificial Sequence
<220>
   <223> Synthetic DNA/RNA primer, F318
<400> 421
   gagtgcccta tuacctcaat catccug 27
<210> 422
   <211> 31
   <212> DNA
   <213> Artificial Sequence
<220>
   <223> Synthetic DNA/RNA primer, F319
<400> 422
   gatcagttac taccugaaaa tgacacttug t 31
<210> 423
   <211> 26
   <212> DNA
   <213> Artificial Sequence
<220>
   <223> Synthetic DNA/RNA primer, F320
<400> 423
   gataaagacc tucttccgtg tgucct 26
<210> 424
   <211> 35
   <212> DNA
   <213> Artificial Sequence
<220>
   <223> Synthetic DNA/RNA primer, F321
<400> 424
   gatacattta tttugagaaa cttgagagaa cutca 35
<210> 425
   <211> 33
   <212> DNA
   <213> Artificial Sequence
<220>
   <223> Synthetic DNA/RNA primer, F322
<400> 425
   gaagatggtg atagaucttt aagagaattg cut 33
<210> 426
   <211> 34
   <212> DNA
   <213> Artificial Sequence
<220>
   <223> Synthetic DNA/RNA primer, F323
<400> 426
   gaagcttttg ataagaguta ggaaatcact aguc 34
<210> 427
   <211> 33
   <212> DNA
   <213> Artificial Sequence
<220>
   <223> Synthetic DNA/RNA primer, F324
<400> 427
   gaaggauaaa aaccagcatt atttattuga gca 33
<210> 428
   <211> 29
   <212> DNA
   <213> Artificial Sequence
<220>
   <223> Synthetic DNA/RNA primer, F325
<400> 428
   gactgaccca uaatcttgca ccattuacc 29
<210> 429
   <211> 29
   <212> DNA
   <213> Artificial Sequence
<220>
   <223> Synthetic DNA/RNA primer, F326
<400> 429
   gatttgaaat gaaugttcac gacaaaugc 29
<210> 430
   <211> 31
   <212> DNA
   <213> Artificial Sequence
<220>
   <223> Synthetic DNA/RNA primer, F327
<400> 430
   gaacaaccaa aacaauacac acagagattu t 31
<210> 431
   <211> 30
   <212> DNA
   <213> Artificial Sequence
<220>
   <223> Synthetic DNA/RNA primer, F328
<400> 431
   gaacggaggg ucatgtgtat attaaguaag 30
<210> 432
   <211> 33
   <212> DNA
   <213> Artificial Sequence
<220>
   <223> Synthetic DNA/RNA primer, F329
<400> 432
   gagaattaag ugtgtactac ucccaagaga aaa 33
<210> 433
   <211> 34
   <212> DNA
   <213> Artificial Sequence
<220>
   <223> Synthetic DNA/RNA primer, F330
<400> 433
   gatataggat gaguagctcc aaattaatga augt 34
<210> 434
   <211> 29
   <212> DNA
   <213> Artificial Sequence
<220>
   <223> Synthetic DNA/RNA primer, F331
<400> 434
   gagctgtaga auagtcaaga ggaatugca 29
<210> 435
   <211> 26
   <212> DNA
   <213> Artificial Sequence
<220>
   <223> Synthetic DNA/RNA primer, F332
<400> 435
   gactcagtgc uctaaatcca gagcug 26
<210> 436
   <211> 33
   <212> DNA
   <213> Artificial Sequence
<220>
   <223> Synthetic DNA/RNA primer, F333
<400> 436
   gaatgaaata tugtcaactc tctuaggcaa aat 33
<210> 437
   <211> 29
   <212> DNA
   <213> Artificial Sequence
<220>
   <223> Synthetic DNA/RNA primer, F334
<400> 437
   gatacttugc aaagctgaat uagacagca 29
<210> 438
   <211> 26
   <212> DNA
   <213> Artificial Sequence
<220>
   <223> Synthetic DNA/RNA primer, F335
<400> 438
   gaacatgagc aucacatttt cctugg 26
<210> 439
   <211> 29
   <212> DNA
   <213> Artificial Sequence
<220>
   <223> Synthetic DNA/RNA primer, F336
<400> 439
   gatgccttat gaauatattc acgcugact 29
<210> 440
   <211> 22
   <212> RNA
   <213> Artificial Sequence
<220>
   <223> Synthetic Primer, F337
<400> 440
   gaaccaggua agcaccgaag uc 22
<210> 441
   <211> 19
   <212> DNA
   <213> Artificial Sequence
<220>
   <223> Synthetic DNA/RNA primer, F338
<400> 441
   gaaccaagcc gcuggtuca 19
<210> 442
   <211> 25
   <212> DNA
   <213> Artificial Sequence
<220>
   <223> Synthetic DNA/RNA primer, F339
<400> 442
   gaagaccccu ttaactcaag acugc 25
<210> 443
   <211> 27
   <212> DNA
   <213> Artificial Sequence
<220>
   <223> Synthetic DNA/RNA primer, F340
<400> 443
   gagcgaggau atctggaaga aatucga 27
<210> 444
   <211> 19
   <212> DNA
   <213> Artificial Sequence
<220>
   <223> Synthetic DNA/RNA primer, F341
<400> 444
   gautcuccac ggccgacca 19
<210> 445
   <211> 27
   <212> DNA
   <213> Artificial Sequence
<220>
   <223> Synthetic DNA/RNA primer, F342
<400> 445
   gaaagtcccu caaaaatagg aggugct 27
<210> 446
   <211> 27
   <212> RNA
   <213> Artificial Sequence
<220>
   <223> Synthetic Primer, F343
<400> 446
   gagacagauc agcaacaacc gaaaaug 27
<210> 447
   <211> 29
   <212> DNA
   <213> Artificial Sequence
<220>
   <223> Synthetic DNA/RNA primer, F344
<400> 447
   gatttgtcca gagaccuttc taacgtaut 29
<210> 448
   <211> 24
   <212> DNA
   <213> Artificial Sequence
<220>
   <223> Synthetic DNA/RNA primer, F345
<400> 448
   gatttcugaa gaggacttgt ugcg 24
<210> 449
   <211> 32
   <212> DNA
   <213> Artificial Sequence
<220>
   <223> Synthetic DNA/RNA primer, F346
<400> 449
   gagattttuc agttaataat aucccccgag ct 32
<210> 450
   <211> 19
   <212> DNA
   <213> Artificial Sequence
<220>
   <223> Synthetic DNA/RNA primer, F347
<400> 450
   gaaatcgccu ccggauccc 19
<210> 451
   <211> 34
   <212> DNA
   <213> Artificial Sequence
<220>
   <223> Synthetic DNA/RNA primer, F348
<400> 451
   gagtcattcc ttcuttttaa aatggtgctu aagt 34
<210> 452
   <211> 22
   <212> DNA
   <213> Artificial Sequence
<220>
   <223> Synthetic DNA/RNA primer, F349
<400> 452
   gaggtccccc accuctcttt ug 22
<210> 453
   <211> 23
   <212> DNA
   <213> Artificial Sequence
<220>
   <223> Synthetic DNA/RNA primer, F350
<400> 453
   gactcuccag gaaggctcac auc 23
<210> 454
   <211> 21
   <212> DNA
   <213> Artificial Sequence
<220>
   <223> Synthetic DNA/RNA primer, F351
<400> 454
   gatggcatug ccttgtcctu g 21
<210> 455
   <211> 27
   <212> DNA
   <213> Artificial Sequence
<220>
   <223> Synthetic DNA/RNA primer, F352
<400> 455
   gaggcagaaa accaaaacau tggctua 27
<210> 456
   <211> 35
   <212> DNA
   <213> Artificial Sequence
<220>
   <223> Synthetic DNA/RNA primer, F353
<400> 456
   gaaattgttc ctcaaguttg tttaaggact uaaaa 35
<210> 457
   <211> 29
   <212> DNA
   <213> Artificial Sequence
<220>
   <223> Synthetic DNA/RNA primer, F354
<400> 457
   gaagtggtat cauccccatt taatagcug 29
<210> 458
   <211> 32
   <212> DNA
   <213> Artificial Sequence
<220>
   <223> Synthetic DNA/RNA primer, F355
<400> 458
   gaacaaatac aaaacugtcc acatctatgt ug 32
<210> 459
   <211> 34
   <212> DNA
   <213> Artificial Sequence
<220>
   <223> Synthetic DNA/RNA primer, F356
<400> 459
   gaaacaaacc atagcuataa tgaagaactt gcua 34
<210> 460
   <211> 34
   <212> DNA
   <213> Artificial Sequence
<220>
   <223> Synthetic DNA/RNA primer, F257
<400> 460
   gaaatagttg atcauacttt gtaacagaau caca 34
<210> 461
   <211> 32
   <212> DNA
   <213> Artificial Sequence
<220>
   <223> Synthetic DNA/RNA primer, F358
<400> 461
   gactcatctc cctutaattt tggcacatta ut 32
<210> 462
   <211> 31
   <212> DNA
   <213> Artificial Sequence
<220>
   <223> Synthetic DNA/RNA primer, F359
<400> 462
   gaaactatct uctttggact tcugaagaga c 31
<210> 463
   <211> 34
   <212> DNA
   <213> Artificial Sequence
<220>
   <223> Synthetic DNA/RNA primer, F360
<400> 463
   gaagtaaata atggttuctc cttctcttac ttug 34
<210> 464
   <211> 26
   <212> DNA
   <213> Artificial Sequence
<220>
   <223> Synthetic DNA/RNA primer, F361
<400> 464
   gattcctggu ggcattcaau aaagca 26
<210> 465
   <211> 24
   <212> DNA
   <213> Artificial Sequence
<220>
   <223> Synthetic DNA/RNA primer, F362
<400> 465
   gaaggagcaa cutagggatc uggt 24
<210> 466
   <211> 23
   <212> DNA
   <213> Artificial Sequence
<220>
   <223> Synthetic DNA/RNA primer, F363
<400> 466
   gaacccctaa uctggtcaac cug 23
<210> 467
   <211> 32
   <212> DNA
   <213> Artificial Sequence
<220>
   <223> Synthetic DNA/RNA primer, F364
<400> 467
   gaagaaatag aaaacuacag gacgttaucc ag 32
<210> 468
   <211> 27
   <212> DNA
   <213> Artificial Sequence
<220>
   <223> Synthetic DNA/RNA primer, F365
<400> 468
   gatccgcttt cuaaaatgtc agttguc 27
<210> 469
   <211> 22
   <212> RNA
   <213> Artificial Sequence
<220>
   <223> Synthetic Primer, R97
<400> 469
   gaggcaggag acccuguagg ag 22
<210> 470
   <211> 26
   <212> DNA
   <213> Artificial Sequence
<220>
   <223> Synthetic DNA/RNA primer, R98
<400> 470
   gaggatauat gccauacccc agcaaa 26
<210> 471
   <211> 29
   <212> DNA
   <213> Artificial Sequence
<220>
   <223> Synthetic DNA/RNA primer, R99
<400> 471
   gaccaagaaa ggcutgtgtc tacatttut 29
<210> 472
   <211> 29
   <212> DNA
   <213> Artificial Sequence
<220>
   <223> Synthetic DNA/RNA primer, R100
<400> 472
   gaccaaauca agaaacctgt tugagagaa 29
<210> 473
   <211> 25
   <212> DNA
   <213> Artificial Sequence
<220>
   <223> Synthetic DNA/RNA primer, R101
<400> 473
   gacacttccc utgtgggaat gucaa 25
<210> 474
   <211> 25
   <212> DNA
   <213> Artificial Sequence
<220>
   <223> Synthetic DNA/RNA primer, R102
<400> 474
   gatgcatcag aacccucctt gaauc 25
<210> 475
   <211> 24
   <212> RNA
   <213> Artificial Sequence
<220>
   <223> Synthetic Primer, R103
<400> 475
   gagguccacg gauccagaaa caag 24
<210> 476
   <211> 23
   <212> DNA
   <213> Artificial Sequence
<220>
   <223> Synthetic DNA/RNA primer, R104
<400> 476
   gaggttcctc cuctcctggt cuc 23
<210> 477
   <211> 29
   <212> DNA
   <213> Artificial Sequence
<220>
   <223> Synthetic DNA/RNA primer, R105
<400> 477
   gagctcacta acuaacgtga aagcctuac 29
<210> 478
   <211> 29
   <212> DNA
   <213> Artificial Sequence
<220>
   <223> Synthetic DNA/RNA primer, R106
<400> 478
   gaggttttgc acaagutagg tttgtttug 29
<210> 479
   <211> 35
   <212> DNA
   <213> Artificial Sequence
<220>
   <223> Synthetic DNA/RNA primer, R107
<400> 479
   gaacctttat atcgtuactc tgaatcttat ctucc 35
<210> 480
   <211> 27
   <212> DNA
   <213> Artificial Sequence
<220>
   <223> Synthetic DNA/RNA primer, R108
<400> 480
   gatgactctg tcucctcttg tcttcuc 27
<210> 481
   <211> 28
   <212> DNA
   <213> Artificial Sequence
<220>
   <223> Synthetic DNA/RNA primer, R109
<400> 481
   gaaaacgttt tucaccttag catttugt 28
<210> 482
   <211> 30
   <212> DNA
   <213> Artificial Sequence
<220>
   <223> Synthetic DNA/RNA primer, R110
<400> 482
   gacaccatcu ccatatcatu gagaccaaat 30
<210> 483
   <211> 29
   <212> DNA
   <213> Artificial Sequence
<220>
   <223> Synthetic DNA/RNA primer, R111
<400> 483
   gacgacagac uactttggtt ctctttugt 29
<210> 484
   <211> 22
   <212> DNA
   <213> Artificial Sequence
<220>
   <223> Synthetic DNA/RNA primer, R112
<400> 484
   gactcacuga caagctccuc gt 22
<210> 485
   <211> 26
   <212> DNA
   <213> Artificial Sequence
<220>
   <223> Synthetic DNA/RNA primer, R113
<400> 485
   gagccttttc ttutgcttcc cttgut 26
<210> 486
   <211> 19
   <212> RNA
   <213> Artificial Sequence
<220>
   <223> Synthetic Primer, R114
<400> 486
   gacaggaccu ggcccugac 19
<210> 487
   <211> 25
   <212> DNA
   <213> Artificial Sequence
<220>
   <223> Synthetic DNA/RNA primer, R115
<400> 487
   gacccaucac acaccataac uccac 25
<210> 488
   <211> 22
   <212> DNA
   <213> Artificial Sequence
<220>
   <223> Synthetic DNA/RNA primer, R116
<400> 488
   gagctguccc ctcaccatuc ag 22
<210> 489
   <211> 30
   <212> DNA
   <213> Artificial Sequence
<220>
   <223> Synthetic DNA/RNA primer, R117
<400> 489
   gatcacaacc cacugaggta tatgtauagg 30
<210> 490
   <211> 22
   <212> RNA
   <213> Artificial Sequence
<220>
   <223> Synthetic Primer, R118
<400> 490
   gacaugcacc ggaaaagcga ug 22
<210> 491
   <211> 26
   <212> DNA
   <213> Artificial Sequence
<220>
   <223> Synthetic DNA/RNA primer, R119
<400> 491
   gacatttcta ggutacaggc cuggat 26
<210> 492
   <211> 20
   <212> DNA
   <213> Artificial Sequence
<220>
   <223> Synthetic DNA/RNA primer, R120
<400> 492
   gactccaugc ccctcacuca 20
<210> 493
   <211> 26
   <212> DNA
   <213> Artificial Sequence
<220>
   <223> Synthetic DNA/RNA primer, R121
<400> 493
   gaattgaaaa tcutcctgcc tuccct 26
<210> 494
   <211> 26
   <212> DNA
   <213> Artificial Sequence
<220>
   <223> Synthetic DNA/RNA primer, R122
<400> 494
   gagcaaaagt ggucctctct gaauct 26
<210> 495
   <211> 26
   <212> DNA
   <213> Artificial Sequence
<220>
   <223> Synthetic DNA/RNA primer, R123
<400> 495
   gaatatcauc cagcctgtgt cttucc 26
<210> 496
   <211> 24
   <212> DNA
   <213> Artificial Sequence
<220>
   <223> Synthetic DNA/RNA primer, R124
<400> 496
   gagagggaag gcaggauctc uaac 24
<210> 497
   <211> 25
   <212> DNA
   <213> Artificial Sequence
<220>
   <223> Synthetic DNA/RNA primer, R125
<400> 497
   gaccaggcaa uggaaagggt acaua 25
<210> 498
   <211> 27
   <212> DNA
   <213> Artificial Sequence
<220>
   <223> Synthetic DNA/RNA primer, R126
<400> 498
   gagaauaaag aggagcaggt ugaggaa 27
<210> 499
   <211> 22
   <212> DNA
   <213> Artificial Sequence
<220>
   <223> Synthetic DNA/RNA primer, R127
<400> 499
   gagggcaaau gagcctcuca gt 22
<210> 500
   <211> 29
   <212> DNA
   <213> Artificial Sequence
<220>
   <223> Synthetic DNA/RNA primer, R128
<400> 500
   gatccagatt gaucttggga gtguaaaaa 29
<210> 501
   <211> 25
   <212> DNA
   <213> Artificial Sequence
<220>
   <223> Synthetic DNA/RNA primer, R129
<400> 501
   gagtctttgt gutcccggac auagt 25
<210> 502
   <211> 22
   <212> DNA
   <213> Artificial Sequence
<220>
   <223> Synthetic DNA/RNA primer, R130
<400> 502
   gagggtcuga cgggtagagu gt 22
<210> 503
   <211> 30
   <212> DNA
   <213> Artificial Sequence
<220>
   <223> Synthetic DNA/RNA primer, R131
<400> 503
   gagcttgctc tgauaggaaa atgagatcua 30
<210> 504
   <211> 24
   <212> DNA
   <213> Artificial Sequence
<220>
   <223> Synthetic DNA/RNA primer, R132
<400> 504
   gacctcttcc ucaggattgc ctut 24
<210> 505
   <211> 30
   <212> DNA
   <213> Artificial Sequence
<220>
   <223> Synthetic DNA/RNA primer, R133
<400> 505
   gatcagtccg gtuttatttg catcatagut 30
<210> 506
   <211> 27
   <212> DNA
   <213> Artificial Sequence
<220>
   <223> Synthetic DNA/RNA primer, R134
<400> 506
   gacccaaaga cuctccaaga tgggaua 27
<210> 507
   <211> 24
   <212> DNA
   <213> Artificial Sequence
<220>
   <223> Synthetic DNA/RNA primer, R135
<400> 507
   gatccagacc agggugttgt ttuc 24
<210> 508
   <211> 25
   <212> DNA
   <213> Artificial Sequence
<220>
   <223> Synthetic DNA/RNA primer, R136
<400> 508
   gagtgccagg gaccutacct tauac 25
<210> 509
   <211> 22
   <212> DNA
   <213> Artificial Sequence
<220>
   <223> Synthetic DNA/RNA primer, R137
<400> 509
   gactgaggut cagagccaug ga 22
<210> 510
   <211> 25
   <212> DNA
   <213> Artificial Sequence
<220>
   <223> Synthetic DNA/RNA primer, R138
<400> 510
   gagucatatc uccccaaacc ccaat 25
<210> 511
   <211> 24
   <212> DNA
   <213> Artificial Sequence
<220>
   <223> Synthetic DNA/RNA primer, R139
<400> 511
   gagccatagg gcauaagctg tguc 24
<210> 512
   <211> 25
   <212> DNA
   <213> Artificial Sequence
<220>
   <223> Synthetic DNA/RNA primer, R140
<400> 512
   gaccttggtc cutcacctaa cctug 25
<210> 513
   <211> 23
   <212> DNA
   <213> Artificial Sequence
<220>
   <223> Synthetic DNA/RNA primer, R141
<400> 513
   gaccctcutt agccauggca agg 23
<210> 514
   <211> 24
   <212> DNA
   <213> Artificial Sequence
<220>
   <223> Synthetic DNA/RNA primer, R142
<400> 514
   gatggtctcu cattctccca uccc 24
<210> 515
   <211> 24
   <212> DNA
   <213> Artificial Sequence
<220>
   <223> Synthetic DNA/RNA primer, R143
<400> 515
   gactcctccu gtgatctgca auct 24
<210> 516
   <211> 30
   <212> DNA
   <213> Artificial Sequence
<220>
   <223> Synthetic DNA/RNA primer, R144
<400> 516
   gagatgauga agatgatugg gaaacacaag 30
<210> 517
   <211> 20
   <212> DNA
   <213> Artificial Sequence
<220>
   <223> Synthetic DNA/RNA primer, R145
<400> 517
   gagggctgug cgtcactgua 20
<210> 518
   <211> 20
   <212> DNA
   <213> Artificial Sequence
<220>
   <223> Synthetic DNA/RNA primer, R146
<400> 518
   gaggagccca ggccuttcut 20
<210> 519
   <211> 21
   <212> DNA
   <213> Artificial Sequence
<220>
   <223> Synthetic DNA/RNA primer, R147
<400> 519
   gagcgtccua ctggcaugac c 21

<210> 520
   <211> 21
   <212> DNA
   <213> Artificial Sequence
<220>
   <223> Synthetic DNA/RNA primer, R148
<400> 520
   gaccacucac aggtcgtgug t 21
<210> 521
   <211> 27
   <212> DNA
   <213> Artificial Sequence
<220>
   <223> Synthetic DNA/RNA primer, R149
<400> 521
   gaacatgatg gaugtcacgt tcucaaa 27
<210> 522
   <211> 26
   <212> DNA
   <213> Artificial Sequence
<220>
   <223> Synthetic DNA/RNA primer, R150
<400> 522
   gatgttaacc utgcagaatg gucgat 26
<210> 523
   <211> 22
   <212> DNA
   <213> Artificial Sequence
<220>
   <223> Synthetic DNA/RNA primer, R151
<400> 523
   gacugcagga ttccuaccgg aa 22
<210> 524
   <211> 24
   <212> DNA
   <213> Artificial Sequence
<220>
   <223> Synthetic DNA/RNA primer, R152
<400> 524
   gaatcaccaa auggcaccau acga 24
<210> 525
   <211> 27
   <212> DNA
   <213> Artificial Sequence
<220>
   <223> Synthetic DNA/RNA primer, R153
<400> 525
   gaagttcaag cugaagaaga tguggaa 27
<210> 526
   <211> 24
   <212> DNA
   <213> Artificial Sequence
<220>
   <223> Synthetic DNA/RNA primer, R154
<400> 526
   gactgacacc uagctgtgat ccug 24
<210> 527
   <211> 30
   <212> DNA
   <213> Artificial Sequence
<220>
   <223> Synthetic DNA/RNA primer, R155
<400> 527
   gaactgatat gguagacaga gccuaaacat 30
<210> 528
   <211> 26
   <212> DNA
   <213> Artificial Sequence
<220>
   <223> Synthetic DNA/RNA primer, R156
<400> 528
   gagtctcagt cautagagca ctcugg 26
<210> 529
   <211> 27
   <212> DNA
   <213> Artificial Sequence
<220>
   <223> Synthetic DNA/RNA primer, R157
<400> 529
   gatttcatac ugaccaaaac ucagcct 27
<210> 530
   <211> 23
   <212> DNA
   <213> Artificial Sequence
<220>
   <223> Synthetic DNA/RNA primer, R158
<400> 530
   gagacacggc uttacctcca aug 23
<210> 531
   <211> 29
   <212> DNA
   <213> Artificial Sequence
<220>
   <223> Synthetic DNA/RNA primer, R159
<400> 531
   gaaggcctgc ugaaaatgac tgaatauaa 29
<210> 532
   <211> 31
   <212> DNA
   <213> Artificial Sequence
<220>
   <223> Synthetic DNA/RNA primer, R160
<400> 532
   gagtaaaagg ugcactgtaa taauccagac t 31
<210> 533
   <211> 29
   <212> DNA
   <213> Artificial Sequence
<220>
   <223> Synthetic DNA/RNA primer, R161
<400> 533
   gagactctga agaugtacct atggtccua 29
<210> 534
   <211> 29
   <212> DNA
   <213> Artificial Sequence
<220>
   <223> Synthetic DNA/RNA primer, R162
<400> 534
   gagctttuca aaaggctuaa acacaggat 29
<210> 535
   <211> 29
   <212> DNA
   <213> Artificial Sequence
<220>
   <223> Synthetic DNA/RNA primer, R163
<400> 535
   gagcaaacca caaaaguata ctccauggt 29
<210> 536
   <211> 29
   <212> DNA
   <213> Artificial Sequence
<220>
   <223> Synthetic DNA/RNA primer, R164
<400> 536
   gatctgactt gguggtaaac ttttgagut 29
<210> 537
   <211> 26
   <212> DNA
   <213> Artificial Sequence
<220>
   <223> Synthetic DNA/RNA primer, R165
<400> 537
   gagttcttgc uggtgtgaaa tgacug 26
<210> 538
   <211> 26
   <212> DNA
   <213> Artificial Sequence
<220>
   <223> Synthetic DNA/RNA primer, R166
<400> 538
   gacaccccca ggautctuac agaaaa 26
<210> 539
   <211> 27
   <212> DNA
   <213> Artificial Sequence
<220>
   <223> Synthetic DNA/RNA primer, R167
<400> 539
   gacatctctu ggaaactccc atctuga 27
<210> 540
   <211> 26
   <212> DNA
   <213> Artificial Sequence
<220>
   <223> Synthetic DNA/RNA primer, R168
<400> 540
   gaccacatgu gtccagtgaa aaucct 26
<210> 541
   <211> 23
   <212> DNA
   <213> Artificial Sequence
<220>
   <223> Synthetic DNA/RNA primer, R169
<400> 541
   gaagtgaagg aggaugagcc uga 23
<210> 542
   <211> 30
   <212> DNA
   <213> Artificial Sequence
<220>
   <223> Synthetic DNA/RNA primer, R170
<400> 542
   gagtggaaga tccaauccat ttttgttguc 30
<210> 543
   <211> 30
   <212> DNA
   <213> Artificial Sequence
<220>
   <223> Synthetic DNA/RNA primer, R171
<400> 543
   gaagcatcag cauttgactt taccttauca 30
<210> 544
   <211> 29
   <212> DNA
   <213> Artificial Sequence
<220>
   <223> Synthetic DNA/RNA primer, R172
<400> 544
   gacataagag agaagguttg actgccaua 29
<210> 545
   <211> 28
   <212> DNA
   <213> Artificial Sequence
<220>
   <223> Synthetic DNA/RNA primer, R173
<400> 545
   gaagaaaacc atuacttgtc catcguct 28
<210> 546
   <211> 23
   <212> DNA
   <213> Artificial Sequence
<220>
   <223> Synthetic DNA/RNA primer, R174
<400> 546
   gaccttgtug ggacctcaga ugt 23
<210> 547
   <211> 23
   <212> DNA
   <213> Artificial Sequence
<220>
   <223> Synthetic DNA/RNA primer, R175
<400> 547
   gagtgguagc agtggaugca gaa 23
<210> 548
   <211> 25
   <212> DNA
   <213> Artificial Sequence
<220>
   <223> Synthetic DNA/RNA primer, R176
<400> 548
   gaaggcccca uacaatttga ugaca 25
<210> 549
   <211> 20
   <212> DNA
   <213> Artificial Sequence
<220>
   <223> Synthetic DNA/RNA primer, R177
<400> 549
   gaccatggug caccugggat 20
<210> 550
   <211> 28
   <212> DNA
   <213> Artificial Sequence
<220>
   <223> Synthetic DNA/RNA primer, R178
<400> 550
   gaactttgcg uggtgtagat atgaucaa 28
<210> 551
   <211> 23
   <212> DNA
   <213> Artificial Sequence
<220>
   <223> Synthetic DNA/RNA primer, R179
<400> 551
   gagtcttcac ucacctcgga uga 23
<210> 552
   <211> 22
   <212> DNA
   <213> Artificial Sequence
<220>
   <223> Synthetic DNA/RNA primer, R180
<400> 552
   gacagguacg cctccagaug ag 22
<210> 553
   <211> 21
   <212> DNA
   <213> Artificial Sequence
<220>
   <223> Synthetic DNA/RNA primer, R181
<400> 553
   gaaacucccg caggttuccc t 21
<210> 554
   <211> 22
   <212> DNA
   <213> Artificial Sequence
<220>
   <223> Synthetic DNA/RNA primer, R182
<400> 554
   gagtgcctug ccctttttgu gg 22
<210> 555
   <211> 20
   <212> RNA
   <213> Artificial Sequence
<220>
   <223> Synthetic Primer, R183
<400> 555
   gaccgggaug ccaggauacg 20
<210> 556
   <211> 22
   <212> DNA
   <213> Artificial Sequence
<220>
   <223> Synthetic DNA/RNA primer, R184
<400> 556
   gagggcugta cctccucaga ga 22
<210> 557
   <211> 21
   <212> RNA
   <213> Artificial Sequence
<220>
   <223> Synthetic Primer, R185
<400> 557
   gaacaggcug cccaagggcu a 21
<210> 558
   <211> 23
   <212> DNA
   <213> Artificial Sequence
<220>
   <223> Synthetic DNA/RNA primer, R186
<400> 558
   gacagugatc agaugagcag cag 23
<210> 559
   <211> 22
   <212> DNA
   <213> Artificial Sequence
<220>
   <223> Synthetic DNA/RNA primer, R187
<400> 559
   gaacgguctu ggaacccaga ga 22
<210> 560
   <211> 24
   <212> DNA
   <213> Artificial Sequence
<220>
   <223> Synthetic DNA/RNA primer, R188
<400> 560
   gagctattga ugtctgcagt cugg 24
<210> 561
   <211> 22
   <212> DNA
   <213> Artificial Sequence
<220>
   <223> Synthetic DNA/RNA primer, R189
<400> 561
   gatugactug ccggaagagc ct 22
<210> 562
   <211> 22
   <212> DNA
   <213> Artificial Sequence
<220>
   <223> Synthetic DNA/RNA primer, R190
<400> 562
   gaagaccucc gagtcactcc ug 22
<210> 563
   <211> 28
   <212> DNA
   <213> Artificial Sequence
<220>
   <223> Synthetic DNA/RNA primer, R191
<400> 563
   gaaaaagact cggaugatgt acctaugg 28
<210> 564
   <211> 26
   <212> DNA
   <213> Artificial Sequence
<220>
   <223> Synthetic DNA/RNA primer, R192
<400> 564
   gattcctttc tucccagaga catugc 26
<210> 565
   <211> 22
   <212> DNA
   <213> Artificial Sequence
<220>
   <223> Synthetic DNA/RNA primer, R193
<400> 565
   gaacatcccu ctctgctcug ca 22
<210> 566
   <211> 30
   <212> DNA
   <213> Artificial Sequence
<220>
   <223> Synthetic DNA/RNA primer, R194
<400> 566
   gaggctggtt autgaaacct tgtttuacat 30
<210> 567
   <211> 25
   <212> DNA
   <213> Artificial Sequence
<220>
   <223> Synthetic DNA/RNA primer, R195
<400> 567
   gactaccccc guaccaagua caaac 25
<210> 568
   <211> 19
   <212> DNA
   <213> Artificial Sequence
<220>
   <223> Synthetic DNA/RNA primer, R196
<400> 568
   gatcgucgaa gcggcugac 19
<210> 569
   <211> 24
   <212> DNA
   <213> Artificial Sequence
<220>
   <223> Synthetic DNA/RNA primer, R197
<400> 569
   gagactctgu aggctgcagt tcuc 24
<210> 570
   <211> 27
   <212> DNA
   <213> Artificial Sequence
<220>
   <223> Synthetic DNA/RNA primer, R198
<400> 570
   gacttcttcc uacctgtttc ccaugac 27
<210> 571
   <211> 25
   <212> DNA
   <213> Artificial Sequence
<220>
   <223> Synthetic DNA/RNA primer, R199
<400> 571
   gaggacccau tagaaccaac uccat 25
<210> 572
   <211> 23
   <212> DNA
   <213> Artificial Sequence
<220>
   <223> Synthetic DNA/RNA primer, R200
<400> 572
   gaggcttgug ggagacctug aac 23
<210> 573
   <211> 29
   <212> DNA
   <213> Artificial Sequence
<220>
   <223> Synthetic DNA/RNA primer, R201
<400> 573
   gacctggtag tcucaagcag atgttaaug 29
<210> 574
   <211> 29
   <212> DNA
   <213> Artificial Sequence
<220>
   <223> Synthetic DNA/RNA primer, R202
<400> 574
   gaaacggaca tgaguttgtt ttccttcua 29
<210> 575
   <211> 28
   <212> DNA
   <213> Artificial Sequence
<220>
   <223> Synthetic DNA/RNA primer, R203
<400> 575
   gacagccaac aagautctga agaacaug 28
<210> 576
   <211> 24
   <212> DNA
   <213> Artificial Sequence
<220>
   <223> Synthetic DNA/RNA primer, R204
<400> 576
   gatccctagg uagctaaccc cuac 24
<210> 577
   <211> 26
   <212> DNA
   <213> Artificial Sequence
<220>
   <223> Synthetic DNA/RNA primer, R205
<400> 577
   gaaaaacacg gcaugtgaac attcug 26
<210> 578
   <211> 28
   <212> DNA
   <213> Artificial Sequence
<220>
   <223> Synthetic DNA/RNA primer, R206
<400> 578
   gagtattcau cgagattuag cagccaga 28
<210> 579
   <211> 26
   <212> DNA
   <213> Artificial Sequence
<220>
   <223> Synthetic DNA/RNA primer, R207
<400> 579
   gagagagagg acugactatc ggacug 26
<210> 580
   <211> 26
   <212> DNA
   <213> Artificial Sequence
<220>
   <223> Synthetic DNA/RNA primer, R208
<400> 580
   gagactguca agcagagaat ggguac 26
<210> 581
   <211> 35
   <212> DNA
   <213> Artificial Sequence
<220>
   <223> Synthetic DNA/RNA primer, R209
<400> 581
   gagaatagga tattguatca taccaatttc ucgat 35
<210> 582
   <211> 34
   <212> DNA
   <213> Artificial Sequence
<220>
   <223> Synthetic DNA/RNA primer, R210
<400> 582
   gaacgaaaat guaagaagat tcatctugaa gaag 34
<210> 583
   <211> 30
   <212> DNA
   <213> Artificial Sequence
<220>
   <223> Synthetic DNA/RNA primer, R211
<400> 583
   gaaaagccat tttuccagat actagagugt 30
<210> 584
   <211> 22
   <212> RNA
   <213> Artificial Sequence
<220>
   <223> Synthetic Primer, R212
<400> 584
   gagggucccc aagacaccua cg 22
<210> 585
   <211> 23
   <212> DNA
   <213> Artificial Sequence
<220>
   <223> Synthetic DNA/RNA primer, R213
<400> 585
   gaccgagaac ugagggtggu aca 23
<210> 586
   <211> 28
   <212> DNA
   <213> Artificial Sequence
<220>
   <223> Synthetic DNA/RNA primer, R214
<400> 586
   gatactagaa cucaaaacac tggctgut 28
<210> 587
   <211> 32
   <212> DNA
   <213> Artificial Sequence
<220>
   <223> Synthetic DNA/RNA primer, R215
<400> 587
   gagtaagtct tcacuttcag attttagtug gg 32
<210> 588
   <211> 27
   <212> DNA
   <213> Artificial Sequence
<220>
   <223> Synthetic DNA/RNA primer, R216
<400> 588
   gatgcttcct gguctttagg atttcut 27
<210> 589
   <211> 25
   <212> DNA
   <213> Artificial Sequence
<220>
   <223> Synthetic DNA/RNA primer, R217
<400> 589
   gattttactt cugcttggtg gcaug 25
<210> 590
   <211> 32
   <212> DNA
   <213> Artificial Sequence
<220>
   <223> Synthetic DNA/RNA primer, R218
<400> 590
   gattttaccc tcauggctta gtagcattat ut 32
<210> 591
   <211> 31
   <212> DNA
   <213> Artificial Sequence
<220>
   <223> Synthetic DNA/RNA primer, R219
<400> 591
   gaaaaaatat ucatccagct ucaggaaaag g 31
<210> 592
   <211> 25
   <212> DNA
   <213> Artificial Sequence
<220>
   <223> Synthetic DNA/RNA primer, R220
<400> 592
   gaatcagtcu ggtggatggg uaaca 25
<210> 593
   <211> 35
   <212> DNA
   <213> Artificial Sequence
<220>
   <223> Synthetic DNA/RNA primer, R221
<400> 593
   gactaataat gaataautgg gtatgaggcu acagt 35
<210> 594
   <211> 25
   <212> DNA
   <213> Artificial Sequence
<220>
   <223> Synthetic DNA/RNA primer, R222
<400> 594
   gatgtgagag agcaaucaag gagug 25
<210> 595
   <211> 29
   <212> DNA
   <213> Artificial Sequence
<220>
   <223> Synthetic DNA/RNA primer, R223
<400> 595
   gagtctgaga guagaaggca gattctgua 29
<210> 596
   <211> 26
   <212> DNA
   <213> Artificial Sequence
<220>
   <223> Synthetic DNA/RNA primer, R224
<400> 596
   gaaacttugc ggagatcuga aaacca 26
<210> 597
   <211> 30
   <212> DNA
   <213> Artificial Sequence
<220>
   <223> Synthetic DNA/RNA primer, R225
<400> 597
   gactctaaag aaggaaguga gaacttcucc 30
<210> 598
   <211> 31
   <212> DNA
   <213> Artificial Sequence
<220>
   <223> Synthetic DNA/RNA primer, R226
<400> 598
   gactttcttc acucaaagtg cctatttuga c 31
<210> 599
   <211> 32
   <212> DNA
   <213> Artificial Sequence
<220>
   <223> Synthetic DNA/RNA primer, R227
<400> 599
   gattcttttg agaacugagt gatttaugac ct 32
<210> 600
   <211> 33
   <212> DNA
   <213> Artificial Sequence
<220>
   <223> Synthetic DNA/RNA primer, R228
<400> 600
   gaagaagtua gaaacagaac tgtatguaag cat 33
<210> 601
   <211> 34
   <212> DNA
   <213> Artificial Sequence
<220>
   <223> Synthetic DNA/RNA primer, R229
<400> 601
   gagctatacg aactuagaag tgagaaataa tcut 34
<210> 602
   <211> 34
   <212> DNA
   <213> Artificial Sequence
<220>
   <223> Synthetic DNA/RNA primer, R230
<400> 602
   gatttctcca gguccaaaat gaataactat tuga 34
<210> 603
   <211> 25
   <212> DNA
   <213> Artificial Sequence
<220>
   <223> Synthetic DNA/RNA primer, R231
<400> 603
   gaatccagga uaggaagcac acaug 25
<210> 604
   <211> 34
   <212> DNA
   <213> Artificial Sequence
<220>
   <223> Synthetic DNA/RNA primer, R232
<400> 604
   gattttataa ctagatuttc cttctctcca tucc 34
<210> 605
   <211> 33
   <212> DNA
   <213> Artificial Sequence
<220>
   <223> Synthetic DNA/RNA primer, R233
<400> 605
   gaaattcata cauttttctc taacugcaaa cat 33
<210> 606
   <211> 29
   <212> DNA
   <213> Artificial Sequence
<220>
   <223> Synthetic DNA/RNA primer, R234
<400> 606
   gagcagttgu gagattatct tttcauggc 29
<210> 607
   <211> 34
   <212> DNA
   <213> Artificial Sequence
<220>
   <223> Synthetic DNA/RNA primer, R235
<400> 607
   gatgtttttc taaugtgtta aagttcatug gaac 34
<210> 608
   <211> 24
   <212> DNA
   <213> Artificial Sequence
<220>
   <223> Synthetic DNA/RNA primer, R236
<400> 608
   gaccatgact gucacagtga ccut 24
<210> 609
   <211> 22
   <212> DNA
   <213> Artificial Sequence
<220>
   <223> Synthetic DNA/RNA primer, R237
<400> 609
   gattguggcc caaacaaagc uc 22
<210> 610
   <211> 30
   <212> DNA
   <213> Artificial Sequence
<220>
   <223> Synthetic DNA/RNA primer, R238
<400> 610
   gagtgcttgg aaauggaatg gtttuagaat 30
<210> 611
   <211> 28
   <212> DNA
   <213> Artificial Sequence
<220>
   <223> Synthetic DNA/RNA primer, R239
<400> 611
   gactactgua accaagaggt gactucag 28
<210> 612
   <211> 21
   <212> DNA
   <213> Artificial Sequence
<220>
   <223> Synthetic DNA/RNA primer, R240
<400> 612
   gaagccgaua tcccugcaga c 21
<210> 613
   <211> 22
   <212> DNA
   <213> Artificial Sequence
<220>
   <223> Synthetic DNA/RNA primer, R241
<400> 613
   gagtcggugt agaugcacag ct 22
<210> 614
   <211> 22
   <212> DNA
   <213> Artificial Sequence
<220>
   <223> Synthetic DNA/RNA primer, R242
<400> 614
   gatgcccaag guactgcaug gt 22
<210> 615
   <211> 22
   <212> RNA
   <213> Artificial Sequence
<220>
   <223> Synthetic Primer, R243
<400> 615
   gacucacgcc uaaaccagaa cc 22
<210> 616
   <211> 20
   <212> DNA
   <213> Artificial Sequence
<220>
   <223> Synthetic DNA/RNA primer, R244
<400> 616
   gatagctggc uccgcaccut 20
<210> 617
   <211> 19
   <212> DNA
   <213> Artificial Sequence
<220>
   <223> Synthetic DNA/RNA primer, R245
<400> 617
   gactgcuggg cgccguaac 19
<210> 618
   <211> 23
   <212> DNA
   <213> Artificial Sequence
<220>
   <223> Synthetic DNA/RNA primer, R246
<400> 618
   gaacccacac aagcgaauct cug 23
<210> 619
   <211> 26
   <212> DNA
   <213> Artificial Sequence
<220>
   <223> Synthetic DNA/RNA primer, R247
<400> 619
   gaactttgcu gccttaatga catucc 26
<210> 620
   <211> 23
   <212> DNA
   <213> Artificial Sequence
<220>
   <223> Synthetic DNA/RNA primer, R248
<400> 620
   gaggtaccug agatggagga guc 23
<210> 621
   <211> 27
   <212> DNA
   <213> Artificial Sequence
<220>
   <223> Synthetic DNA/RNA primer, R249
<400> 621
   gattgagcca cuaagcagta accatuc 27
<210> 622
   <211> 23
   <212> DNA
   <213> Artificial Sequence
<220>
   <223> Synthetic DNA/RNA primer, R250
<400> 622
   gacccgaagu tcttctgcag ucc 23
<210> 623
   <211> 23
   <212> DNA
   <213> Artificial Sequence
<220>
   <223> Synthetic DNA/RNA primer, R251
<400> 623
   gagccgaaac gaucaaggug agt 23
<210> 624
   <211> 30
   <212> DNA
   <213> Artificial Sequence
<220>
   <223> Synthetic DNA/RNA primer, R252
<400> 624
   gatcagtaga aagauggtac caaaaugggt 30
<210> 625
   <211> 20
   <212> DNA
   <213> Artificial Sequence
<220>
   <223> Synthetic DNA/RNA primer, R253
<400> 625
   gagaccgagc ucgggtguat 20
<210> 626
   <211> 27
   <212> DNA
   <213> Artificial Sequence
<220>
   <223> Synthetic DNA/RNA primer, R254
<400> 626
   gagcctttgt ggucatggga aagtaua 27
<210> 627
   <211> 33
   <212> DNA
   <213> Artificial Sequence
<220>
   <223> Synthetic DNA/RNA primer, R255
<400> 627
   gaatttgcct gaaatuacac atagaacttt cug 33
<210> 628
   <211> 23
   <212> DNA
   <213> Artificial Sequence
<220>
   <223> Synthetic DNA/RNA primer, R256
<400> 628
   gagggatggg ugactgagau ggt 23
<210> 629
   <211> 22
   <212> DNA
   <213> Artificial Sequence
<220>
   <223> Synthetic DNA/RNA primer, R257
<400> 629
   gagtggacau gcgaauggag ga 22
<210> 630
   <211> 34
   <212> DNA
   <213> Artificial Sequence
<220>
   <223> Synthetic DNA/RNA primer, R258
<400> 630
   gatttactct gacagcuaaa tgaactcaaa tgua 34
<210> 631
   <211> 29
   <212> DNA
   <213> Artificial Sequence
<220>
   <223> Synthetic DNA/RNA primer, R259
<400> 631
   gaaggctcag aacacuttac tgaatttug 29
<210> 632
   <211> 32
   <212> DNA
   <213> Artificial Sequence
<220>
   <223> Synthetic DNA/RNA primer, R260
<400> 632
   gattacttag aagaaaautg ctccttguca ga 32
<210> 633
   <211> 28
   <212> DNA
   <213> Artificial Sequence
<220>
   <223> Synthetic DNA/RNA primer, R261
<400> 633
   gagtgaagga aaccautcgt gauaaagc 28
<210> 634
   <211> 26
   <212> DNA
   <213> Artificial Sequence
<220>
   <223> Synthetic DNA/RNA primer, R262
<400> 634
   gagaaaatug gacccagttc tcugct 26
<210> 635
   <211> 31
   <212> DNA
   <213> Artificial Sequence
<220>
   <223> Synthetic DNA/RNA primer, R263
<400> 635
   gaatctttca acugtaaaat tcactguggg t 31
<210> 636
   <211> 27
   <212> DNA
   <213> Artificial Sequence
<220>
   <223> Synthetic DNA/RNA primer, R264
<400> 636
   gaccattctc auatcctagg tcugcct 27
<210> 637
   <211> 23
   <212> DNA
   <213> Artificial Sequence
<220>
   <223> Synthetic DNA/RNA primer, R265
<400> 637
   gagcacucca tttuggacag caa 23
<210> 638
   <211> 34
   <212> DNA
   <213> Artificial Sequence
<220>
   <223> Synthetic DNA/RNA primer, R266
<400> 638
   gagtttattt tctgguttca atagaacaag tuga 34
<210> 639
   <211> 34
   <212> DNA
   <213> Artificial Sequence
<220>
   <223> Synthetic DNA/RNA primer, R267
<400> 639
   gatttgaaag gtagautgcc ataatgtatc atug 34
<210> 640
   <211> 33
   <212> DNA
   <213> Artificial Sequence
<220>
   <223> Synthetic DNA/RNA primer, R268
<400> 640
   gaatgtggaa tctutgttta gttttactcu ggt 33
<210> 641
   <211> 32
   <212> DNA
   <213> Artificial Sequence
<220>
   <223> Synthetic DNA/RNA primer, R269
<400> 641
   gaatggttta gtcugacaca tatttaacac ut 32
<210> 642
   <211> 34
   <212> DNA
   <213> Artificial Sequence
<220>
   <223> Synthetic DNA/RNA primer, R270
<400> 642
   gattttcaag ttauagaaac atgtcatgtt guca 34
<210> 643
   <211> 34
   <212> DNA
   <213> Artificial Sequence
<220>
   <223> Synthetic DNA/RNA primer, R271
<400> 643
   gactagactt ugagacctgc taaataatua gatg 34
<210> 644
   <211> 23
   <212> RNA
   <213> Artificial Sequence
<220>
   <223> Synthetic Primer, R272
<400> 644
   gacaggucca agugaaccag gga 23
<210> 645
   <211> 20
   <212> DNA
   <213> Artificial Sequence
<220>
   <223> Synthetic DNA/RNA primer, R273
<400> 645
   gaagggcacu gaccctggua 20
<210> 646
   <211> 22
   <212> DNA
   <213> Artificial Sequence
<220>
   <223> Synthetic DNA/RNA primer, R274
<400> 646
   gatctgcagg agggugctct ua 22
<210> 647
   <211> 23
   <212> DNA
   <213> Artificial Sequence
<220>
   <223> Synthetic DNA/RNA primer, R275
<400> 647
   gagggcaacu acacctgcat ugt 23
<210> 648
   <211> 26
   <212> DNA
   <213> Artificial Sequence
<220>
   <223> Synthetic DNA/RNA primer, R276
<400> 648
   gatgccaaga cagugaagtt caaaug 26
<210> 649
   <211> 24
   <212> DNA
   <213> Artificial Sequence
<220>
   <223> Synthetic DNA/RNA primer, R277
<400> 649
   gagacaccac cuacttctcc guca 24
<210> 650
   <211> 31
   <212> DNA
   <213> Artificial Sequence
<220>
   <223> Synthetic DNA/RNA primer, R278
<400> 650
   gaaagaccaa aagagaaugg aaagtacuga c 31
<210> 651
   <211> 25
   <212> DNA
   <213> Artificial Sequence
<220>
   <223> Synthetic DNA/RNA primer, R279
<400> 651
   gaagaguatc catcuccagg agacg 25
<210> 652
   <211> 20
   <212> DNA
   <213> Artificial Sequence
<220>
   <223> Synthetic DNA/RNA primer, R280
<400> 652
   gatgtggcuc tccgcccaut 20
<210> 653
   <211> 28
   <212> DNA
   <213> Artificial Sequence
<220>
   <223> Synthetic DNA/RNA primer, R281
<400> 653
   gagctacacc atuagcttca ctgattut 28
<210> 654
   <211> 29
   <212> DNA
   <213> Artificial Sequence
<220>
   <223> Synthetic DNA/RNA primer, R282
<400> 654
   gactggatgg uaagaggagt ttcttcauc 29
<210> 655
   <211> 23
   <212> DNA
   <213> Artificial Sequence
<220>
   <223> Synthetic DNA/RNA primer, R283
<400> 655
   gacctttccc cucccctacc uag 23
<210> 656
   <211> 34
   <212> DNA
   <213> Artificial Sequence
<220>
   <223> Synthetic DNA/RNA primer, R284
<400> 656
   gacaaaacaa agucaaagag aattatgaaa tgug 34
<210> 657
   <211> 31
   <212> DNA
   <213> Artificial Sequence
<220>
   <223> Synthetic DNA/RNA primer, R285
<400> 657
   gacagggatt tuggttacta ctttgcuaag a 31
<210> 658
   <211> 26
   <212> DNA
   <213> Artificial Sequence
<220>
   <223> Synthetic DNA/RNA primer, R286
<400> 658
   gattgtcttc uggacacgtt cugaaa 26
<210> 659
   <211> 34
   <212> DNA
   <213> Artificial Sequence
<220>
   <223> Synthetic DNA/RNA primer, R287
<400> 659
   gatattaagc tttcutggaa aattctcttu ccct 34
<210> 660
   <211> 27
   <212> DNA
   <213> Artificial Sequence
<220>
   <223> Synthetic DNA/RNA primer, R288
<400> 660
   gattcagaag tuaggaaagg aguccag 27
<210> 661
   <211> 20
   <212> RNA
   <213> Artificial Sequence
<220>
   <223> Synthetic Primer, R289
<400> 661
   gacaccuguc acccgcacac 20
<210> 662
   <211> 23
   <212> DNA
   <213> Artificial Sequence
<220>
   <223> Synthetic DNA/RNA primer, R290
<400> 662
   gacagtcagu aacgccagug agt 23
<210> 663
   <211> 20
   <212> DNA
   <213> Artificial Sequence
<220>
   <223> Synthetic DNA/RNA primer, R291
<400> 663
   gagucccgtg gugcaaaggc 20
<210> 664
   <211> 32
   <212> DNA
   <213> Artificial Sequence
<220>
   <223> Synthetic DNA/RNA primer, R292
<400> 664
   gaatgtacac tagttuccgg aataaacctt ut 32
<210> 665
   <211> 20
   <212> DNA
   <213> Artificial Sequence
<220>
   <223> Synthetic DNA/RNA primer, R293
<400> 665
   gactugaccc cugcgagcca 20
<210> 666
   <211> 23
   <212> DNA
   <213> Artificial Sequence
<220>
   <223> Synthetic DNA/RNA primer, R294
<400> 666
   gacccacuca agctcagctg uaa 23
<210> 667
   <211> 27
   <212> DNA
   <213> Artificial Sequence
<220>
   <223> Synthetic DNA/RNA primer, R295
<400> 667
   gactgagaat ggcuacctct cgataug 27
<210> 668
   <211> 27
   <212> DNA
   <213> Artificial Sequence
<220>
   <223> Synthetic DNA/RNA primer, R296
<400> 668
   gaggctggag utggtgttat agtucaa 27
<210> 669
   <211> 29
   <212> DNA
   <213> Artificial Sequence
<220>
   <223> Synthetic DNA/RNA primer, R297
<400> 669
   gactcacaca tcutgagccc atttttauc 29
<210> 670
   <211> 25
   <212> DNA
   <213> Artificial Sequence
<220>
   <223> Synthetic DNA/RNA primer, R298
<400> 670
   gactgagagg gugtcacata ccaug 25
<210> 671
   <211> 27
   <212> DNA
   <213> Artificial Sequence
<220>
   <223> Synthetic DNA/RNA primer, R299
<400> 671
   gattcatacc gacaugtagg acctugt 27
<210> 672
   <211> 22
   <212> DNA
   <213> Artificial Sequence
<220>
   <223> Synthetic DNA/RNA primer, R300
<400> 672
   gaacttcuca caccgctgtg ut 22
<210> 673
   <211> 22
   <212> DNA
   <213> Artificial Sequence
<220>
   <223> Synthetic DNA/RNA primer, R301
<400> 673
   gacctcaccu ccgtttccug ca 22
<210> 674
   <211> 21
   <212> DNA
   <213> Artificial Sequence
<220>
   <223> Synthetic DNA/RNA primer, R302
<400> 674
   gatcuggccc cctuaggagg a 21
<210> 675
   <211> 27
   <212> DNA
   <213> Artificial Sequence
<220>
   <223> Synthetic DNA/RNA primer, R303
<400> 675
   gatcatcctc uccccataga aaagucc 27
<210> 676
   <211> 25
   <212> DNA
   <213> Artificial Sequence
<220>
   <223> Synthetic DNA/RNA primer, R304
<400> 676
   gatctcugcc atcattuccg gaaag 25
<210> 677
   <211> 25
   <212> DNA
   <213> Artificial Sequence
<220>
   <223> Synthetic DNA/RNA primer, R305
<400> 677
   gatgcaagga augcgatgaa guaga 25
<210> 678
   <211> 23
   <212> DNA
   <213> Artificial Sequence
<220>
   <223> Synthetic DNA/RNA primer, R306
<400> 678
   gagtcgcuaa cacgtgtgtg tuc 23
<210> 679
   <211> 29
   <212> DNA
   <213> Artificial Sequence
<220>
   <223> Synthetic DNA/RNA primer, R307
<400> 679
   gatggctaaa ctugaccttt ttactcugc 29
<210> 680
   <211> 22
   <212> DNA
   <213> Artificial Sequence
<220>
   <223> Synthetic DNA/RNA primer, R308
<400> 680
   gattcctcag caucgacctu gc 22
<210> 681
   <211> 32
   <212> DNA
   <213> Artificial Sequence
<220>
   <223> Synthetic DNA/RNA primer, R309
<400> 681
   gaaatctata tactucctta cctgggatug ga 32
<210> 682
   <211> 25
   <212> DNA
   <213> Artificial Sequence
<220>
   <223> Synthetic DNA/RNA primer, R310
<400> 682
   gaacatgctg agaucagcca aatuc 25
<210> 683
   <211> 27
   <212> DNA
   <213> Artificial Sequence
<220>
   <223> Synthetic DNA/RNA primer, R311
<400> 683
   gagcagugaa aagagtcuca aacacaa 27
<210> 684
   <211> 21
   <212> DNA
   <213> Artificial Sequence
<220>
   <223> Synthetic DNA/RNA primer, R312
<400> 684
   gacccacagg ccutctucga g 21
<210> 685
   <211> 25
   <212> DNA
   <213> Artificial Sequence
<220>
   <223> Synthetic DNA/RNA primer, R313
<400> 685
   gactgguatg agaaacugca cgagt 25
<210> 686
   <211> 27
   <212> DNA
   <213> Artificial Sequence
<220>
   <223> Synthetic DNA/RNA primer, R314
<400> 686
   gaaataccaa tcuattgtgg gctcugg 27
<210> 687
   <211> 27
   <212> DNA
   <213> Artificial Sequence
<220>
   <223> Synthetic DNA/RNA primer, R315
<400> 687
   gacctccttc ugcatggtat tcttuct 27
<210> 688
   <211> 26
   <212> DNA
   <213> Artificial Sequence
<220>
   <223> Synthetic DNA/RNA primer, R316
<400> 688
   gaattaaagc agugctcatg atuggg 26
<210> 689
   <211> 23
   <212> DNA
   <213> Artificial Sequence
<220>
   <223> Synthetic DNA/RNA primer, R317
<400> 689
   gacgggacuc gagtgatgat ugg 23
<210> 690
   <211> 26
   <212> DNA
   <213> Artificial Sequence
<220>
   <223> Synthetic DNA/RNA primer, R318
<400> 690
   gacttcacct tuaacacctc cagucc 26
<210> 691
   <211> 30
   <212> DNA
   <213> Artificial Sequence
<220>
   <223> Synthetic DNA/RNA primer, R319
<400> 691
   gactcctcta gcuatcttaa tgactuggac 30
<210> 692
   <211> 34
   <212> DNA
   <213> Artificial Sequence
<220>
   <223> Synthetic DNA/RNA primer, R320
<400> 692
   gactgctttc atucataggg aaatacauaa gaaa 34
<210> 693
   <211> 34
   <212> DNA
   <213> Artificial Sequence
<220>
   <223> Synthetic DNA/RNA primer, R321
<400> 693
   gattcaatat tttaaauagt ctggccuaaa cggt 34
<210> 694
   <211> 34
   <212> DNA
   <213> Artificial Sequence
<220>
   <223> Synthetic DNA/RNA primer, R322
<400> 694
   gatgatttcc aguattaatt ggcaauaaga gaat 34
<210> 695
   <211> 34
   <212> DNA
   <213> Artificial Sequence
<220>
   <223> Synthetic DNA/RNA primer, R323
<400> 695
   gaatgaaagc uaaaacataa gatgaauggg aaaa 34
<210> 696
   <211> 33
   <212> DNA
   <213> Artificial Sequence
<220>
   <223> Synthetic DNA/RNA primer, R324
<400> 696
   gaattatttc ttaccacutt tcctttctcc ugt 33
<210> 697
   <211> 28
   <212> DNA
   <213> Artificial Sequence
<220>
   <223> Synthetic DNA/RNA primer, R325
<400> 697
   gaattgtgag atuaacagca gggauacc 28
<210> 698
   <211> 35
   <212> DNA
   <213> Artificial Sequence
<220>
   <223> Synthetic DNA/RNA primer, R326
<400> 698
   gagcttcatt gtctugataa aatttatggt atcut 35
<210> 699
   <211> 34
   <212> DNA
   <213> Artificial Sequence
<220>
   <223> Synthetic DNA/RNA primer, R327
<400> 699
   gaccagctct tucatatctt aacattuagc aaca 34
<210> 700
   <211> 33
   <212> DNA
   <213> Artificial Sequence
<220>
   <223> Synthetic DNA/RNA primer, R328
<400> 700
   gagccaaaac atttugtccc tttctataat tug 33
<210> 701
   <211> 25
   <212> DNA
   <213> Artificial Sequence
<220>
   <223> Synthetic DNA/RNA primer, R329
<400> 701
   gatggacttc aagugatcac ttgug 25
<210> 702
   <211> 22
   <212> DNA
   <213> Artificial Sequence
<220>
   <223> Synthetic DNA/RNA primer, R330
<400> 702
   gaagcctgug gtgcttttug cg 22
<210> 703
   <211> 29
   <212> DNA
   <213> Artificial Sequence
<220>
   <223> Synthetic DNA/RNA primer, R331
<400> 703
   gaaagtcaaa cuacactcag aaccugaat 29
<210> 704
   <211> 30
   <212> DNA
   <213> Artificial Sequence
<220>
   <223> Synthetic DNA/RNA primer, R332
<400> 704
   gagcaaaggc caaagauaaa atgcttacug 30
<210> 705
   <211> 32
   <212> DNA
   <213> Artificial Sequence
<220>
   <223> Synthetic DNA/RNA primer, R333
<400> 705
   gaaagctaca gaaugtgaac agtcttctua aa 32
<210> 706
   <211> 30
   <212> DNA
   <213> Artificial Sequence
<220>
   <223> Synthetic DNA/RNA primer, R334
<400> 706
   gagaggtaga ugctgtaatt gctgauacaa 30
<210> 707
   <211> 29
   <212> DNA
   <213> Artificial Sequence
<220>
   <223> Synthetic DNA/RNA primer, R335
<400> 707
   gacaaacacc tccugataaa ttggcttug 29
<210> 708
   <211> 23
   <212> DNA
   <213> Artificial Sequence
<220>
   <223> Synthetic DNA/RNA primer, R336
<400> 708
   gaccctacuc caaggagcuc agg 23
<210> 709
   <211> 30
   <212> DNA
   <213> Artificial Sequence
<220>
   <223> Synthetic DNA/RNA primer, R337
<400> 709
   gacccagtua ccataactac tcugagaaaa 30
<210> 710
   <211> 25
   <212> DNA
   <213> Artificial Sequence
<220>
   <223> Synthetic DNA/RNA primer, R338
<400> 710
   gacttgcaga gcuatccccu aaagc 25
<210> 711
   <211> 21
   <212> DNA
   <213> Artificial Sequence
<220>
   <223> Synthetic DNA/RNA primer, R339
<400> 711
   gagctgcacc gagucgtagu c 21
<210> 712
   <211> 21
   <212> DNA
   <213> Artificial Sequence
<220>
   <223> Synthetic DNA/RNA primer, R340
<400> 712
   gagtcgutgt cuccccgaag g 21
<210> 713
   <211> 30
   <212> DNA
   <213> Artificial Sequence
<220>
   <223> Synthetic DNA/RNA primer, R341
<400> 713
   gaatacagtc cuggatgatg atgttttuga 30
<210> 714
   <211> 26
   <212> DNA
   <213> Artificial Sequence
<220>
   <223> Synthetic DNA/RNA primer, R342
<400> 714
   gaggacaaga aaagugcaac tuccca 26
<210> 715
   <211> 27
   <212> DNA
   <213> Artificial Sequence
<220>
   <223> Synthetic DNA/RNA primer, R343
<400> 715
   gatttcattg utttccaacu ccgggat 27
<210> 716
   <211> 30
   <212> DNA
   <213> Artificial Sequence
<220>
   <223> Synthetic DNA/RNA primer, R344
<400> 716
   gattuccaca gaaacaacau cgatttcttc 30
<210> 717
   <211> 30
   <212> DNA
   <213> Artificial Sequence
<220>
   <223> Synthetic DNA/RNA primer, R345
<400> 717
   gatgcattug atcatgcatt ugaaacaagt 30
<210> 718
   <211> 19
   <212> DNA
   <213> Artificial Sequence
<220>
   <223> Synthetic DNA/RNA primer, R346
<400> 718
   gaagtctgug cgcgctugc 19
<210> 719
   <211> 19
   <212> DNA
   <213> Artificial Sequence
<220>
   <223> Synthetic DNA/RNA primer, R347
<400> 719
   gagtgcgcac gucgcaauc 19
<210> 720
   <211> 26
   <212> DNA
   <213> Artificial Sequence
<220>
   <223> Synthetic DNA/RNA primer, R348
<400> 720
   gaggatgtau acaaaaggcg gatgug 26
<210> 721
   <211> 22
   <212> DNA
   <213> Artificial Sequence
<220>
   <223> Synthetic DNA/RNA primer, R349
<400> 721
   gagccagaga gucccttuca cc 22
<210> 722
   <211> 23
   <212> DNA
   <213> Artificial Sequence
<220>
   <223> Synthetic DNA/RNA primer, R350
<400> 722
   gatgccactc uttgggttga gut 23
<210> 723
   <211> 27
   <212> DNA
   <213> Artificial Sequence
<220>
   <223> Synthetic DNA/RNA primer, R351
<400> 723
   gatttcaaac uggaggctta ucaccaa 27
<210> 724
   <211> 35
   <212> DNA
   <213> Artificial Sequence
<220>
   <223> Synthetic DNA/RNA primer, R352
<400> 724
   gaatacaagc atgaaaauca aaacatatct tcugc 35
<210> 725
   <211> 34
   <212> DNA
   <213> Artificial Sequence
<220>
   <223> Synthetic DNA/RNA primer, R353
<400> 725
   gagtaaatgg tagctuttat cataatcacc aguc 34
<210> 726
   <211> 28
   <212> DNA
   <213> Artificial Sequence
<220>
   <223> Synthetic DNA/RNA primer, R354
<400> 726
   gatccattca agacuttagc aggtggua 28
<210> 727
   <211> 32
   <212> DNA
   <213> Artificial Sequence
<220>
   <223> Synthetic DNA/RNA primer, R355
<400> 727
   gagttactct catgugagaa ccatttgaau ga 32
<210> 728
   <211> 35
   <212> DNA
   <213> Artificial Sequence
<220>
   <223> Synthetic DNA/RNA primer, R356
<400> 728
   gagtttttct tatcucttaa aatgtttctg cuaca 35
<210> 729
   <211> 28
   <212> DNA
   <213> Artificial Sequence
<220>
   <223> Synthetic DNA/RNA primer, R257
<400> 729
   gaaagguaca agtuaaggca cacagaag 28
<210> 730
   <211> 27
   <212> DNA
   <213> Artificial Sequence
<220>
   <223> Synthetic DNA/RNA primer, R358
<400> 730
   gatggatctu ggcacaatga uaacagg 27
<210> 731
   <211> 32
   <212> DNA
   <213> Artificial Sequence
<220>
   <223> Synthetic DNA/RNA primer, R359
<400> 731
   gatgctatag taccaguacc ttttaaggtu ca 32
<210> 732
   <211> 23
   <212> DNA
   <213> Artificial Sequence
<220>
   <223> Synthetic DNA/RNA primer, R360
<400> 732
   gaccgtaagg uggcctactt ugc 23
<210> 733
   <211> 31
   <212> DNA
   <213> Artificial Sequence
<220>
   <223> Synthetic DNA/RNA primer, R361
<400> 733
   gacagcgttt tcutgtattc ctgtattuag c 31
<210> 734
   <211> 27
   <212> DNA
   <213> Artificial Sequence
<220>
   <223> Synthetic DNA/RNA primer, R362
<400> 734
   gaggaactgt gaaugaactt gtaggug 27
<210> 735
   <211> 20
   <212> RNA
   <213> Artificial Sequence
<220>
   <223> Synthetic Primer, R363
<400> 735
   gaccugacca gggcgucaaa 20
<210> 736
   <211> 29
   <212> DNA
   <213> Artificial Sequence
<220>
   <223> Synthetic DNA/RNA primer, R364
<400> 736
   gaagttcatc tucgaagctc aaattucag 29
<210> 737
   <211> 31
   <212> DNA
   <213> Artificial Sequence
<220>
   <223> Synthetic DNA/RNA primer, R365
<400> 737
   gatacaacaa aaugtttgac ttcaugcagg t 31
<210> 738
   <211> 28
   <212> DNA
   <213> Artificial Sequence
<220>
   <223> Synthetic DNA/RNA primer, F366
<400> 738
   aaaactcagt aucaacaact accgguac 28
<210> 739
   <211> 28
   <212> DNA
   <213> Artificial Sequence
<220>
   <223> Synthetic DNA/RNA primer, F367
<400> 739
   ctcagaaaug gaaaaaacct gcaguaaa 28
<210> 740
   <211> 23
   <212> DNA
   <213> Artificial Sequence
<220>
   <223> Synthetic DNA/RNA primer, F368
<400> 740
   gttccctcug cgtgttctca uaa 23
<210> 741
   <211> 27
   <212> DNA
   <213> Artificial Sequence
<220>
   <223> Synthetic DNA/RNA primer, F369
<400> 741
   aagaacctgu gtgaaagtat cuagcac 27
<210> 742
   <211> 25
   <212> RNA
   <213> Artificial Sequence
<220>
   <223> Synthetic Primer, F370
<400> 742
   auaaaccaaa cccaugcaaa aggac 25
<210> 743
   <211> 25
   <212> DNA
   <213> Artificial Sequence
<220>
   <223> Synthetic DNA/RNA primer, F371
<400> 743
   gcattgaugg aaggaagcaa auaca 25
<210> 744
   <211> 25
   <212> DNA
   <213> Artificial Sequence
<220>
   <223> Synthetic DNA/RNA primer, F372
<400> 744
   ccagcttcau agacaaaggt tcuct 25
<210> 745
   <211> 24
   <212> DNA
   <213> Artificial Sequence
<220>
   <223> Synthetic DNA/RNA primer, F373
<400> 745
   gtggtttctu ccattgacca cauc 24
<210> 746
   <211> 22
   <212> DNA
   <213> Artificial Sequence
<220>
   <223> Synthetic DNA/RNA primer, F374
<400> 746
   caaaugggca ggactctuag gt 2
<210> 747
   <211> 22
   <212> DNA
   <213> Artificial Sequence
<220>
   <223> Synthetic DNA/RNA primer, F375
<400> 747
   gugaggaaac tuctgcagag gt 22
<210> 748
   <211> 22
   <212> DNA
   <213> Artificial Sequence
<220>
   <223> Synthetic DNA/RNA primer, F376
<400> 748
   ggaagcaggg aagcucttca uc 22
<210> 749
   <211> 27
   <212> DNA
   <213> Artificial Sequence
<220>
   <223> Synthetic DNA/RNA primer, F377
<400> 749
   tggttugaag aactttctuc agaagct 27
<210> 750
   <211> 25
   <212> DNA
   <213> Artificial Sequence
<220>
   <223> Synthetic DNA/RNA primer, F378
<400> 750
   agggagacug ugtgtaatat ttgcg 25
<210> 751
   <211> 30
   <212> DNA
   <213> Artificial Sequence
<220>
   <223> Synthetic DNA/RNA primer, F379
<400> 751
   gccagtatug aagaatgttg aagaucaaaa 30
<210> 752
   <211> 23
   <212> DNA
   <213> Artificial Sequence
<220>
   <223> Synthetic DNA/RNA primer, F380
<400> 752
   gccaaaagga aguctgttuc cac 23
<210> 753
   <211> 27
   <212> DNA
   <213> Artificial Sequence
<220>
   <223> Synthetic DNA/RNA primer, F381
<400> 753
   catgccacac autctctttt tacaugt 27
<210> 754
   <211> 23
   <212> DNA
   <213> Artificial Sequence
<220>
   <223> Synthetic DNA/RNA primer, F382
<400> 754
   gtagagugct acactgucca aca 23
<210> 755
   <211> 28
   <212> DNA
   <213> Artificial Sequence
<220>
   <223> Synthetic DNA/RNA primer, F383
<400> 755
   ctctgagaaa gaaugaaatg gagtugga 28
<210> 756
   <211> 24
   <212> DNA
   <213> Artificial Sequence
<220>
   <223> Synthetic DNA/RNA primer, F384
<400> 756
   aaacaaattt uccagcgctt cuga 24
<210> 757
   <211> 30
   <212> DNA
   <213> Artificial Sequence
<220>
   <223> Synthetic DNA/RNA primer, F385
<400> 757
   agcaataaaa gtguataaat gcctgtaugc 30
<210> 758
   <211> 30
   <212> DNA
   <213> Artificial Sequence
<220>
   <223> Synthetic DNA/RNA primer, F386
<400> 758
   tcaacaagtt gacuaaatct cgtacttuct 30
<210> 759
   <211> 28
   <212> DNA
   <213> Artificial Sequence
<220>
   <223> Synthetic DNA/RNA primer, F387
<400> 759
   cattcttaca uaaaggacac tgugaagg 28
<210> 760
   <211> 24
   <212> DNA
   <213> Artificial Sequence
<220>
   <223> Synthetic DNA/RNA primer, F388
<400> 760
   cccttacaga uggagtcttt uggc 24
<210> 761
   <211> 27
   <212> DNA
   <213> Artificial Sequence
<220>
   <223> Synthetic DNA/RNA primer, F389
<400> 761
   aaagaccttt uggtaactca gacucag 27
<210> 762
   <211> 32
   <212> DNA
   <213> Artificial Sequence
<220>
   <223> Synthetic DNA/RNA primer, F390
<400> 762
   acattcacug aaaatugtaa agcctataat tg 32
<210> 763
   <211> 35
   <212> DNA
   <213> Artificial Sequence
<220>
   <223> Synthetic DNA/RNA primer, F391
<400> 763
   ggttgtgctt ttuaaatttc aattttattt tugct 35
<210> 764
   <211> 19
   <212> DNA
   <213> Artificial Sequence
<220>
   <223> Synthetic DNA/RNA primer, F392
<400> 764
   ggatgucaca accgugtgg 19
<210> 765
   <211> 29
   <212> DNA
   <213> Artificial Sequence
<220>
   <223> Synthetic DNA/RNA primer, F393
<400> 765
   agtgaaaacu aaaatggauc aagcagatg 29
<210> 766
   <211> 33
   <212> DNA
   <213> Artificial Sequence
<220>
   <223> Synthetic DNA/RNA primer, F394
<400> 766
   aaactagttt tugccagttt ttuaaaataa cct 33
<210> 767
   <211> 23
   <212> DNA
   <213> Artificial Sequence
<220>
   <223> Synthetic DNA/RNA primer, F395
<400> 767
   tttttacccc caguggtatg ugg 23
<210> 768
   <211> 27
   <212> RNA
   <213> Artificial Sequence
<220>
   <223> Synthetic Primer, F396
<400> 768
   gaaaacacaa aucaaagaga agcugca 27
<210> 769
   <211> 28
   <212> DNA
   <213> Artificial Sequence
<220>
   <223> Synthetic DNA/RNA primer, F397
<400> 769
   atatttagua gccaggacag uagaagga 28
<210> 770
   <211> 28
   <212> DNA
   <213> Artificial Sequence
<220>
   <223> Synthetic DNA/RNA primer, F398
<400> 770
   aaatatttca gugtccgtuc acacacaa 28
<210> 771
   <211> 25
   <212> DNA
   <213> Artificial Sequence
<220>
   <223> Synthetic DNA/RNA primer, F399
<400> 771
   gcagaugcaa ggtattctgu aaagg 25
<210> 772
   <211> 30
   <212> DNA
   <213> Artificial Sequence
<220>
   <223> Synthetic DNA/RNA primer, F400
<400> 772
   acctacataa aacuctttcc agaatgtugt 30
<210> 773
   <211> 25
   <212> DNA
   <213> Artificial Sequence
<220>
   <223> Synthetic DNA/RNA primer, F401
<400> 773
   ccctttctgt ugaagctgtc aatuc 25
<210> 774
   <211> 23
   <212> DNA
   <213> Artificial Sequence
<220>
   <223> Synthetic DNA/RNA primer, F402
<400> 774
   agauggtatg tugccaacac gag 23
<210> 775
   <211> 23
   <212> DNA
   <213> Artificial Sequence
<220>
   <223> Synthetic DNA/RNA primer, F403
<400> 775
   gatgtttccg ucaaatcgtg ugg 23
<210> 776
   <211> 27
   <212> DNA
   <213> Artificial Sequence
<220>
   <223> Synthetic DNA/RNA primer, F404
<400> 776
   gtagaactat cugcagacac cucaaac 27
<210> 777
   <211> 27
   <212> DNA
   <213> Artificial Sequence
<220>
   <223> Synthetic DNA/RNA primer, F405
<400> 777
   ccagaaccac cauctttcag taattug 27
<210> 778
   <211> 27
   <212> DNA
   <213> Artificial Sequence
<220>
   <223> Synthetic DNA/RNA primer, F406
<400> 778
   atcataaaat gtuggagcta ggtccut 27
<210> 779
   <211> 27
   <212> DNA
   <213> Artificial Sequence
<220>
   <223> Synthetic DNA/RNA primer, F407
<400> 779
   tatgatggaa ggguagctgt uagaagg 27
<210> 780
   <211> 28
   <212> DNA
   <213> Artificial Sequence
<220>
   <223> Synthetic DNA/RNA primer, F408
<400> 780
   ggttaaaatg tcacuctgag aggauagc 28
<210> 781
   <211> 27
   <212> DNA
   <213> Artificial Sequence
<220>
   <223> Synthetic DNA/RNA primer, F409
<400> 781
   ggaaatttgu aaaatgtgcu ccccaaa 27
<210> 782
   <211> 25
   <212> DNA
   <213> Artificial Sequence

<220>
   <223> Synthetic DNA/RNA primer, F410
<400> 782
   aattccttgt cacucagacc aacuc 25
<210> 783
   <211> 24
   <212> DNA
   <213> Artificial Sequence
<220>
   <223> Synthetic DNA/RNA primer, F411
<400> 783
   actaaggtga ugttcctgag augc 24
<210> 784
   <211> 30
   <212> DNA
   <213> Artificial Sequence
<220>
   <223> Synthetic DNA/RNA primer, F412
<400> 784
   actttccuta atgtcatttu cagcaaaact 30
<210> 785
   <211> 30
   <212> DNA
   <213> Artificial Sequence
<220>
   <223> Synthetic DNA/RNA primer, F413
<400> 785
   cagtctgaac uacttcttca tattctugct 30
<210> 786
   <211> 28
   <212> DNA
   <213> Artificial Sequence
<220>
   <223> Synthetic DNA/RNA primer, F414
<400> 786
   ctagttctgc utgaatgttt tcaucact 28
<210> 787
   <211> 28
   <212> DNA
   <213> Artificial Sequence
<220>
   <223> Synthetic DNA/RNA primer, F415
<400> 787
   tggaatgttc tcauttccca tttctcut 28
<210> 788
   <211> 24
   <212> DNA
   <213> Artificial Sequence
<220>
   <223> Synthetic DNA/RNA primer, F416
<400> 788
   gtttcgtugc ctctgaacug agat 24
<210> 789
   <211> 30
   <212> DNA
   <213> Artificial Sequence
<220>
   <223> Synthetic DNA/RNA primer, F417
<400> 789
   ccttgatttt ctuccttttg ttcacatuca 30
<210> 790
   <211> 24
   <212> DNA
   <213> Artificial Sequence
<220>
   <223> Synthetic DNA/RNA primer, F418
<400> 790
   tttctatgct ugtttcccga cugt 24
<210> 791
   <211> 24
   <212> DNA
   <213> Artificial Sequence
<220>
   <223> Synthetic DNA/RNA primer, F419
<400> 791
   gaugaaagct cctucaccac agaa 24
<210> 792
   <211> 25
   <212> DNA
   <213> Artificial Sequence
<220>
   <223> Synthetic DNA/RNA primer, F420
<400> 792
   cctagagtgc uaacttccag uaacg 25
<210> 793
   <211> 27
   <212> DNA
   <213> Artificial Sequence
<220>
   <223> Synthetic DNA/RNA primer, F421
<400> 793
   cttggaaggc uaggattgac aaatuct 27
<210> 794
   <211> 25
   <212> DNA
   <213> Artificial Sequence
<220>
   <223> Synthetic DNA/RNA primer, F422
<400> 794
   ttgttactct tcutggctcc agtug 25
<210> 795
   <211> 27
   <212> DNA
   <213> Artificial Sequence
<220>
   <223> Synthetic DNA/RNA primer, F423
<400> 795
   ttaggtgggc utagatttct acugact 27
<210> 796
   <211> 26
   <212> DNA
   <213> Artificial Sequence
<220>
   <223> Synthetic DNA/RNA primer, F424
<400> 796
   tgcttatagg tucagctttc gtttug 26
<210> 797
   <211> 24
   <212> DNA
   <213> Artificial Sequence
<220>
   <223> Synthetic DNA/RNA primer, F425
<400> 797
   ccactatgua agacaaaggc uggt 24
<210> 798
   <211> 27
   <212> DNA
   <213> Artificial Sequence
<220>
   <223> Synthetic DNA/RNA primer, F426
<400> 798
   tccgtttggt uagttccctg atttauc 27
<210> 799
   <211> 28
   <212> DNA
   <213> Artificial Sequence
<220>
   <223> Synthetic DNA/RNA primer, F427
<400> 799
   gtattatctg uggctcagta acaaaugc 28
<210> 800
   <211> 24
   <212> DNA
   <213> Artificial Sequence
<220>
   <223> Synthetic DNA/RNA primer, F428
<400> 800
   ttaaagcctc augaggatca cugg 24
<210> 801
   <211> 26
   <212> DNA
   <213> Artificial Sequence
<220>
   <223> Synthetic DNA/RNA primer, F429
<400> 801
   agttcatcac tuctggaaaa ccacuc 26
<210> 802
   <211> 27
   <212> DNA
   <213> Artificial Sequence
<220>
   <223> Synthetic DNA/RNA primer, F430
<400> 802
   gggatcagca tucagatcta cctttut 27
<210> 803
   <211> 28
   <212> DNA
   <213> Artificial Sequence
<220>
   <223> Synthetic DNA/RNA primer, F431
<400> 803
   ttcagccttt tcuacattca ttctguct 28
<210> 804
   <211> 25
   <212> DNA
   <213> Artificial Sequence
<220>
   <223> Synthetic DNA/RNA primer, F432
<400> 804
   taccctgaua cttttctgga ugcct 25
<210> 805
   <211> 26
   <212> DNA
   <213> Artificial Sequence
<220>
   <223> Synthetic DNA/RNA primer, F433
<400> 805
   gaatccaaac ugatttcatc ccuggt 26
<210> 806
   <211> 27
   <212> DNA
   <213> Artificial Sequence
<220>
   <223> Synthetic DNA/RNA primer, F434
<400> 806
   agctgccuac cacaaataca aattaug 27
<210> 807
   <211> 26
   <212> DNA
   <213> Artificial Sequence
<220>
   <223> Synthetic DNA/RNA primer, F435
<400> 807
   cagagttcuc acagttccaa ggtuag 26
<210> 808
   <211> 31
   <212> DNA
   <213> Artificial Sequence
<220>
   <223> Synthetic DNA/RNA primer, F436
<400> 808
   gaagaagaag aaaacaaaug gtttuaccaa g 31
<210> 809
   <211> 28
   <212> DNA
   <213> Artificial Sequence
<220>
   <223> Synthetic DNA/RNA primer, F437
<400> 809
   atcaccacgt cauagaaagt aattgugc 28
<210> 810
   <211> 30
   <212> DNA
   <213> Artificial Sequence
<220>
   <223> Synthetic DNA/RNA primer, F438
<400> 810
   cattcaaact tactugcaaa atatgtgguc 30
<210> 811
   <211> 31
   <212> DNA
   <213> Artificial Sequence
<220>
   <223> Synthetic DNA/RNA primer, F439
<400> 811
   gcataggaga taaucatagg aatcccaaau t 31
<210> 812
   <211> 30
   <212> DNA
   <213> Artificial Sequence
<220>
   <223> Synthetic DNA/RNA primer, F440
<400> 812
   agttgtagtt gtugaattca gtatcaucct 30
<210> 813
   <211> 28
   <212> DNA
   <213> Artificial Sequence
<220>
   <223> Synthetic DNA/RNA primer, F441
<400> 813
   tgtgcctttc cuaaggaatt tgctaaua 28
<210> 814
   <211> 28
   <212> DNA
   <213> Artificial Sequence
<220>
   <223> Synthetic DNA/RNA primer, F442
<400> 814
   aaaagataau ggaaagggau gacacagc 28
<210> 815
   <211> 24
   <212> DNA
   <213> Artificial Sequence
<220>
   <223> Synthetic DNA/RNA primer, F443
<400> 815
   ctgttaaggc ccagutagat ccuc 24
<210> 816
   <211> 28
   <212> DNA
   <213> Artificial Sequence
<220>
   <223> Synthetic DNA/RNA primer, F444
<400> 816
   aggcagttcu agaagaatga aaactcut 28
<210> 817
   <211> 24
   <212> DNA
   <213> Artificial Sequence
<220>
   <223> Synthetic DNA/RNA primer, F445
<400> 817
   tgtacctagc autctgcctc auac 24
<210> 818
   <211> 25
   <212> DNA
   <213> Artificial Sequence
<220>
   <223> Synthetic DNA/RNA primer, F446
<400> 818
   tagacctttt ccuctgccct tauca 25
<210> 819
   <211> 28
   <212> DNA
   <213> Artificial Sequence
<220>
   <223> Synthetic DNA/RNA primer, F447
<400> 819
   cacatuatta cagtggaugg agaagaca 28
<210> 820
   <211> 26
   <212> DNA
   <213> Artificial Sequence
<220>
   <223> Synthetic DNA/RNA primer, F448
<400> 820
   cttctttggg ugttttatgc ttggut 26
<210> 821
   <211> 25
   <212> DNA
   <213> Artificial Sequence
<220>
   <223> Synthetic DNA/RNA primer, F449
<400> 821
   gcagagctut atgaagcagu gaaga 25
<210> 822
   <211> 28
   <212> DNA
   <213> Artificial Sequence
<220>
   <223> Synthetic DNA/RNA primer, F450
<400> 822
   tcttaaatgg ucacagggtt attucagt 28
<210> 823
   <211> 27
   <212> DNA
   <213> Artificial Sequence
<220>
   <223> Synthetic DNA/RNA primer, F451
<400> 823
   ttccattgca tcuttctcat ctttcuc 27
<210> 824
   <211> 29
   <212> DNA
   <213> Artificial Sequence
<220>
   <223> Synthetic DNA/RNA primer, F452
<400> 824
   ttcactucag caaattttta gauccagac 29
<210> 825
   <211> 23
   <212> DNA
   <213> Artificial Sequence
<220>
   <223> Synthetic DNA/RNA primer, F453
<400> 825
   tgccccttuc gtctatttgu cag 23
<210> 826
   <211> 28
   <212> DNA
   <213> Artificial Sequence
<220>
   <223> Synthetic DNA/RNA primer, F454
<400> 826
   ggagattttt ctgugttttc tgctaguc 28
<210> 827
   <211> 27
   <212> DNA
   <213> Artificial Sequence
<220>
   <223> Synthetic DNA/RNA primer, F455
<400> 827
   tgacauactt tgcaaugaag cagaaaa 27
<210> 828
   <211> 27
   <212> DNA
   <213> Artificial Sequence
<220>
   <223> Synthetic DNA/RNA primer, F456
<400> 828
   ggatcctgat augtcttggt caagtuc 27
<210> 829
   <211> 24
   <212> RNA
   <213> Artificial Sequence
<220>
   <223> Synthetic Primer, F457
<400> 829
   ggcaccaaau acgaaacacc caua 24
<210> 830
   <211> 28
   <212> DNA
   <213> Artificial Sequence
<220>
   <223> Synthetic DNA/RNA primer, F458
<400> 830
   atatctgtca gtgaauccac taggacug 28
<210> 831
   <211> 28
   <212> DNA
   <213> Artificial Sequence
<220>
   <223> Synthetic DNA/RNA primer, F459
<400> 831
   tgaagaagca ucugaaactg tatttcct 28
<210> 832
   <211> 31
   <212> DNA
   <213> Artificial Sequence
<220>
   <223> Synthetic DNA/RNA primer, F460
<400> 832
   ggactactac tataugtgca ttgagagttu t 31
<210> 833
   <211> 32
   <212> DNA
   <213> Artificial Sequence
<220>
   <223> Synthetic DNA/RNA primer, F461
<400> 833
   tggcttataa aatatuaatg tgcttctgtt ut 32
<210> 834
   <211> 28
   <212> DNA
   <213> Artificial Sequence
<220>
   <223> Synthetic DNA/RNA primer, F462
<400> 834
   ggtaaaaaug cctattggau ccaaagag 28
<210> 835
   <211> 30
   <212> DNA
   <213> Artificial Sequence
<220>
   <223> Synthetic DNA/RNA primer, F463
<400> 835
   aatctacaaa aaguaagaac uagcaagact 30
<210> 836
   <211> 28
   <212> DNA
   <213> Artificial Sequence
<220>
   <223> Synthetic DNA/RNA primer, F464
<400> 836
   aagtgacaaa atcuccaagg aagttgua 28
<210> 837
   <211> 26
   <212> DNA
   <213> Artificial Sequence
<220>
   <223> Synthetic DNA/RNA primer, F465
<400> 837
   gaattcttug ccacgtattt cuagcc 26
<210> 838
   <211> 27
   <212> DNA
   <213> Artificial Sequence
<220>
   <223> Synthetic DNA/RNA primer, F466
<400> 838
   ggcttcttca uttcagggta ucaaaaa 27
<210> 839
   <211> 28
   <212> DNA
   <213> Artificial Sequence
<220>
   <223> Synthetic DNA/RNA primer, F467
<400> 839
   aatacauact gtttgcucac agaaggag 28
<210> 840
   <211> 31
   <212> DNA
   <213> Artificial Sequence
<220>
   <223> Synthetic DNA/RNA primer, F468
<400> 840
   accgaaagac caaaaaucag aactaatuaa c 31
<210> 841
   <211> 27
   <212> DNA
   <213> Artificial Sequence
<220>
   <223> Synthetic DNA/RNA primer, F469
<400> 841
   tcacagaaug attcugaaga accaact 27
<210> 842
   <211> 25
   <212> DNA
   <213> Artificial Sequence
<220>
   <223> Synthetic DNA/RNA primer, F470
<400> 842
   attaccccag aagcugattc tcugt 25
<210> 843
   <211> 28
   <212> DNA
   <213> Artificial Sequence
<220>
   <223> Synthetic DNA/RNA primer, F471
<400> 843
   tatatgatca tgaaaaugcc agcacuct 28
<210> 844
   <211> 28
   <212> DNA
   <213> Artificial Sequence
<220>
   <223> Synthetic DNA/RNA primer, F472
<400> 844
   ttcccatgga aaagaaucaa gatgtaug 28
<210> 845
   <211> 27
   <212> DNA
   <213> Artificial Sequence
<220>
   <223> Synthetic DNA/RNA primer, F473
<400> 845
   actgtcaatc cagacuctga agaacut 27
<210> 846
   <211> 25
   <212> DNA
   <213> Artificial Sequence
<220>
   <223> Synthetic DNA/RNA primer, F474
<400> 846
   caggugauaa acaagcaacc caagt 25
<210> 847
   <211> 29
   <212> DNA
   <213> Artificial Sequence
<220>
   <223> Synthetic DNA/RNA primer, F475
<400> 847
   tggcattaga uaatcaaaag aaacugagc 29
<210> 848
   <211> 30
   <212> DNA
   <213> Artificial Sequence
<220>
   <223> Synthetic DNA/RNA primer, F476
<400> 848
   gaatcaggaa gucagtttga atttacucag 30
<210> 849
   <211> 28
   <212> DNA
   <213> Artificial Sequence
<220>
   <223> Synthetic DNA/RNA primer, F477
<400> 849
   gcctgtugaa aaatgactgu aacaaaag 28
<210> 850
   <211> 28
   <212> DNA
   <213> Artificial Sequence
<220>
   <223> Synthetic DNA/RNA primer, F478
<400> 850
   tgaagataac aaauatactg cugccagt 28
<210> 851
   <211> 28
   <212> DNA
   <213> Artificial Sequence
<220>
   <223> Synthetic DNA/RNA primer, F479
<400> 851
   aggagggaaa cacucagatu aaagaaga 28
<210> 852
   <211> 27
   <212> DNA
   <213> Artificial Sequence
<220>
   <223> Synthetic DNA/RNA primer, F480
<400> 852
   tttcagactg caagugggaa aaataut 27
<210> 853
   <211> 30
   <212> DNA
   <213> Artificial Sequence
<220>
   <223> Synthetic DNA/RNA primer, F481
<400> 853
   tcttcttaca acuccctata cattctcaut 30
<210> 854
   <211> 27
   <212> DNA
   <213> Artificial Sequence
<220>
   <223> Synthetic DNA/RNA primer, F482
<400> 854
   ccagttggta cuggaaatca actagug 27
<210> 855
   <211> 28
   <212> DNA
   <213> Artificial Sequence
<220>
   <223> Synthetic DNA/RNA primer, F483
<400> 855
   aaaagagcaa gguactagtg aaaucacc 28
<210> 856
   <211> 28
   <212> DNA
   <213> Artificial Sequence
<220>
   <223> Synthetic DNA/RNA primer, F484
<400> 856
   aaaaaccttg ttuctattga gactgugg 28
<210> 857
   <211> 28
   <212> DNA
   <213> Artificial Sequence
<220>
   <223> Synthetic DNA/RNA primer, F485
<400> 857
   aattcagcct uagcttttta cacaagut 28
<210> 858
   <211> 28
   <212> DNA
   <213> Artificial Sequence
<220>
   <223> Synthetic DNA/RNA primer, F486
<400> 858
   tgacaaaaau catctcuccg aaaaacaa 28
<210> 859
   <211> 25
   <212> DNA
   <213> Artificial Sequence
<220>
   <223> Synthetic DNA/RNA primer, F487
<400> 859
   aataattttg agguagggcc accug 25
<210> 860
   <211> 32
   <212> DNA
   <213> Artificial Sequence
<220>
   <223> Synthetic DNA/RNA primer, F488
<400> 860
   tcataactct cuagataatg atgaatguag ca 32
<210> 861
   <211> 28
   <212> DNA
   <213> Artificial Sequence
<220>
   <223> Synthetic DNA/RNA primer, F489
<400> 861
   gtatagggaa gcutcataag tcagtcuc 28
<210> 862
   <211> 28
   <212> DNA
   <213> Artificial Sequence
<220>
   <223> Synthetic DNA/RNA primer, F490
<400> 862
   agaagatagu accaagcaag tctttucc 28
<210> 863
   <211> 25
   <212> DNA
   <213> Artificial Sequence
<220>
   <223> Synthetic DNA/RNA primer, F491
<400> 863
   tagtacagca aguggaaagc aagut 25
<210> 864
   <211> 27
   <212> DNA
   <213> Artificial Sequence
<220>
   <223> Synthetic DNA/RNA primer, F492
<400> 864
   caggcttcac cuaaaaacgt aaaaaug 27
<210> 865
   <211> 32
   <212> DNA
   <213> Artificial Sequence
<220>
   <223> Synthetic DNA/RNA primer, F493
<400> 865
   atgaaataut tcttttuagg agaaccctca at 32
<210> 866
   <211> 27
   <212> DNA
   <213> Artificial Sequence
<220>
   <223> Synthetic DNA/RNA primer, F494
<400> 866
   atatattutc tccccatugc agcacaa 27
<210> 867
   <211> 28
   <212> DNA
   <213> Artificial Sequence
<220>
   <223> Synthetic DNA/RNA primer, F495
<400> 867
   aggacatcca utttatcaag tttcugct 28
<210> 868
   <211> 33
   <212> DNA
   <213> Artificial Sequence
<220>
   <223> Synthetic DNA/RNA primer, F496
<400> 868
   tggctctgat gauagtaaaa ataagattaa uga 33
<210> 869
   <211> 28
   <212> DNA
   <213> Artificial Sequence
<220>
   <223> Synthetic DNA/RNA primer, F497
<400> 869
   gctgtatacg uatggcgttt cuaaacat 28
<210> 870
   <211> 25
   <212> DNA
   <213> Artificial Sequence
<220>
   <223> Synthetic DNA/RNA primer, R366
<400> 870
   tcccgtggcu ggtaaatctg aaaua 25
<210> 871
   <211> 27
   <212> DNA
   <213> Artificial Sequence
<220>
   <223> Synthetic DNA/RNA primer, R367
<400> 871
   ccaaaacatg aaugttctca acaagug 27
<210> 872
   <211> 25
   <212> DNA
   <213> Artificial Sequence
<220>
   <223> Synthetic DNA/RNA primer, R368
<400> 872
   attcctgcac uaatgtgttc atuct 25
<210> 873
   <211> 23
   <212> RNA
   <213> Artificial Sequence
<220>
   <223> Synthetic Primer, R369
<400> 873
   guccaaagcg agcaagagaa ucc 23
<210> 874
   <211> 26
   <212> DNA
   <213> Artificial Sequence
<220>
   <223> Synthetic DNA/RNA primer, R370
<400> 874
   agttccagua gtcctacttu gacact 26
<210> 875
   <211> 23
   <212> DNA
   <213> Artificial Sequence
<220>
   <223> Synthetic DNA/RNA primer, R371
<400> 875
   agagcacgtu cttctgctgt aug 23
<210> 876
   <211> 33
   <212> DNA
   <213> Artificial Sequence
<220>
   <223> Synthetic DNA/RNA primer, R372
<400> 876
   agttgaatat ctgttuttca acaagtacat tut 33
<210> 877
   <211> 21
   <212> DNA
   <213> Artificial Sequence
<220>
   <223> Synthetic DNA/RNA primer, R373
<400> 877
   gcctggccug aatgcctuaa a 21
<210> 878
   <211> 30
   <212> DNA
   <213> Artificial Sequence
<220>
   <223> Synthetic DNA/RNA primer, R374
<400> 878
   caatttcaac acaagcuaaa ctaguaggat 30
<210> 879
   <211> 26
   <212> DNA
   <213> Artificial Sequence
<220>
   <223> Synthetic DNA/RNA primer, R375
<400> 879
   tcaacaaaag ugccagtagu catttc 26
<210> 880
   <211> 24
   <212> DNA
   <213> Artificial Sequence
<220>
   <223> Synthetic DNA/RNA primer, R376
<400> 880
   ctgttttuag caaaagcguc caga 24
<210> 881
   <211> 22
   <212> DNA
   <213> Artificial Sequence
<220>
   <223> Synthetic DNA/RNA primer, R377
<400> 881
   agtcagcccu tgctcttuga at 22
<210> 882
   <211> 27
   <212> DNA
   <213> Artificial Sequence
<220>
   <223> Synthetic DNA/RNA primer, R378
<400> 882
   ttggccauac aaagtgauaa aggactt 27
<210> 883
   <211> 22
   <212> DNA
   <213> Artificial Sequence
<220>
   <223> Synthetic DNA/RNA primer, R379
<400> 883
   tttgcagggu gaagagctag uc 22
<210> 884
   <211> 25
   <212> DNA
   <213> Artificial Sequence
<220>
   <223> Synthetic DNA/RNA primer, R380
<400> 884
   tgtacaaaug ggactaacag gugga 25
<210> 885
   <211> 30
   <212> DNA
   <213> Artificial Sequence
<220>
   <223> Synthetic DNA/RNA primer, R381
<400> 885
   agcataccaa gtcuactgaa taaacactut 30
<210> 886
   <211> 25
   <212> DNA
   <213> Artificial Sequence
<220>
   <223> Synthetic DNA/RNA primer, R382
<400> 886
   cctggagtcg autgattaga gccua 25
<210> 887
   <211> 28
   <212> DNA
   <213> Artificial Sequence
<220>
   <223> Synthetic DNA/RNA primer, R383
<400> 887
   aatgtgttat guggctccat tatuagct 28
<210> 888
   <211> 29
   <212> DNA
   <213> Artificial Sequence
<220>
   <223> Synthetic DNA/RNA primer, R384
<400> 888
   gcatttttac cuacgatatt cctccaaug 29
<210> 889
   <211> 28
   <212> DNA
   <213> Artificial Sequence
<220>
   <223> Synthetic DNA/RNA primer, R385
<400> 889
   accagtaaaa auaaagaacc aggagugg 28
<210> 890
   <211> 28
   <212> DNA
   <213> Artificial Sequence
<220>
   <223> Synthetic DNA/RNA primer, R386
<400> 890
   ttatagaggt ttuctactgt tgcugcat 28
<210> 891
   <211> 27
   <212> DNA
   <213> Artificial Sequence
<220>
   <223> Synthetic DNA/RNA primer, R387
<400> 891
   gcagttgtga gautatcttt tcauggc 27
<210> 892
   <211> 24
   <212> DNA
   <213> Artificial Sequence
<220>
   <223> Synthetic DNA/RNA primer, R388
<400> 892
   catcattcac ccutggcaca guaa 24
<210> 893
   <211> 27
   <212> DNA
   <213> Artificial Sequence
<220>
   <223> Synthetic DNA/RNA primer, R389
<400> 893
   aaauattttc taggaatugc gggagga 27
<210> 894
   <211> 26
   <212> DNA
   <213> Artificial Sequence
<220>
   <223> Synthetic DNA/RNA primer, R390
<400> 894
   cagguaaucg gctctaaaga aacatg 26
<210> 895
   <211> 22
   <212> DNA
   <213> Artificial Sequence
<220>
   <223> Synthetic DNA/RNA primer, R391
<400> 895
   cagagagatu cgaggcagag ug 22
<210> 896
   <211> 31
   <212> DNA
   <213> Artificial Sequence
<220>
   <223> Synthetic DNA/RNA primer, R392
<400> 896
   agtaguggat ttugcttctc tgatataaac t 31
<210> 897
   <211> 32
   <212> DNA
   <213> Artificial Sequence
<220>
   <223> Synthetic DNA/RNA primer, R393
<400> 897
   gctctuagcc aaaatatuag cataaaaatc ag 32
<210> 898
   <211> 31
   <212> DNA
   <213> Artificial Sequence
<220>
   <223> Synthetic DNA/RNA primer, R394
<400> 898
   aaaaagcatu gtttttaatc auacctgact t 31
<210> 899
   <211> 27
   <212> DNA
   <213> Artificial Sequence
<220>
   <223> Synthetic DNA/RNA primer, R395
<400> 899
   ggtacagaut tgtaaatcuc agggcaa 27
<210> 900
   <211> 20
   <212> RNA
   <213> Artificial Sequence
<220>
   <223> Synthetic Primer, R396
<400> 900
   gagaucacgg gugacagagc 20
<210> 901
   <211> 23
   <212> DNA
   <213> Artificial Sequence
<220>
   <223> Synthetic DNA/RNA primer, R397
<400> 901
   acctacctga uaccccagau ccc 23
<210> 902
   <211> 27
   <212> DNA
   <213> Artificial Sequence
<220>
   <223> Synthetic DNA/RNA primer, R398
<400> 902
   tccagattga ucttgggagt guaaaaa 27
<210> 903
   <211> 28
   <212> DNA
   <213> Artificial Sequence
<220>
   <223> Synthetic DNA/RNA primer, R399
<400> 903
   gtgtgctaga gguaactcat gataaugg 28
<210> 904
   <211> 26
   <212> DNA
   <213> Artificial Sequence
<220>
   <223> Synthetic DNA/RNA primer, R400
<400> 904
   gaaaggguca acaaaagaat guccat 26
<210> 905
   <211> 26
   <212> DNA
   <213> Artificial Sequence
<220>
   <223> Synthetic DNA/RNA primer, R401
<400> 905
   gaaagttccc caautgaaag tugcag 26
<210> 906
   <211> 30
   <212> DNA
   <213> Artificial Sequence
<220>
   <223> Synthetic DNA/RNA primer, R402
<400> 906
   aactttgtaa tucaacattc atcgttgugt 30
<210> 907
   <211> 27
   <212> DNA
   <213> Artificial Sequence
<220>
   <223> Synthetic DNA/RNA primer, R403
<400> 907
   tagatgatag guggtacatg cacagut 27
<210> 908
   <211> 29
   <212> DNA
   <213> Artificial Sequence
<220>
   <223> Synthetic DNA/RNA primer, R404
<400> 908
   accugaatta tcactaucag aacaaagca 29
<210> 909
   <211> 21
   <212> DNA
   <213> Artificial Sequence
<220>
   <223> Synthetic DNA/RNA primer, R405
<400> 909
   gaacaguacc cgttccctug a 21
<210> 910
   <211> 22
   <212> DNA
   <213> Artificial Sequence
<220>
   <223> Synthetic DNA/RNA primer, R406
<400> 910
   cttgaggacc ugcgaaaucc ag 22
<210> 911
   <211> 30
   <212> DNA
   <213> Artificial Sequence
<220>
   <223> Synthetic DNA/RNA primer, R407
<400> 911
   tggaaagctt cucaaagtat ttcatttuct 30
<210> 912
   <211> 27
   <212> DNA
   <213> Artificial Sequence
<220>
   <223> Synthetic DNA/RNA primer, R408
<400> 912
   gcagcgttta uagtctgctt ttacauc 27
<210> 913
   <211> 26
   <212> DNA
   <213> Artificial Sequence
<220>
   <223> Synthetic DNA/RNA primer, R409
<400> 913
   aacgggctug gaagaaaata aucaag 26
<210> 914
   <211> 25
   <212> DNA
   <213> Artificial Sequence
<220>
   <223> Synthetic DNA/RNA primer, R410
<400> 914
   tctgctagcu tgttttctuc acagt 25
<210> 915
   <211> 30
   <212> DNA
   <213> Artificial Sequence
<220>
   <223> Synthetic DNA/RNA primer, R411
<400> 915
   aacaatatac ctuctcagtc tacuaggcat 30
<210> 916
   <211> 28
   <212> DNA
   <213> Artificial Sequence
<220>
   <223> Synthetic DNA/RNA primer, R412
<400> 916
   cagataactu agaacagcct augggaag 28
<210> 917
   <211> 27
   <212> DNA
   <213> Artificial Sequence
<220>
   <223> Synthetic DNA/RNA primer, R413
<400> 917
   ggccaaaatu gaatgctatg ctuagat 27
<210> 918
   <211> 30
   <212> DNA
   <213> Artificial Sequence
<220>
   <223> Synthetic DNA/RNA primer, R414
<400> 918
   agcacaatua gccgtaataa catuagagaa 30
<210> 919
   <211> 28
   <212> DNA
   <213> Artificial Sequence
<220>
   <223> Synthetic DNA/RNA primer, R415
<400> 919
   tggactcatt acuccaaata aacaugga 28
<210> 920
   <211> 28
   <212> DNA
   <213> Artificial Sequence
<220>
   <223> Synthetic DNA/RNA primer, R416
<400> 920
   gtctaatatc aagccugtac agacagut 28
<210> 921
   <211> 27
   <212> DNA
   <213> Artificial Sequence
<220>
   <223> Synthetic DNA/RNA primer, R417
<400> 921
   tgcagaatac autcaaggtt ucaaagc 27
<210> 922
   <211> 25
   <212> DNA
   <213> Artificial Sequence
<220>
   <223> Synthetic DNA/RNA primer, R418
<400> 922
   aataaatgtg ugagtcagtg ugcag 25
<210> 923
   <211> 22
   <212> DNA
   <213> Artificial Sequence
<220>
   <223> Synthetic DNA/RNA primer, R419
<400> 923
   aagccttcau ccggagagtg ua 22
<210> 924
   <211> 25
   <212> DNA
   <213> Artificial Sequence
<220>
   <223> Synthetic DNA/RNA primer, R420
<400> 924
   taatgcugaa gaccccaaag atcuc 25
<210> 925
   <211> 27
   <212> RNA
   <213> Artificial Sequence
<220>
   <223> Synthetic Primer, R421
<400> 925
   gccaaaugaa cagacaagua aaagaca 27
<210> 926
   <211> 28
   <212> DNA
   <213> Artificial Sequence
<220>
   <223> Synthetic DNA/RNA primer, R422
<400> 926
   gcaaattgat agutgttcta gcagugaa 28
<210> 927
   <211> 28
   <212> DNA
   <213> Artificial Sequence
<220>
   <223> Synthetic DNA/RNA primer, R423
<400> 927
   cagcagtaua agcaatatgg aacucgaa 28
<210> 928
   <211> 26
   <212> DNA
   <213> Artificial Sequence
<220>
   <223> Synthetic DNA/RNA primer, R424
<400> 928
   ggagcagaat ggucaagtga tgaaua 26
<210> 929
   <211> 32
   <212> DNA
   <213> Artificial Sequence
<220>
   <223> Synthetic DNA/RNA primer, R425
<400> 929
   ttttataact agatttucct tctctccatu cc 32
<210> 930
   <211> 24
   <212> DNA
   <213> Artificial Sequence
<220>
   <223> Synthetic DNA/RNA primer, R426
<400> 930
   agagcgtccc cucacaaata aaut 24
<210> 931
   <211> 28
   <212> DNA
   <213> Artificial Sequence
<220>
   <223> Synthetic DNA/RNA primer, R427
<400> 931
   gaaagagttc acuccaaatc aguagaga 28
<210> 932
   <211> 24
   <212> DNA
   <213> Artificial Sequence
<220>
   <223> Synthetic DNA/RNA primer, R428
<400> 932
   ggttctgaug actcacatga uggg 24
<210> 933
   <211> 28
   <212> DNA
   <213> Artificial Sequence
<220>
   <223> Synthetic DNA/RNA primer, R429
<400> 933
   ccctgtguga gagaaaagaa tggaauaa 28
<210> 934
   <211> 28
   <212> DNA
   <213> Artificial Sequence
<220>
   <223> Synthetic DNA/RNA primer, R430
<400> 934
   aggcugaatt ctgtaauaaa agcaaaca 28
<210> 935
   <211> 26
   <212> DNA
   <213> Artificial Sequence
<220>
   <223> Synthetic DNA/RNA primer, R431
<400> 935
   agggtagttc ugtttcaaac tugcat 26
<210> 936
   <211> 30
   <212> DNA
   <213> Artificial Sequence
<220>
   <223> Synthetic DNA/RNA primer, R432
<400> 936
   tgtatatttt cagcugcttg tgaatttuct 30
<210> 937
   <211> 28
   <212> DNA
   <213> Artificial Sequence
<220>
   <223> Synthetic DNA/RNA primer, R433
<400> 937
   gacagttctg cauacatgta actagugt 28
<210> 938
   <211> 22
   <212> RNA
   <213> Artificial Sequence
<220>
   <223> Synthetic Primer, R434
<400> 938
   gcggauacaa ccucaaaaga cg 22
<210> 939
   <211> 27
   <212> DNA
   <213> Artificial Sequence
<220>
   <223> Synthetic DNA/RNA primer, R435
<400> 939
   tgucaagttt ctctucagga ggaaaag 27
<210> 940
   <211> 32
   <212> DNA
   <213> Artificial Sequence
<220>
   <223> Synthetic DNA/RNA primer, R436
<400> 940
   aaggaaaata acuctcctga acatcuaaaa ga 32
<210> 941
   <211> 30
   <212> DNA
   <213> Artificial Sequence
<220>
   <223> Synthetic DNA/RNA primer, R437
<400> 941
   tgttgaagag cuattgaaaa tcatttgugc 30
<210> 942
   <211> 30
   <212> DNA
   <213> Artificial Sequence
<220>
   <223> Synthetic DNA/RNA primer, R438
<400> 942
   acagctcaaa gutgaactta ttcacuaaga 30
<210> 943
   <211> 31
   <212> DNA
   <213> Artificial Sequence
<220>
   <223> Synthetic DNA/RNA primer, R439
<400> 943
   atgtttttct aatgugttaa agttcatugg a 31
<210> 944
   <211> 28
   <212> DNA
   <213> Artificial Sequence
<220>
   <223> Synthetic DNA/RNA primer, R440
**<400>** 944
   gccagtttcc auatgatcca tctauagt 28
<210> 945
   <211> 28
   <212> DNA
   <213> Artificial Sequence
<220>
   <223> Synthetic DNA/RNA primer, R441
<400> 945
   agaaacctta accauactgc cgtataug 28
<210> 946
   <211> 24
   <212> DNA
   <213> Artificial Sequence
<220>
   <223> Synthetic DNA/RNA primer, R442
<400> 946
   gccacttttu gggtatctgc acua 24
<210> 947
   <211> 23
   <212> DNA
   <213> Artificial Sequence
<220>
   <223> Synthetic DNA/RNA primer, R443
<400> 947
   ttcaagaggu gtacaggcau cag 23
<210> 948
   <211> 28
   <212> DNA
   <213> Artificial Sequence
<220>
   <223> Synthetic DNA/RNA primer, R444
<400> 948
   ggtcaggaaa gaauccaagt ttggtaua 28
<210> 949
   <211> 28
   <212> DNA
   <213> Artificial Sequence
<220>
   <223> Synthetic DNA/RNA primer, R445
<400> 949
   cctcagctcc uagactttca gaaataug 28
<210> 950
   <211> 32
   <212> DNA
   <213> Artificial Sequence
<220>
   <223> Synthetic DNA/RNA primer, R446
<400> 950
   aaactccatc ucaaacaaac aaacaaatua at 32
<210> 951
   <211> 29
   <212> DNA
   <213> Artificial Sequence
<220>
   <223> Synthetic DNA/RNA primer, R447
<400> 951
   cctcctgaat ttuagtgaat aaggctuct 29
<210> 952
   <211> 20
   <212> DNA
   <213> Artificial Sequence
<220>
   <223> Synthetic DNA/RNA primer, R448
<400> 952
   gcaaagcacg aacutgcugt 20
<210> 953
   <211> 25
   <212> DNA
   <213> Artificial Sequence
<220>
   <223> Synthetic DNA/RNA primer, R449
<400> 953
   gtgauggcca gagagtcuaa aacag 25
<210> 954
   <211> 26
   <212> DNA
   <213> Artificial Sequence
<220>
   <223> Synthetic DNA/RNA primer, R450
<400> 954
   tgacatccct ugataaacct tgtucc 26
<210> 955
   <211> 26
   <212> DNA
   <213> Artificial Sequence
<220>
   <223> Synthetic DNA/RNA primer, R451
<400> 955
   tttttgtcgc ugctaactgt atgtua 26
<210> 956
   <211> 32
   <212> DNA
   <213> Artificial Sequence
<220>
   <223> Synthetic DNA/RNA primer, R452
<400> 956
   gctccaacta aucataagag atttuaaaag ac 32
<210> 957
   <211> 26
   <212> DNA
   <213> Artificial Sequence
<220>
   <223> Synthetic DNA/RNA primer, R453
<400> 957
   aagtaagaag gccugatttg gatuct 26
<210> 958
   <211> 28
   <212> DNA
   <213> Artificial Sequence
<220>
   <223> Synthetic DNA/RNA primer, R454
<400> 958
   gctatttcct ugatactgga ctgucaaa 28
<210> 959
   <211> 31
   <212> DNA
   <213> Artificial Sequence
<220>
   <223> Synthetic DNA/RNA primer, R455
<400> 959
   attccttgag uttacattaa ctuaccagaa g 31
<210> 960
   <211> 28
   <212> DNA
   <213> Artificial Sequence
<220>
   <223> Synthetic DNA/RNA primer, R456
<400> 960
   atgacaatta tcaaccucat ctgctcut 28
<210> 961
   <211> 30
   <212> DNA
   <213> Artificial Sequence
<220>
   <223> Synthetic DNA/RNA primer, R457
<400> 961
   taaattgutt ttctcctgtu gaaccagaca 30
<210> 962
   <211> 27
   <212> DNA
   <213> Artificial Sequence
<220>
   <223> Synthetic DNA/RNA primer, R458
<400> 962
   cctgcttatt tutctcacat tctuccg 27
<210> 963
   <211> 29
   <212> DNA
   <213> Artificial Sequence
<220>
   <223> Synthetic DNA/RNA primer, R459
<400> 963
   ggttuagaga ctttcucaaa ggcttagat 29
<210> 964
   <211> 25
   <212> DNA
   <213> Artificial Sequence
<220>
   <223> Synthetic DNA/RNA primer, R460
<400> 964
   gtgtttucac tgtctgucac agaag 25
<210> 965
   <211> 31
   <212> DNA
   <213> Artificial Sequence
<220>
   <223> Synthetic DNA/RNA primer, R461
<400> 965
   aaaactatct tcutcagagg tatcuacaac t 31
<210> 966
   <211> 24
   <212> DNA
   <213> Artificial Sequence
<220>
   <223> Synthetic DNA/RNA primer, R462
<400> 966
   gtgacguact gggttttuag caag 24
<210> 967
   <211> 27
   <212> DNA
   <213> Artificial Sequence
<220>
   <223> Synthetic DNA/RNA primer, R463
<400> 967
   ggcttctgat tugctacatt tgaauct 27
<210> 968
   <211> 31
   <212> DNA
   <213> Artificial Sequence
<220>
   <223> Synthetic DNA/RNA primer, R464
<400> 968
   aggtcttttt ctgaaauatt ttggtcacau g 31
<210> 969
   <211> 26
   <212> DNA
   <213> Artificial Sequence
<220>
   <223> Synthetic DNA/RNA primer, R465
<400> 969
   agatattgcc ugctttacug caagaa 26
<210> 970
   <211> 25
   <212> DNA
   <213> Artificial Sequence
<220>
   <223> Synthetic DNA/RNA primer, R466
<400> 970
   tgtatttcca guccacttuc agagg 25
<210> 971
   <211> 32
   <212> DNA
   <213> Artificial Sequence
<220>
   <223> Synthetic DNA/RNA primer, R467
<400> 971
   ttgttttctt ttucaaagtg gatatuaaac ct 32
<210> 972
   <211> 32
   <212> DNA
   <213> Artificial Sequence
<220>
   <223> Synthetic DNA/RNA primer, R468
<400> 972
   cagaaggaat cgucatctat aaaactatau gt 32
<210> 973
   <211> 32
   <212> DNA
   <213> Artificial Sequence
<220>
   <223> Synthetic DNA/RNA primer, R469
<400> 973
   ctgtagtttt tcctuattac attttgcttc ut 32
<210> 974
   <211> 28
   <212> DNA
   <213> Artificial Sequence
<220>
   <223> Synthetic DNA/RNA primer, R470
<400> 974
   tgggattgaa agucagtatc actgtaut 28
<210> 975
   <211> 28
   <212> DNA
   <213> Artificial Sequence
<220>
   <223> Synthetic DNA/RNA primer, R471
<400> 975
   gttacctttg agcutgtctg acatttug 28
<210> 976
   <211> 31
   <212> DNA
   <213> Artificial Sequence
<220>
   <223> Synthetic DNA/RNA primer, R472
<400> 976
   ttggattact ctuagatttg tgttttggtu g 31
<210> 977
   <211> 26
   <212> DNA
   <213> Artificial Sequence
<220>
   <223> Synthetic DNA/RNA primer, R473
<400> 977
   atggtagagt tcutgaaaat gggtuc 26
<210> 978
   <211> 33
   <212> DNA
   <213> Artificial Sequence
<220>
   <223> Synthetic DNA/RNA primer, R474
<400> 978
   gtattttatc tatatucaag gagatgtccg aut 33
<210> 979
   <211> 27
   <212> DNA
   <213> Artificial Sequence
<220>
   <223> Synthetic DNA/RNA primer, R475
<400> 979
   gccttttggc uaggtgttaa attaugg 27
<210> 980
   <211> 22
   <212> DNA
   <213> Artificial Sequence
<220>
   <223> Synthetic DNA/RNA primer, R476
<400> 980
   gtctaccuga ccaatcgaug gg 22
<210> 981
   <211> 24
   <212> DNA
   <213> Artificial Sequence
<220>
   <223> Synthetic DNA/RNA primer, R477
<400> 981
   agcttttugc agagcttcag uaga 24
<210> 982
   <211> 29
   <212> DNA
   <213> Artificial Sequence
<220>
   <223> Synthetic DNA/RNA primer, R478
<400> 982
   ggccagataa ttuaagacat atgttgugc 29
<210> 983
   <211> 26
   <212> DNA
   <213> Artificial Sequence
<220>
   <223> Synthetic DNA/RNA primer, R479
<400> 983
   gctccgtttu agtagcagtt aacugt 26
<210> 984
   <211> 30
   <212> DNA
   <213> Artificial Sequence
<220>
   <223> Synthetic DNA/RNA primer, R480
<400> 984
   gtctgtttcc ucataactta gaatguccat 30
<210> 985
   <211> 26
   <212> DNA
   <213> Artificial Sequence
<220>
   <223> Synthetic DNA/RNA primer, R481
<400> 985
   tcactgtgcg aagacuttta tgtcua 26
<210> 986
   <211> 27
   <212> DNA
   <213> Artificial Sequence
<220>
   <223> Synthetic DNA/RNA primer, R482
<400> 986
   tttcactttg uccaaagatt ccttugc 27
<210> 987
   <211> 28
   <212> DNA
   <213> Artificial Sequence
<220>
   <223> Synthetic DNA/RNA primer, R483
<400> 987
   agaattctgc auttctttac acttuggg 28
<210> 988
   <211> 26
   <212> DNA
   <213> Artificial Sequence
<220>
   <223> Synthetic DNA/RNA primer, R484
<400> 988
   ggactgattu gtgtaacaag tugcag 26
<210> 989
   <211> 32
   <212> DNA
   <213> Artificial Sequence
<220>
   <223> Synthetic DNA/RNA primer, R485
<400> 989
   tcatacaaat aatutcctac ataatcugca gt 32
<210> 990
   <211> 28
   <212> DNA
   <213> Artificial Sequence
<220>
   <223> Synthetic DNA/RNA primer, R486
<400> 990
   caatactggc ucaataccag aaucaagt 28
<210> 991
   <211> 24
   <212> DNA
   <213> Artificial Sequence
<220>
   <223> Synthetic DNA/RNA primer, R487
<400> 991
   aacctgccau aattttcgtt uggc 24
<210> 992
   <211> 28
   <212> DNA
   <213> Artificial Sequence
<220>
   <223> Synthetic DNA/RNA primer, R488
<400> 992
   gaagtttcca aacuaacatc acaaggug 28
<210> 993
   <211> 26
   <212> DNA
   <213> Artificial Sequence
<220>
   <223> Synthetic DNA/RNA primer, R489
<400> 993
   atttcagaaa acacutgtct tgcgut 26
<210> 994
   <211> 28
   <212> DNA
   <213> Artificial Sequence
<220>
   <223> Synthetic DNA/RNA primer, R490
<400> 994
   accacattat augaaaagcc ttttuggg 28
<210> 995
   <211> 26
   <212> DNA
   <213> Artificial Sequence
<220>
   <223> Synthetic DNA/RNA primer, R491
<400> 995
   gguttctctt aucaacacga ggaagt 26
<210> 996
   <211> 28
   <212> DNA
   <213> Artificial Sequence
<220>
   <223> Synthetic DNA/RNA primer, R492
<400> 996
   ctgtcagttc aucatcttcc auaaaagc 28
<210> 997
   <211> 31
   <212> DNA
   <213> Artificial Sequence
<220>
   <223> Synthetic DNA/RNA primer, R493
<400> 997
   tataccauac ctauagaggg agaacagata t 31
<210> 998
   <211> 28
   <212> DNA
   <213> Artificial Sequence
<220>
   <223> Synthetic DNA/RNA primer, R494
<400> 998
   gcttgaagat ttutccaaag tcagaugt 28
<210> 999
   <211> 27
   <212> DNA
   <213> Artificial Sequence
<220>
   <223> Synthetic DNA/RNA primer, R495
<400> 999
   gttttgcttt ugtctgtttt ccuccaa 27
<210> 1000
   <211> 28
   <212> DNA
   <213> Artificial Sequence
<220>
   <223> Synthetic DNA/RNA primer, R496
<400> 1000
   aggcaaaaat tcaucacaca aattguca 28
<210> 1001
   <211> 23
   <212> DNA
   <213> Artificial Sequence
<220>
   <223> Synthetic DNA/RNA primer, R497
<400> 1001
   tcattggagg guatgagcca ucc 23
<210> 1002
   <211> 21
   <212> DNA
   <213> Artificial Sequence
<220>
   <223> Synthetic DNA/RNA primer, F499
<400> 1002
   acaacugcag caaagacugg t 21
<210> 1003
   <211> 28
   <212> RNA
   <213> Artificial Sequence
<220>
   <223> Synthetic Primer, F500
<400> 1003
   aguuaauuuu gguuacaucc cucucugc 28
<210> 1004
   <211> 25
   <212> RNA
   <213> Artificial Sequence
<220>
   <223> Synthetic Primer, F501
<400> 1004
   aucgaucugu uagaaaccuc uccag 25
<210> 1005
   <211> 19
   <212> DNA
   <213> Artificial Sequence
<220>
   <223> Synthetic DNA/RNA primer, F502
<400> 1005
   ggacucugua ggcugcagt 19
<210> 1006
   <211> 22
   <212> RNA
   <213> Artificial Sequence
<220>
   <223> Synthetic Primer, F503
<400> 1006
   ugaggcaguc uuuacucacc ug 22
<210> 1007
   <211> 28
   <212> DNA
   <213> Artificial Sequence
<220>
   <223> Synthetic DNA/RNA primer, F504
<400> 1007
   acaagcaaag ucucuauggu gauuaugt 28
<210> 1008
   <211> 25
   <212> DNA
   <213> Artificial Sequence
<220>
   <223> Synthetic DNA/RNA primer, F505
<400> 1008
   caacuaccau ccagcaacag aaaat 25
<210> 1009
   <211> 28
   <212> RNA
   <213> Artificial Sequence
<220>
   <223> Synthetic Primer, F506
<400> 1009
   gacagaugag agaaaugcac uuagaaga 28
<210> 1010
   <211> 25
   <212> RNA
   <213> Artificial Sequence
<220>
   <223> Synthetic Primer, F507
<400> 1010
   aggaaugugu uucuccauac agguc 25
<210> 1011
   <211> 24
   <212> RNA
   <213> Artificial Sequence
<220>
   <223> Synthetic Primer, F508
<400> 1011
   cuucaagcag ugagaauacg ucca 24
<210> 1012
   <211> 20
   <212> RNA
   <213> Artificial Sequence
<220>
   <223> Synthetic Primer, F509
<400> 1012
   aggguccagg uucuuccaga 20
<210> 1013
   <211> 26
   <212> RNA
   <213> Artificial Sequence
<220>
   <223> Synthetic Primer, F510
<400> 1013
   gauaguuuug agagucguuc gauugc 26
<210> 1014
   <211> 21
   <212> RNA
   <213> Artificial Sequence
<220>
   <223> Synthetic Primer, F511
<400> 1014
   cuccaccacc uccucaaaca g 21
<210> 1015
   <211> 18
   <212> RNA
   <213> Artificial Sequence
<220>
   <223> Synthetic Primer, F512
<400> 1015
   aucagccagg cacaaagc 18
<210> 1016
   <211> 28
   <212> DNA
   <213> Artificial Sequence
<220>
   <223> Synthetic DNA/RNA primer, F513
<400> 1016
   caucuuuguc aucagcugaa gaugaaat 28
<210> 1017
   <211> 29
   <212> RNA
   <213> Artificial Sequence
<220>
   <223> Synthetic Primer, F514
<400> 1017
   gccuaaagaa ucaaaugaaa accaagaga 29
<210> 1018
   <211> 18
   <212> RNA
   <213> Artificial Sequence
<220>
   <223> Synthetic Primer, F515
<400> 1018
   gugacccgga gcacuucc 18
<210> 1019
   <211> 26
   <212> DNA
   <213> Artificial Sequence
<220>
   <223> Synthetic DNA/RNA primer, F516
<400> 1019
   ccacauuaca uacuuaccau gccact 26
<210> 1020
   <211> 18
   <212> RNA
   <213> Artificial Sequence
<220>
   <223> Synthetic Primer, F517
<400> 1020
   augggaccca cuccaucg 18
<210> 1021
   <211> 22
   <212> RNA
   <213> Artificial Sequence
<220>
   <223> Synthetic Primer, F518
<400> 1021
   cccuucuaag gacccccucu uc 22
<210> 1022
   <211> 20
   <212> DNA
   <213> Artificial Sequence
<220>
   <223> Synthetic DNA/RNA primer, F519
<400> 1022
   cucugccggg cuuugaucut 20
<210> 1023
   <211> 19
   <212> DNA
   <213> Artificial Sequence
<220>
   <223> Synthetic DNA/RNA primer, F520
<400> 1023
   uacuaccgcc ucacacgct 19
<210> 1024
   <211> 21
   <212> RNA
   <213> Artificial Sequence
<220>
   <223> Synthetic Primer, F521
<400> 1024
   uucccucucu ccuucugccu c 21
<210> 1025
   <211> 25
   <212> DNA
   <213> Artificial Sequence
<220>
   <223> Synthetic Primer, F522
<400> 1025
   ccagcagaag acaaaaagac aaaca 25
<210> 1026
   <211> 21
   <212> DNA
   <213> Artificial Sequence
<220>
   <223> Synthetic DNA/RNA primer, F523
<400> 1026
   ggagagggag gagagcuaac t 21
<210> 1027
   <211> 22
   <212> RNA
   <213> Artificial Sequence
<220>
   <223> Synthetic Primer, F524
<400> 1027
   agaagcugug cauuuacacc ga 22
<210> 1028
   <211> 24
   <212> RNA
   <213> Artificial Sequence
<220>
   <223> Synthetic Primer, F525
<400> 1028
   uuuugcugau gcuaugcucu ccac 24
<210> 1029
   <211> 19
   <212> RNA
   <213> Artificial Sequence
<220>
   <223> Synthetic Primer, F526
<400> 1029
   cugauccgca agcaugcuc 19
<210> 1030
   <211> 21
   <212> RNA
   <213> Artificial Sequence
<220>
   <223> Synthetic Primer, F527
<400> 1030
   ugagcucgcu cacuugugau g 21
<210> 1031
   <211> 23
   <212> RNA
   <213> Artificial Sequence
<220>
   <223> Synthetic Primer, F528
<400> 1031
   caggauccaa auucguucug ugc 23
<210> 1032
   <211> 25
   <212> DNA
   <213> Artificial Sequence
<220>
   <223> Synthetic DNA/RNA primer, F529
<400> 1032
   uacuaacaac ucuggucugg accat 25
<210> 1033
   <211> 28
   <212> RNA
   <213> Artificial Sequence
<220>
   <223> Synthetic Primer, F530
<400> 1033
   augcuauuug gacaauaaac ucaccuug 28
<210> 1034
   <211> 25
   <212> RNA
   <213> Artificial Sequence
<220>
   <223> Synthetic Primer, F531
<400> 1034
   cuccuuaccu cauacagugc agaaa 25
<210> 1035
   <211> 20
   <212> RNA
   <213> Artificial Sequence
<220>
   <223> Synthetic Primer, F532
<400> 1035
   cccugagugc agcuucgauc 20
<210> 1036
   <211> 20
   <212> RNA
   <213> Artificial Sequence
<220>
   <223> Synthetic Primer, F533
<400> 1036
   cgaucaugga uggcggguac 20
<210> 1037
   <211> 22
   <212> RNA
   <213> Artificial Sequence
<220>
   <223> Synthetic Primer, F534
<400> 1037
   uuuaugaaug gagaggcugc ug 22
<210> 1038
   <211> 26
   <212> RNA
   <213> Artificial Sequence
<220>
   <223> Synthetic Primer, F535
<400> 1038
   accucucacc cuuauaaguc uucuga 26
<210> 1039
   <211> 21
   <212> RNA
   <213> Artificial Sequence
<220>
   <223> Synthetic Primer, F536
<400> 1039
   acuugagcuu cccuaggacc a 21
<210> 1040
   <211> 20
   <212> RNA
   <213> Artificial Sequence
<220>
   <223> Synthetic Primer, F537
<400> 1040
   gggagacaau acgugucggg 20
<210> 1041
   <211> 23
   <212> RNA
   <213> Artificial Sequence
<220>
   <223> Synthetic Primer, F538
<400> 1041
   aagacaggua gcgauccagg uag 23
<210> 1042
   <211> 21
   <212> RNA
   <213> Artificial Sequence
<220>
   <223> Synthetic Primer, F539
<400> 1042
   cuaggugccc auguccaucu g 21
<210> 1043
   <211> 23
   <212> RNA
   <213> Artificial Sequence
<220>
   <223> Synthetic Primer, F540
<400> 1043
   gauguccaau guaccugagg caa 23
<210> 1044
   <211> 30
   <212> RNA
   <213> Artificial Sequence
<220>
   <223> Synthetic Primer, F541
<400> 1044
   cagcagaaag aggacucaga auagaaaauc 30

<210> 1045
   <211> 24
   <212> RNA
   <213> Artificial Sequence
<220>
   <223> Synthetic Primer, F542
<400> 1045
   agagauucgc uugugugggu uaaa 24
<210> 1046
   <211> 21
   <212> RNA
   <213> Artificial Sequence
<220>
   <223> Synthetic Primer, F543
<400> 1046
   accaggguua ccuugaucuc c 21
<210> 1047
   <211> 24
   <212> RNA
   <213> Artificial Sequence
<220>
   <223> Synthetic Primer, F544
<400> 1047
   acuucucaau ugcuacgggc aauc 24
<210> 1048
   <211> 22
   <212> DNA
   <213> Artificial Sequence
<220>
   <223> Synthetic DNA/RNA primer, F545
<400> 1048
   agaucucggu gaacgaugca at 22
<210> 1049
   <211> 24
   <212> RNA
   <213> Artificial Sequence
<220>
   <223> Synthetic Primer, F546
<400> 1049
   uauguggacu gcagaagaac uucg 24
<210> 1050
   <211> 25
   <212> RNA
   <213> Artificial Sequence
<220>
   <223> Synthetic Primer, F547
<400> 1050
   ugugguuuau gaacaagcga uuugg 25
<210> 1051
   <211> 24
   <212> RNA
   <213> Artificial Sequence
<220>
   <223> Synthetic Primer, F548
<400> 1051
   gcucauaucg agagguagcc auuc 24
<210> 1052
   <211> 23
   <212> RNA
   <213> Artificial Sequence
<220>
   <223> Synthetic Primer, F549
<400> 1052
   gcagacgagc uugacaucag aaa 23
<210> 1053
   <211> 21
   <212> RNA
   <213> Artificial Sequence
<220>
   <223> Synthetic Primer, F550
<400> 1053
   aagacuucgg gugcucugua c 21
<210> 1054
   <211> 26
   <212> RNA
   <213> Artificial Sequence
<220>
   <223> Synthetic Primer, F551
<400> 1054
   gggacagacu gucauucaaa auagga 26
<210> 1055
   <211> 22
   <212> RNA
   <213> Artificial Sequence
<220>
   <223> Synthetic Primer, F552
<400> 1055
   ugaagaaagu ccagaccucg ga 22
<210> 1056
   <211> 23
   <212> RNA
   <213> Artificial Sequence
<220>
   <223> Synthetic Primer, F553
<400> 1056
   gucgacugcc ugauaagaca uga 23
<210> 1057
   <211> 26
   <212> RNA
   <213> Artificial Sequence
<220>
   <223> Synthetic Primer, F554
<400> 1057
   uaguuugguu ucucugucug uucgug 26
<210> 1058
   <211> 19
   <212> DNA
   <213> Artificial Sequence
<220>
   <223> Synthetic DNA/RNA primer, F555
<400> 1058
   aagccuccau cgcuaccct 19
<210> 1059
   <211> 17
   <212> DNA
   <213> Artificial Sequence
<220>
   <223> Synthetic DNA/RNA primer, F556
<400> 1059
   ucaggcgcca aguaggt 17
<210> 1060
   <211> 21
   <212> RNA
   <213> Artificial Sequence
<220>
   <223> Synthetic Primer, F557
<400> 1060
   aaagcggcug uuagucacug g 21
<210> 1061
   <211> 25
   <212> RNA
   <213> Artificial Sequence
<220>
   <223> Synthetic Primer, F558
<400> 1061
   uaaagaucau gucucggcuc aagga 25
<210> 1062
   <211> 24
   <212> RNA
   <213> Artificial Sequence
<220>
   <223> Synthetic Primer, F559
<400> 1062
   gugcacaggu uauucugauu uccc 24
<210> 1063
   <211> 21
   <212> RNA
   <213> Artificial Sequence
<220>
   <223> Synthetic Primer, F560
<400> 1063
   agaagggcua ggccaauuga c 21
<210> 1064
   <211> 24
   <212> RNA
   <213> Artificial Sequence
<220>
   <223> Synthetic Primer, F561
<400> 1064
   gucagccuga acauaacauc cuug 24
<210> 1065
   <211> 20
   <212> RNA
   <213> Artificial Sequence
<220>
   <223> Synthetic Primer, F562
<400> 1065
   gggaccuccg gucagaaaac 20
<210> 1066
   <211> 21
   <212> RNA
   <213> Artificial Sequence
<220>
   <223> Synthetic Primer, F563
<400> 1066
   cucccaacca agcucucuug a 21
<210> 1067
   <211> 27
   <212> RNA
   <213> Artificial Sequence
<220>
   <223> Synthetic Primer, F564
<400> 1067
   cccagaaggu gagaaaguua aaauucc 27
<210> 1068
   <211> 21
   <212> RNA
   <213> Artificial Sequence
<220>
   <223> Synthetic Primer, F565
<400> 1068
   agggcaugaa cuacuuggag g 21
<210> 1069
   <211> 21
   <212> RNA
   <213> Artificial Sequence
<220>
   <223> Synthetic Primer, F566
<400> 1069
   gccucucccu cccuccagga a 21
<210> 1070
   <211> 17
   <212> DNA
   <213> Artificial Sequence
<220>
   <223> Synthetic DNA/RNA primer, F567
<400> 1070
   ugccucaccu ccaccgt 17
<210> 1071
   <211> 21
   <212> RNA
   <213> Artificial Sequence
<220>
   <223> Synthetic Primer, F568
<400> 1071
   aaguguaaga agugcgaagg g 21
<210> 1072
   <211> 26
   <212> RNA
   <213> Artificial Sequence
<220>
   <223> Synthetic Primer, F569
<400> 1072
   gccuuuuuaa cugguagaga uuggug 26
<210> 1073
   <211> 24
   <212> RNA
   <213> Artificial Sequence
<220>
   <223> Synthetic Primer, F570
<400> 1073
   ucaucaccuu ccuuucaugc ucuc 24
<210> 1074
   <211> 22
   <212> RNA
   <213> Artificial Sequence
<220>
   <223> Synthetic Primer, F571
<400> 1074
   uccuacgugg ugugugucug aa 22
<210> 1075
   <211> 25
   <212> RNA
   <213> Artificial Sequence
<220>
   <223> Synthetic Primer, F572
<400> 1075
   ugaucaucga auucuccaaa auggc 25
<210> 1076
   <211> 25
   <212> RNA
   <213> Artificial Sequence
<220>
   <223> Synthetic Primer, F573
<400> 1076
   gaugagaugu gguacaagca uucca 25
<210> 1077
   <211> 28
   <212> DNA
   <213> Artificial Sequence
<220>
   <223> Synthetic DNA/RNA primer, F574
<400> 1077
   ccccuacagc auuguuaaga aaguauut 28
<210> 1078
   <211> 20
   <212> RNA
   <213> Artificial Sequence
<220>
   <223> Synthetic Primer, F575
<400> 1078
   cugggacuag caugcugacc 20
<210> 1079
   <211> 20
   <212> DNA
   <213> Artificial Sequence
<220>
   <223> Synthetic DNA/RNA primer, F576
<400> 1079
   cugccugucu cugguucugt 20
<210> 1080
   <211> 22
   <212> RNA
   <213> Artificial Sequence
<220>
   <223> Synthetic Primer, F577
<400> 1080
   acuuggagug aguuuggaug gg 22
<210> 1081
   <211> 26
   <212> DNA
   <213> Artificial Sequence
<220>
   <223> Synthetic DNA/RNA primer, F578
<400> 1081
   uccugaucuc cuuagacaac uaccut 26
<210> 1082
   <211> 22
   <212> RNA
   <213> Artificial Sequence
<220>
   <223> Synthetic Primer, F579
<400> 1082
   uguuccuauu ucagccccac uc 22
<210> 1083
   <211> 23
   <212> RNA
   <213> Artificial Sequence
<220>
   <223> Synthetic Primer, F580
<400> 1083
   ggaaaggguc cucugaucau ugc 23
<210> 1084
   <211> 20
   <212> RNA
   <213> Artificial Sequence
<220>
   <223> Synthetic Primer, F581
<400> 1084
   cgagggccgg uauacauucg 20
<210> 1085
   <211> 24
   <212> DNA
   <213> Artificial Sequence
<220>
   <223> Synthetic DNA/RNA primer, F582
<400> 1085
   gaaugugaaa auuccagugg ccat 24
<210> 1086
   <211> 21
   <212> DNA
   <213> Artificial Sequence
<220>
   <223> Synthetic DNA/RNA primer, F583
<400> 1086
   auacccucuc agcguacccu t 21
<210> 1087
   <211> 19
   <212> RNA
   <213> Artificial Sequence
<220>
   <223> Synthetic Primer, F584
<400> 1087
   agcgcuuugu ggucaucca 19
<210> 1088
   <211> 22
   <212> RNA
   <213> Artificial Sequence
<220>
   <223> Synthetic Primer, F585
<400> 1088
   aaacuagccc ucaaucccug ac 22
<210> 1089
   <211> 23
   <212> RNA
   <213> Artificial Sequence
<220>
   <223> Synthetic Primer, F586
<400> 1089
   cacaguugga ggacuuccuc uuc 23
<210> 1090
   <211> 22
   <212> RNA
   <213> Artificial Sequence
<220>
   <223> Synthetic Primer, F587
<400> 1090
   cuacaugggu gcuucccauu cc 22
<210> 1091
   <211> 21
   <212> RNA
   <213> Artificial Sequence
<220>
   <223> Synthetic Primer, F588
<400> 1091
   guccucgugg ccaugaauga a 21
<210> 1092
   <211> 22
   <212> RNA
   <213> Artificial Sequence
<220>
   <223> Synthetic Primer, F589
<400> 1092
   cccaaucccc acaccaagua uc 22
<210> 1093
   <211> 25
   <212> DNA
   <213> Artificial Sequence
<220>
   <223> Synthetic DNA/RNA primer, F590
<400> 1093
   auguuccucc cucaucucua auggt 25
<210> 1094
   <211> 22
   <212> RNA
   <213> Artificial Sequence
<220>
   <223> Synthetic Primer, F591
<400> 1094
   uggacucgag caacauugau gg 22
<210> 1095
   <211> 21
   <212> RNA
   <213> Artificial Sequence
<220>
   <223> Synthetic Primer, F592
<400> 1095
   gcugaaguac cagaccugcu a 21
<210> 1096
   <211> 23
   <212> RNA
   <213> Artificial Sequence
<220>
   <223> Synthetic Primer, F593
<400> 1096
   ggauuugacc cuccaugauc agg 23
<210> 1097
   <211> 21
   <212> RNA
   <213> Artificial Sequence
<220>
   <223> Synthetic Primer, F594
<400> 1097
   ucacucucuc ucugcgcauu c 21
<210> 1098
   <211> 21
   <212> DNA
   <213> Artificial Sequence
<220>
   <223> Synthetic DNA/RNA primer, F595
<400> 1098
   caugaagugc aagaacgugg t 21
<210> 1099
   <211> 19
   <212> RNA
   <213> Artificial Sequence
<220>
   <223> Synthetic Primer, F596
<400> 1099
   gcggaucaga gccucaaac 19
<210> 1100
   <211> 28
   <212> DNA
   <213> Artificial Sequence
<220>
   <223> Synthetic DNA/RNA primer, F597
<400> 1100
   acuguccugu uuugauaucc cagauuut 28
<210> 1101
   <211> 25
   <212> RNA
   <213> Artificial Sequence
<220>
   <223> Synthetic Primer, F598
<400> 1101
   cugucucaau aucccaaacc cuaag 25
<210> 1102
   <211> 23
   <212> RNA
   <213> Artificial Sequence
<220>
   <223> Synthetic Primer, F599
<400> 1102
   uauuaguaug ccccugcaac gug 23
<210> 1103
   <211> 23
   <212> RNA
   <213> Artificial Sequence
<220>
   <223> Synthetic Primer, F600
<400> 1103
   caacccuccu gccaucauau uga 23
<210> 1104
   <211> 26
   <212> RNA
   <213> Artificial Sequence
<220>
   <223> Synthetic Primer, F601
<400> 1104
   caaccaugac aagauuuucc cuuacc 26
<210> 1105
   <211> 21
   <212> RNA
   <213> Artificial Sequence
<220>
   <223> Synthetic Primer, F602
<400> 1105
   ugccugugga ggaacuuuuc a 21
<210> 1106
   <211> 19
   <212> RNA
   <213> Artificial Sequence
<220>
   <223> Synthetic Primer, F603
<400> 1106
   cuuccucucg cccaucaca 19
<210> 1107
   <211> 20
   <212> DNA
   <213> Artificial Sequence
<220>
   <223> Synthetic DNA/RNA primer, F604
<400> 1107
   agugccuccu cucccaucut 20
<210> 1108
   <211> 25
   <212> RNA
   <213> Artificial Sequence
<220>
   <223> Synthetic Primer, F605
<400> 1108
   cacuccuugc uucucagaug aaacc 25
<210> 1109
   <211> 19
   <212> RNA
   <213> Artificial Sequence
<220>
   <223> Synthetic Primer, F606
<400> 1109
   cagguacucc cgcagguug 19
<210> 1110
   <211> 21
   <212> RNA
   <213> Artificial Sequence
<220>
   <223> Synthetic Primer, F607
<400> 1110
   cacgcauacg guuugguuug g 21
<210> 1111
   <211> 24
   <212> DNA
   <213> Artificial Sequence
<220>
   <223> Synthetic DNA/RNA primer, F608
<400> 1111
   cuagaagcuc ucuaucccac acct 24
<210> 1112
   <211> 25
   <212> RNA
   <213> Artificial Sequence
<220>
   <223> Synthetic Primer, F609
<400> 1112
   caaggaaugc cuucaaaaag uuggg 25
<210> 1113
   <211> 23
   <212> RNA
   <213> Artificial Sequence
<220>
   <223> Synthetic Primer, F610
<400> 1113
   agaugaugau cuccagguac agg 23
<210> 1114
   <211> 21
   <212> DNA
   <213> Artificial Sequence
<220>
   <223> Synthetic DNA/RNA primer, F611
<400> 1114
   cggcacugca ugcaauuucu t 21
<210> 1115
   <211> 26
   <212> RNA
   <213> Artificial Sequence
<220>
   <223> Synthetic Primer, F612
<400> 1115
   ccauuuauag cugagucucc auccug 26
<210> 1116
   <211> 23
   <212> DNA
   <213> Artificial Sequence
<220>
   <223> Synthetic DNA/RNA primer, F613
<400> 1116
   cccaguugug gguaccuuua gat 23
<210> 1117
   <211> 26
   <212> RNA
   <213> Artificial Sequence
<220>
   <223> Synthetic Primer, F614
<400> 1117
   cuuucaaacg agucaagcaa gaaugg 26
<210> 1118
   <211> 23
   <212> DNA
   <213> Artificial Sequence
<220>
   <223> Synthetic DNA/RNA primer, F615
<400> 1118
   accacacuuu ccauaaugag get 23
<210> 1119
   <211> 25
   <212> RNA
   <213> Artificial Sequence
<220>
   <223> Synthetic Primer, F616
<400> 1119
   cuuuuccauc uuuucugugu ugguc 25
<210> 1120
   <211> 25
   <212> RNA
   <213> Artificial Sequence
<220>
   <223> Synthetic Primer, F617
<400> 1120
   cagacaaauc ccaaaacaaa ccuga 25
<210> 1121
   <211> 24
   <212> RNA
   <213> Artificial Sequence
<220>
   <223> Synthetic Primer, F618
<400> 1121
   guagcuacag gacucagaua cgug 24
<210> 1122
   <211> 24
   <212> RNA
   <213> Artificial Sequence
<220>
   <223> Synthetic Primer, F619
<400> 1122
   guauuugggc gaaugcaguu uuuc 24
<210> 1123
   <211> 26
   <212> RNA
   <213> Artificial Sequence
<220>
   <223> Synthetic Primer, F620
<400> 1123
   ccagagaaaa gagaguuacu cacaca 26
<210> 1124
   <211> 24
   <212> RNA
   <213> Artificial Sequence
<220>
   <223> Synthetic Primer, F621
<400> 1124
   acuguguuac ugccaucgac uuac 24
<210> 1125
   <211> 22
   <212> DNA
   <213> Artificial Sequence
<220>
   <223> Synthetic DNA/RNA primer, F622
<400> 1125
   gguauucucg gagguugccu ut 22
<210> 1126
   <211> 22
   <212> RNA
   <213> Artificial Sequence
<220>
   <223> Synthetic Primer, F623
<400> 1126
   cuuggucgug uucuucauuc gg 22
<210> 1127
   <211> 19
   <212> RNA
   <213> Artificial Sequence
<220>
   <223> Synthetic Primer, F624
<400> 1127
   accacugugg aggcauuug 19
<210> 1128
   <211> 25
   <212> RNA
   <213> Artificial Sequence
<220>
   <223> Synthetic Primer, F625
<400> 1128
   agugaagauc ucccacauua acacc 25
<210> 1129
   <211> 21
   <212> RNA
   <213> Artificial Sequence
<220>
   <223> Synthetic Primer, F626
<400> 1129
   cuugcccaaa gcaaccuucu c 21
<210> 1130
   <211> 19
   <212> DNA
   <213> Artificial Sequence
<220>
   <223> Synthetic DNA/RNA primer, F627
<400> 1130
   auugguugcg gccaucuct 19
<210> 1131
   <211> 20
   <212> RNA
   <213> Artificial Sequence
<220>
   <223> Synthetic Primer, F628
<400> 1131
   accaauuuca uaggcguggc 20
<210> 1132
   <211> 20
   <212> RNA
   <213> Artificial Sequence
<220>
   <223> Synthetic Primer, F629
<400> 1132
   gccuaucgcu cugcucucuc 20
<210> 1133
   <211> 21
   <212> RNA
   <213> Artificial Sequence
<220>
   <223> Synthetic Primer, F630
<400> 1133
   uaacccagcg acgaacuuuc c 21
<210> 1134
   <211> 18
   <212> RNA
   <213> Artificial Sequence
<220>
   <223> Synthetic Primer, F631
<400> 1134
   gccccugagc gucaucug 18
<210> 1135
   <211> 17
   <212> RNA
   <213> Artificial Sequence
<220>
   <223> Synthetic Primer, F632
<400> 1135
   cugguggagg cugacga 17
<210> 1136
   <211> 22
   <212> RNA
   <213> Artificial Sequence
<220>
   <223> Synthetic Primer, F633
<400> 1136
   acaacgugau gaagaucgca ga 22
<210> 1137
   <211> 19
   <212> RNA
   <213> Artificial Sequence
<220>
   <223> Synthetic Primer, F634
<400> 1137
   gggagaucuu cacgcuggg 19
<210> 1138
   <211> 17
   <212> DNA
   <213> Artificial Sequence
<220>
   <223> Synthetic DNA/RNA primer, F635
<400> 1138
   gucugaggag cccgugt 17
<210> 1139
   <211> 18
   <212> RNA
   <213> Artificial Sequence
<220>
   <223> Synthetic Primer, F636
<400> 1139
   uccucggagc agugaggg 18
<210> 1140
   <211> 19
   <212> RNA
   <213> Artificial Sequence
<220>
   <223> Synthetic Primer, F637
<400> 1140
   agccucucca cgcucccuc 19
<210> 1141
   <211> 23
   <212> RNA
   <213> Artificial Sequence
<220>
   <223> Synthetic Primer, F638
<400> 1141
   cucacauugc cccugacaac aua 23
<210> 1142
   <211> 23
   <212> RNA
   <213> Artificial Sequence
<220>
   <223> Synthetic Primer, F639
<400> 1142
   guguccuuuc aggauggugg aug 23
<210> 1143
   <211> 26
   <212> RNA
   <213> Artificial Sequence
<220>
   <223> Synthetic Primer, F640
<400> 1143
   ggugacauuu ucaaagcagu guaucc 26
<210> 1144
   <211> 22
   <212> RNA
   <213> Artificial Sequence
<220>
   <223> Synthetic Primer, F641
<400> 1144
   ggguauucga ugaucccugu gg 22
<210> 1145
   <211> 19
   <212> DNA
   <213> Artificial Sequence
<220>
   <223> Synthetic DNA/RNA primer, F642
<400> 1145
   ccuccccacc agcauguut 19
<210> 1146
   <211> 22
   <212> RNA
   <213> Artificial Sequence
<220>
   <223> Synthetic Primer, F643
<400> 1146
   ggcuuuggug agauccauug ac 22
<210> 1147
   <211> 19
   <212> DNA
   <213> Artificial Sequence
<220>
   <223> Synthetic DNA/RNA primer, F644
<400> 1147
   gcauguacug gucccgcat 19
<210> 1148
   <211> 21
   <212> RNA
   <213> Artificial Sequence
<220>
   <223> Synthetic Primer, F645
<400> 1148
   ugguucugga ucagcuggau g 21
<210> 1149
   <211> 23
   <212> RNA
   <213> Artificial Sequence
<220>
   <223> Synthetic Primer, R646
<400> 1149
   ugccaacaug acuuacuuga ucc 23
<210> 1150
   <211> 22
   <212> DNA
   <213> Artificial Sequence
<220>
   <223> Synthetic DNA/RNA primer, F647
<400> 1150
   ggacuaggcg ugggauguuu ut 22
<210> 1151
   <211> 19
   <212> RNA
   <213> Artificial Sequence
<220>
   <223> Synthetic Primer, F648
<400> 1151
   aguggauccc cucuccacc 19
<210> 1152
   <211> 25
   <212> DNA
   <213> Artificial Sequence
<220>
   <223> Synthetic DNA/RNA primer, F649
<400> 1152
   gagguuuucc agcacucuga cauat 25
<210> 1153
   <211> 24
   <212> RNA
   <213> Artificial Sequence
<220>
   <223> Synthetic Primer, F650
<400> 1153
   cgguugaaug uaaggcuuac aacg 24
<210> 1154
   <211> 20
   <212> RNA
   <213> Artificial Sequence
<220>
   <223> Synthetic Primer, F651
<400> 1154
   gaacgggaag cccucauguc 20
<210> 1155
   <211> 25
   <212> RNA
   <213> Artificial Sequence
<220>
   <223> Synthetic Primer, F652
<400> 1155
   ccuuacucau ggucggauca caaag 25
<210> 1156
   <211> 20
   <212> RNA
   <213> Artificial Sequence
<220>
   <223> Synthetic Primer, F653
<400> 1156
   cccuuucucc ccacagaaac 20
<210> 1157
   <211> 22
   <212> RNA
   <213> Artificial Sequence
<220>
   <223> Synthetic Primer, F654
<400> 1157
   guagagcaaa uccaucccca ca 22
<210> 1158
   <211> 19
   <212> RNA
   <213> Artificial Sequence
<220>
   <223> Synthetic Primer, F655
<400> 1158
   ugugcuuuua gggcccacc 19
<210> 1159
   <211> 29
   <212> DNA
   <213> Artificial Sequence
<220>
   <223> Synthetic DNA/RNA primer, F656
<400> 1159
   ucuguucaau uuuguugagc uucugaaut 29
<210> 1160
   <211> 27
   <212> DNA
   <213> Artificial Sequence
<220>
   <223> Synthetic DNA/RNA primer, F657
<400> 1160
   ucaguguuac uuaccugucu ugucuut 27
<210> 1161
   <211> 24
   <212> RNA
   <213> Artificial Sequence
<220>
   <223> Synthetic Primer, F658
<400> 1161
   ugaauuagcu guaucgucaa ggca 24
<210> 1162
   <211> 25
   <212> RNA
   <213> Artificial Sequence
<220>
   <223> Synthetic Primer, F659
<400> 1162
   uguuucuccc uucucaggau uccua 25
<210> 1163
   <211> 18
   <212> RNA
   <213> Artificial Sequence
<220>
   <223> Synthetic Primer, F660
<400> 1163
   aaacccgcaa uccggaac 18
<210> 1164
   <211> 23
   <212> RNA
   <213> Artificial Sequence
<220>
   <223> Synthetic Primer, F661
<400> 1164
   cccuccaaca uccuagucaa cuc 23
<210> 1165
   <211> 22
   <212> RNA
   <213> Artificial Sequence
<220>
   <223> Synthetic Primer, F662
<400> 1165
   gcuagagcuu gaugagcagc ag 22
<210> 1166
   <211> 20
   <212> RNA
   <213> Artificial Sequence
<220>
   <223> Synthetic Primer, F663
<400> 1166
   ccauggaguc gaugagcugg 20
<210> 1167
   <211> 21
   <212> RNA
   <213> Artificial Sequence
<220>
   <223> Synthetic Primer, F664
<400> 1167
   gcccagcucu gagauccuuu c 21
<210> 1168
   <211> 25
   <212> RNA
   <213> Artificial Sequence
<220>
   <223> Synthetic Primer, F665
<400> 1168
   cauuucugac aacugaacug cucuc 25
<210> 1169
   <211> 26
   <212> RNA
   <213> Artificial Sequence
<220>
   <223> Synthetic Primer, F666
<400> 1169
   uuaccagcuu guucaugucu ggauuc 26
<210> 1170
   <211> 26
   <212> DNA
   <213> Artificial Sequence
<220>
   <223> Synthetic DNA/RNA primer, F667
<400> 1170
   acugagcuug uuggaauaag gaugut 26
<210> 1171
   <211> 22
   <212> RNA
   <213> Artificial Sequence
<220>
   <223> Synthetic Primer, F668
<400> 1171
   uuguaagugc ccgaagugua ag 22
<210> 1172
   <211> 25
   <212> RNA
   <213> Artificial Sequence
<220>
   <223> Synthetic Primer, F669
<400> 1172
   uacgcagugc uaaccaaguu cuuuc 25
<210> 1173
   <211> 24
   <212> RNA
   <213> Artificial Sequence
<220>
   <223> Synthetic Primer, F670
<400> 1173
   cagucaaggu ugcugauuuu gguc 24
<210> 1174
   <211> 26
   <212> RNA
   <213> Artificial Sequence
<220>
   <223> Synthetic Primer, F671
<400> 1174
   uauggauguu gccaagcugu auucug 26
<210> 1175
   <211> 25
   <212> DNA
   <213> Artificial Sequence
<220>
   <223> Synthetic DNA/RNA primer, F672
<400> 1175
   ggugguccua ccauacauga aacat 25
<210> 1176
   <211> 25
   <212> RNA
   <213> Artificial Sequence
<220>
   <223> Synthetic Primer, F673
<400> 1176
   gcaagcaaaa aguuugucca cagag 25
<210> 1177
   <211> 23
   <212> DNA
   <213> Artificial Sequence
<220>
   <223> Synthetic DNA/RNA primer, F674
<400> 1177
   acaucucuca ccucaucugu cct 23
<210> 1178
   <211> 20
   <212> RNA
   <213> Artificial Sequence
<220>
   <223> Synthetic Primer, F675
<400> 1178
   ucccuguagu cccggaugag 20
<210> 1179
   <211> 25
   <212> RNA
   <213> Artificial Sequence
<220>
   <223> Synthetic Primer, F676
<400> 1179
   aauuguugcc auuucagggu uucug 25
<210> 1180
   <211> 26
   <212> RNA
   <213> Artificial Sequence
<220>
   <223> Synthetic Primer, F677
<400> 1180
   cucaccuauc ucccaggccu aaaaua 26
<210> 1181
   <211> 22
   <212> RNA
   <213> Artificial Sequence
<220>
   <223> Synthetic Primer, F678
<400> 1181
   acaaacgaga ugccucuucc ag 22
<210> 1182
   <211> 21
   <212> RNA
   <213> Artificial Sequence
<220>
   <223> Synthetic Primer, F679
<400> 1182
   ggcugucgug guagacuuag a 21
<210> 1183
   <211> 24
   <212> RNA
   <213> Artificial Sequence
<220>
   <223> Synthetic Primer, F680
<400> 1183
   cugaguguau ccuggagguu guug 24
<210> 1184
   <211> 26
   <212> RNA
   <213> Artificial Sequence
<220>
   <223> Synthetic Primer, F681
<400> 1184
   gcuugguucu gauguuugua guguag 26
<210> 1185
   <211> 25
   <212> DNA
   <213> Artificial Sequence
<220>
   <223> Synthetic DNA/RNA primer, F682
<400> 1185
   uccuuguugg uguccauuuu cuugt 25
<210> 1186
   <211> 24
   <212> RNA
   <213> Artificial Sequence
<220>
   <223> Synthetic Primer, F683
<400> 1186
   acaugccauc auucuaggaa gcuc 24
<210> 1187
   <211> 21
   <212> RNA
   <213> Artificial Sequence
<220>
   <223> Synthetic Primer, F684
<400> 1187
   caggacccgc uucucugaaa g 21
<210> 1188
   <211> 24
   <212> RNA
   <213> Artificial Sequence
<220>
   <223> Synthetic Primer, F685
<400> 1188
   aagaccccuu uaacucaaga cugc 24
<210> 1189
   <211> 19
   <212> RNA
   <213> Artificial Sequence
<220>
   <223> Synthetic Primer, F686
<400> 1189
   ugcuccauga ggagacacc 19
<210> 1190
   <211> 29
   <212> DNA
   <213> Artificial Sequence
<220>
   <223> Synthetic DNA/RNA primer, F687
<400> 1190
   aauguaaccu ugcuaaagga gugauuuct 29
<210> 1191
   <211> 29
   <212> RNA
   <213> Artificial Sequence
<220>
   <223> Synthetic Primer, F688
<400> 1191
   aacuggcaaa uauaucauug agccaaauc 29
<210> 1192
   <211> 26
   <212> RNA
   <213> Artificial Sequence
<220>
   <223> Synthetic Primer, F689
<400> 1192
   ggugugaaau gacugaguac aaacug 26
<210> 1193
   <211> 25
   <212> DNA
   <213> Artificial Sequence
<220>
   <223> Synthetic DNA/RNA primer, F690
<400> 1193
   auggugaaac cuguuuguug gacat 25
<210> 1194
   <211> 25
   <212> RNA
   <213> Artificial Sequence
<220>
   <223> Synthetic Primer, F691
<400> 1194
   ccugcucaug gucuuugagu auaug 25
<210> 1195
   <211> 19
   <212> RNA
   <213> Artificial Sequence
<220>
   <223> Synthetic Primer, F692
<400> 1195
   gccacacgca acugucuag 19
<210> 1196
   <211> 25
   <212> RNA
   <213> Artificial Sequence
<220>
   <223> Synthetic Primer, F693
<400> 1196
   gacaauccuu gcuuaccuga ggaac 25
<210> 1197
   <211> 23
   <212> DNA
   <213> Artificial Sequence
<220>
   <223> Synthetic DNA/RNA primer, F694
<400> 1197
   gcucgggauc cauauguggu aat 23
<210> 1198
   <211> 22
   <212> DNA
   <213> Artificial Sequence
<220>
   <223> Synthetic DNA/RNA primer, F695
<400> 1198
   ggcccuauac uuaggcccuu ut 22
<210> 1199
   <211> 17
   <212> DNA
   <213> Artificial Sequence
<220>
   <223> Synthetic DNA/RNA primer, F696
<400> 1199
   aacucacggu ggcugct 17
<210> 1200
   <211> 25
   <212> RNA
   <213> Artificial Sequence
<220>
   <223> Synthetic Primer, F697
<400> 1200
   uguccugguc auuuauagaa accga 25
<210> 1201
   <211> 28
   <212> DNA
   <213> Artificial Sequence
<220>
   <223> Synthetic DNA/RNA primer, F698
<400> 1201
   ucucaugucu gaacugaaga uaaugact 28
<210> 1202
   <211> 24
   <212> DNA
   <213> Artificial Sequence
<220>
   <223> Synthetic DNA/RNA primer, F699
<400> 1202
   uugguagcuc agcuggacug auat 24
<210> 1203
   <211> 24
   <212> RNA
   <213> Artificial Sequence
<220>
   <223> Synthetic Primer, F700
<400> 1203
   augaagcagg cugauacuac acag 24
<210> 1204
   <211> 23
   <212> RNA
   <213> Artificial Sequence
<220>
   <223> Synthetic Primer, F701
<400> 1204
   uugugaagau cugugacuuu ggc 23
<210> 1205
   <211> 28
   <212> RNA
   <213> Artificial Sequence
<220>
   <223> Synthetic Primer, F702
<400> 1205
   ccuuuggguu auaaauagug cacucaga 28
<210> 1206
   <211> 27
   <212> DNA
   <213> Artificial Sequence
<220>
   <223> Synthetic DNA/RNA primer, F703
<400> 1206
   gggaagaaaa guguuuugaa augugut 27
<210> 1207
   <211> 23
   <212> RNA
   <213> Artificial Sequence
<220>
   <223> Synthetic Primer, F704
<400> 1207
   uggcuuugaa ucuuuggcca gua 23
<210> 1208
   <211> 21
   <212> RNA
   <213> Artificial Sequence
<220>
   <223> Synthetic Primer, F705
<400> 1208
   gucgaggcaa uggaaaagcu c 21
<210> 1209
   <211> 25
   <212> RNA
   <213> Artificial Sequence
<220>
   <223> Synthetic Primer, F706
<400> 1209
   agaacagcuc aaagcaauuu cuaca 25
<210> 1210
   <211> 26
   <212> RNA
   <213> Artificial Sequence
<220>
   <223> Synthetic Primer, F707
<400> 1210
   agcaagaggc uuuggaguau uucaug 26
<210> 1211
   <211> 29
   <212> DNA
   <213> Artificial Sequence
<220>
   <223> Synthetic DNA/RNA primer, F708
<400> 1211
   uguucaugcu guguauguaa uagaaugut 29
<210> 1212
   <211> 28
   <212> RNA
   <213> Artificial Sequence
<220>
   <223> Synthetic Primer, F709
<400> 1212
   cuggaaugcc agaacuacaa ucuuuuga 28
<210> 1213
   <211> 28
   <212> DNA
   <213> Artificial Sequence
<220>
   <223> Synthetic DNA/RNA primer, F710
<400> 1213
   cucaagaagc agaaagggaa gaauuuut 28
<210> 1214
   <211> 24
   <212> RNA
   <213> Artificial Sequence
<220>
   <223> Synthetic Primer, F711
<400> 1214
   ugacagccau caucaaagag aucg 24
<210> 1215
   <211> 24
   <212> RNA
   <213> Artificial Sequence
<220>
   <223> Synthetic Primer, F712
<400> 1215
   gggauuuccu gcagaaagac uuga 24
<210> 1216
   <211> 19
   <212> RNA
   <213> Artificial Sequence
<220>
   <223> Synthetic Primer, F713
<400> 1216
   aaggcacaag aggcccuag 19
<210> 1217
   <211> 26
   <212> DNA
   <213> Artificial Sequence
<220>
   <223> Synthetic DNA/RNA primer, F714
<400> 1217
   accaauggcu aagugaagau gacaat 26
<210> 1218
   <211> 26
   <212> RNA
   <213> Artificial Sequence
<220>
   <223> Synthetic Primer, F715
<400> 1218
   agguuaucuu uuuaccacag uugcac 26
<210> 1219
   <211> 25
   <212> RNA
   <213> Artificial Sequence
<220>
   <223> Synthetic Primer, F716
<400> 1219
   uuuucugucc accagggagu aacua 25
<210> 1220
   <211> 26
   <212> RNA
   <213> Artificial Sequence
<220>
   <223> Synthetic Primer, F717
<400> 1220
   gacaaguuca uguacuuuga guuccc 26
<210> 1221
   <211> 24
   <212> RNA
   <213> Artificial Sequence
<220>
   <223> Synthetic Primer, F718
<400> 1221
   agcaaauaaa gacaaagcca accg 24
<210> 1222
   <211> 30
   <212> DNA
   <213> Artificial Sequence
<220>
   <223> Synthetic DNA/RNA primer, F719
<400> 1222
   aguuuaagau gagucauauu uguggguuut 30
<210> 1223
   <211> 24
   <212> RNA
   <213> Artificial Sequence
<220>
   <223> Synthetic Primer, F720
<400> 1223
   cugaccaugu ggacauuagg ugug 24
<210> 1224
   <211> 22
   <212> RNA
   <213> Artificial Sequence
<220>
   <223> Synthetic Primer, F721
<400> 1224
   ccuucccucg ggaaaaacug ac 22
<210> 1225
   <211> 26
   <212> RNA
   <213> Artificial Sequence
<220>
   <223> Synthetic Primer, F722
<400> 1225
   guuugguuuu guaggucuug uggaug 26
<210> 1226
   <211> 21
   <212> RNA
   <213> Artificial Sequence
<220>
   <223> Synthetic Primer, F723
<400> 1226
   gagguggccu gaucuucaca a 21
<210> 1227
   <211> 25
   <212> DNA
   <213> Artificial Sequence
<220>
   <223> Synthetic DNA/RNA primer, F724
<400> 1227
   cgcuuaugca uacucaggau gagut 25
<210> 1228
   <211> 23
   <212> RNA
   <213> Artificial Sequence
<220>
   <223> Synthetic Primer, F725
<400> 1228
   uaagguuccu ucaagcugcc cua 23
<210> 1229
   <211> 25
   <212> DNA
   <213> Artificial Sequence
<220>
   <223> Synthetic DNA/RNA primer, F726
<400> 1229
   caugggagga uguucuuucc cauut 25
<210> 1230
   <211> 25
   <212> RNA
   <213> Artificial Sequence
<220>
   <223> Synthetic Primer, F727
<400> 1230
   uuuucuuccu aagguugcac auagg 25
<210> 1231
   <211> 21
   <212> DNA
   <213> Artificial Sequence
<220>
   <223> Synthetic DNA/RNA primer, F728
<400> 1231
   auuuuuggcu uccuggccuu t 21
<210> 1232
   <211> 21
   <212> RNA
   <213> Artificial Sequence
<220>
   <223> Synthetic Primer, F729
<400> 1232
   ggaaagccuc accugucuac g 21
<210> 1233
   <211> 18
   <212> RNA
   <213> Artificial Sequence
<220>
   <223> Synthetic Primer, F730
<400> 1233
   ucaagaaucg cccgagcc 18
<210> 1234
   <211> 20
   <212> RNA
   <213> Artificial Sequence
<220>
   <223> Synthetic Primer, F731
<400> 1234
   uugguucgga cagacaaccc 20
<210> 1235
   <211> 22
   <212> RNA
   <213> Artificial Sequence
<220>
   <223> Synthetic Primer, F732
<400> 1235
   cucugcacag cuccaaugag ac 22
<210> 1236
   <211> 23
   <212> DNA
   <213> Artificial Sequence
<220>
   <223> Synthetic DNA/RNA primer, F733
<400> 1236
   gcuacaagaa cuaccgauac cgt 23
<210> 1237
   <211> 20
   <212> RNA
   <213> Artificial Sequence
<220>
   <223> Synthetic Primer, F734
<400> 1237
   cucggagagg agccauacug 20
<210> 1238
   <211> 26
   <212> RNA
   <213> Artificial Sequence
<220>
   <223> Synthetic Primer, F735
<400> 1238
   uauaaugaca guuaacccug ccagga 26
<210> 1239
   <211> 22
   <212> RNA
   <213> Artificial Sequence
<220>
   <223> Synthetic Primer, F736
<400> 1239
   aggaagagca cagucacuuu ga 22
<210> 1240
   <211> 22
   <212> RNA
   <213> Artificial Sequence
<220>
   <223> Synthetic Primer, F737
<400> 1240
   caguggagcg aauuccuuug ga 22
<210> 1241
   <211> 19
   <212> RNA
   <213> Artificial Sequence
<220>
   <223> Synthetic Primer, F738
<400> 1241
   uugggucguu gggcauucc 19
<210> 1242
   <211> 21
   <212> RNA
   <213> Artificial Sequence
<220>
   <223> Synthetic Primer, F739
<400> 1242
   caguucacag ugcagcgaaa a 21
<210> 1243
   <211> 28
   <212> RNA
   <213> Artificial Sequence
<220>
   <223> Synthetic Primer, F740
<400> 1243
   aaauaucuac acacaggucu acaagguc 28
<210> 1244
   <211> 22
   <212> RNA
   <213> Artificial Sequence
<220>
   <223> Synthetic Primer, F741
<400> 1244
   cauccgggcu uuacgcaaau aa 22
<210> 1245
   <211> 25
   <212> DNA
   <213> Artificial Sequence
<220>
   <223> Synthetic DNA/RNA primer, F742
<400> 1245
   gccuccuuca ggaauucaau cuuct 25
<210> 1246
   <211> 19
   <212> RNA
   <213> Artificial Sequence
<220>
   <223> Synthetic Primer, F743
<400> 1246
   augaguucug ggcacuggg 19
<210> 1247
   <211> 24
   <212> DNA
   <213> Artificial Sequence
<220>
   <223> Synthetic DNA/RNA primer, F744
<400> 1247
   gaugcaaacu cuugcacaaa ugct 24
<210> 1248
   <211> 21
   <212> RNA
   <213> Artificial Sequence
<220>
   <223> Synthetic Primer, F745
<400> 1248
   gaaccccgag ggcaaauaca g 21
<210> 1249
   <211> 24
   <212> RNA
   <213> Artificial Sequence
<220>
   <223> Synthetic Primer, F746
<400> 1249
   caguucgugg gcuuguuuug uauc 24
<210> 1250
   <211> 22
   <212> RNA
   <213> Artificial Sequence
<220>
   <223> Synthetic Primer, F747
<400> 1250
   uuaaagcugg cuauggcacc 22
<210> 1251
   <211> 20
   <212> RNA
   <213> Artificial Sequence
<220>
   <223> Synthetic Primer, F748
<400> 1251
   caucucucac caucccaagg 20
<210> 1252
   <211> 18
   <212> RNA
   <213> Artificial Sequence
<220>
   <223> Synthetic Primer, F749
<400> 1252
   auacgcagcc uguaccca 18
<210> 1253
   <211> 24
   <212> RNA
   <213> Artificial Sequence
<220>
   <223> Synthetic Primer, F750
<400> 1253
   caccucucuc aagaguuugg augg 24
<210> 1254
   <211> 20
   <212> DNA
   <213> Artificial Sequence
<220>
   <223> Synthetic DNA/RNA primer, F751
<400> 1254
   agauugcgag 20
<210> 1255
   <211> 22
   <212> RNA
   <213> Artificial Sequence
<220>
   <223> Synthetic Primer, F752
<400> 1255
   cugugcugca uuucagagaa cg 22
<210> 1256
   <211> 19
   <212> RNA
   <213> Artificial Sequence
<220>
   <223> Synthetic Primer, F753
<400> 1256
   aagacccaag cugccugac 19
<210> 1257
   <211> 24
   <212> RNA
   <213> Artificial Sequence
<220>
   <223> Synthetic Primer, F754
<400> 1257
   gcuauuuuuc cucacagcuc guuc 24
<210> 1258
   <211> 28
   <212> RNA
   <213> Artificial Sequence
<220>
   <223> Synthetic Primer, F755
<400> 1258
   cuccuuccua gagaguuaga guaacuuc 28
<210> 1259
   <211> 26
   <212> RNA
   <213> Artificial Sequence
<220>
   <223> Synthetic Primer, F756
<400> 1259
   gagccuguuu ugugucuacu guucua 26
<210> 1260
   <211> 19
   <212> DNA
   <213> Artificial Sequence
<220>
   <223> Synthetic DNA/RNA primer, F757
<400> 1260
   cucuugcagc agccagact 19
<210> 1261
   <211> 20
   <212> RNA
   <213> Artificial Sequence
<220>
   <223> Synthetic Primer, F758
<400> 1261
   ccaugggacu gacuuucugc 20
<210> 1262
   <211> 19
   <212> DNA
   <213> Artificial Sequence
<220>
   <223> Synthetic DNA/RNA primer, F759
<400> 1262
   gcugaggacc ugguccuct 19
<210> 1263
   <211> 18
   <212> RNA
   <213> Artificial Sequence
<220>
   <223> Synthetic Primer, F760
<400> 1263
   cugcaccagc agcuccua 18
<210> 1264
   <211> 23
   <212> DNA
   <213> Artificial Sequence
<220>
   <223> Synthetic DNA/RNA primer, F761
<400> 1264
   cccggacgau auugaacaau ggt 23
<210> 1265
   <211> 17
   <212> RNA
   <213> Artificial Sequence
<220>
   <223> Synthetic Primer, F762
<400> 1265
   agccucacca cgagcug 17
<210> 1266
   <211> 23
   <212> RNA
   <213> Artificial Sequence
<220>
   <223> Synthetic Primer, F763
<400> 1266
   caccuuuccu ugccucuuuc cua 23
<210> 1267
   <211> 18
   <212> DNA
   <213> Artificial Sequence
<220>
   <223> Synthetic DNA/RNA primer, F764
<400> 1267
   cucaaggaug cccaggct 18
<210> 1268
   <211> 21
   <212> RNA
   <213> Artificial Sequence
<220>
   <223> Synthetic Primer, F765
<400> 1268
   ccucccugcu ucugucuccu a 21
<210> 1269
   <211> 20
   <212> RNA
   <213> Artificial Sequence
<220>
   <223> Synthetic Primer, F766
<400> 1269
   ccaguugcaa accagaccuc 20
<210> 1270
   <211> 22
   <212> RNA
   <213> Artificial Sequence
<220>
   <223> Synthetic Primer, F767
<400> 1270
   acuccacacg caaauuuccu uc 22
<210> 1271
   <211> 17
   <212> RNA
   <213> Artificial Sequence
<220>
   <223> Synthetic Primer, F768
<400> 1271
   aggguggcaa guggcuc 17
<210> 1272
   <211> 18
   <212> RNA
   <213> Artificial Sequence
<220>
   <223> Synthetic Primer, F769
<400> 1272
   gaggcucccc uuucuugc 18
<210> 1273
   <211> 21
   <212> RNA
   <213> Artificial Sequence
<220>
   <223> Synthetic Primer, F770
<400> 1273
   cugugacugc uuguagaugg c 21
<210> 1274
   <211> 18
   <212> RNA
   <213> Artificial Sequence
<220>
   <223> Synthetic Primer, F771
<400> 1274
   cugucgucuc uccagccc 18
<210> 1275
   <211> 23
   <212> RNA
   <213> Artificial Sequence
<220>
   <223> Synthetic Primer, R498
<400> 1275
   ugccaucauu cuugaggagg aag 23
<210> 1276
   <211> 19
   <212> DNA
   <213> Artificial Sequence
<220>
   <223> Synthetic DNA/RNA primer, R499
<400> 1276
   ugcaaucccu gccccggut 19
<210> 1277
   <211> 21
   <212> DNA
   <213> Artificial Sequence
<220>
   <223> Synthetic DNA/RNA primer, R500
<400> 1277
   ggauugcagg cucaccccaa t 21
<210> 1278
   <211> 21
   <212> RNA
   <213> Artificial Sequence
<220>
   <223> Synthetic Primer, R501
<400> 1278
   cuggauuucc ucauggaagc c 21
<210> 1279
   <211> 21
   <212> RNA
   <213> Artificial Sequence
<220>
   <223> Synthetic Primer, R502
<400> 1279
   aaauccaguu cguccuguuc a 21
<210> 1280
   <211> 22
   <212> RNA
   <213> Artificial Sequence
<220>
   <223> Synthetic Primer, R503
<400> 1280
   gaaacugccu cuugaccugu cc 22
<210> 1281
   <211> 28
   <212> DNA
   <213> Artificial Sequence
<220>
   <223> Synthetic DNA/RNA primer, R504
<400> 1281
   aggacaguca uguugccagu auuaaaat 28
<210> 1282
   <211> 23
   <212> RNA
   <213> Artificial Sequence
<220>
   <223> Synthetic Primer, R505
<400> 1282
   cuuccaugac uuuggcaauc ugg 23
<210> 1283
   <211> 29
   <212> DNA
   <213> Artificial Sequence
<220>
   <223> Synthetic DNA/RNA primer, R506
<400> 1283
   gaacaugucc uauuugaauu uuccgacut 29
<210> 1284
   <211> 27
   <212> DNA
   <213> Artificial Sequence
<220>
   <223> Synthetic DNA/RNA primer, R507
<400> 1284
   gacacaaaga cuggcuuaca uuuugat 27
<210> 1285
   <211> 22
   <212> DNA
   <213> Artificial Sequence
<220>
   <223> Synthetic DNA/RNA primer, R508
<400> 1285
   aggcugacca cuucuacucu gt 22
<210> 1286
   <211> 21
   <212> RNA
   <213> Artificial Sequence
<220>
   <223> Synthetic Primer, R509
<400> 1286
   gcacucaggc uggaugaaca a 21
<210> 1287
   <211> 23
   <212> RNA
   <213> Artificial Sequence
<220>
   <223> Synthetic Primer, R510
<400> 1287
   uguccagggc uaucuggaag auc 23
<210> 1288
   <211> 20
   <212> RNA
   <213> Artificial Sequence
<220>
   <223> Synthetic Primer, R511
<400> 1288
   gcagcauuua cugcagcuug 20
<210> 1289
   <211> 27
   <212> RNA
   <213> Artificial Sequence
<220>
   <223> Synthetic Primer, R512
<400> 1289
   ugacagaagu acaucugcua aacauga 27
<210> 1290
   <211> 22
   <212> RNA
   <213> Artificial Sequence
<220>
   <223> Synthetic Primer, R513
<400> 1290
   cucacaggau cuucagcuga cc 22
<210> 1291
   <211> 22
   <212> DNA
   <213> Artificial Sequence
<220>
   <223> Synthetic DNA/RNA primer, R514
<400> 1291
   acuuuguugg cauggcagaa at 22
<210> 1292
   <211> 17
   <212> DNA
   <213> Artificial Sequence
<220>
   <223> Synthetic DNA/RNA primer, R515
<400> 1292
   gccguggugc ugaccat 17
<210> 1293
   <211> 24
   <212> RNA
   <213> Artificial Sequence
<220>
   <223> Synthetic Primer, R516
<400> 1293
   gugaugauug ggagauuccu gaug 24
<210> 1294
   <211> 29
   <212> DNA
   <213> Artificial Sequence
<220>
   <223> Synthetic DNA/RNA primer, R517
<400> 1294
   gcucugauag gaaaaugaga ucuacugut 29
<210> 1295
   <211> 18
   <212> DNA
   <213> Artificial Sequence
<220>
   <223> Synthetic DNA/RNA primer, R518
<400> 1295
   cuccagcagg gcuucgat 18
<210> 1296
   <211> 25
   <212> RNA
   <213> Artificial Sequence
<220>
   <223> Synthetic Primer, R519
<400> 1296
   ggacuuugca acuucaacaa aacuc 25
<210> 1297
   <211> 21
   <212> RNA
   <213> Artificial Sequence
<220>
   <223> Synthetic Primer, R520
<400> 1297
   cuaggugucu cccccuguaa g 21
<210> 1298
   <211> 18
   <212> DNA
   <213> Artificial Sequence
<220>
   <223> Synthetic DNA/RNA primer, R521
<400> 1298
   agguucaggc cuugcact 18
<210> 1299
   <211> 20
   <212> DNA
   <213> Artificial Sequence
<220>
   <223> Synthetic Primer, R522
<400> 1299
   ccagcccagg aagcaaagag 20
<210> 1300
   <211> 22
   <212> RNA
   <213> Artificial Sequence
<220>
   <223> Synthetic Primer, R523
<400> 1300
   uuaaaacugg ucucgcucuc cc 22
<210> 1301
   <211> 20
   <212> RNA
   <213> Artificial Sequence
<220>
   <223> Synthetic Primer, R524
<400> 1301
   gaaagcggga aucgcagaaa 20
<210> 1302
   <211> 22
   <212> DNA
   <213> Artificial Sequence
<220>
   <223> Synthetic DNA/RNA primer, R525
<400> 1302
   ggaagaccuc uucuucgcac ut 22
<210> 1303
   <211> 21
   <212> RNA
   <213> Artificial Sequence
<220>
   <223> Synthetic Primer, R526
<400> 1303
   caaaagagcu cccccaucuc c 21
<210> 1304
   <211> 25
   <212> RNA
   <213> Artificial Sequence
<220>
   <223> Synthetic Primer, R527
<400> 1304
   agaagagaca ucuggacuua gccaa 25
<210> 1305
   <211> 21
   <212> RNA
   <213> Artificial Sequence
<220>
   <223> Synthetic Primer, R528
<400> 1305
   aucaucgacg guggguacau g 21
<210> 1306
   <211> 20
   <212> RNA
   <213> Artificial Sequence
<220>
   <223> Synthetic Primer, R529
<400> 1306
   cguugguccu gacgguacug 20
<210> 1307
   <211> 25
   <212> RNA
   <213> Artificial Sequence

<220>
   <223> Synthetic Primer, R530
<400> 1307
   ccauucugag gacugcuggu uuaua 25
<210> 1308
   <211> 23
   <212> RNA
   <213> Artificial Sequence
<220>
   <223> Synthetic Primer, R531
<400> 1308
   ccaggaccau cauccuacug uaa 23
<210> 1309
   <211> 22
   <212> RNA
   <213> Artificial Sequence
<220>
   <223> Synthetic Primer, R532
<400> 1309
   gaguguuugc uccucacucu uc 22
<210> 1310
   <211> 25
   <212> DNA
   <213> Artificial Sequence
<220>
   <223> Synthetic DNA/RNA primer, R533
<400> 1310
   cuucagguuu ccuucucuca uggut 25
<210> 1311
   <211> 20
   <212> RNA
   <213> Artificial Sequence
<220>
   <223> Synthetic Primer, R534
<400> 1311
   ggaugagcuc acagagcugc 20
<210> 1312
   <211> 23
   <212> DNA
   <213> Artificial Sequence
<220>
   <223> Synthetic DNA/RNA primer, R535
<400> 1312
   ucaaaaagca ugcccagacc uut 23
<210> 1313
   <211> 23
   <212> RNA
   <213> Artificial Sequence
<220>
   <223> Synthetic Primer, R536
<400> 1313
   acuuugagac uucugcuuug cuc 23
<210> 1314
   <211> 24
   <212> RNA
   <213> Artificial Sequence
<220>
   <223> Synthetic Primer, R537
<400> 1314
   aucgaaaaac ugugcaucua cacc 24
<210> 1315
   <211> 18
   <212> DNA
   <213> Artificial Sequence
<220>
   <223> Synthetic DNA/RNA primer, R538
<400> 1315
   agacccagca gugacugt 18
<210> 1316
   <211> 24
   <212> DNA
   <213> Artificial Sequence
<220>
   <223> Synthetic DNA/RNA primer, R539
<400> 1316
   acaaguauaa ugagcacccc uuct 24
<210> 1317
   <211> 23
   <212> RNA
   <213> Artificial Sequence
<220>
   <223> Synthetic Primer, R540
<400> 1317
   guggacacac cuguauuccu gag 23
<210> 1318
   <211> 20
   <212> RNA
   <213> Artificial Sequence
<220>
   <223> Synthetic Primer, R541
<400> 1318
   ccgaccaguu gggcaaaauc 20
<210> 1319
   <211> 25
   <212> DNA
   <213> Artificial Sequence
<220>
   <223> Synthetic DNA/RNA primer, R542
<400> 1319
   gcagaagucu guuuucuuca uggut 25
<210> 1320
   <211> 20
   <212> RNA
   <213> Artificial Sequence
<220>
   <223> Synthetic Primer, R543
<400> 1320
   aggccauguu ggguuaaagg 20
<210> 1321
   <211> 24
   <212> RNA
   <213> Artificial Sequence
<220>
   <223> Synthetic Primer, R544
<400> 1321
   agaaucuaca gcuaccagau ggca 24
<210> 1322
   <211> 24
   <212> RNA
   <213> Artificial Sequence
<220>
   <223> Synthetic Primer, R545
<400> 1322
   ccuaguuucc agugcaucug uacc 24
<210> 1323
   <211> 24
   <212> RNA
   <213> Artificial Sequence
<220>
   <223> Synthetic Primer, R546
<400> 1323
   gguccccauc cauucuuccu auuc 24
<210> 1324
   <211> 24
   <212> RNA
   <213> Artificial Sequence
<220>
   <223> Synthetic Primer, R547
<400> 1324
   uguggagugu uggcuguauc uuug 24
<210> 1325
   <211> 25
   <212> RNA
   <213> Artificial Sequence
<220>
   <223> Synthetic Primer, R548
<400> 1325
   cucuguaagc gacuuuuggu gauag 25
<210> 1326
   <211> 25
   <212> RNA
   <213> Artificial Sequence
<220>
   <223> Synthetic Primer, R549
<400> 1326
   gcccaaccaa uugagaaguu uguaa 25
<210> 1327
   <211> 24
   <212> RNA
   <213> Artificial Sequence
<220>
   <223> Synthetic Primer, R550
<400> 1327
   uuagucagga gucuaagcca acag 24
<210> 1328
   <211> 23
   <212> RNA
   <213> Artificial Sequence
<220>
   <223> Synthetic Primer, R551
<400> 1328
   cuugcccgca ucuauaguuu cca 23
<210> 1329
   <211> 28
   <212> DNA
   <213> Artificial Sequence
<220>
   <223> Synthetic DNA/RNA primer, R552
<400> 1329
   aacuuccguu uugaguguuu acugauut 28
<210> 1330
   <211> 25
   <212> DNA
   <213> Artificial Sequence
<220>
   <223> Synthetic DNA/RNA primer, R553
<400> 1330
   uuacuuggau aaaguuccag agcct 25
<210> 1331
   <211> 25
   <212> DNA
   <213> Artificial Sequence
<220>
   <223> Synthetic DNA/RNA primer, R554
<400> 1331
   gcuuauugcc accuacuuaa ccuct 25
<210> 1332
   <211> 19
   <212> RNA
   <213> Artificial Sequence
<220>
   <223> Synthetic Primer, R555
<400> 1332
   cuucagcccu gcagggaaa 19
<210> 1333
   <211> 28
   <212> RNA
   <213> Artificial Sequence
<220>
   <223> Synthetic Primer, R556
<400> 1333
   ggcaaguuca acauuauucc cuuuugua 28
<210> 1334
   <211> 23
   <212> RNA
   <213> Artificial Sequence
<220>
   <223> Synthetic Primer, R557
<400> 1334
   ucuuccucag gauugccuuu acc 23
<210> 1335
   <211> 26
   <212> RNA
   <213> Artificial Sequence
<220>
   <223> Synthetic Primer, R558
<400> 1335
   cauacagaga gggucaucag ugauac 26
<210> 1336
   <211> 23
   <212> RNA
   <213> Artificial Sequence
<220>
   <223> Synthetic Primer, R559
<400> 1336
   gaaagucucc cacaaaguaa ccc 23
<210> 1337
   <211> 20
   <212> RNA
   <213> Artificial Sequence
<220>
   <223> Synthetic Primer, R560
<400> 1337
   auagucauag ccgggccaca 20
<210> 1338
   <211> 26
   <212> RNA
   <213> Artificial Sequence
<220>
   <223> Synthetic Primer, R561
<400> 1338
   ccaguuuauu guauuugcau agcaca 26
<210> 1339
   <211> 26
   <212> RNA
   <213> Artificial Sequence
<220>
   <223> Synthetic Primer, R562
<400> 1339
   ggacccauua gaaccaacuc cauaaa 26
<210> 1340
   <211> 21
   <212> RNA
   <213> Artificial Sequence
<220>
   <223> Synthetic Primer, R563
<400> 1340
   uaccuuauac accgugccga a 21
<210> 1341
   <211> 22
   <212> RNA
   <213> Artificial Sequence
<220>
   <223> Synthetic Primer, R564
<400> 1341
   ccacacagca aagcagaaac uc 22
<210> 1342
   <211> 20
   <212> RNA
   <213> Artificial Sequence
<220>
   <223> Synthetic Primer, R565
<400> 1342
   uucuuucucu uccgcaccca 20
<210> 1343
   <211> 19
   <212> RNA
   <213> Artificial Sequence
<220>
   <223> Synthetic Primer, R566
<400> 1343
   gugaggcaga ugcccagca 19
<210> 1344
   <211> 27
   <212> RNA
   <213> Artificial Sequence
<220>
   <223> Synthetic Primer, R567
<400> 1344
   ccaauauugu cuuuguguuc ccggaca 27
<210> 1345
   <211> 20
   <212> RNA
   <213> Artificial Sequence
<220>
   <223> Synthetic Primer, R568
<400> 1345
   uguguuccuu uggagguggc 20
<210> 1346
   <211> 24
   <212> RNA
   <213> Artificial Sequence
<220>
   <223> Synthetic Primer, R569
<400> 1346
   gauccagagg aggaguaugu guga 24
<210> 1347
   <211> 24
   <212> RNA
   <213> Artificial Sequence
<220>
   <223> Synthetic Primer, R570
<400> 1347
   ucuuccucca ucucauagcu gucg 24
<210> 1348
   <211> 20
   <212> RNA
   <213> Artificial Sequence
<220>
   <223> Synthetic Primer, R571
<400> 1348
   cguccuguuu ucaggccaag 20
<210> 1349
   <211> 24
   <212> RNA
   <213> Artificial Sequence
<220>
   <223> Synthetic Primer, R572
<400> 1349
   auuagaggga cucuucccaa ugga 24
<210> 1350
   <211> 20
   <212> RNA
   <213> Artificial Sequence
<220>
   <223> Synthetic Primer, R573
<400> 1350
   ccacggugga auuguugcug 20
<210> 1351
   <211> 27
   <212> RNA
   <213> Artificial Sequence
<220>
   <223> Synthetic Primer, R574
<400> 1351
   auaccaggcu aguauagaug cuuaggg 27
<210> 1352
   <211> 21
   <212> RNA
   <213> Artificial Sequence
<220>
   <223> Synthetic Primer, R575
<400> 1352
   cagacaccaa cucccggaau c 21
<210> 1353
   <211> 20
   <212> RNA
   <213> Artificial Sequence
<220>
   <223> Synthetic Primer, R576
<400> 1353
   cagaacucuc uccccagcag 20
<210> 1354
   <211> 19
   <212> RNA
   <213> Artificial Sequence
<220>
   <223> Synthetic Primer, R577
<400> 1354
   cagcuucaug ucugugccg 19
<210> 1355
   <211> 20
   <212> RNA
   <213> Artificial Sequence
<220>
   <223> Synthetic Primer, R578
<400> 1355
   ucacaccgcu guguuccauc 20
<210> 1356
   <211> 21
   <212> RNA
   <213> Artificial Sequence
<220>
   <223> Synthetic Primer, R579
<400> 1356
   guugugagcg augagcacgu a 21
<210> 1357
   <211> 23
   <212> RNA
   <213> Artificial Sequence
<220>
   <223> Synthetic Primer, R580
<400> 1357
   aaaaucgugu ccugguagca gag 23
<210> 1358
   <211> 25
   <212> DNA
   <213> Artificial Sequence
<220>
   <223> Synthetic DNA/RNA primer, R581
<400> 1358
   cccaccaaaa ugagaaaacu gugut 25
<210> 1359
   <211> 20
   <212> RNA
   <213> Artificial Sequence
<220>
   <223> Synthetic Primer, R582
<400> 1359
   uguccuccua gcaggagagg 20
<210> 1360
   <211> 20
   <212> RNA
   <213> Artificial Sequence
<220>
   <223> Synthetic Primer, R583
<400> 1360
   ccguggaugu caggcagaug 20
<210> 1361
   <211> 24
   <212> RNA
   <213> Artificial Sequence
<220>
   <223> Synthetic Primer, R584
<400> 1361
   auacuggacu caucucuccu uccc 24
<210> 1362
   <211> 19
   <212> DNA
   <213> Artificial Sequence
<220>
   <223> Synthetic Primer, R585
<400> 1362
   aaagaccacc cccaagacc 19
<210> 1363
   <211> 23
   <212> DNA
   <213> Artificial Sequence
<220>
   <223> Synthetic DNA/RNA primer, R586
<400> 1363
   auaacuccac acaucacucu ggt 23
<210> 1364
   <211> 23
   <212> RNA
   <213> Artificial Sequence
<220>
   <223> Synthetic Primer, R587
<400> 1364
   uugacauggu ugggacucuu gac 23
<210> 1365
   <211> 22
   <212> RNA
   <213> Artificial Sequence
<220>
   <223> Synthetic Primer, R588
<400> 1365
   uggcaaacuu cccaucguag ac 22
<210> 1366
   <211> 24
   <212> RNA
   <213> Artificial Sequence
<220>
   <223> Synthetic Primer, R589
<400> 1366
   guugaucauu guuccuuccc cuca 24
<210> 1367
   <211> 21
   <212> RNA
   <213> Artificial Sequence
<220>
   <223> Synthetic Primer, R590
<400> 1367
   ccaucuuguc aggaggacag g 21
<210> 1368
   <211> 23
   <212> RNA
   <213> Artificial Sequence
<220>
   <223> Synthetic Primer, R591
<400> 1368
   ggcaggaucu cuaacccauu gag 23
<210> 1369
   <211> 22
   <212> RNA
   <213> Artificial Sequence
<220>
   <223> Synthetic Primer, R592
<400> 1369
   cucagcaggu aacucacacu ug 22
<210> 1370
   <211> 18
   <212> DNA
   <213> Artificial Sequence
<220>
   <223> Synthetic DNA/RNA primer, R593
<400> 1370
   cuucccuggg ugcuccat 18
<210> 1371
   <211> 22
   <212> RNA
   <213> Artificial Sequence
<220>
   <223> Synthetic Primer, R594
<400> 1371
   guggauaugg uccuucucuu cc 22
<210> 1372
   <211> 19
   <212> RNA
   <213> Artificial Sequence
<220>
   <223> Synthetic Primer, R595
<400> 1372
   ggcuaguggg cgcauguag 19
<210> 1373
   <211> 20
   <212> RNA
   <213> Artificial Sequence
<220>
   <223> Synthetic Primer, R596
<400> 1373
   aucaaagucc agcaccagca 20
<210> 1374
   <211> 24
   <212> RNA
   <213> Artificial Sequence
<220>
   <223> Synthetic Primer, R597
<400> 1374
   gggaauugca uucacacguu aaca 24
<210> 1375
   <211> 29
   <212> RNA
   <213> Artificial Sequence
<220>
   <223> Synthetic Primer, R598
<400> 1375
   uuuguuuugu uuuucuguuu cucccucug 29
<210> 1376
   <211> 24
   <212> RNA
   <213> Artificial Sequence
<220>
   <223> Synthetic Primer, R599
<400> 1376
   gaggguuguu aguggagcau auga 24
<210> 1377
   <211> 23
   <212> RNA
   <213> Artificial Sequence
<220>
   <223> Synthetic Primer, R600
<400> 1377
   ugagacaggc caguguuuac aug 23
<210> 1378
   <211> 26
   <212> DNA
   <213> Artificial Sequence
<220>
   <223> Synthetic DNA/RNA primer, R601
<400> 1378
   gagacuggag aauguauaca caccut 26
<210> 1379
   <211> 18
   <212> DNA
   <213> Artificial Sequence
<220>
   <223> Synthetic DNA/RNA primer, R602
<400> 1379
   cgacaucucc ucgggcut 18
<210> 1380
   <211> 18
   <212> RNA
   <213> Artificial Sequence
<220>
   <223> Synthetic Primer, R603
<400> 1380
   cguagagcuc cggguguc 18
<210> 1381
   <211> 20
   <212> DNA
   <213> Artificial Sequence
<220>
   <223> Synthetic DNA/RNA primer, R604
<400> 1381
   cuacccaggg ccacuguuut 20
<210> 1382
   <211> 22
   <212> RNA
   <213> Artificial Sequence
<220>
   <223> Synthetic Primer, R605
<400> 1382
   gggacauuca ccacaucgac ua 22
<210> 1383
   <211> 18
   <212> RNA
   <213> Artificial Sequence
<220>
   <223> Synthetic Primer, R606
<400> 1383
   uggccucuuc uccugugc 18
<210> 1384
   <211> 25
   <212> RNA
   <213> Artificial Sequence
<220>
   <223> Synthetic Primer, R607
<400> 1384
   cuucuucuuc ccauagaugc ucucc 25
<210> 1385
   <211> 24
   <212> RNA
   <213> Artificial Sequence
<220>
   <223> Synthetic Primer, R608
<400> 1385
   gaggcauuau uugaccggau cuac 24
<210> 1386
   <211> 23
   <212> RNA
   <213> Artificial Sequence
<220>
   <223> Synthetic Primer, R609
<400> 1386
   cugaguauga gcuucccgaa gac 23
<210> 1387
   <211> 19
   <212> RNA
   <213> Artificial Sequence
<220>
   <223> Synthetic Primer, R610
<400> 1387
   ccugcuaaca cccuguucg 19
<210> 1388
   <211> 21
   <212> DNA
   <213> Artificial Sequence
<220>
   <223> Synthetic DNA/RNA primer, R611
<400> 1388
   cugccugucu cucuuggcuu t 21
<210> 1389
   <211> 26
   <212> RNA
   <213> Artificial Sequence
<220>
   <223> Synthetic Primer, R612
<400> 1389
   uaugaacuuc cagaggaccc aaaaug 26
<210> 1390
   <211> 24
   <212> RNA
   <213> Artificial Sequence
<220>
   <223> Synthetic Primer, R613
<400> 1390
   ggaaaagaac ggcaguaaau acgg 24
<210> 1391
   <211> 23
   <212> RNA
   <213> Artificial Sequence
<220>
   <223> Synthetic Primer, R614
<400> 1391
   aauacggguc caucaaucac acg 23
<210> 1392
   <211> 25
   <212> RNA
   <213> Artificial Sequence
<220>
   <223> Synthetic Primer, R615
<400> 1392
   caguacuugg uauucugugc uagga 25
<210> 1393
   <211> 25
   <212> RNA
   <213> Artificial Sequence
<220>
   <223> Synthetic Primer, R616
<400> 1393
   ggaagcuguc caucaguaua cauuc 25
<210> 1394
   <211> 23
   <212> RNA
   <213> Artificial Sequence
<220>
   <223> Synthetic Primer, R617
<400> 1394
   ggcccuccuu caguuuaguu gag 23
<210> 1395
   <211> 21
   <212> RNA
   <213> Artificial Sequence
<220>
   <223> Synthetic Primer, R618
<400> 1395
   gguggaggcg auaguggaua g 21
<210> 1396
   <211> 26
   <212> RNA
   <213> Artificial Sequence
<220>
   <223> Synthetic Primer, R619
<400> 1396
   agauggagau gaugaagaug auuggg 26
<210> 1397
   <211> 22
   <212> RNA
   <213> Artificial Sequence
<220>
   <223> Synthetic Primer, R620
<400> 1397
   gucaagugga uggcuccaga ag 22
<210> 1398
   <211> 28
   <212> RNA
   <213> Artificial Sequence
<220>
   <223> Synthetic Primer, R621
<400> 1398
   ccagaaaugu uuugguaaca gaaaacaa 28
<210> 1399
   <211> 26
   <212> RNA
   <213> Artificial Sequence
<220>
   <223> Synthetic Primer, R622
<400> 1399
   auucucucuu uagggagcuu cucuuc 26
<210> 1400
   <211> 27
   <212> RNA
   <213> Artificial Sequence
<220>
   <223> Synthetic Primer, R623
<400> 1400
   uggaagagaa aaggagauua cagcuuc 27
<210> 1401
   <211> 23
   <212> RNA
   <213> Artificial Sequence
<220>
   <223> Synthetic Primer, R624
<400> 1401
   auuggucucu cauucuccca ucc 23
<210> 1402
   <211> 23
   <212> DNA
   <213> Artificial Sequence
<220>
   <223> Synthetic DNA/RNA primer, R625
<400> 1402
   guuuagguuu uggcaacgug gat 23
<210> 1403
   <211> 24
   <212> RNA
   <213> Artificial Sequence
<220>
   <223> Synthetic Primer, R626
<400> 1403
   ucaccagaug cuaugugcua aucc 24
<210> 1404
   <211> 19
   <212> RNA
   <213> Artificial Sequence
<220>
   <223> Synthetic Primer, R627
<400> 1404
   uccuaccugu guccacacc 19
<210> 1405
   <211> 19
   <212> DNA
   <213> Artificial Sequence
<220>
   <223> Synthetic DNA/RNA primer, R628
<400> 1405
   ggcaugggac agagucgut 19
<210> 1406
   <211> 22
   <212> RNA
   <213> Artificial Sequence
<220>
   <223> Synthetic Primer, R629
<400> 1406
   uugugcaagg agagaaccuc ua 22
<210> 1407
   <211> 25
   <212> RNA
   <213> Artificial Sequence
<220>
   <223> Synthetic Primer, R630
<400> 1407
   ccuaucccag aacuggagac agaaa 25
<210> 1408
   <211> 21
   <212> DNA
   <213> Artificial Sequence
<220>
   <223> Synthetic DNA/RNA primer, R631
<400> 1408
   uguacaccuu gcaguggaac t 21
<210> 1409
   <211> 18
   <212> RNA
   <213> Artificial Sequence
<220>
   <223> Synthetic Primer, R632
<400> 1409
   agcccaggcc uuucuugg 18
<210> 1410
   <211> 17
   <212> RNA
   <213> Artificial Sequence
<220>
   <223> Synthetic Primer, R633
<400> 1410
   acuggcauga cccccac 17
<210> 1411
   <211> 17
   <212> DNA
   <213> Artificial Sequence
<220>
   <223> Synthetic DNA/RNA primer, R634
<400> 1411
   ugccacucac aggucgt 17
<210> 1412
   <211> 18
   <212> DNA
   <213> Artificial Sequence
<220>
   <223> Synthetic DNA/RNA primer, R635
<400> 1412
   gcagaaacuc ccgcaggt 18
<210> 1413
   <211> 20
   <212> DNA
   <213> Artificial Sequence
<220>
   <223> Synthetic DNA/RNA primer, R636
<400> 1413
   acuccagaua cugcaugcct 20
<210> 1414
   <211> 17
   <212> RNA
   <213> Artificial Sequence
<220>
   <223> Synthetic Primer, R637
<400> 1414
   acucccgcag guuuccc 17
<210> 1415
   <211> 24
   <212> RNA
   <213> Artificial Sequence
<220>
   <223> Synthetic Primer, R638
<400> 1415
   acgggaaagu ggugaagaua ugug 24
<210> 1416
   <211> 26
   <212> RNA
   <213> Artificial Sequence
<220>
   <223> Synthetic Primer, R639
<400> 1416
   agaaacauga uggaugucac guucuc 26
<210> 1417
   <211> 24
   <212> DNA
   <213> Artificial Sequence
<220>
   <223> Synthetic DNA/RNA primer, R640
<400> 1417
   uguuaaccuu gcagaauggu cgat 24
<210> 1418
   <211> 19
   <212> RNA
   <213> Artificial Sequence
<220>
   <223> Synthetic Primer, R641
<400> 1418
   augacuugga ccgcguagc 19
<210> 1419
   <211> 21
   <212> DNA
   <213> Artificial Sequence
<220>
   <223> Synthetic DNA/RNA primer, R642
<400> 1419
   gcauccuacc guugaagcac t 21
<210> 1420
   <211> 25
   <212> DNA
   <213> Artificial Sequence
<220>
   <223> Synthetic DNA/RNA primer, R643
<400> 1420
   caccuggaac uuggucucaa agaut 25
<210> 1421
   <211> 19
   <212> DNA
   <213> Artificial Sequence
<220>
   <223> Synthetic DNA/RNA primer, R644
<400> 1421
   auuccuaccg gaagcaggt 19
<210> 1422
   <211> 23
   <212> DNA
   <213> Artificial Sequence
<220>
   <223> Synthetic DNA/RNA primer, R645
<400> 1422
   augacggaau auaagcuggu ggt 23
<210> 1423
   <211> 22
   <212> RNA
   <213> Artificial Sequence
<220>
   <223> Synthetic Primer, R646
<400> 1423
   aaaauauccc ccggcuugug ag 22
<210> 1424
   <211> 26
   <212> RNA
   <213> Artificial Sequence
<220>
   <223> Synthetic Primer, R647
<400> 1424
   gaagaagaug uggaaaaguc ccaaug 26
<210> 1425
   <211> 17
   <212> RNA
   <213> Artificial Sequence
<220>
   <223> Synthetic Primer, R648
<400> 1425
   gucccuggcu ggaccaa 17
<210> 1426
   <211> 22
   <212> RNA
   <213> Artificial Sequence
<220>
   <223> Synthetic Primer, R649
<400> 1426
   cacacauugg agcaugccau uc 22
<210> 1427
   <211> 27
   <212> DNA
   <213> Artificial Sequence
<220>
   <223> Synthetic DNA/RNA primer, R650
<400> 1427
   agccuaaaca uccccuuaaa uuggaut 27
<210> 1428
   <211> 20
   <212> RNA
   <213> Artificial Sequence
<220>
   <223> Synthetic Primer, R651
<400> 1428
   cggcuuuacc uccaauggug 20
<210> 1429
   <211> 24
   <212> RNA
   <213> Artificial Sequence
<220>
   <223> Synthetic Primer, R652
<400> 1429
   gcagagaaug gguacucacg uuuc 24
<210> 1430
   <211> 21
   <212> DNA
   <213> Artificial Sequence
<220>
   <223> Synthetic DNA/RNA primer, R653
<400> 1430
   ucagccuguu ucugggaaac t 21
<210> 1431
   <211> 29
   <212> RNA
   <213> Artificial Sequence
<220>
   <223> Synthetic Primer, R654
<400> 1431
   uggagagaga acaaauaaau gguuaccug 29
<210> 1432
   <211> 27
   <212> DNA
   <213> Artificial Sequence
<220>
   <223> Synthetic DNA/RNA primer, R655
<400> 1432
   gauucuuaua aagugcagcu ucugcat 27
<210> 1433
   <211> 24
   <212> DNA
   <213> Artificial Sequence
<220>
   <223> Synthetic DNA/RNA primer, R656
<400> 1433
   cagacgucac uuucaaacgu guat 24
<210> 1434
   <211> 22
   <212> RNA
   <213> Artificial Sequence
<220>
   <223> Synthetic Primer, R657
<400> 1434
   caggcucagg acuuagcaag aa 22
<210> 1435
   <211> 24
   <212> RNA
   <213> Artificial Sequence
<220>
   <223> Synthetic Primer, R658
<400> 1435
   uaaggccugc ugaaaaugac ugaa 24
<210> 1436
   <211> 21
   <212> DNA
   <213> Artificial Sequence
<220>
   <223> Synthetic DNA/RNA primer, R659
<400> 1436
   aguccucaug uacugguccc t 21
<210> 1437
   <211> 20
   <212> RNA
   <213> Artificial Sequence
<220>
   <223> Synthetic Primer, R660
<400> 1437
   cugaucucgc caucgcugua 20
<210> 1438
   <211> 24
   <212> DNA
   <213> Artificial Sequence
<220>
   <223> Synthetic DNA/RNA primer, R661
<400> 1438
   guucauaccg acauguagga ccut 24
<210> 1439
   <211> 23
   <212> RNA
   <213> Artificial Sequence
<220>
   <223> Synthetic Primer, R662
<400> 1439
   ucaaagucgu cauccuucag uuc 23
<210> 1440
   <211> 19
   <212> DNA
   <213> Artificial Sequence
<220>
   <223> Synthetic DNA/RNA primer, R663
<400> 1440
   ccuccagaug ugaagccct 19
<210> 1441
   <211> 19
   <212> DNA
   <213> Artificial Sequence
<220>
   <223> Synthetic DNA/RNA primer, R664
<400> 1441
   gcuggaggag cuggaacut 19
<210> 1442
   <211> 21
   <212> RNA
   <213> Artificial Sequence
<220>
   <223> Synthetic Primer, R665
<400> 1442
   uaaacaggag cacgaggaug c 21
<210> 1443
   <211> 19
   <212> DNA
   <213> Artificial Sequence
<220>
   <223> Synthetic DNA/RNA primer, R666
<400> 1443
   uauucaucac ggcgcgcut 19
<210> 1444
   <211> 26
   <212> RNA
   <213> Artificial Sequence
<220>
   <223> Synthetic Primer, R667
<400> 1444
   gaguccagga gaaaauucac augagg 26
<210> 1445
   <211> 30
   <212> DNA
   <213> Artificial Sequence
<220>
   <223> Synthetic DNA/RNA primer, R668
<400> 1445
   acaacccacu gagguauaug uauagguaut 30
<210> 1446
   <211> 24
   <212> RNA
   <213> Artificial Sequence
<220>
   <223> Synthetic Primer, R669
<400> 1446
   agcacaguga auuuucuugc cauc 24
<210> 1447
   <211> 25
   <212> RNA
   <213> Artificial Sequence
<220>
   <223> Synthetic Primer, R670
<400> 1447
   ggugguaaac uuuugaguuu gcaga 25
<210> 1448
   <211> 22
   <212> DNA
   <213> Artificial Sequence
<220>
   <223> Synthetic DNA/RNA primer, R671
<400> 1448
   gggaaggagu gguacaacag at 22
<210> 1449
   <211> 25
   <212> RNA
   <213> Artificial Sequence
<220>
   <223> Synthetic Primer, R672
<400> 1449
   acagcuaguu ugccaguuag uaagc 25
<210> 1450
   <211> 25
   <212> RNA
   <213> Artificial Sequence
<220>
   <223> Synthetic Primer, R673
<400> 1450
   cacuuaauuu ggauuguggc acaga 25
<210> 1451
   <211> 21
   <212> RNA
   <213> Artificial Sequence
<220>
   <223> Synthetic Primer, R674
<400> 1451
   cucuugucau cagcucccag a 21
<210> 1452
   <211> 20
   <212> RNA
   <213> Artificial Sequence
<220>
   <223> Synthetic Primer, R675
<400> 1452
   gcgccagcau ccagagauac 20
<210> 1453
   <211> 20
   <212> DNA
   <213> Artificial Sequence
<220>
   <223> Synthetic DNA/RNA primer, R676
<400> 1453
   gagcguguga ugcagcucut 20
<210> 1454
   <211> 26
   <212> RNA
   <213> Artificial Sequence
<220>
   <223> Synthetic Primer, R677
<400> 1454
   guuugaccga agaaccaauu auaccc 26
<210> 1455
   <211> 25
   <212> RNA
   <213> Artificial Sequence
<220>
   <223> Synthetic Primer, R678
<400> 1455
   gaugcuucuc uccuucuucu cuugg 25
<210> 1456
   <211> 26
   <212> RNA
   <213> Artificial Sequence
<220>
   <223> Synthetic Primer, R679
<400> 1456
   uuccccaacc cacauuuccu uuauag 26
<210> 1457
   <211> 23
   <212> RNA
   <213> Artificial Sequence
<220>
   <223> Synthetic Primer, R680
<400> 1457
   ccaaaacccu ccugauguac acg 23
<210> 1458
   <211> 21
   <212> RNA
   <213> Artificial Sequence
<220>
   <223> Synthetic Primer, R681
<400> 1458
   gucacagcuc cagugucugu c 21
<210> 1459
   <211> 24
   <212> RNA
   <213> Artificial Sequence
<220>
   <223> Synthetic Primer, R682
<400> 1459
   gagauccagg cuaccuggua ugag 24
<210> 1460
   <211> 20
   <212> DNA
   <213> Artificial Sequence
<220>
   <223> Synthetic DNA/RNA primer, R683
<400> 1460
   aaggacgacc cagagcugat 20
<210> 1461
   <211> 25
   <212> RNA
   <213> Artificial Sequence
<220>
   <223> Synthetic Primer, R684
<400> 1461
   aaauuaaaag gcaaguggac uucgg 25
<210> 1462
   <211> 23
   <212> RNA
   <213> Artificial Sequence
<220>
   <223> Synthetic Primer, R685
<400> 1462
   cuguugguga agcuaacguu gag 23
<210> 1463
   <211> 22
   <212> RNA
   <213> Artificial Sequence
<220>
   <223> Synthetic Primer, R686
<400> 1463
   aaaaugggaa agguauccag cc 22
<210> 1464
   <211> 26
   <212> RNA
   <213> Artificial Sequence
<220>
   <223> Synthetic Primer, R687
<400> 1464
   ccacagaaac aacaucgauu ucuucc 26
<210> 1465
   <211> 18
   <212> DNA
   <213> Artificial Sequence
<220>
   <223> Synthetic DNA/RNA primer, R688
<400> 1465
   acagggaugg ugguggut 18
<210> 1466
   <211> 25
   <212> RNA
   <213> Artificial Sequence
<220>
   <223> Synthetic Primer, R689
<400> 1466
   uucuggauua gcuggauugu cagug 25
<210> 1467
   <211> 23
   <212> DNA
   <213> Artificial Sequence
<220>
   <223> Synthetic DNA/RNA primer, R690
<400> 1467
   ggcaaauaca cagaggaagc cut 23
<210> 1468
   <211> 23
   <212> RNA
   <213> Artificial Sequence
<220>
   <223> Synthetic Primer, R691
<400> 1468
   augucuauag ggaagggaag acg 23
<210> 1469
   <211> 21
   <212> DNA
   <213> Artificial Sequence
<220>
   <223> Synthetic DNA/RNA primer, R692
<400> 1469
   gucggugcug uagauauccc t 21
<210> 1470
   <211> 26
   <212> RNA
   <213> Artificial Sequence
<220>
   <223> Synthetic Primer, R693
<400> 1470
   acauugucaa guucuaugga gugugc 26
<210> 1471
   <211> 22
   <212> DNA
   <213> Artificial Sequence
<220>
   <223> Synthetic DNA/RNA primer, R694
<400> 1471
   cuggcugaag guggguuuga ut 22
<210> 1472
   <211> 17
   <212> RNA
   <213> Artificial Sequence
<220>
   <223> Synthetic Primer, R695
<400> 1472
   aagucacacg gcccucc 17
<210> 1473
   <211> 28
   <212> RNA
   <213> Artificial Sequence
<220>
   <223> Synthetic Primer, R696
<400> 1473
   uuguucucau uggcuucaaa gaucuuua 28
<210> 1474
   <211> 24
   <212> RNA
   <213> Artificial Sequence
<220>
   <223> Synthetic Primer, R697
<400> 1474
   ucucuuggaa acucccaucu ugag 24
<210> 1475
   <211> 18
   <212> RNA
   <213> Artificial Sequence
<220>
   <223> Synthetic Primer, R698
<400> 1475
   ugagcccacc ugacuugg 18
<210> 1476
   <211> 24
   <212> RNA
   <213> Artificial Sequence
<220>
   <223> Synthetic Primer, R699
<400> 1476
   acaugagagc uuguuuuuca cugg 24
<210> 1477
   <211> 25
   <212> RNA
   <213> Artificial Sequence
<220>
   <223> Synthetic Primer, R700
<400> 1477
   agagugaucu cuggaugucg gaaua 25
<210> 1478
   <211> 21
   <212> RNA
   <213> Artificial Sequence
<220>
   <223> Synthetic Primer, R701
<400> 1478
   accagugagg gaagugagga c 21
<210> 1479
   <211> 28
   <212> DNA
   <213> Artificial Sequence
<220>
   <223> Synthetic DNA/RNA primer, R702
<400> 1479
   uaagcaucag cauuugacuu uaccuuat 28
<210> 1480
   <211> 27
   <212> RNA
   <213> Artificial Sequence
<220>
   <223> Synthetic Primer, R703
<400> 1480
   caaacaaguu uauauuuccc caugcca 27
<210> 1481
   <211> 28
   <212> DNA
   <213> Artificial Sequence
<220>
   <223> Synthetic DNA/RNA primer, R704
<400> 1481
   gauuugaucc aguaacacca auagggut 28
<210> 1482
   <211> 26
   <212> DNA
   <213> Artificial Sequence
<220>
   <223> Synthetic DNA/RNA primer, R705
<400> 1482
   aaacacaaac uagagucaca caccut 26
<210> 1483
   <211> 23
   <212> RNA
   <213> Artificial Sequence
<220>
   <223> Synthetic Primer, R706
<400> 1483
   agcacuuacc ugugacucca uag 23
<210> 1484
   <211> 27
   <212> RNA
   <213> Artificial Sequence
<220>
   <223> Synthetic Primer, R707
<400> 1484
   uuguguggaa gauccaaucc auuuuug 27
<210> 1485
   <211> 25
   <212> RNA
   <213> Artificial Sequence
<220>
   <223> Synthetic Primer, R708
<400> 1485
   aaccauauca aauucacaca cuggc 25
<210> 1486
   <211> 27
   <212> RNA
   <213> Artificial Sequence
<220>
   <223> Synthetic Primer, R709
<400> 1486
   cucuugcuca guuuuaucua aggcuag 27
<210> 1487
   <211> 30
   <212> RNA
   <213> Artificial Sequence
<220>
   <223> Synthetic Primer, R710
<400> 1487
   cauaccaauu ucucgauuga ggaucuuuuc 30
<210> 1488
   <211> 21
   <212> RNA
   <213> Artificial Sequence
<220>
   <223> Synthetic Primer, R711
<400> 1488
   ccgcagaaau ggauacaggu c 21
<210> 1489
   <211> 30
   <212> RNA
   <213> Artificial Sequence
<220>
   <223> Synthetic Primer, R712
<400> 1489
   agaaaaucaa agcauucuua ccuuacuaca 30
<210> 1490
   <211> 25
   <212> DNA
   <213> Artificial Sequence
<220>
   <223> Synthetic DNA/RNA primer, R713
<400> 1490
   uccaggaaga ggaaaggaaa aacat 25
<210> 1491
   <211> 27
   <212> RNA
   <213> Artificial Sequence
<220>
   <223> Synthetic Primer, R714
<400> 1491
   auuugccccg auguaauaaa uaugcac 27
<210> 1492
   <211> 26
   <212> RNA
   <213> Artificial Sequence
<220>
   <223> Synthetic Primer, R715
<400> 1492
   gucaagaucu ucacaaaagg guuuga 26
<210> 1493
   <211> 24
   <212> RNA
   <213> Artificial Sequence
<220>
   <223> Synthetic Primer, R716
<400> 1493
   gccacugguc uauaauccag auga 24
<210> 1494
   <211> 25
   <212> RNA
   <213> Artificial Sequence
<220>
   <223> Synthetic Primer, R717
<400> 1494
   gcaucuuguu cuguuugugg aagaa 25
<210> 1495
   <211> 23
   <212> DNA
   <213> Artificial Sequence
<220>
   <223> Synthetic DNA/RNA primer, R718
<400> 1495
   ucaacaaccc ccacaaaaug uut 23
<210> 1496
   <211> 21
   <212> RNA
   <213> Artificial Sequence
<220>
   <223> Synthetic Primer, R719
<400> 1496
   uggauuugac ggcuccucua c 21
<210> 1497
   <211> 24
   <212> RNA
   <213> Artificial Sequence
<220>
   <223> Synthetic Primer, R720
<400> 1497
   uuaacaccuc cagucccuca ucug 24
<210> 1498
   <211> 27
   <212> RNA
   <213> Artificial Sequence
<220>
   <223> Synthetic Primer, R721
<400> 1498
   uaagaugucc acugcuguuc cuucaua 27
<210> 1499
   <211> 20
   <212> RNA
   <213> Artificial Sequence
<220>
   <223> Synthetic Primer, R722
<400> 1499
   cuucagccaa ggcagcaaug 20
<210> 1500
   <211> 28
   <212> RNA
   <213> Artificial Sequence
<220>
   <223> Synthetic Primer, R723
<400> 1500
   gauauggauu cacacagaca cuaucaca 28
<210> 1501
   <211> 24
   <212> DNA
   <213> Artificial Sequence
<220>
   <223> Synthetic DNA/RNA primer, R724
<400> 1501
   caagguguuu cuuugaugcu cugt 24
<210> 1502
   <211> 23
   <212> RNA
   <213> Artificial Sequence
<220>
   <223> Synthetic Primer, R725
<400> 1502
   ccuguggaca uuggagaguu gac 23
<210> 1503
   <211> 26
   <212> RNA
   <213> Artificial Sequence
<220>
   <223> Synthetic Primer, R726
<400> 1503
   gaaccuuaaa ugucucuccu accuga 26
<210> 1504
   <211> 19
   <212> RNA
   <213> Artificial Sequence
<220>
   <223> Synthetic Primer, R727
<400> 1504
   aaggcaccug acccaaaca 19
<210> 1505
   <211> 20
   <212> RNA
   <213> Artificial Sequence
<220>
   <223> Synthetic Primer, R728
<400> 1505
   gcacauaguc ccggaagcug 20
<210> 1506
   <211> 24
   <212> RNA
   <213> Artificial Sequence
<220>
   <223> Synthetic Primer, R729
<400> 1506
   uucuugaucu cacagucagg gaug 24
<210> 1507
   <211> 17
   <212> DNA
   <213> Artificial Sequence
<220>
   <223> Synthetic DNA/RNA primer, R730
<400> 1507
   augagcagcg uggccut 17
<210> 1508
   <211> 20
   <212> RNA
   <213> Artificial Sequence
<220>
   <223> Synthetic Primer, R731
<400> 1508
   uagcugugca uguccuggug 20
<210> 1509
   <211> 23
   <212> RNA
   <213> Artificial Sequence
<220>
   <223> Synthetic Primer, R732
<400> 1509
   uaggugagga ccacaaacca aac 23
<210> 1510
   <211> 21
   <212> DNA
   <213> Artificial Sequence
<220>
   <223> Synthetic DNA/RNA primer, R733
<400> 1510
   uggucuucac ucaccucgga t 21
<210> 1511
   <211> 22
   <212> DNA
   <213> Artificial Sequence
<220>
   <223> Synthetic DNA/RNA primer, R734
<400> 1511
   uuccuccaga agcuugaacu ct 22
<210> 1512
   <211> 23
   <212> DNA
   <213> Artificial Sequence
<220>
   <223> Synthetic DNA/RNA primer, R735
<400> 1512
   cccaagccug ggaccucuau uat 23
<210> 1513
   <211> 19
   <212> RNA
   <213> Artificial Sequence
<220>
   <223> Synthetic Primer, R736
<400> 1513
   caugcuggac cuucugcac 19
<210> 1514
   <211> 26
   <212> RNA
   <213> Artificial Sequence
<220>
   <223> Synthetic Primer, R737
<400> 1514
   agacugcuaa ggcauaggaa uuuucg 26
<210> 1515
   <211> 26
   <212> DNA
   <213> Artificial Sequence
<220>
   <223> Synthetic DNA/RNA primer, R738
<400> 1515
   uuugacucug ucuccucuug ucuuct 26
<210> 1516
   <211> 24
   <212> RNA
   <213> Artificial Sequence
<220>
   <223> Synthetic Primer, R739
<400> 1516
   gagaugaagc aaacaacagu ggag 24
<210> 1517
   <211> 30
   <212> RNA
   <213> Artificial Sequence
<220>
   <223> Synthetic Primer, R740
<400> 1517
   auuucaugca aacuagauaa cuaccuguaa 30
<210> 1518
   <211> 24
   <212> RNA
   <213> Artificial Sequence
<220>
   <223> Synthetic Primer, R741
<400> 1518
   uggaguuugu cugcugaaug aacc 24
<210> 1519
   <211> 26
   <212> RNA
   <213> Artificial Sequence
<220>
   <223> Synthetic Primer, R742
<400> 1519
   agcucacaga aaugucugcu auacug 26
<210> 1520
   <211> 24
   <212> RNA
   <213> Artificial Sequence
<220>
   <223> Synthetic Primer, R743
<400> 1520
   augaggagug uguacucuug cauc 24
<210> 1521
   <211> 22
   <212> DNA
   <213> Artificial Sequence
<220>
   <223> Synthetic DNA/RNA primer, R744
<400> 1521
   gccaagaguu acgggauucc at 22
<210> 1522
   <211> 22
   <212> RNA
   <213> Artificial Sequence
<220>
   <223> Synthetic Primer, R745
<400> 1522
   aggaugccug accaguuaga gg 22
<210> 1523
   <211> 26
   <212> RNA
   <213> Artificial Sequence
<220>
   <223> Synthetic Primer, R746
<400> 1523
   aaaagacucg gaugauguac cuaugg 26
<210> 1524
   <211> 21
   <212> RNA
   <213> Artificial Sequence
<220>
   <223> Synthetic Primer, R747
<400> 1524
   cacucacccu ggaugucuuc g 21
<210> 1525
   <211> 20
   <212> RNA
   <213> Artificial Sequence
<220>
   <223> Synthetic Primer, R748
<400> 1525
   caccguagcu ccagacauca 20
<210> 1526
   <211> 19
   <212> DNA
   <213> Artificial Sequence
<220>
   <223> Synthetic Primer, R749
<400> 1526
   aaggagaaga ggacagcgg 19
<210> 1527
   <211> 24
   <212> RNA
   <213> Artificial Sequence
<220>
   <223> Synthetic Primer, R750
<400> 1527
   ccugcacuuc uaggcacuua cuaa 24
<210> 1528
   <211> 20
   <212> DNA
   <213> Artificial Sequence
<220>
   <223> Synthetic DNA/RNA primer, R751
<400> 1528
   ggcacuugca cagagaugat 20
<210> 1529
   <211> 23
   <212> RNA
   <213> Artificial Sequence
<220>
   <223> Synthetic Primer, R752
<400> 1529
   auuugaugac augugggugg uug 23
<210> 1530
   <211> 17
   <212> DNA
   <213> Artificial Sequence
<220>
   <223> Synthetic DNA/RNA primer, R753
<400> 1530
   ggagccguau uuggcgt 17
<210> 1531
   <211> 24
   <212> RNA
   <213> Artificial Sequence
<220>
   <223> Synthetic Primer, R754
<400> 1531
   ccucuucacg uaggaauccu cuuc 24
<210> 1532
   <211> 26
   <212> DNA
   <213> Artificial Sequence
<220>
   <223> Synthetic DNA/RNA primer, R755
<400> 1532
   aucacuuugc gugguguaga uaugat 26
<210> 1533
   <211> 25
   <212> DNA
   <213> Artificial Sequence
<220>
   <223> Synthetic DNA/RNA primer, R756
<400> 1533
   aggacucuga agauguaccu auggt 25
<210> 1534
   <211> 27
   <212> DNA
   <213> Artificial Sequence
<220>
   <223> Synthetic DNA/RNA primer, R757
<400> 1534
   acaguuucca uaggucugaa aauguut 27
<210> 1535
   <211> 20
   <212> RNA
   <213> Artificial Sequence
<220>
   <223> Synthetic Primer, R758
<400> 1535
   agcccaaccc uuguccuuac 20
<210> 1536
   <211> 24
   <212> RNA
   <213> Artificial Sequence
<220>
   <223> Synthetic Primer, R759
<400> 1536
   gggacagcau caaaucaucc auug 24
<210> 1537
   <211> 20
   <212> RNA
   <213> Artificial Sequence
<220>
   <223> Synthetic Primer, R760
<400> 1537
   ccagacggaa accguagcug 20
<210> 1538
   <211> 18
   <212> DNA
   <213> Artificial Sequence
<220>
   <223> Synthetic DNA/RNA primer, R761
<400> 1538
   ggagcagccu cuggcaut 18
<210> 1539
   <211> 28
   <212> DNA
   <213> Artificial Sequence
<220>
   <223> Synthetic DNA/RNA primer, R762
<400> 1539
   ggcaaggaaa ggugauaaaa gugaauct 28
<210> 1540
   <211> 27
   <212> DNA
   <213> Artificial Sequence
<220>
   <223> Synthetic DNA/RNA primer, R763
<400> 1540
   acuugauaag aggucccaag acuuagt 27
<210> 1541
   <211> 22
   <212> RNA
   <213> Artificial Sequence
<220>
   <223> Synthetic Primer, R764
<400> 1541
   ccuauggcuu uccaaccuag ga 22
<210> 1542
   <211> 25
   <212> DNA
   <213> Artificial Sequence
<220>
   <223> Synthetic DNA/RNA primer, R765
<400> 1542
   cccuucuguc uugaacauga guuut 25
<210> 1543
   <211> 26
   <212> RNA
   <213> Artificial Sequence
<220>
   <223> Synthetic Primer, R766
<400> 1543
   uguggaguau uuggaugaca gaaaca 26
<210> 1544
   <211> 22
   <212> DNA
   <213> Artificial Sequence
<220>
   <223> Synthetic DNA/RNA primer, R767
<400> 1544
   aggccucuga uuccucacug at 22
<210> 1545
   <211> 20
   <212> DNA
   <213> Artificial Sequence
<220>
   <223> Synthetic DNA/RNA primer, R768
<400> 1545
   ccuagguugg cucugacugt 20
<210> 1546
   <211> 22
   <212> DNA
   <213> Artificial Sequence
<220>
   <223> Synthetic DNA/RNA primer, R769
<400> 1546
   ugccucuugc uucucuuuuc ct 22
<210> 1547
   <211> 20
   <212> RNA
   <213> Artificial Sequence
<220>
   <223> Synthetic Primer, R770
<400> 1547
   uuccuacagu acuccccugc 20
<210> 1548
   <211> 19
   <212> RNA
   <213> Artificial Sequence
<220>
   <223> Synthetic Primer, R771
<400> 1548
   agucacagca caugacgga 19
<210> 1549
   <211> 20
   <212> RNA
   <213> Artificial Sequence
<220>
   <223> Synthetic Primer, F772
<400> 1549
   ggaggaggcg auggcuacua 20
<210> 1550
   <211> 24
   <212> RNA
   <213> Artificial Sequence
<220>
   <223> Synthetic Primer, F773
<400> 1550
   ggagaccuac aaacugaagu gcaa 24
<210> 1551
   <211> 22
   <212> RNA
   <213> Artificial Sequence
<220>
   <223> Synthetic Primer, F774
<400> 1551
   ccaugcagaa ugccaccaag ua 22
<210> 1552
   <211> 19
   <212> RNA
   <213> Artificial Sequence
<220>
   <223> Synthetic Primer, F775
<400> 1552
   caggcacucc uuggagcaa 19
<210> 1553
   <211> 22
   <212> RNA
   <213> Artificial Sequence
<220>
   <223> Synthetic Primer, F776
<400> 1553
   cuguuugaaa ugagcaggca cu 22
<210> 1554
   <211> 19
   <212> RNA
   <213> Artificial Sequence
<220>
   <223> Synthetic Primer, F777
<400> 1554
   acuggaggac ccgucuucu 19
<210> 1555
   <211> 24
   <212> RNA
   <213> Artificial Sequence
<220>
   <223> Synthetic Primer, F778
<400> 1555
   agaccuuaag ggaacagcuc ucau 24
<210> 1556
   <211> 26
   <212> RNA
   <213> Artificial Sequence
<220>
   <223> Synthetic Primer, F779
<400> 1556
   guggagucau gcuuauaugg agcaaa 26
<210> 1557
   <211> 27
   <212> RNA
   <213> Artificial Sequence
<220>
   <223> Synthetic Primer, F780
<400> 1557
   gacagaaaaa uaauucugug ggaucau 27
<210> 1558
   <211> 28
   <212> RNA
   <213> Artificial Sequence
<220>
   <223> Synthetic Primer, F781
<400> 1558
   uccugaaaga gaaauagagg uuccugau 28
<210> 1559
   <211> 18
   <212> DNA
   <213> Artificial Sequence
<220>
   <223> Synthetic Primer, F782
<400> 1559
   ggtggccata ggaacgca 18
<210> 1560
   <211> 25
   <212> RNA
   <213> Artificial Sequence
<220>
   <223> Synthetic Primer, F783
<400> 1560
   uggaugcaga aaccagagau cuagu 25
<210> 1561
   <211> 22
   <212> RNA
   <213> Artificial Sequence
<220>
   <223> Synthetic Primer, F784
<400> 1561
   cuggucccca gacaacaagu au 22
<210> 1562
   <211> 23
   <212> RNA
   <213> Artificial Sequence
<220>
   <223> Synthetic Primer, F785
<400> 1562
   gaagaucaug uggccucagu gaa 23
<210> 1563
   <211> 27
   <212> RNA
   <213> Artificial Sequence
<220>
   <223> Synthetic Primer, F786
<400> 1563
   gucgaaaaua ccuucaacac ccaaauu 27
<210> 1564
   <211> 27
   <212> RNA
   <213> Artificial Sequence
<220>
   <223> Synthetic Primer, F787
<400> 1564
   ccaaaacugc agacaagcau aaagaug 27
<210> 1565
   <211> 22
   <212> RNA
   <213> Artificial Sequence
<220>
   <223> Synthetic Primer, F788
<400> 1565
   caggcagaag uugaucgacu cu 22
<210> 1566
   <211> 27
   <212> RNA
   <213> Artificial Sequence
<220>
   <223> Synthetic Primer, F789
<400> 1566
   aaagaagagu gcacaaaugu uagagga 27
<210> 1567
   <211> 24
   <212> RNA
   <213> Artificial Sequence
<220>
   <223> Synthetic Primer, F790
<400> 1567
   ccagcuuccu auaacuugga cgau 24
<210> 1568
   <211> 23
   <212> RNA
   <213> Artificial Sequence
<220>
   <223> Synthetic Primer, F791
<400> 1568
   gaaccacauc auggucucug ucu 23
<210> 1569
   <211> 21
   <212> RNA
   <213> Artificial Sequence
<220>
   <223> Synthetic Primer, F792

<400> 1569
   ucaucgggaa gaccuggcuu a 21
<210> 1570
   <211> 23
   <212> RNA
   <213> Artificial Sequence
<220>
   <223> Synthetic Primer, F793
<400> 1570
   gcugcaggac uaugaggaga aga 23
<210> 1571
   <211> 20
   <212> RNA
   <213> Artificial Sequence
<220>
   <223> Synthetic Primer, F794
<400> 1571
   cucccagaga ccaacguuca 20
<210> 1572
   <211> 20
   <212> RNA
   <213> Artificial Sequence
<220>
   <223> Synthetic Primer, F795
<400> 1572
   ggaccuggac cguguccuua 20
<210> 1573
   <211> 20
   <212> RNA
   <213> Artificial Sequence
<220>
   <223> Synthetic Primer, F796
<400> 1573
   ggaccuggac cguguccuua 20
<210> 1574
   <211> 22
   <212> RNA
   <213> Artificial Sequence
<220>
   <223> Synthetic Primer, R772a
<400> 1574
   cugcaguuag agguugguga ca 22
<210> 1575
   <211> 19
   <212> RNA
   <213> Artificial Sequence
<220>
   <223> Synthetic Primer, R772b
<400> 1575
   cccgccaagc acguauacu 19
<210> 1576
   <211> 20
   <212> RNA
   <213> Artificial Sequence
<220>
   <223> Synthetic Primer, R773a
<400> 1576
   ccggaagagg aguagcugac 20
<210> 1577
   <211> 27
   <212> RNA
   <213> Artificial Sequence
<220>
   <223> Synthetic Primer, R773b
<400> 1577
   cuccuagagu uuuuccaaga accaagu 27
<210> 1578
   <211> 26
   <212> RNA
   <213> Artificial Sequence
<220>
   <223> Synthetic Primer, R774
<400> 1578
   auuugcagcu acuacucuga acugaa 26
<210> 1579
   <211> 26
   <212> RNA
   <213> Artificial Sequence
<220>
   <223> Synthetic Primer, R775
<400> 1579
   ucagugggau uguaacaacc agaaau 26
<210> 1580
   <211> 20
   <212> RNA
   <213> Artificial Sequence
<220>
   <223> Synthetic Primer, R776
<400> 1580
   gcacugucac cccuuccuug 20
<210> 1581
   <211> 19
   <212> RNA
   <213> Artificial Sequence
<220>
   <223> Synthetic Primer, R779
<400> 1581
   gcuccaucug cauggcuug 19
<210> 1582
   <211> 22
   <212> RNA
   <213> Artificial Sequence
<220>
   <223> Synthetic Primer, R780
<400> 1582
   ggguuguagu cggucaugau gg 22
<210> 1583
   <211> 19
   <212> RNA
   <213> Artificial Sequence
<220>
   <223> Synthetic Primer, R781
<400> 1583
   ccuggcccuu gaagcacua 19
<210> 1584
   <211> 20
   <212> RNA
   <213> Artificial Sequence
<220>
   <223> Synthetic Primer, R782
<400> 1584
   accccaucuu ccccauccau 20
<210> 1585
   <211> 24
   <212> RNA
   <213> Artificial Sequence
<220>
   <223> Synthetic Primer, R786
<400> 1585
   cuaccucaca gugacugcag uuua 24
<210> 1586
   <211> 21
   <212> RNA
   <213> Artificial Sequence
<220>
   <223> Synthetic Primer, R787
<400> 1586
   agagaggauc agcgagagug g 21
<210> 1587
   <211> 20
   <212> RNA
   <213> Artificial Sequence
<220>
   <223> Synthetic Primer, R788
<400> 1587
   gucucguugc ccaaauugau 20
<210> 1588
   <211> 27
   <212> RNA
   <213> Artificial Sequence
<220>
   <223> Synthetic Primer, R789
<400> 1588
   aguguuuuca uucgauuccu gucuucu 27
<210> 1589
   <211> 20
   <212> RNA
   <213> Artificial Sequence
<220>
   <223> Synthetic Primer, R791
<400> 1589
   ggugaugccg ugguugaugu 20
<210> 1590
   <211> 20
   <212> RNA
   <213> Artificial Sequence
<220>
   <223> Synthetic Primer, R792
<400> 1590
   aguucucgcu ucagcacgau 20
<210> 1591
   <211> 20
   <212> RNA
   <213> Artificial Sequence
<220>
   <223> Synthetic Primer, R794
<400> 1591
   uggccaagca aucugcguau 20
<210> 1592
   <211> 23
   <212> RNA
   <213> Artificial Sequence
<220>
   <223> Synthetic Primer, R795
<400> 1592
   ugccaggauc auagcguuua cag 23
<210> 1593
   <211> 22
   <212> RNA
   <213> Artificial Sequence
<220>
   <223> Synthetic Primer, R796a
<400> 1593
   cuggagcagg uccacuauag gu 22
<210> 1594
   <211> 19
   <212> RNA
   <213> Artificial Sequence
<220>
   <223> Synthetic Primer, R796b
<400> 1594
   uccucacacc ugcuccuca 19
<210> 1595
   <211> 18
   <212> RNA
   <213> Artificial Sequence
<220>
   <223> Synthetic Primer, R796c
<400> 1595
   gcugaugggu gggcacug 18
<210> 1596
   <211> 20
   <212> RNA
   <213> Artificial Sequence
<220>
   <223> Synthetic Primer, R796d
<400> 1596
   ggucuaccag gacugucccu 20
<210> 1597
   <211> 25
   <212> RNA
   <213> Artificial Sequence
<220>
   <223> Synthetic Primer, R797
<400> 1597
   ggaaggcagg aagauuuuca aucuc 25
<210> 1598
   <211> 24
   <212> RNA
   <213> Artificial Sequence
<220>
   <223> Synthetic Primer, R798
<400> 1598
   cguuuauaag cacugucacc ccuu 24
<210> 1599
   <211> 21
   <212> RNA
   <213> Artificial Sequence
<220>
   <223> Synthetic Primer, R801
<400> 1599
   aggaugaugg cacugaacuc c 21
<210> 1600
   <211> 25
   <212> RNA
   <213> Artificial Sequence
<220>
   <223> Synthetic Primer, R802
<400> 1600
   cacguuaguu agugagccag guaau 25
<210> 1601
   <211> 19
   <212> RNA
   <213> Artificial Sequence
<220>
   <223> Synthetic Primer, R803
<400> 1601
   cucagggcuc ugcagcucc 19
<210> 1602
   <211> 19
   <212> RNA
   <213> Artificial Sequence
<220>
   <223> Synthetic Primer, R804
<400> 1602
   ccuccggaag gucaucuca 19
<210> 1603
   <211> 27
   <212> RNA
   <213> Artificial Sequence
<220>
   <223> Synthetic Primer, R806b
<400> 1603
   cuccuagagu uuuuccaaga accaagu 27
<210> 1604
   <211> 23
   <212> RNA
   <213> Artificial Sequence
<220>
   <223> Synthetic Primer, R807
<400> 1604
   gaaccaaguu cuuccgaggg aau 23
<210> 1605
   <211> 18
   <212> RNA
   <213> Artificial Sequence
<220>
   <223> Synthetic Primer, R809
<400> 1605
   acagcggcug cgaucacc 18
<210> 1606
   <211> 19
   <212> RNA
   <213> Artificial Sequence
<220>
   <223> Synthetic Primer, R811
<400> 1606
   gcugacugca caggacagg 19
<210> 1607
   <211> 21
   <212> RNA
   <213> Artificial Sequence
<220>
   <223> Synthetic Primer, R813a
<400> 1607
   cgagacccca aaagguguuu c 21
<210> 1608
   <211> 23
   <212> RNA
   <213> Artificial Sequence
<220>
   <223> Synthetic Primer, R813b
<400> 1608
   uccacauuug uugagcacaa gga 23
<210> 1609
   <211> 27
   <212> RNA
   <213> Artificial Sequence
<220>
   <223> Synthetic Primer, R815
<400> 1609
   caccuuuaac ugcuucaggg ucaauau 27
<210> 1610
   <211> 18
   <212> RNA
   <213> Artificial Sequence
<220>
   <223> Synthetic Primer, R816
<400> 1610
   uguugucccg uggccauu 18
<210> 1611
   <211> 21
   <212> RNA
   <213> Artificial Sequence
<220>
   <223> Synthetic Primer, R817
<400> 1611
   ggcaugaacc guucugagau g 21
<210> 1612
   <211> 23
   <212> RNA
   <213> Artificial Sequence
<220>
   <223> Synthetic Primer, R818
<400> 1612
   ccaaauucgc cuucuccuag agu 23
<210> 1613
   <211> 22
   <212> RNA
   <213> Artificial Sequence
<220>
   <223> Synthetic Primer, R819
<400> 1613
   cuccucugca ccaagguaaa ca 22
<210> 1614
   <211> 25
   <212> RNA
   <213> Artificial Sequence
<220>
   <223> Synthetic Primer, R820
<400> 1614
   ucccuucuag uaauuuggga augcc 25
<210> 1615
   <211> 28
   <212> RNA
   <213> Artificial Sequence
<220>
   <223> Synthetic Primer, R823
<400> 1615
   ucagcuuucu cccacuguau ugaauuuu 28
<210> 1616
   <211> 20
   <212> RNA
   <213> Artificial Sequence
<220>
   <223> Synthetic Primer, R824
<400> 1616
   ucggaagggc uguggaauug 20
<210> 1617
   <211> 22
   <212> RNA
   <213> Artificial Sequence
<220>
   <223> Synthetic Primer, R824b
<400> 1617
   cguaggcaca cucaaacaac ga 22
<210> 1618
   <211> 24
   <212> RNA
   <213> Artificial Sequence
<220>
   <223> Synthetic Primer, R825
<400> 1618
   cugauuucug aacauggacu gugg 24
<210> 1619
   <211> 23
   <212> RNA
   <213> Artificial Sequence
<220>
   <223> Synthetic Primer, R826
<400> 1619
   acgaagugca auggucuuua ggu 23
<210> 1620
   <211> 25
   <212> RNA
   <213> Artificial Sequence
<220>
   <223> Synthetic Primer, R828
<400> 1620
   gugagucauu ugucuugcuu uuggu 25
<210> 1621
   <211> 25
   <212> RNA
   <213> Artificial Sequence
<220>
   <223> Synthetic Primer, F829
<400> 1621
   gacagucuga aucauguccu ucagu 25
<210> 1622
   <211> 18
   <212> RNA
   <213> Artificial Sequence
<220>
   <223> Synthetic Primer, F830
<400> 1622
   gggcugccca ccaucuuc 18
<210> 1623
   <211> 25
   <212> RNA
   <213> Artificial Sequence
<220>
   <223> Synthetic Primer, F831
<400> 1623
   ucagccugau agucugguac aaacu 25
<210> 1624
   <211> 20
   <212> RNA
   <213> Artificial Sequence
<220>
   <223> Synthetic Primer, R833
<400> 1624
   ccuccaccuu gggcuacuca 20
<210> 1625
   <211> 19
   <212> RNA
   <213> Artificial Sequence
<220>
   <223> Synthetic Primer, R834
<400> 1625
   gggugagccu ugacacaca 19
<210> 1626
   <211> 22
   <212> RNA
   <213> Artificial Sequence
<220>
   <223> Synthetic Primer, R835
<400> 1626
   cagggaucag uucagcugua cc 22
<210> 1627
   <211> 21
   <212> RNA
   <213> Artificial Sequence
<220>
   <223> Synthetic Primer, F838
<400> 1627
   ugggcucugu aaagaauagu g 21
<210> 1628
   <211> 21
   <212> RNA
   <213> Artificial Sequence
<220>
   <223> Synthetic Primer, F839
<400> 1628
   ugcacacuug gacagcauuu c 21
<210> 1629
   <211> 26
   <212> RNA
   <213> Artificial Sequence
<220>
   <223> Synthetic Primer, F840
<400> 1629
   ccaggaccaa ucuggucaca aacaua 26
<210> 1630
   <211> 23
   <212> DNA
   <213> Artificial Sequence
<220>
   <223> Synthetic DNA/RNA primer, F841
<400> 1630
   ggugggagga aaagacauag gat 23
<210> 1631
   <211> 24
   <212> RNA
   <213> Artificial Sequence
<220>
   <223> Synthetic Primer, F842
<400> 1631
   cuccagagag aaagaaucaa cagg 24
<210> 1632
   <211> 20
   <212> RNA
   <213> Artificial Sequence
<220>
   <223> Synthetic Primer, F843
<400> 1632
   gcauccguga cucucuggac 20
<210> 1633
   <211> 28
   <212> RNA
   <213> Artificial Sequence
<220>
   <223> Synthetic Primer, F844
<400> 1633
   ucagugagcc aauuccuugu aauaacuc 28
<210> 1634
   <211> 22
   <212> DNA
   <213> Artificial Sequence
<220>
   <223> Synthetic DNA/RNA primer, F845
<400> 1634
   cagaucccaa gcucuuccuc ut 22
<210> 1635
   <211> 22
   <212> DNA
   <213> Artificial Sequence
<220>
   <223> Synthetic DNA/RNA primer, F846
<400> 1635
   guucaugcca cugcacuuca ct 22
<210> 1636
   <211> 20
   <212> DNA
   <213> Artificial Sequence
<220>
   <223> Synthetic DNA/RNA primer, F847
<400> 1636
   ggugcaccca uuacccgaat 20
<210> 1637
   <211> 25
   <212> DNA
   <213> Artificial Sequence
<220>
   <223> Synthetic DNA/RNA primer, F848
<400> 1637
   uccccauaua aguucaagcc ugugt 25
<210> 1638
   <211> 27
   <212> DNA
   <213> Artificial Sequence
<220>
   <223> Synthetic DNA/RNA primer, F849
<400> 1638
   uuguauagcu acaguuuuuc uguuggt 27
<210> 1639
   <211> 27
   <212> RNA
   <213> Artificial Sequence
<220>
   <223> Synthetic Primer, F850
<400> 1639
   uaaauaugug agucaauucc ccaagug 27
<210> 1640
   <211> 27
   <212> DNA
   <213> Artificial Sequence
<220>
   <223> Synthetic DNA/RNA primer, F851
<400> 1640
   ggcuagauuu uccccgauga uaguagt 27
<210> 1641
   <211> 27
   <212> DNA
   <213> Artificial Sequence
<220>
   <223> Synthetic DNA/RNA primer, F852
<400> 1641
   ggcuagauuu uccccuauga uaguagt 27
<210> 1642
   <211> 24
   <212> RNA
   <213> Artificial Sequence
<220>
   <223> Synthetic Primer, F853
<400> 1642
   caguaaguua aaggauugca ggag 24
<210> 1643
   <211> 28
   <212> RNA
   <213> Artificial Sequence
<220>
   <223> Synthetic Primer, F854
<400> 1643
   uguguauaug cauuuaccug ugaguaug 28
<210> 1644
   <211> 24
   <212> DNA
   <213> Artificial Sequence
<220>
   <223> Synthetic DNA/RNA primer, F855
<400> 1644
   uguaacaagg gcuacaggaa ucat 24
<210> 1645
   <211> 27
   <212> RNA
   <213> Artificial Sequence
<220>
   <223> Synthetic Primer, F856
<400> 1645
   gggcaucucu uauacucaug aaaucaa 27
<210> 1646
   <211> 25
   <212> DNA
   <213> Artificial Sequence
<220>
   <223> Synthetic DNA/RNA primer, F857
<400> 1646
   cuaugcagaa gaaugaacca gggat 25
<210> 1647
   <211> 28
   <212> RNA
   <213> Artificial Sequence
<220>
   <223> Synthetic Primer, F858
<400> 1647
   ugauucauuu ccauagggua agugaaaa 28
<210> 1648
   <211> 26
   <212> RNA
   <213> Artificial Sequence
<220>
   <223> Synthetic Primer, F859
<400> 1648
   gacauuauca ccaauuuuuc uagacg 26
<210> 1649
   <211> 26
   <212> RNA
   <213> Artificial Sequence
<220>
   <223> Synthetic Primer, F860
<400> 1649
   gacauucuca ccaauuuuuc uagacg 26
<210> 1650
   <211> 22
   <212> RNA
   <213> Artificial Sequence
<220>
   <223> Synthetic Primer, F861
<400> 1650
   ugugacaagg gugauuuucc uc 22
<210> 1651
   <211> 25
   <212> DNA
   <213> Artificial Sequence
<220>
   <223> Synthetic DNA/RNA primer, F862
<400> 1651
   cauaauugua ugagccacuu cccat 25
<210> 1652
   <211> 26
   <212> DNA
   <213> Artificial Sequence
<220>
   <223> Synthetic DNA/RNA primer, F863
<400> 1652
   agacucacaa uguacaaaag ccuaut 26
<210> 1653
   <211> 33
   <212> RNA
   <213> Artificial Sequence
<220>
   <223> Synthetic Primer, F864
<400> 1653
   aauauauaua aaggguauga uagaacacuu guc 33
<210> 1654
   <211> 30
   <212> DNA
   <213> Artificial Sequence
<220>
   <223> Synthetic DNA/RNA primer, F865
<400> 1654
   ggccuggcaa cuuauaugua uuuuuguaut 30
<210> 1655
   <211> 30
   <212> DNA
   <213> Artificial Sequence
<220>
   <223> Synthetic DNA/RNA primer, F866
<400> 1655
   ggccugacaa cuuauaugua uuuuuguaut 30
<210> 1656
   <211> 33
   <212> RNA
   <213> Artificial Sequence
<220>
   <223> Synthetic Primer, F867
<400> 1656
   ccauccuuau cucuugugua ucuauucauu caa 33
<210> 1657
   <211> 25
   <212> RNA
   <213> Artificial Sequence
<220>
   <223> Synthetic Primer, F868
<400> 1657
   gauuugucug uaauugccag caaaa 25
<210> 1658
   <211> 22
   <212> RNA
   <213> Artificial Sequence
<220>
   <223> Synthetic Primer, F869
<400> 1658
   gagcaagaca ccaucucaag aa 22
<210> 1659
   <211> 28
   <212> DNA
   <213> Artificial Sequence
<220>
   <223> Synthetic DNA/RNA primer, F870
<400> 1659
   caugauugau acauggaaag aauucuct 28
<210> 1660
   <211> 24
   <212> RNA
   <213> Artificial Sequence
<220>
   <223> Synthetic Primer, F871
<400> 1660
   acccaaauca acucaacucc agug 24
<210> 1661
   <211> 28
   <212> DNA
   <213> Artificial Sequence
<220>
   <223> Synthetic DNA/RNA primer, F872
<400> 1661
   uuagagcauu uaaaguaagc cacagugt 28
<210> 1662
   <211> 21
   <212> DNA
   <213> Artificial Sequence
<220>
   <223> Synthetic DNA/RNA primer, F873
<400> 1662
   cuguacacag ggcuuccgag t 21
<210> 1663
   <211> 24
   <212> RNA
   <213> Artificial Sequence
<220>
   <223> Synthetic Primer, F874
<400> 1663
   uuucagggcu gugaucacua gcac 24
<210> 1664
   <211> 31
   <212> RNA
   <213> Artificial Sequence
<220>
   <223> Synthetic Primer, F875
<400> 1664
   agauacauag guuagauaga gauaggacag a 31
<210> 1665
   <211> 21
   <212> RNA
   <213> Artificial Sequence
<220>
   <223> Synthetic Primer, R838a
<400> 1665
   aucagagcuu aaacugggaa g 21
<210> 1666
   <211> 21
   <212> RNA
   <213> Artificial Sequence
<220>
   <223> Synthetic Primer, R838b
<400> 1666
   aucagagcuu aaacugggaa a 21
<210> 1667
   <211> 29
   <212> RNA
   <213> Artificial Sequence
<220>
   <223> Synthetic Primer, R839
<400> 1667
   gucucaguuu uccuaccugu aaaaugaag 29
<210> 1668
   <211> 28
   <212> DNA
   <213> Artificial Sequence
<220>
   <223> Synthetic DNA/RNA primer, R840
<400> 1668
   acuuauucug acaguucucu uuuuccct 28
<210> 1669
   <211> 23
   <212> RNA
   <213> Artificial Sequence
<220>
   <223> Synthetic Primer, R841
<400> 1669
   gguggcagug agcuguaaca gua 23
<210> 1670
   <211> 22
   <212> RNA
   <213> Artificial Sequence
<220>
   <223> Synthetic Primer, R842
<400> 1670
   ucagccucca uaucacuuga gc 22
<210> 1671
   <211> 21
   <212> RNA
   <213> Artificial Sequence
<220>
   <223> Synthetic Primer, R843
<400> 1671
   aacuuggguu gagccauagg c 21
<210> 1672
   <211> 27
   <212> RNA
   <213> Artificial Sequence
<220>
   <223> Synthetic Primer, R844
<400> 1672
   ccugguucca uggauuccac auuaaga 27
<210> 1673
   <211> 20
   <212> DNA
   <213> Artificial Sequence
<220>
   <223> Synthetic DNA/RNA primer, R845
<400> 1673
   gcguuugugu gugcaucugt 20
<210> 1674
   <211> 23
   <212> RNA
   <213> Artificial Sequence
<220>
   <223> Synthetic Primer, R846
<400> 1674
   ucuggugugu ggagaugucu uac 23
<210> 1675
   <211> 25
   <212> RNA
   <213> Artificial Sequence
<220>
   <223> Synthetic Primer, R847a
<400> 1675
   ggcugcaaaa agcuauaauu guacc 25
<210> 1676
   <211> 25
   <212> RNA
   <213> Artificial Sequence
<220>
   <223> Synthetic Primer, R847b
<400> 1676
   ggcugcaaaa agcuauaacu guacc 25
<210> 1677
   <211> 25
   <212> RNA
   <213> Artificial Sequence
<220>
   <223> Synthetic Primer, R848
<400> 1677
   uguguuaguc aggauucuuc agaga 25
<210> 1678
   <211> 31
   <212> RNA
   <213> Artificial Sequence
<220>
   <223> Synthetic Primer, R849
<400> 1678
   uucaguuaua uguguauaaa ugugugcauu g 31
<210> 1679
   <211> 26
   <212> RNA
   <213> Artificial Sequence
<220>
   <223> Synthetic Primer, R850
<400> 1679
   cuccagagac agacuaauag gaggua 26
<210> 1680
   <211> 22
   <212> DNA
   <213> Artificial Sequence
<220>
   <223> Synthetic DNA/RNA primer, R851
<400> 1680
   ccugugccca aguugagaga at 22
<210> 1681
   <211> 20
   <212> RNA
   <213> Artificial Sequence
<220>
   <223> Synthetic Primer, R853
<400> 1681
   uaauccagcu gugggaggga 20
<210> 1682
   <211> 20
   <212> RNA
   <213> Artificial Sequence
<220>
   <223> Synthetic Primer, R854
<400> 1682
   ggugcuaggu gugcucagga 20
<210> 1683
   <211> 27
   <212> RNA
   <213> Artificial Sequence
<220>
   <223> Synthetic Primer, R855
<400> 1683
   cuucacucuc cuucccaaau guuuaug 27
<210> 1684
   <211> 22
   <212> RNA
   <213> Artificial Sequence
<220>
   <223> Synthetic Primer, R856
<400> 1684
   cuaugauucc cccacugcag uc 22
<210> 1685
   <211> 28
   <212> RNA
   <213> Artificial Sequence
<220>
   <223> Synthetic Primer, R857
<400> 1685
   agaccccaaa auuacuugag ccaauuua 28
<210> 1686
   <211> 27
   <212> RNA
   <213> Artificial Sequence
<220>
   <223> Synthetic Primer, R858
<400> 1686
   acuucaacuu caauucaucc acugaaa 27
<210> 1687
   <211> 24
   <212> RNA
   <213> Artificial Sequence
<220>
   <223> Synthetic Primer, R859
<400> 1687
   ugcuugccug uaugaaaaua ucuc 24
<210> 1688
   <211> 24
   <212> RNA
   <213> Artificial Sequence
<220>
   <223> Synthetic Primer, R861
<400> 1688
   uccaaucaua gccacaguuu acaa 24
<210> 1689
   <211> 26
   <212> DNA
   <213> Artificial Sequence
<220>
   <223> Synthetic DNA/RNA primer, R862
<400> 1689
   gcacucuuau ucaucuaguu gccugt 26
<210> 1690
   <211> 23
   <212> RNA
   <213> Artificial Sequence
<220>
   <223> Synthetic Primer, R863a
<400> 1690
   caucauguga gccaauuccu cuc 23
<210> 1691
   <211> 23
   <212> RNA
   <213> Artificial Sequence
<220>
   <223> Synthetic Primer, R863b
<400> 1691
   caucauguga gccaaguccu cuc 23
<210> 1692
   <211> 32
   <212> DNA
   <213> Artificial Sequence
<220>
   <223> Synthetic DNA/RNA primer, R864a
<400> 1692
   uugcaccaaa uauugguaau uaaauguuua ct 32
<210> 1693
   <211> 32
   <212> DNA
   <213> Artificial Sequence
<220>
   <223> Synthetic DNA/RNA primer, R864b
<400> 1693
   uugcaccaca uauugguaau uaaauguuua ct 32
<210> 1694
   <211> 22
   <212> RNA
   <213> Artificial Sequence
<220>
   <223> Synthetic Primer, R865
<400> 1694
   cacuguaucg uaucccauug cg 22
<210> 1695
   <211> 24
   <212> RNA
   <213> Artificial Sequence
<220>
   <223> Synthetic Primer, R867
<400> 1695
   uugcaagcaa uugccauaga ggga 24
<210> 1696
   <211> 33
   <212> RNA
   <213> Artificial Sequence
<220>
   <223> Synthetic Primer, R868
<400> 1696
   acagauuaaa cuguaaccaa aauaaaauua ggc 33
<210> 1697
   <211> 22
   <212> RNA
   <213> Artificial Sequence
<220>
   <223> Synthetic Primer, R869a
<400> 1697
   ugccuaaccu auggucauaa cg 22
<210> 1698
   <211> 22
   <212> RNA
   <213> Artificial Sequence
<220>
   <223> Synthetic Primer, R869b
<400> 1698
   ugccuaaccu auggucauac cg 22
<210> 1699
   <211> 20
   <212> RNA
   <213> Artificial Sequence
<220>
   <223> Synthetic Primer, R870
<400> 1699
   cccaggaggu ggagauugaa 20
<210> 1700
   <211> 24
   <212> RNA
   <213> Artificial Sequence
<220>
   <223> Synthetic Primer, R871
<400> 1700
   uccauguacu uuguccaaug cuga 24
<210> 1701
   <211> 25
   <212> RNA
   <213> Artificial Sequence
<220>
   <223> Synthetic Primer, R872
<400> 1701
   ugucaacacg auuaacaugc aaaga 25
<210> 1702
   <211> 26
   <212> DNA
   <213> Artificial Sequence
<220>
   <223> Synthetic DNA/RNA primer, R873
<400> 1702
   caaaauucaa aggguaucug ggcuct 26
<210> 1703
   <211> 23
   <212> DNA
   <213> Artificial Sequence
<220>
   <223> Synthetic DNA/RNA primer, R874
<400> 1703
   ugugcgcugg ucuuacuccu gut 23
<210> 1704
   <211> 27
   <212> RNA
   <213> Artificial Sequence
<220>
   <223> Synthetic Primer, R875
<400> 1704
   gcccuagugg augauaagaa uaaucag 27
<210> 1705
   <211> 60
   <212> DNA
   <213> Artificial Sequence
<220>
   <223> Synthetic DNA/RNA, Rev Adaptor I
<220>
   <221> misc_feature
   <222> (21)..(23)
   <223> n is a, c, g, t or u
<220>
   <221> misc_feature
   <222> (27)..(29)
   <223> n is a, c, g, t or u
<400> 1705
   tgacaaggcg tagtcacggu nnnactnnnt gauccttctg cauggtattc tttctctucc 60
<210> 1706
   <211> 90
   <212> DNA
   <213> Artificial Sequence
<220>
   <223> Synthetic Primer, 5-01-Ah
<400> 1706
   aatgatacgg cgaccaccga gatctacaca gcgctagtcg tcggcagcgt cagatgtgta 60
   taagagacag tctgtacggt gacaaggcgt 90
<210> 1707
   <211> 90
   <212> DNA
   <213> Artificial Sequence
<220>
   <223> Synthetic Primer, 5-02-Ah
<400> 1707
   aatgatacgg cgaccaccga gatctacacg atatcgatcg tcggcagcgt cagatgtgta 60
   taagagacag tctgtacggt gacaaggcgt 90
<210> 1708
   <211> 90
   <212> DNA
   <213> Artificial Sequence
<220>
   <223> Synthetic Primer, 5-07-Ah
<400> 1708
   aatgatacgg cgaccaccga gatctacaca catagcgtcg tcggcagcgt cagatgtgta 60
   taagagacag tctgtacggt gacaaggcgt 90
<210> 1709
   <211> 90
   <212> DNA
   <213> Artificial Sequence
<220>
   <223> Synthetic Primer, 5-08-Ah
<400> 1709
   aatgatacgg cgaccaccga gatctacacg tgcgatatcg tcggcagcgt cagatgtgta 60
   taagagacag tctgtacggt gacaaggcgt 90
<210> 1710
   <211> 90
   <212> DNA
   <213> Artificial Sequence
<220>
   <223> Synthetic Primer, 5-09-Ah
<400> 1710
   aatgatacgg cgaccaccga gatctacacc caacagatcg tcggcagcgt cagatgtgta 60
   taagagacag tctgtacggt gacaaggcgt 90
<210> 1711
   <211> 90
   <212> DNA
   <213> Artificial Sequence
<220>
   <223> Synthetic Primer, 5-010-Ah
<400> 1711
   aatgatacgg cgaccaccga gatctacact tggtgagtcg tcggcagcgt cagatgtgta 60
   taagagacag tctgtacggt gacaaggcgt 90
<210> 1712
   <211> 90
   <212> DNA
   <213> Artificial Sequence
<220>
   <223> Synthetic Primer, 5-013-Ah
<400> 1712
   aatgatacgg cgaccaccga gatctacaca accgcggtcg tcggcagcgt cagatgtgta 60
   taagagacag tctgtacggt gacaaggcgt 90
<210> 1713
   <211> 90
   <212> DNA
   <213> Artificial Sequence
<220>
   <223> Synthetic Primer, 5-014-Ah
<400> 1713
   aatgatacgg cgaccaccga gatctacacg gttataatcg tcggcagcgt cagatgtgta 60
   taagagacag tctgtacggt gacaaggcgt 90
<210> 1714
   <211> 90
   <212> DNA
   <213> Artificial Sequence
<220>
   <223> Synthetic Primer, 01-Ih
<400> 1714
   aatgatacgg cgaccaccga gatctacaca gcgctagtcg tcggcagcgt cagatgtgta 60
   taagagacag tgacaaggcg tagtcacggt 90
<210> 1715
   <211> 90
   <212> DNA
   <213> Artificial Sequence
<220>
   <223> Synthetic Primer, 02-Ih
<400> 1715
   aatgatacgg cgaccaccga gatctacacg atatcgatcg tcggcagcgt cagatgtgta 60
   taagagacag tgacaaggcg tagtcacggt 90
<210> 1716
   <211> 90
   <212> DNA
   <213> Artificial Sequence
<220>
   <223> Synthetic Primer, 5-07-Ih
<400> 1716
   aatgatacgg cgaccaccga gatctacaca catagcgtcg tcggcagcgt cagatgtgta 60
   taagagacag tgacaaggcg tagtcacggt 90
<210> 1717
   <211> 90
   <212> DNA
   <213> Artificial Sequence
<220>
   <223> Synthetic Primer, 5-08-Ih
<400> 1717
   aatgatacgg cgaccaccga gatctacacg tgcgatatcg tcggcagcgt cagatgtgta 60
   taagagacag tgacaaggcg tagtcacggt 90
<210> 1718
   <211> 90
   <212> DNA
   <213> Artificial Sequence
<220>
   <223> Synthetic Primer, 5-09-Ih
<400> 1718
   aatgatacgg cgaccaccga gatctacacc caacagatcg tcggcagcgt cagatgtgta 60
   taagagacag tgacaaggcg tagtcacggt 90
<210> 1719
   <211> 90
   <212> DNA
   <213> Artificial Sequence
<220>
   <223> Synthetic Primer, 5-010-Ih
<400> 1719
   aatgatacgg cgaccaccga gatctacact tggtgagtcg tcggcagcgt cagatgtgta 60
   taagagacag tgacaaggcg tagtcacggt 90
<210> 1720
   <211> 90
   <212> DNA
   <213> Artificial Sequence
<220>
   <223> Synthetic Primer, 5-013-Ih
<400> 1720
   aatgatacgg cgaccaccga gatctacaca accgcggtcg tcggcagcgt cagatgtgta 60
   taagagacag tgacaaggcg tagtcacggt 90
<210> 1721
   <211> 90
   <212> DNA
   <213> Artificial Sequence
<220>
   <223> Synthetic Primer, 5-014-Ih
<400> 1721
   aatgatacgg cgaccaccga gatctacacg gttataatcg tcggcagcgt cagatgtgta 60
   taagagacag tgacaaggcg tagtcacggt 90
<210> 1722
   <211> 86
   <212> DNA
   <213> Artificial Sequence
<220>
   <223> Synthetic Primer, 7-1-Ah
<400> 1722
   caagcagaag acggcatacg agatatattc acgtctcgtg ggctcggaga tgtgtataag 60
   agacagtctg tacggtgaca aggcgt 86
<210> 1723
   <211> 86
   <212> DNA
   <213> Artificial Sequence
<220>
   <223> Synthetic Primer, 7-2-Ah
<400> 1723
   caagcagaag acggcatacg agatgcgcct gtgtctcgtg ggctcggaga tgtgtataag 60
   agacagtctg tacggtgaca aggcgt 86
<210> 1724
   <211> 86
   <212> DNA
   <213> Artificial Sequence
<220>
   <223> Synthetic Primer, 7-3-Ah
<400> 1724
   caagcagaag acggcatacg agatactcta tggtctcgtg ggctcggaga tgtgtataag 60
   agacagtctg tacggtgaca aggcgt 86
<210> 1725
   <211> 86
   <212> DNA
   <213> Artificial Sequence
<220>
   <223> Synthetic Primer, 7-4-Ah
<400> 1725
   caagcagaag acggcatacg agatgtctcg cagtctcgtg ggctcggaga tgtgtataag 60
   agacagtctg tacggtgaca aggcgt 86
<210> 1726
   <211> 86
   <212> DNA
   <213> Artificial Sequence
<220>
   <223> Synthetic Primer, 7-5-Ah
<400> 1726
   caagcagaag acggcatacg agatagtaga gagtctcgtg ggctcggaga tgtgtataag 60
   agacagtctg tacggtgaca aggcgt 86
<210> 1727
   <211> 86
   <212> DNA
   <213> Artificial Sequence
<220>
   <223> Synthetic Primer, 7-6-Ah
<400> 1727
   caagcagaag acggcatacg agatgacgag aggtctcgtg ggctcggaga tgtgtataag 60
   agacagtctg tacggtgaca aggcgt 86
<210> 1728
   <211> 86
   <212> DNA
   <213> Artificial Sequence
<220>
   <223> Synthetic Primer, 7-7-Ah
<400> 1728
   caagcagaag acggcatacg agatagactt gggtctcgtg ggctcggaga tgtgtataag 60
   agacagtctg tacggtgaca aggcgt 86
<210> 1729
   <211> 86
   <212> DNA
   <213> Artificial Sequence
<220>
   <223> Synthetic Primer, 7-8-Ah
<400> 1729
   caagcagaag acggcatacg agatgagtcc aagtctcgtg ggctcggaga tgtgtataag 60
   agacagtctg tacggtgaca aggcgt 86
<210> 1730
   <211> 86
   <212> DNA
   <213> Artificial Sequence
<220>
   <223> Synthetic Primer, 7-9-Ah
<400> 1730
   caagcagaag acggcatacg agataattct gcgtctcgtg ggctcggaga tgtgtataag 60
   agacagtctg tacggtgaca aggcgt 86
<210> 1731
   <211> 86
   <212> DNA
   <213> Artificial Sequence
<220>
   <223> Synthetic Primer, 7-10-Ah
<400> 1731
   caagcagaag acggcatacg agatggcctc atgtctcgtg ggctcggaga tgtgtataag 60
   agacagtctg tacggtgaca aggcgt 86
<210> 1732
   <211> 86
   <212> DNA
   <213> Artificial Sequence
<220>
   <223> Synthetic Primer, 7-1-Ah
<400> 1732
   caagcagaag acggcatacg agatatctta gtgtctcgtg ggctcggaga tgtgtataag 60
   agacagtctg tacggtgaca aggcgt 86
<210> 1733
   <211> 86
   <212> DNA
   <213> Artificial Sequence
<220>
   <223> Synthetic Primer, 7-12-Ah
<400> 1733
   caagcagaag acggcatacg agatgctccg acgtctcgtg ggctcggaga tgtgtataag 60
   agacagtctg tacggtgaca aggcgt 86
<210> 1734
   <211> 86
   <212> DNA
   <213> Artificial Sequence
<220>
   <223> Synthetic Primer, 7-1-Ih
<400> 1734
   caagcagaag acggcatacg agatatattc acgtctcgtg ggctcggaga tgtgtataag 60
   agacagtgac aaggcgtagt cacggt 86
<210> 1735
   <211> 86
   <212> DNA
   <213> Artificial Sequence
<220>
   <223> Synthetic Primer, 7-2-Ih
<400> 1735
   caagcagaag acggcatacg agatgcgcct gtgtctcgtg ggctcggaga tgtgtataag 60
   agacagtgac aaggcgtagt cacggt 86
<210> 1736
   <211> 86
   <212> DNA
   <213> Artificial Sequence
<220>
   <223> Synthetic Primer, 7-3-Ih
<400> 1736
   caagcagaag acggcatacg agatactcta tggtctcgtg ggctcggaga tgtgtataag 60
   agacagtgac aaggcgtagt cacggt 86
<210> 1737
   <211> 86
   <212> DNA
   <213> Artificial Sequence
<220>
   <223> Synthetic Primer, 7-4-Ih
<400> 1737
   caagcagaag acggcatacg agatgtctcg cagtctcgtg ggctcggaga tgtgtataag 60
   agacagtgac aaggcgtagt cacggt 86
<210> 1738
   <211> 86
   <212> DNA
   <213> Artificial Sequence
<220>
   <223> Synthetic Primer, 7-5-Ih
<400> 1738
   caagcagaag acggcatacg agatagtaga gagtctcgtg ggctcggaga tgtgtataag 60
   agacagtgac aaggcgtagt cacggt 86
<210> 1739
   <211> 86
   <212> DNA
   <213> Artificial Sequence
<220>
   <223> Synthetic Primer, 7-6-Ih
<400> 1739
   caagcagaag acggcatacg agatgacgag aggtctcgtg ggctcggaga tgtgtataag 60
   agacagtgac aaggcgtagt cacggt 86
<210> 1740
   <211> 86
   <212> DNA
   <213> Artificial Sequence
<220>
   <223> Synthetic Primer, 7-7-Ih
<400> 1740
   caagcagaag acggcatacg agatagactt gggtctcgtg ggctcggaga tgtgtataag 60
   agacagtgac aaggcgtagt cacggt 86
<210> 1741
   <211> 86
   <212> DNA
   <213> Artificial Sequence
<220>
   <223> Synthetic Primer, 7-8-Ih
<400> 1741
   caagcagaag acggcatacg agatgagtcc aagtctcgtg ggctcggaga tgtgtataag 60
   agacagtgac aaggcgtagt cacggt 86
<210> 1742
   <211> 86
   <212> DNA
   <213> Artificial Sequence
<220>
   <223> Synthetic Primer, 7-9-Ih
<400> 1742
   caagcagaag acggcatacg agataattct gcgtctcgtg ggctcggaga tgtgtataag 60
   agacagtgac aaggcgtagt cacggt 86
<210> 1743
   <211> 86
   <212> DNA
   <213> Artificial Sequence
<220>
   <223> Synthetic Primer, 7-10-Ih
<400> 1743
   caagcagaag acggcatacg agatggcctc atgtctcgtg ggctcggaga tgtgtataag 60
   agacagtgac aaggcgtagt cacggt 86
<210> 1744
   <211> 86
   <212> DNA
   <213> Artificial Sequence
<220>
   <223> Synthetic Primer, 7-11-Ih
<400> 1744
   caagcagaag acggcatacg agatatctta gtgtctcgtg ggctcggaga tgtgtataag 60
   agacagtgac aaggcgtagt cacggt 86
<210> 1745
   <211> 86
   <212> DNA
   <213> Artificial Sequence
<220>
   <223> Synthetic Primer, 7-12-Ih
<400> 1745
   caagcagaag acggcatacg agatgctccg acgtctcgtg ggctcggaga tgtgtataag 60
   agacagtgac aaggcgtagt cacggt 86
<210> 1746
   <211> 18
   <212> RNA
   <213> Artificial Sequence
<220>
   <223> Synthetic Primer, F498
<400> 1746
   gagugugcgu ggcucuca 18
<210> 1747
   <211> 18
   <212> RNA
   <213> Artificial Sequence
<220>
   <223> Synthetic Primer, F825
<400> 1747
   cuggcuccgg gugacagc 18
<210> 1748
   <211> 19
   <212> RNA
   <213> Artificial Sequence
<220>
   <223> Synthetic Primer, F826
<400> 1748
   gacucccaug acccccauc 19
<210> 1749
   <211> 28
   <212> RNA
   <213> Artificial Sequence
<220>
   <223> Synthetic Primer, F827
<400> 1749
   aaaaauguua ugucagcguu uggcuuaa 28
<210> 1750
   <211> 18
   <212> RNA
   <213> Artificial Sequence
<220>
   <223> Synthetic Primer, F828
<400> 1750
   guaggcgcga gcuaagca 18
<210> 1751
   <211> 28
   <212> RNA
   <213> Artificial Sequence
<220>
   <223> Synthetic Primer, F829
<400> 1751
   caggucauau ugaacauucc agauaccu 28
<210> 1752
   <211> 18
   <212> RNA
   <213> Artificial Sequence
<220>
   <223> Synthetic Primer, F830
<400> 1752
   gguccugacg caggcuuc 18
<210> 1753
   <211> 22
   <212> RNA
   <213> Artificial Sequence
<220>
   <223> Synthetic Primer, F831
<400> 1753
   guaccugcau caaccccucu aa 22
<210> 1754
   <211> 20
   <212> RNA
   <213> Artificial Sequence
<220>
   <223> Synthetic Primer, F832
<400> 1754
   cagagacccg ugcugaguuu 20
<210> 1755
   <211> 23
   <212> RNA
   <213> Artificial Sequence
<220>
   <223> Synthetic Primer, F833
<400> 1755
   ggagagaaga gugcacaaua cca 23
<210> 1756
   <211> 23
   <212> RNA
   <213> Artificial Sequence
<220>
   <223> Synthetic Primer, F834
<400> 1756
   ccuguaaucc cugcacuuua gga 23
<210> 1757
   <211> 25
   <212> RNA
   <213> Artificial Sequence
<220>
   <223> Synthetic Primer, F835
<400> 1757
   acuuuccagu ugagcauccc aaauu 25
<210> 1758
   <211> 28
   <212> RNA
   <213> Artificial Sequence
<220>
   <223> Synthetic Primer, F836
<400> 1758
   cgucagcgug auauguaccg uauuuuau 28
<210> 1759
   <211> 27
   <212> RNA
   <213> Artificial Sequence
<220>
   <223> Synthetic Primer, F837
<400> 1759
   caccucagua auauggaagu ccaaguu 27
<210> 1760
   <211> 26
   <212> RNA
   <213> Artificial Sequence
<220>
   <223> Synthetic Primer, F797
<400> 1760
   cccuucguag acauauagcu guucuc 26
<210> 1761
   <211> 23
   <212> RNA
   <213> Artificial Sequence
<220>
   <223> Synthetic Primer, F798
<400> 1761
   uggugcuagu ugcaaagaca caa 23
<210> 1762
   <211> 18
   <212> RNA
   <213> Artificial Sequence
<220>
   <223> Synthetic Primer, F799
<400> 1762
   agcgacgcca uugcucau 18
<210> 1763
   <211> 21
   <212> RNA
   <213> Artificial Sequence
<220>
   <223> Synthetic Primer, F800
<400> 1763
   ccucaaccau uuccggcaaa u 21
<210> 1764
   <211> 18
   <212> RNA
   <213> Artificial Sequence
<220>
   <223> Synthetic Primer, F801
<400> 1764
   ccagcucccu gcgaagag 18
<210> 1765
   <211> 26
   <212> RNA
   <213> Artificial Sequence
<220>
   <223> Synthetic Primer, F802
<400> 1765
   aaucccugca guagauacga agacua 26
<210> 1766
   <211> 25
   <212> RNA
   <213> Artificial Sequence
<220>
   <223> Synthetic Primer, F803
<400> 1766
   agaccuugca gaaauaggaa uugcu 25
<210> 1767
   <211> 27
   <212> RNA
   <213> Artificial Sequence
<220>
   <223> Synthetic Primer, F805
<400> 1767
   aaagaaaaga caguuggagg aaucugu 27
<210> 1768
   <211> 28
   <212> RNA
   <213> Artificial Sequence
<220>
   <223> Synthetic Primer, F806
<400> 1768
   gaagaaaaug aaaaggaguu agcagcau 28
<210> 1769
   <211> 27
   <212> RNA
   <213> Artificial Sequence
<220>
   <223> Synthetic Primer, F807
<400> 1769
   aguggcaaaa gaacuucaga cuuuaca 27
<210> 1770
   <211> 19
   <212> RNA
   <213> Artificial Sequence
<220>
   <223> Synthetic Primer, F808
<400> 1770
   gcgcugcuca gaagcaaaa 19
<210> 1771
   <211> 25
   <212> RNA
   <213> Artificial Sequence
<220>
   <223> Synthetic Primer, F809
<400> 1771
   guagaucgca uaaaggaagc aguca 25
<210> 1772
   <211> 26
   <212> RNA
   <213> Artificial Sequence
<220>
   <223> Synthetic Primer, F810
<400> 1772
   ggaagcaguc aggucaaaga auaugg 26
<210> 1773
   <211> 27
   <212> RNA
   <213> Artificial Sequence
<220>
   <223> Synthetic Primer, F812
<400> 1773
   caagcagaaa cacuguacaa agagauu 27
<210> 1774
   <211> 18
   <212> RNA
   <213> Artificial Sequence
<220>
   <223> Synthetic Primer, F813
<400> 1774
   gagggcgagc ugcaugau 18
<210> 1775
   <211> 25
   <212> RNA
   <213> Artificial Sequence
<220>
   <223> Synthetic Primer, F814
<400> 1775
   cacaucuuca ggugcuggau uuuuc 25
<210> 1776
   <211> 27
   <212> RNA
   <213> Artificial Sequence
<220>
   <223> Synthetic Primer, F815
<400> 1776
   cuuuugaaaa gccagugaug aucucaa 27
<210> 1777
   <211> 24
   <212> RNA
   <213> Artificial Sequence
<220>
   <223> Synthetic Primer, F816
<400> 1777
   gcaccuugac uuuaagugag agca 24
<210> 1778
   <211> 28
   <212> RNA
   <213> Artificial Sequence
<220>
   <223> Synthetic Primer, F817
<400> 1778
   acagcacugu uauuacuacu uggguuuu 28
<210> 1779
   <211> 22
   <212> RNA
   <213> Artificial Sequence
<220>
   <223> Synthetic Primer, F818
<400> 1779
   caagcuccuu acauacccag ca 22
<210> 1780
   <211> 19
   <212> RNA
   <213> Artificial Sequence
<220>
   <223> Synthetic Primer, F819
<400> 1780
   gcguuuccuc gcuugcauu 19
<210> 1781
   <211> 22
   <212> DNA
   <213> Artificial Sequence
<220>
   <223> Synthetic Primer, F820
<400> 1781
   cgggcaggaa tctgatgact tt 22
<210> 1782
   <211> 20
   <212> RNA
   <213> Artificial Sequence
<220>
   <223> Synthetic Primer, F821
<400> 1782
   gcagggcagc aacaucuuug 20
<210> 1783
   <211> 27
   <212> RNA
   <213> Artificial Sequence
<220>
   <223> Synthetic Primer, F822
<400> 1783
   ggcuccugag accuuugaua acauaac 27
<210> 1784
   <211> 26
   <212> RNA
   <213> Artificial Sequence
<220>
   <223> Synthetic Primer, F823
<400> 1784
   cgugugcucc cuggauauuc uuagua 26
<210> 1785
   <211> 18
   <212> RNA
   <213> Artificial Sequence
<220>
   <223> Synthetic Primer, F824
<400> 1785
   cuggcuccgg gugacagc 18